# EUROPEAN PATENT SPECIFICATION

(11) **EP 3 932 917 B1**
(45) Date of publication and mention of the grant of the patent: **25.11.2026**
(21) Application number: 21185896.4
(22) Date of filing: 15.07.2016
(51) Int. Cl.: C07D 413/06, A61P 31/04, A61K 31/422

(54) **SUBSTITUTED PHENYLOXAZOLIDINONES FOR ANTIMICROBIAL THERAPY**
SUBSTITUIERTE PHENYLOXAZOLIDINONE FÜR ANTIMIKROBIELLE THERAPIE
PHÉNYLOXAZOLIDINONES SUBSTITUÉES POUR THÉRAPIE ANTIMICROBIENNE

(30) Priority: 17.07.2015 US 201562193963 P
(43) Date of publication of application: 05.01.2022
(62) Document number(s) of the earlier application(s) in accordance with Art. 76 EPC: 16828300.0 / 3 307 268
(73) Proprietor: The Global Alliance for TB Drug Development, Inc., New York, NY 10005 (US)
(72) Inventor: COOPER, Christopher B., New York, 10005 (US); HUANG, Haihong, New York, 10005 (US); ZHANG, Doongfeng, New York, 10005 (US); FOTOUHI, Nader, New York, 10005 (US); KANEKO, Takushi, New York, 10005 (US)
(74) Representative: Vossius & Partner Patentanwälte Rechtsanwälte mbB

(56) References cited:
- WO-A1-2007/000644
- WO-A1-95/07271
- WO-A1-96/15130
- WO-A2-03/006440
- US-A1- 2004 077 626

## Description

### FIELD OF THE INVENTION

The invention relates generally to compounds with antibacterial activity and, more specifically, with anti-tuberculosis properties. In particular, it relates to substituted phenyloxazolidinone compounds useful for the treatment of tuberculosis in patients in need thereof.

### BACKGROUND OF THE INVENTION

Linezolid is the first-in-class drug and was approved in 2000 for a number of clinical applications including the treatment of nosocomial and community-acquired pneumonia and skin infections caused by *Staphylococcus* aureus/Methicillin-resistant *S. aureus,* Vancomycin-resistant *Enterococci,* and *Streptococcus pneumoniae* (Pen-S). Linezolid exhibits *in vitro* bacteriostatic activity against *Mycobacterium tuberculosis,* including multidrug-resistant (MDR) and extensively drug resistant (XDR) strains, with a minimum inhibitory concentration (MIC) of less than 1 µg/ml. However, it has demonstrated only modest activity in murine models of tuberculosis. Nonetheless, Linezolid has been used off-label in combination regimens to treat multidrug-resistant tuberculosis.

Oxazolidinones currently in clinical development show bone marrow toxicity in animals after long term administration (i.e., greater than one month) that is believed to be related to mitochondrial protein synthesis (MPS) inhibition, with very narrow safety margins or no safety margins. Since the antimicrobial mode of action of this class of compounds is inhibition of microbial protein synthesis, the MPS inhibition and consequent bone marrow toxicity exhibited by these compounds is considered mechanism specific. These oxazolidinones generally show high clearance and so require administration of high doses in clinical treatment of TB or the other indications for which they are being developed (e.g., 500 mg to 1600 mg daily) to achieve efficacious exposures. Therefore, it would be highly desirable to identify a new generation of oxazolidinones for TB treatment that would demonstrate improved potency and efficacy against TB, reduced systemic clearance to reduce the daily dose below 500 mg, and diminished MPS inhibition and related bone marrow toxicity, resulting in an improved safety margin for long term administration.

### SUMMARY OF THE INVENTION

The present invention relates to novel oxazolidinones of Formula I, or a pharmaceutically acceptable salt, hydrate, or solvate thereof: 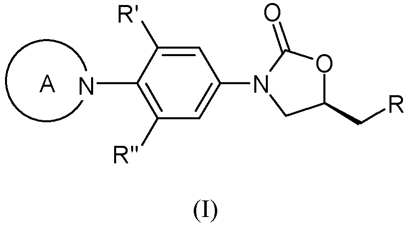 wherein,
R is independently OR₁, OC(O)R₂, OC(O)NHR₂, OS(O₂)R₂, NHS(O)₂R₂, NR₃R₄;
R' and R" are independently H, F, Cl or OMe;
each R₁ is independently H, C₁-C₆ alkyl, C₃-C₈ cycloalkyl, wherein said alkyl, cycloalkyl are optionally substituted with 1 to 4 groups selected from halo, hydroxy, C₁-C₆ alkyl, C₁-C₆ alkyloxy;
each R₂ is independently C₁-C₆ alkyl, C₃-C₈ cycloalkyl, heterocyclyl, heteroaryl or aryl, wherein said alkyl, cycloalkyl, heterocyclyl, heteroaryl, or aryl are optionally substituted with 1 to 4 groups selected from halo, hydroxyl, C₁-C₆ alkyl, C₁-C₆ alkoxy, C₁-C₆ acyloxy, CF₃, NO₂, CN and NH₂;
each R₃ is C₁-C₆ alkyl, C₃-C₈ cycloalkyl, heterocyclyl, heteroaryl, or aryl, and R₄ is H, C₁-C₆ alkyl, C₃-C₈ cycloalkyl, heterocyclyl, heteroaryl, or aryl; wherein said alkyl, cycloalkyl, heterocyclyl, heteroaryl, or aryl are optionally substituted with 1 to 4 groups selected from halo, C₁-C₆ alkyl, C₁-C₆ alkoxy, CF₃, NO₂, CN;
Ring A is 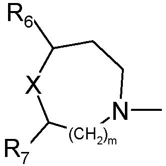
wherein,
each R₆ and R₇ is independently H, F, CH₃, CH₂CH₃, CF₃, phenyl;
X = O, S, SO, SO₂;
m is 1, or 2.

The present invention also relates to a pharmaceutical composition comprising at least one compound of Formula I, or a salt, hydrate, or solvate thereof, as defined above, and one or more pharmaceutically acceptable carriers and/or additives.

The pharmaceutical composition preferably further comprises one or more additional anti-infective treatments.

The compound of Formula I, or a salt, hydrate, or solvate thereof, as defined above is preferably for use in preventing or treating microbial infections in humans. The microbial infection is preferably a microbial infection caused by *Mycobacterium tuberculosis.*

Any references to methods of treatment herein are to be interpreted as references to the compounds, pharmaceutical compositions and medicaments of the present invention for use in a method for treatment of the human (or animal) body by therapy (or for diagnosis).

### DETAILED DESCRIPTION OF THE INVENTION

One aspect of the present invention is to provide novel compounds according to Formula I shown and described above. Specifically, the compounds of the invention are useful antimicrobial agents, effective against a number of human and veterinary pathogens, including gram-positive aerobic bacteria, *Mycobacterium tuberculosis, Mycobacterium avium,* and the like. As a result, this invention provides novel compounds according to Formula I, as well as pharmaceutically acceptable salts, hydrates, or solvates thereof. Values for the variables in Formula I are provided in the following paragraphs.

The present invention preferably relates to a compound of formula I, wherein the compound is represented by Formula II: 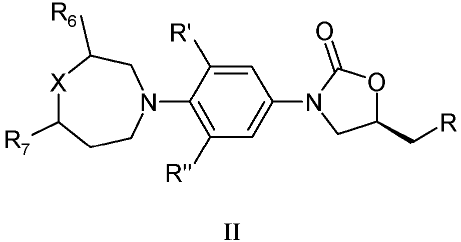 wherein,
R is independently OR₁, OC(O)R₂, NR₃R₄, NHS(O)₂R₂;
R' and R" are independently H, or F;
R₁ is independently H, C₁-C₆ alkyl, C₃-C₆ cycloalkyl;
R₂ is independently C₁-C₆ alkyl, C₃-C₆ cycloalkyl;
R₃ is C₁-C₆ alkyl, C₃-C₆ cycloalkyl, phenyl, and R₄ is H, C₁-C₆ alkyl, C₃-C₆ cycloalkyl, phenyl;
R₆ and R₇ is independently H, F, CH₃, CH₂CH₃, CF₃;
X = O, S, SO, SO₂.

Alternatively, the compound is preferably represented by Formula III: 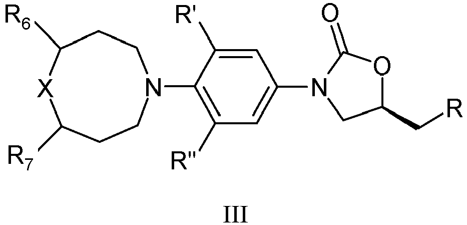 wherein,
R is independently OR₁, OC(O)R₂, NR₃R₄, NHS(O)₂R₂;
R' and R" are independently H, or F;
R₁ is independently H, C₁-C₆ alkyl, C₃-C₆ cycloalkyl;
R₂ is independently C₁-C₆ alkyl, C₃-C₆ cycloalkyl;
R₃ is C₁-C₆ alkyl, C₃-C₆ cycloalkyl, or phenyl, and R₄ is H, C₁-C₆ alkyl, C₃-C₆ cycloalkyl, or phenyl;
R₆ and R₇ is independently H, F, CH₃, CH₂CH₃, CF₃;
X = O, S, SO, SO₂;

The compound of formula (I) is more preferably a compound represented by Formula IIa, IIb, IIIa, IIIb, IVa, IVb, Va, Vb, IXa, or IXb: 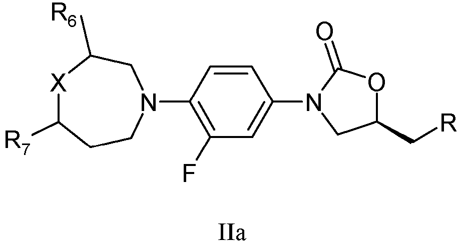 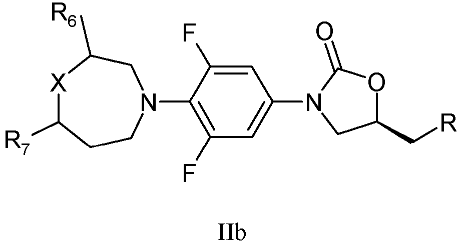 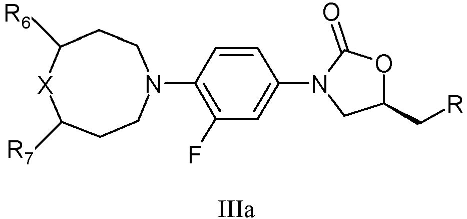 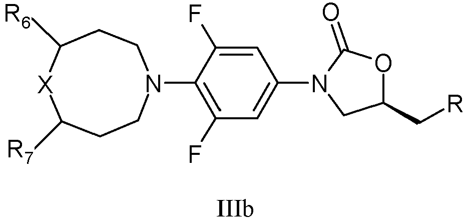 wherein,
R is independently OH, OCH₃, OCH₂CH₃, OC(O)CH₃, NHCH₃, NHC₆H₅, NHS(O)₂R₂;
R₂ is independently C₁-C₆ alkyl; and
X = O, S, SO, SO₂.

Specific preferred examples of the compound of the present invention include:
OTB-109 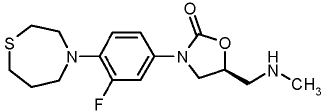
OTB-108 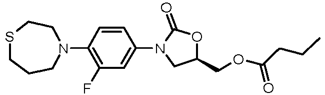
OTB-116 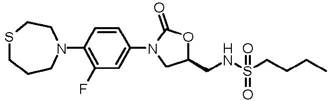
OTB-126 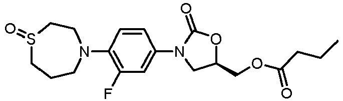
OTB-110 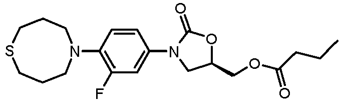 and
OTB-120

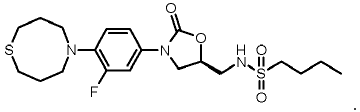

The present invention also relates to a pharmaceutical composition comprising at least one compound of Formula I, or a salt, hydrate, or solvate thereof, as defined above, and one or more pharmaceutically acceptable carriers and/or additives.

The pharmaceutical composition preferably further comprises one or more additional anti-infective treatments.

The compound of Formula I, or a salt, hydrate, or solvate thereof, as defined above is preferably for use in preventing or treating microbial infections in humans. The microbial infection is preferably a microbial infection caused by *Mycobacterium tuberculosis.*

Table 1 below shows some specific examples of compounds, by indicating their structures as well as their *in vitro* activity against *Mycobacterium tuberculosis* H37Rv strains, and *in vitro* MPS inhibition activity when tested as described in Example 9 and 10 below, respectively. It is to be understood that only compounds of Formula I as defined in the claims are accoriding to the present invention. In Table I, these are represented by the inventive compounds OTB-109, OTB-108, OTB-116, OTB-126, OTB-110 and OTB-120.

As shown in Table 1 below, potent anti-tubercular agents demonstrate low MIC values (particular compounds with MIC's below 1 □g/mL). Conversely, high MPS inhibition IC₅₀'s indicate diminished mitochondrial protein synthesis activity *in vitro,* and are indicative of reduced myelosuppression toxicity *in vivo.* In certain embodiments of the invention, compounds having the best therapeutic index are those demonstrating a relatively lower MIC value combined a relatively higher MPS inhibition IC₅₀. Representative compounds of the invention are provided in Table 1 (wherein the entry "NA" (i.e., "not available") indicates that a particular value was not measured):

**TABLE 1**

| **Compound** | **Structure** | **HRMS [M+H]⁺** | **MIC (µg/mL) against H₃₇Rv** | **IC₅₀ (µM) MPS inhibition** |
|---|---|---|---|---|
| OTB-107 | 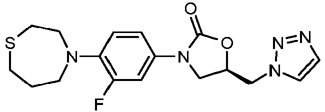 | 378.1396 | 0.03 | 84.85 |
| OTB-106 | 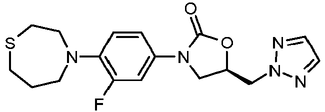 | 378.1403 | 2 | > 100 |
| OTB-109 | 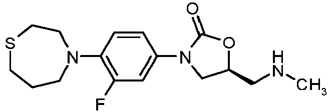 | 340.1484 | 32 | NA |
| OTB-108 | 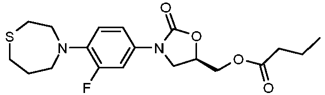 | 397.1613 | 0.125 | > 100 |
| OTB-111 | 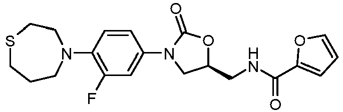 | 420.1400 | 0.125 | 60.32 |
| OTB-112 | 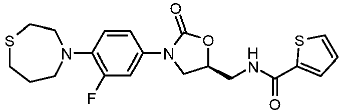 | 382.1620 | 0.5 | > 100 |
| OTB-115 | 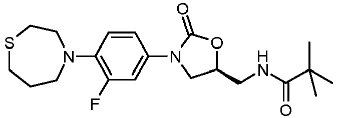 | 410.1942 | 3.733 | > 100 |
| OBD-005 | 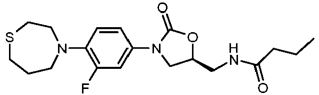 | 395.1679 | 0.055 | 15.63 |
| OTB-116 | 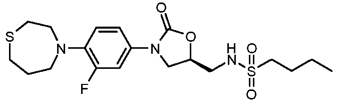 | 446.1623 | 3.812 | > 100 |
| OTB-119 | 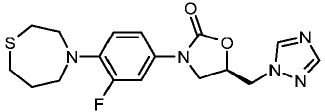 | 378.1421 | 0.456 | > 100 |
| OTB-412 | 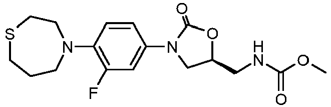 | 384.1371 | 0.199 | 10 |
| OTB-413 | 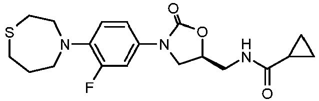 | 394.1580 | 0.108 | 9.13 |
| OTB-414 | 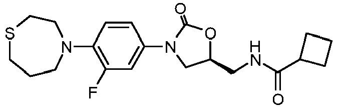 | 408.1736 | 0.171 | NA |
| OTB-407 | 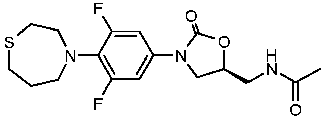 | 386.1330 | 0.097 | 3.079 |
| OTB-410 | 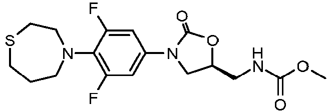 | 402.1287 | 0.125 | > 100 |
| OTB-408 | 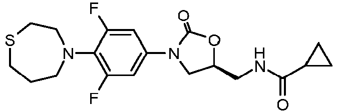 | 412.1485 | 0.342 | 3.608 |
| OTB-409 | 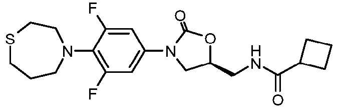 | 426.1643 | 0.477 | 8.51 |
| OTB-411 | 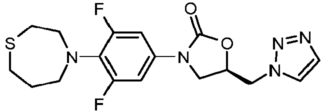 | 396.1296 | 0.124 | 8.73 |
| OTB-126 | 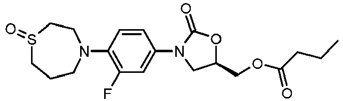 | 413.1573 | 0.847 | 35.60 |
| OTB-127 | 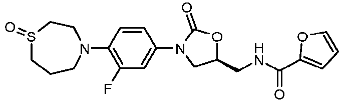 | 436.1371 | 1.234 | 10.15 |
| OTB-137 | 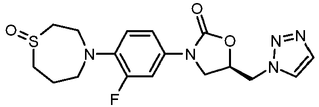 | 394.1328 | 0.277 | 7.64 |
| OTB-138 | 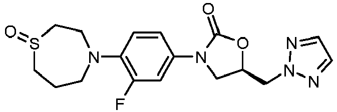 | 394.1338 | 7.565 | > 100 |
| OTB-140 | 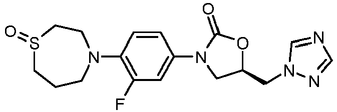 | 394.1339 | 3.695 | > 100 |
| OBD-006 | 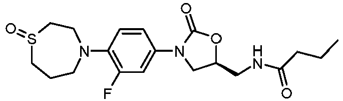 | 411.1628 | 0.49 | 7.485 |
| OBD-007 | 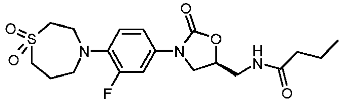 | 427.1577 | 0.46 | 19.81 |
| OTB-110 | 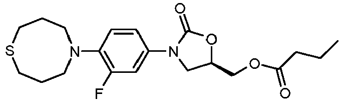 | 411.1786 | 0.125 | > 100 |
| OTB-113 | 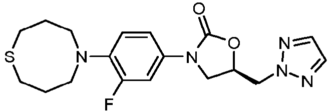 | 392.1590 | 2 | > 100 |
| OTB-114 | 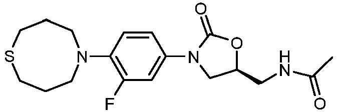 | 382.1620 | 0.05-0.11 | 28-29 |
| OTB-124 | 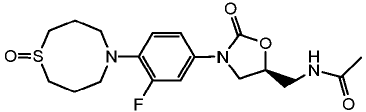 | 398.1540 | 0.26-1.4 | 94->100 |
| OTB-117 | 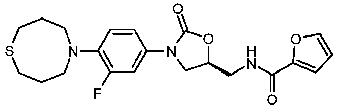 | 434.1581 | 0.665 | 56.32 |
| OTB-118 | 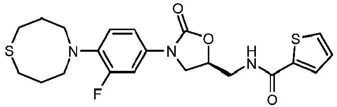 | 450.1356 | 1.548 | NA |
| OTB-120 | 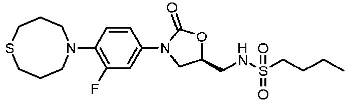 | 460.1778 | 3.877 | 7.27 |
| OTB-121 | 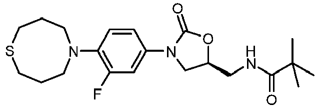 | 424.2096 | 2.785 | 17.63 |
| OBD-001 | 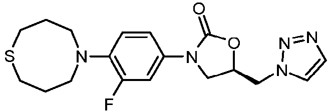 | 391.1478 | 0.3 | 12 |
| OBD-002 | 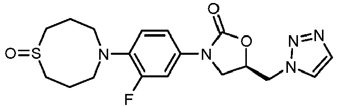 | 407.1427 | 1.6 | 29 |
| OBD-003 | 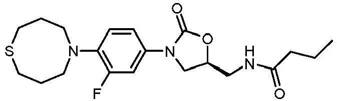 | 409.1835 | 0.33 | > 100 |
| OBD-004 | 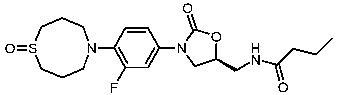 | 425.1785 | 3.338 | 15.76 |
| OBD-008 | 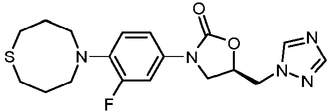 | 391.1478 | 3.513 | 29.89 |
| OBD-009 | 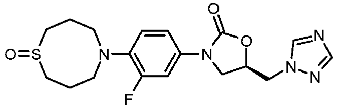 | 407.1427 | 21.052 | > 100 |
| OBD-027 | 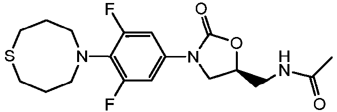 | | 0.3 | NA |
| OBD-240 | 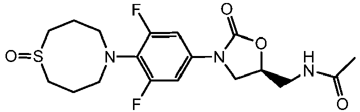 | | NA | NA |
| OBD-026 | 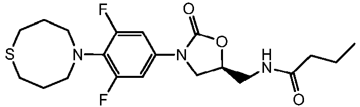 | | 0.4 | NA |
| OBD-241 | 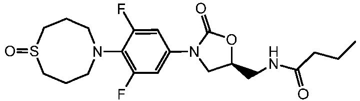 | | NA | NA |
| OTB-227 | 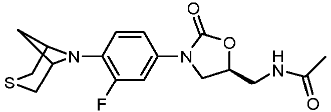 | 366.1277 | 0.889 | 15.22 |
| OTB-501 | 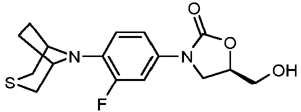 | 339.1169 | 4.5 | NA |
| OBD-081 | 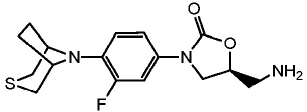 | 407.9 | 0.47 | 77 |
| OBD-085 | 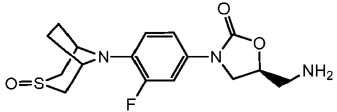 | | 1.1 | >100 |
| OTB-502 | 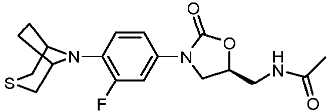 | 380.1435 | 0.246 | 13.55 |
| OTB-503 | 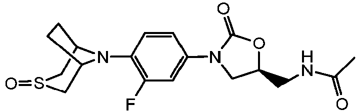 | 396.1379 | 0.2-1.3 | 78->100 |
| OTB-504 | 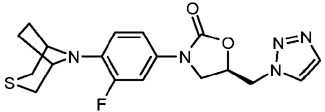 | 390.1385 | 0.5-0.7 | 17-> 100 |
| OTB-505 | 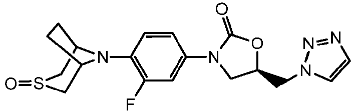 | 406.1339 | 1.8-3.5 | 57->100 |
| OTB-236 | 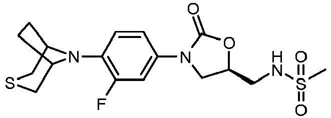 | 416.1097 | 14.256 | NA |
| OTB-237 | 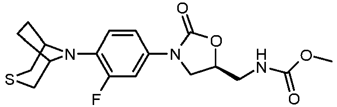 | 396.1388 | 0,03-0. 11 | 15-23 |
| OTB-518 | 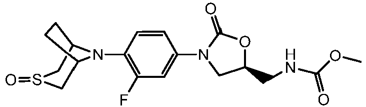 | | 3.5 | 73 |
| OBD-016 | 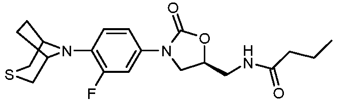 | 407.1679 | 0.486 | 13 |
| OBD-017 | 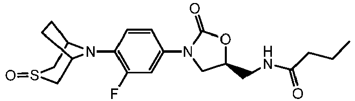 | 423.8 | 5.8-6.3 | 41 |
| OBD-021 | 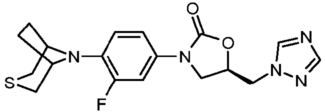 | 389.1322 | 27.456 | NA |
| OBD-018 | 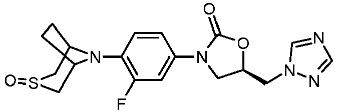 | 405.1271 | > 32 | NA |
| OTB-506 | 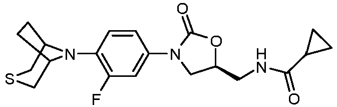 | 406.1527 | 1 | 8 |
| OTB-507 | 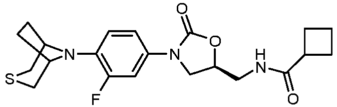 | 420.1736 | 0.7 | 10 |
| OTB-510 | 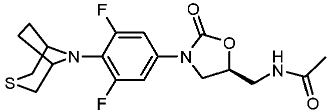 | 398.1329 | 0.03-0. 22 | 3-7 |
| OTB-514 | 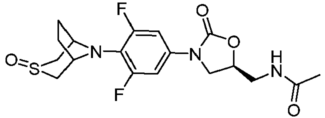 | 414.1275 | 0.5 | 25 |
| OTB-512 | 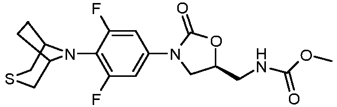 | 414.1278 | 0.063 | 42.47 |
| OTB-519 | 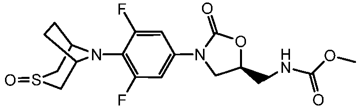 | | 0.9 | 51 |
| OTB-511 | 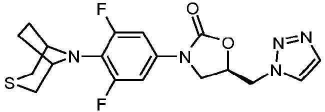 | 408.1295 | 0.06-0.2 | 9-> 100 |
| OTB-517 | 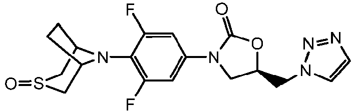 | | 1.3-2 | 30-55 |
| OTB-508 | 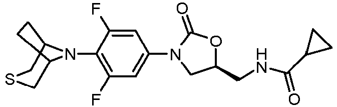 | 424.1484 | 0.2 | 8 |
| OTB-509 | 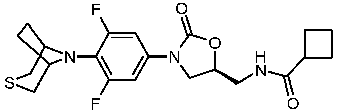 | 438.1642 | 0.04 | 5 |
| OTB-513 | 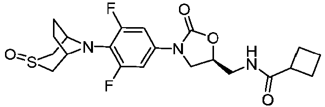 | 454.1588 | 1.664 | 12.96 |
| OBD-083 | 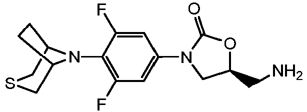 | | 0.05 | 20 |
| OBD-087 | 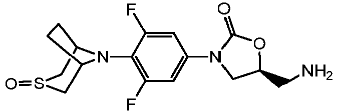 | | 0.86 | >100 |
| OBD-029 | 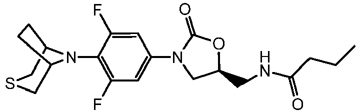 | | 0.11 | >100 |
| OBD-242 | 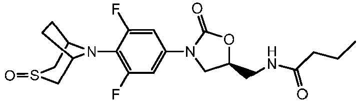 | | 0.61 | >100 |
| OTB-260 | 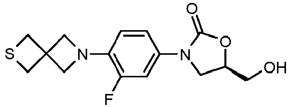 | 325.1010 | 1.49 | 30.03 |
| OTB-261 | 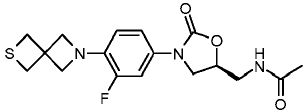 | 366.1274 | 0.12 | 2 |
| OTB-523 | 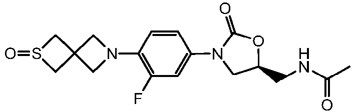 | | 0.5 | 4 |
| OTB-515 | 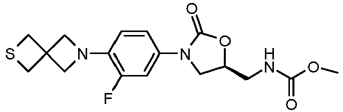 | NA | 0.03-0.06 | 23-71 |
| OTB-256 | 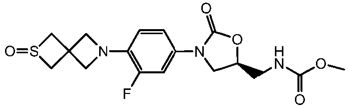 | | 4 | 46 |
| OTB-241 | 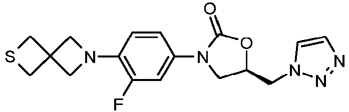 | 376.1231 | 0.116 | 19.81 |
| OTB-247 | 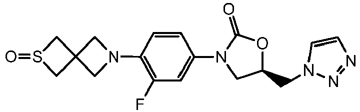 | 392.0 | 1.2-1.4 | 10 |
| OTB-249 | 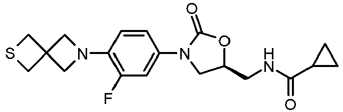 | 392.1426 | 0.06-0.15 | 7->100 |
| OTB-255 | 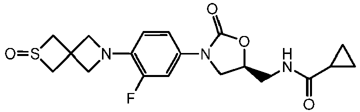 | 408.1378 | 1.9 | 10 |
| OTB-250 | 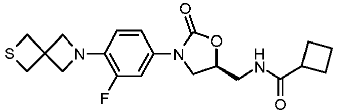 | 428.2 | 0.06 | 10->100 |
| OTB-254 | 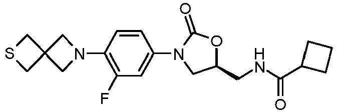 | 422.1531 | 1.8 | 23 |
| OTB-260 -2A | 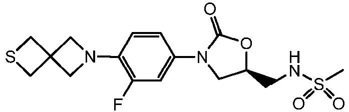 | 402.1 | 0.82 | >100 |
| OTB-260 -2B | 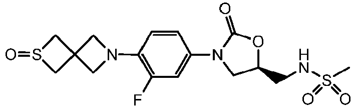 | 418.0 | 0.49 | >100 |
| OTB-260 -5A | 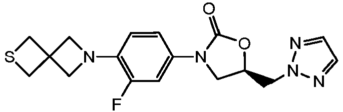 | 376.1 | 0.44 | >100 |
| OTB-260 -5B | 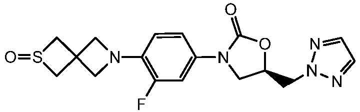 | 392.1 | >32 | >100 |
| OTB-260 -4A | 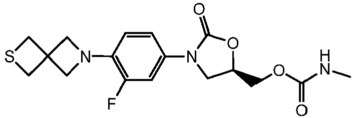 | 382.0 | 0.39 | >100 |
| OTB-260 -4B | 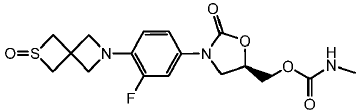 | 398.0 | 20 | >100 |
| OTB-516 | 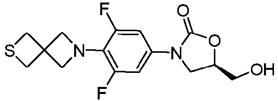 | 343.0912 | 0.465 | 15.33 |
| OTB-515 | 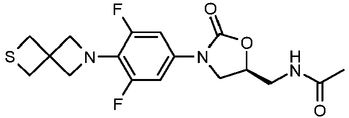 | 384.1168 | 0.03-0.57 | 14-21 |
| OTB-520 | 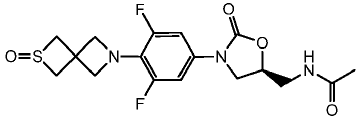 | 400.1158 | 0.4 | 17 |
| OTB-242 | 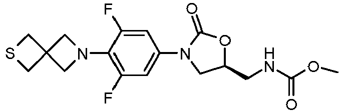 | 400.1125 | 0.02-0.06 | 23-71 |
| OTB-253 | 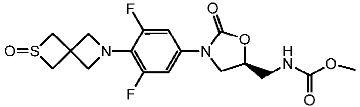 | 416.1073 | 0.7 | 10 |
| OTB-245 | 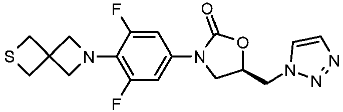 | 394.1129 | 0.03-0.5 | 25-35 |
| OTB-522 | 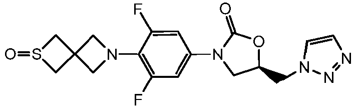 | 410.1 | 1 | 38 |
| OTB-243 | 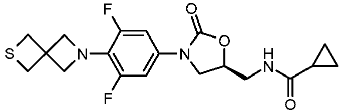 | 410.1331 | 0.03 | 14 |
| OTB-252 | 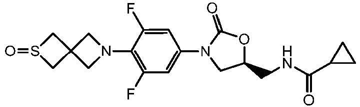 | 426.1278 | 0.7 | 52 |
| OTB-244 | 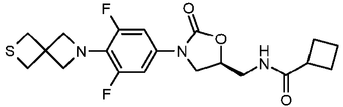 | 424.1483 | 0.02-0. 6 | 6->22 |
| OTB-251 | 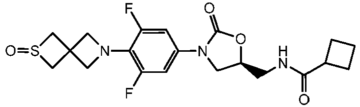 | 440.1441 | 0.9 | 24 |
| OTB-516 -2A | 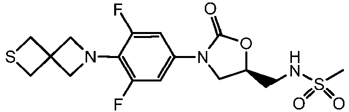 | 420.1 | 0.05 | >100 |
| OTB-516 -2B | 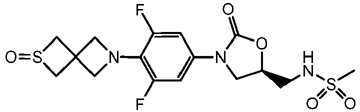 | 436.0 | 3 | >100 |
| OTB-516 -4A | 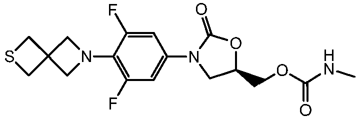 | 400.1 | 0.03 | >100 |
| OTB-516 -4B | 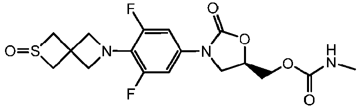 | 416.0 | 4.7 | >100 |
| OTB-201 | 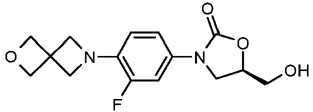 | 309.1269 | 16 | NA |
| OBD-057 | 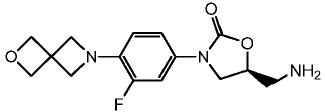 | | 0.4 | 67 |
| OTB-202 | 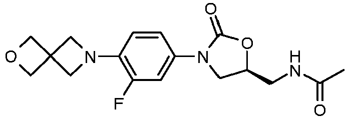 | 350.1497 | 0.6-1.2 | 17-29 |
| OTB-203 | 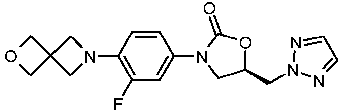 | 360.1451 | > 32 | NA |
| OTB-204 | 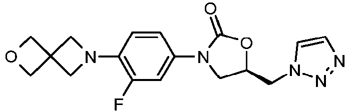 | 360.1451 | 3.2-3.7 | > 100 |
| OTB-205 | 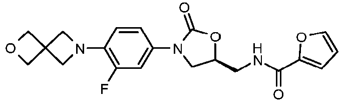 | 402.1561 | 3.9 | NA |
| OTB-206 | 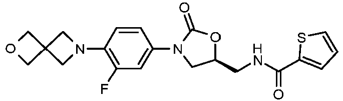 | 418.1331 | 2.8 | NA |
| OBD-056 | 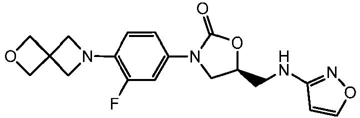 | | 1.9 | 17 |
| OTB-222 | 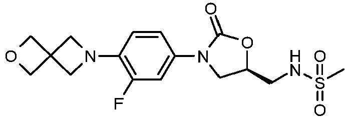 | 386.1185 | 7.4 | NA |
| OTB-223 | 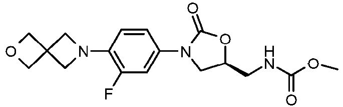 | 366.1466 | 0.8-2.6 | 38->100 |
| OTB-238 | 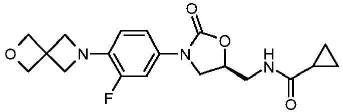 | 376.1652 | 2-4 | 20 |
| OTB-239 | 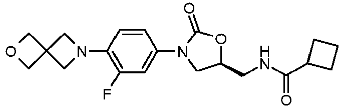 | 390.1808 | 2.1-3.8 | 19-67 |
| OTB-229 | 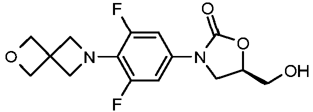 | 327.1135 | 1.9-7.4 | > 100 |
| OBD-062 | 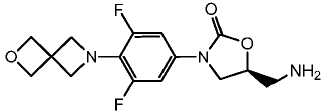 | | 0.1-0.2 5 | 21->100 |
| OTB-230 | 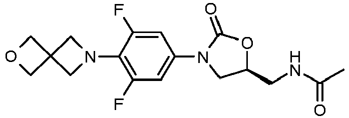 | 368.1418 | 0.2 | 13 |
| OTB-231 | 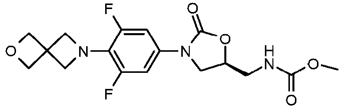 | 384.1367 | 0.24-0. 7 | 37-63 |
| OTB-232 | 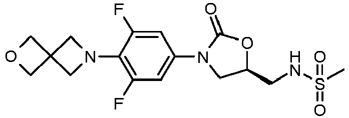 | 404.1087 | 1.5-4 | > 100 |
| OTB-233 | 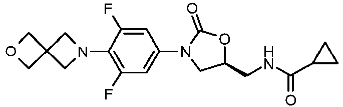 | 394.1575 | 0.36-1 | 8.4-50 |
| OTB-234 | 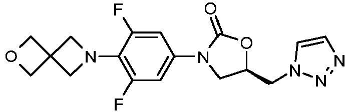 | 378.1365 | 0.39-4 | 43->100 |
| OBD-061 | 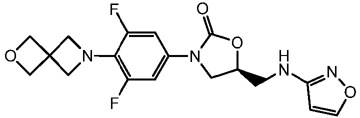 | | 0.8 | 29 |
| OTB-240 | 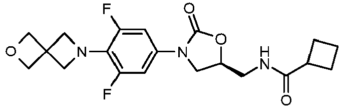 | 408.1716 | 0.24-0. 6 | 19-67 |
| OBD-051 | 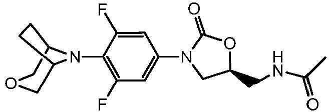 | 381.9 | 0.6 | 6 |
| OBD-052 | 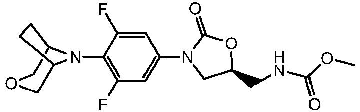 | 398.0 | 0.95 | 13 |
| OBD-055 | 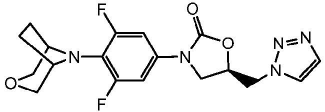 | 391.8 | 1.3 | 15 |
| OBD-112 | 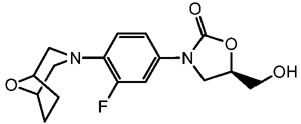 | | 3.5 | >100 |
| OBD-113 | 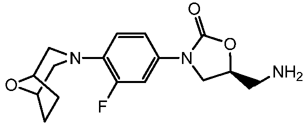 | | 0.39 | >100 |
| OBD-110 | 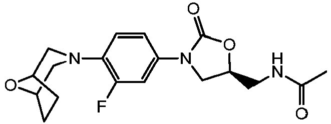 | | 0.45 | 7 |
| OBD-111 | 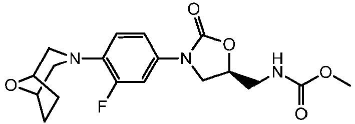 | | 0.49 | 7 |
| OBD-114 | 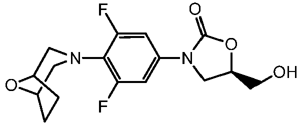 | | 1.7 | >100 |
| OBD-115 | 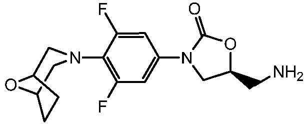 | | 0.2=0.3 | 87->100 |
| OBD-048 | 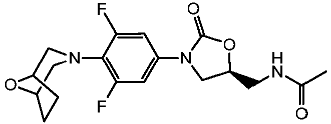 | | 0.39 | 5 |
| OBD-049 | 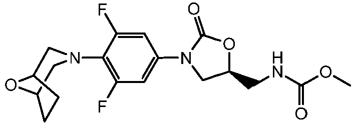 | | 0.25-1 | 6->100 |
| OBD-252 | 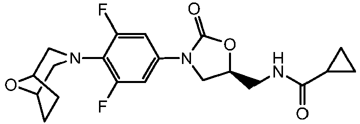 | | 0.47 | 32.3 |
| OBD-253 | 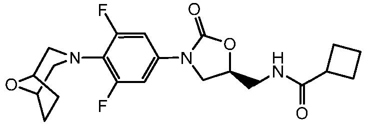 | | 0.53 | 65 |
| OBD-054 | 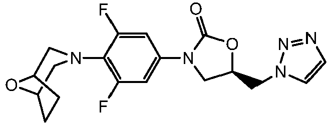 | | 0.5 | 6-31 |
| OBD-254 | 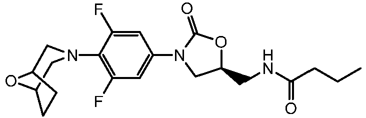 | | 0.73 | 36 |

### Definitions

As used herein unless otherwise indicated, "alkyl" includes branched- and straight-chain saturated aliphatic hydrocarbon groups having the specified carbon atom numbers. Commonly used abbreviations for alkyl groups are used throughout the application, e.g. methyl may be represented by conventional abbreviations including "Me" or CH₃ or a symbol that is an extended bond without defined terminal group, e.g. " 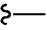 ", ethyl is represented by "Et" or CH₂CH₃, propyl is represented by "Pr" or CH₂CH₂CH₃, butyl can be represented by "Bu" or CH₂CH₂CH₂CH₃ , etc. "C₁₋₆ alkyl" (or "C1-C6 alkyl") means branched or linear chain alkyl groups, including all isomers, having the specified number of carbon atoms. C₁₋₆ alkyl includes all of the hexyl alkyl and pentyl alkyl isomers as well as n-, iso-, sec- and t-butyl, n- and isopropyl, ethyl and methyl. If no number is provided, 1-10 carbon atoms are intended for linear or branched alkyl groups. C₁₋₆ alkyl may be unsubstituted or substituted with 1-3 fluorine or 1-3 chlorine atoms.

"Cycloalkyl" means C₃₋₁₀ carbocycles not containing heteroatoms. For example, cycloalkyl includes cyclopropyl, cyclobutyl, cyclopentyl, cyclohexyl, cycloheptyl, decahydronaphthyl, and the like.

"Aryl" means mono- and bicyclic aromatic rings containing 6-12 carbon atoms. Examples of aryl include, but are not limited to, phenyl, naphthyl, indenyl and so on. Aryl also includes monocyclic rings fused to an aryl group. Examples include tetrahydronaphthyl, indanyl and the like.

"Heterocyclyl," unless otherwise indicated, means a 4-, 5-, 6-, 7- or 8-membered monocyclic saturated ring containing 1-2 heteroatoms selected from N, O and S, in which the point of attachment may be carbon or nitrogen. Examples of "heterocyclyl" include, but are not limited to, piperidinyl, piperazinyl, morpholinyl, pyrrolidinyl, oxazolidinyl, imidazolidinyl, and so on. The term also includes partially unsaturated monocyclic rings that are not aromatic, such as 2- or 4-pyridones attached through the nitrogen or N-substituted-(1H, 3H)-pyrimidine-2, 4-diones (N-substituted uracils). Heterocyclyl may also include such moieties in charged form, e.g., piperidinium.

"Heteroaryl" means a mono- or bicyclic aromatic ring or ring system having 5 to 10 atoms and containing 1-3 heteroatoms selected from N, O, and S. Examples include, but are not limited to, oxadiazolyl, thiadiazolyl, pyrrolyl, furanyl, triazinyl, thienyl, pyrimidyl, pyrimidinyl, pyridazinyl, pyrazinyl, isoxazolyl, triazolyl, isothiazolyl, pyrazolyl, imidazolyl, pyridyl, pyridinyl, oxazolyl, thiazolyl, tetrazolyl, and the like. Heteroaryl also includes aromatic heterocyclic groups fused to heterocycles that are non-aromatic or partially aromatic, and aromatic heterocyclic groups fused to cycloalkyl rings. Additional examples of heteroaryls include, but are not limited to, imidazopyridinyl, imidazopyridazinyl, pyrazolopyrazolyl, indazolyl, thienopyrazolyl, pyrazolopyridinyl, and imidazothiazolyl. Heteroaryl also includes such groups in charged form, such as pyridinium. In an embodiment, heteroaryl is triazolyl, imidazolyl, oxadiazolyl, pyrazolyl, oxazolyl, and pyridinyl.

"Heterocyclic alkyl," unless otherwise indicated, includes both branched- and straight-chain saturated aliphatic hydrocarbon groups which is bonded to a carbon or nitrogen atom of a heterocyclyl, as described above.

"Halogen (or halo)" includes fluorine (fluoro), chlorine (chloro), bromine (bromo) and iodine (iodo). In one embodiment, halogen is chlorine or fluorine.

Substitution by a named substituent is permitted on any atom in a ring (e.g., aryl, a heteroaryl ring, or a saturated heterocyclic ring) provided such ring substitution is chemically allowed and results in a stable compound. A "stable" compound can be prepared and isolated, and whose structure and properties remain or can be caused to remain essentially unchanged for a period of time that allows use of the compound for the described purposes.

Under standard nomenclature used throughout this disclosure, the terminal portion of the designated side chain is described first, followed by the adjacent functionality toward the point of attachment. For example, a C₁₋₅ alkyl COOR is equivalent to

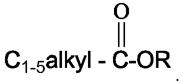

When a variable (e.g., R, Rₓ, etc.) occurs more than once in any constituent or formula, its definition on each occurrence is independent of its definition at every other occurrence. In addition, combinations of substituents and/or variables are allowed only if such combinations lead to stable compounds.

In choosing compounds of the present disclosure, one of ordinary skill in the art will recognize that the various substituents, i.e. R¹, R², R, etc., are to be chosen in conformity with common principles of chemical structure connectivity and stability.

The term "substituted" is used to include multiple degrees of substitution by a named substituent. Where multiple substituents are **itemized**, the substituted compound can be independently substituted by one or more of the disclosed substituents. By independently substituted, it is meant that the (two or more) substituents can be the identical or different.

Where a substituent or variable has multiple definitions, the substituent or variable is defined as being selected from the group consisting of the indicated definitions.

### Salts:

Compounds of structural Formula I also cover the pharmaceutically acceptable salts. The compounds of the present invention may be administered in the form of a pharmaceutically acceptable salt. The term "pharmaceutically acceptable salt" means salts prepared from pharmaceutically acceptable bases or acids including inorganic or organic bases or acids. Pharmaceutically acceptable salts of basic compounds refer to non-toxic salts of the compounds of this invention which are generally prepared by mixing the free base with a suitable organic or inorganic acid. Representative salts of basic compounds of the present invention include, but are not limited to, the following: acetate, ascorbate, benzenesulfonate, benzoate, bicarbonate, bisulfate, bitartrate, borate, butyrate, camphorate, camphorsulfonate, camsylate, carbonate, clavulanate, citrate, edetate, edisylate, estolate, esylate, fumarate, gluceptate, gluconate, glutamate, hydrobromide, hydrochloride, lactobionate, laurate, malate, maleate, mandelate, mesylate, methylbromide, methylnitrate, methylsulfate, methanesulfonate, phosphate/diphosphate, polygalacturonate, propionate, salicylate, stearate, sulfate, subacetate, succinate, tannate, tartrate, teoclate, thiocyanate, tosylate, triethiodide, valerate and the like. Suitable pharmaceutically acceptable salts of acids covered by Formula I include, but are not limited to, salts generated from inorganic bases including aluminum, ammonium, calcium, copper, ferric, ferrous, lithium, magnesium, manganic, mangamous, potassium, sodium, zinc, and the like. Salts derived from pharmaceutically acceptable organic non-toxic bases include salts of primary, secondary, and tertiary amines, cyclic amines, dicyclohexyl amines and basic ion-exchange resins, such as arginine, betaine, caffeine, choline, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and so on.

Solvates and hydrates of the compounds of Formula I are also included in the present invention.

The present invention also discloses processes to synthesize the compounds of Formula I, as described below.

One aspect of the invention that is of interest relates to a compound in accordance with Formula I, or a pharmaceutically acceptable salt, hydrate, or solvate thereof, for use in a method of treatment of microbial infections in humans.

Another aspect of the invention that is of interest is a method of treating microbial infections in a human patient in need of such treatment, comprising administering a therapeutically effective amount of a compound of Formula I or a pharmaceutically acceptable salt, hydrate, or solvate thereof to said patient.

In a further aspect, the present invention provides pharmaceutical compositions of Formula I, or a salt, hydrate, or solvate thereof, further comprising one or more additional anti-infective agents.

In still a further aspect, the present invention relates to a compound in accordance with Formula I or a pharmaceutically acceptable salt, hydrate, or solvate thereof for use as an anti-tuberculosis (TB) agent in a human.

While it may be possible for the compounds of the invention to be administered as the raw chemical, it is preferable to present these as a pharmaceutical composition. Thus, according to a further aspect, the present invention provides a pharmaceutical composition comprising a compound of Formula (I) or a pharmaceutically acceptable salt or solvate thereof, together with one or more pharmaceutically carriers thereof and optionally one or more other therapeutic ingredients. The carrier(s) must be "acceptable" in the sense of being compatible with the other ingredients of the formulation and not deleterious to the recipient thereof.

The formulations include those suitable for oral, parenteral (including subcutaneous, intradermal, intramuscular, intravenous and intraarticular), rectal and topical (including dermal, buccal, sublingual and intraocular) administration. The most suitable route may depend upon the condition and disorder of the recipient. Tablets, capsules, intraocular topical formulations and parenteral solutions are common among aminoglycosides. The formulations may conveniently be presented in unit dosage form and may be prepared by any of the methods well known in the art of pharmacy. All methods include the step of bringing into association a compound of Formula (I) or a pharmaceutically acceptable salt or solvate thereof ("active ingredient") with the carrier which constitutes one or more accessory ingredients. In general, the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or molding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Molded tablets may be made by molding in a suitable machine a mixture of the powdered compound moistened with an inert liquid diluent. The tablets may optionally be coated or scored and may be formulated so as to provide sustained, delayed or controlled release of the active ingredient therein.

Formulations for parenteral administration include aqueous and non-aqueous sterile injection solutions which may contain anti-oxidants, buffers, bacteriostats and solutes which render the formulation isotonic with the blood of the intended recipient. Formulations for parenteral administration also include aqueous and non-aqueous sterile suspensions, which may include suspending agents and thickening agents. The formulations may be presented in unit-dose of multi-dose containers, for example sealed ampoules and vials, and may be stored in a freeze-dried (lyophilized) condition requiring only the addition of a sterile liquid carrier, for example saline, phosphate-buffered saline (PBS) or the like, immediately prior to use. Extemporaneous injection solutions and suspensions may be prepared from sterile powders, granules and tablets of the kind previously described.

Preferred unit dosage formulations are those containing an effective dose, as hereinbelow recited, or an appropriate fraction thereof, of the active ingredient.

It should be understood that in addition to the ingredients particularly mentioned above, the formulations of this invention may include other agents conventional in the art having regard to the type of formulation in question, for example those suitable for oral administration may include flavoring agents.

### Abbreviations

Throughout the synthetic schemes and examples below, abbreviations are used with the following meanings unless otherwise indicated:
Ac is acetate, or acetyl;
aq. is aqueous;
Ar is Aryl;
Bn is benzyl;
BnNH₂ is benzylamine;
Boc is tert-butylcarbamoyl;
br is broad;
Bu is butyl;
*^{t}*Bu is *tert*-butyl;
n-BuLi is n-butyllithium;
CbzCl is benzyl chloroformate;
CFU is colony forming units
CO₂ is carbon dioxide
COX-1 is cyclooxygenase I
*^{c}*Pr is cyclopropyl;
DCM is dichloromethane;
DIPEA is N,N-diisopropylethylamine;
DMAP is 4-dimethylaminopyridine
DMEM is Dulbecco's Modified Eagle Medium
DMF is NN-dimethylformamide;
DMSO is dimethyl sulfoxide;
ELISA is enzyme-linked immunosorbent assay
ESI is electrospray ionization;
Et is ethyl;
Et₃N is triethylaimne;
Et₂O is diethyl ether;
EtOH is ethanol,
EtOAc is ethyl acetate;
FBS is Fetal Bovine Serum
Halo is a halogen (e.g., fluorine or chlorine);
¹H-NMR is proton nuclear magnetic resonance;
¹³C-NMR is carbon nuclear magnetic resonance;
H9C2 is a cell line from rat heart myoblasts
HPLC is high performance liquid chromatography;
HRMS is high-resolution mass spectrometry;
Hz is hertz;
*i* is Iso;
IC₅₀ is half-maximum inhibitory concentration;
Kg is kilogram;
M is molar;
Me is methyl;
µg is microgram;
MeCN is acetonitrile;
MeOH is methanol;
MsCl is methanesulfonyl chloride;
MHz is megahertz;
mm is millimeter;
µL is microliter;
mM is milimolar;
µM is micromolar;
mmol is milimoles;
MABA is microplate alamar blue assay;
MIC is minimum inhibitory concentration;
MPS is mitochondrial protein synthesis;
*m*/*z* is mass to charge ratio;
*n* is normal;
NEAA is non-essential amino acids
nm is nanometer;
*n*Pr is *n*-propyl;
*p* is para;
PE is petroleum ether;
Ph is phenyl;
Pr is propyl;
rt is room temperature;
*sec* is secondary;
SDH-A is succinate dehydrogenase-A
*tert* is tertiary;
TFA is trifluoroacetic acid;
TsCl is p-toluene sulfonyl chloride;
TMSI is trimethylsilyl iodide;
TPP is triphenylphosphine;
TsNH₂ is p-toluenesulfonamide;
Tosyl is p-toluenesulfonyl;
THF is tetrahydrofuran;
TLC is thin layer chromatography.

### EXAMPLES

Synthetic methods for preparing the representative compounds of the present invention are illustrated in the following Examples. Starting materials are commercially available or may be made according to procedures known in the art or as illustrated herein. The following Examples are intended to help illustrate the invention, and are not intended to, nor should they be constructed to limit its scope.

### Example 1

### Preparation of [1, 4]Thiazepane (1a)

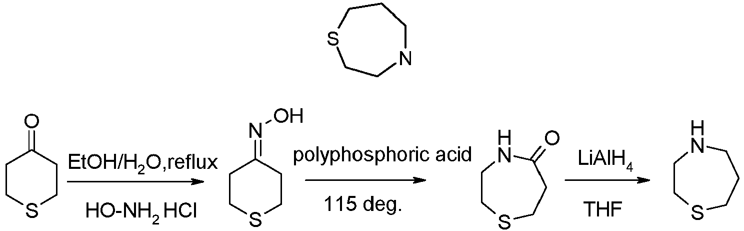

### Step 1: Synthesis of Dihydro-2H-thiopyran-4(3H)-one oxime (1a-1)

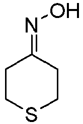

To a solution of dihydro-2*H*-thiopyran-4(3H)-one (10 g, 0.086 mol) and hydroxylamine hydrochloride (10.4 g, 0.15 mol) in H₂O (100 mL) and ethanol (40 mL) was added sodium acetate (13.1 g, 0.16 mol). The mixture was refluxed for 4h, the organic solvent was removed in vacuum and the residue was cooled in an ice bath, 8.92 g solid was obtained in 79% yield by filtration. ¹H-NMR (400 MHz, CDCl₃) δ: 2.88 (m, 2H), 2.80 (m, 2H), 2.74 (m, 2H), 2.57 (m, 2H).

### Step 2: Synthesis of 1,4-Thiazepan-5-one (1a-2)

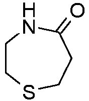

The mixture of dihydro-2*H*-thiopyran-4(3*H*)-one oxime (4.01 g, 0.03 mol) in polyphosphoric acid was heated at 115□ for 15min, and cooled to rt, ice-water was added, then the mixture was extracted with EtOAc 5 times. The combined organic layer was dried over Na₂SO₄ and concentrated under vacuum to give 2.4 g product as brown solid in 60% yield. ¹H-NMR (400 MHz, CDCl₃) δ: 6.79 (brs, 1H), 3.63 (m, 2H), 2.94 (m, 2H), 2.74 (m, 4H).

### Step 3: Synthesis of 1,4-Thiazepane (1a)

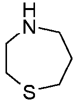

To a solution of 1,4-thiazepan-5-one (2.07 g, 15.7 mmol) in dry THF was added LiAlH₄ (0.66 g, 17.3 mmol) at 0□, then the mixture was stirred at rt for 4h. H₂O (0.7 mL), 15% NaOH (0.7 mL) and H₂O (2.1 mL) were added to the reaction in successively. The mixture was filtrated to give 1.77 g product in 96% yield. ¹H-NMR (400 MHz, CDCl₃) δ: 3.07 (m, 2H), 2.98 (m, 2H), 2.75 (m, 4H), 1.93 (m, 2H).

### Example 2

### Preparation of 1,5-Thiazocane hydrochloride (1b)

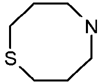

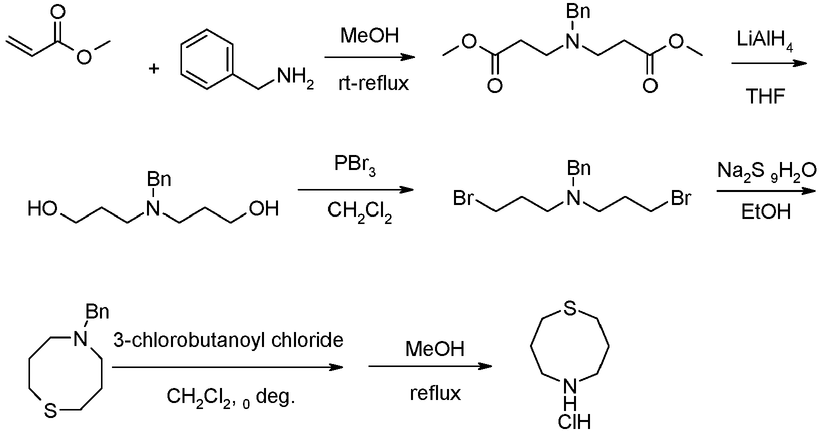

### Step 1: Synthesis of Dimethyl 3,3'-(benzylazanediyl)dipropanoate (1b-1)

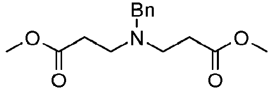

A solution of benzylamine (10.7 g, 0.1 mol) in MeOH (50 mL) was added in dropwise to a solution of methyl acrylate (18.9 g, .022 mol) in MeOH (100 mL) at rt. The result mixture was refluxed for 8h, and evaporated in vacuum to give 27.9 g product in quantitative yield. ¹H-NMR (400 MHz, CDCl₃) δ: 7.28 (m., 5H), 3.64 (s, 2H), 3.59 (s, 6H), 2.80 (m, 4H), 2.47 (m, 4H).

### Step 2: Synthesis of 3,3'-(Benzylazanediyl)bis(propan-1-ol) (1b-2)

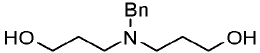

To a solution of dimethyl 3,3'-(benzylazanediyl)dipropanoate (4.47 g, 16.0 mmol) in dry THF was added LiAlH₄ (0.77 g, 20.2 mmol) at 0□, then the mixture was stirred at rt for 24h. MeOH (1.5 mL), 15% NaOH (1.0 mL) and H₂O (1.0 mL) were added to the reaction in successively. The mixture was filtrated to give 3.4 g product in 91% yield. ¹H-NMR (400 MHz, CDCl₃) δ: 7.31 (m, 5H), 3.68 (t, *J =* 5.6 Hz, 5.6 Hz, 4H), 3.57 (s, 2H), 2.63 (t, *J =* 6.4 Hz, 6.0 Hz, 4H), 1.76 (m, 4H).

### Step 3: Synthesis of N-Benzyl-3-bromo-N-(3-bromopropyl)propan-1-amine (1b-3)

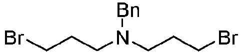

To a solution of 3,3'-(benzylazanediyl)bis(propan-1-ol) (447 mg, 2.0 mmol) in dry CH₂Cl₂ was added PBr3 in dropwise at 0□, then the mixture was stirred at rt for 12h. The reaction mixture was diluted with water and extracted with CH₂Cl₂. The combined organic layer was washed with sat. NaHCO₃ and brine, dried over Na₂SO₄, and concentrated. The 0.43 g product was obtained as yellow oil in 61% yield. ¹H-NMR (400 MHz, CDCl₃) δ: 7.27 (m, 5 H), 3.56 (s, 2 H), 3.44 (t, *J* = 6.8 Hz, 6.4 Hz, 4 H), 2.58 (t, *J =* 6.4 Hz, 6.4 Hz, 4 H), 2.02 (m, 4 H).

### Step 4: Synthesis of 5-Benzyl-1,5-thiazocane (1b-4)

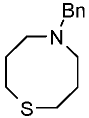

To a solution of N-benzyl-3-bromo-N-(3-bromopropyl)propan-1-amine (1.0 g, 2.9 mmol) in ethanol was added Na₂S·9H₂O (697 mg, 2.9 mmol). The mixture was refluxed for 18 h. The mixture was then cooled to r.t., and the solvent was removed in vacuum. To the residue was added H₂O and Et₂O. The aqueous layer was extracted with Et₂O, and the combined organic layer was washed with brine, dried over Na₂SO₄ and concentrated under vacuum. The crude product was used without purification.

### Step 5: Synthesis of 1,5-Thiazocane hydrochloride (1b)

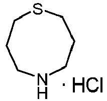

To a solution of 5-benzyl-1,5-thiazocane (8.6 g, 39 mmol) in CH₂Cl₂ was added (6.15 g, 43 mmol) at 0□. The mixture was stirred at rt for 6 h. the solvent was evaporated in vacuum and the residue was refluxed in MeOH for 3h. The mixture was concentrated and washed with Et₂O. The crude product was used without purification.

### Example 3

### Preparation of (1R,5S)-3-thia-6-azabicyclo[3.1.1]heptane (1c)

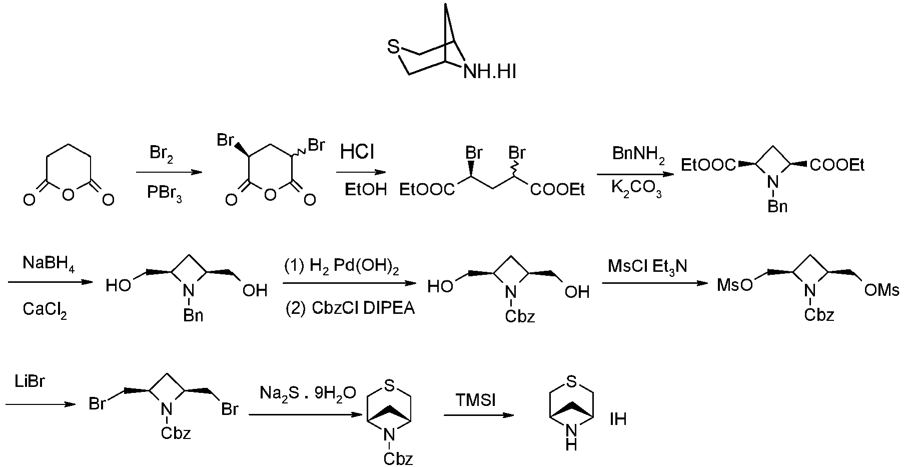

### Step 1: Synthesis of diethyl 2,4-dibromopentanedioate (1c-1)

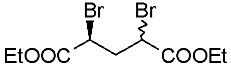

To a solution of dihydro-2H-pyran-2,6(3H)-dione (11.4 g, 0.1mol) and PBr₃ (0.1 mL) was added Br₂ (32 g, 0.2 mol) dropwise at 100°C, the mixture was stirred at 100°C for 7h and cooled to rt. HCl/EtOH (10mL) was added to the reaction mixture and stirred overnight at rt. After EtOH was evaporated, Et₂O was added to the residue and washed with sat. NaHCO₃ and brine, dried over Na₂SO₄, concentrated to give 32 g product was used for next step without purification.

### Step 2: Synthesis of (2R,4S)-diethyl 1-benzylazetidine-2,4-dicarboxylate (1c-2)

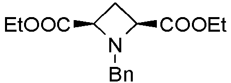

A mixture of (2R,4S)-diethyl 2,4-dibromopentanedioate (54 g, 156 mmol), benzylamine (17 g, 159 mmol) and K₂CO₃ (25.9 g, 187.2 mmol) in toluene was refluxed for 24h. the mixture was washed with brine, dried over and concentrated. The crude product was purified by chromatography on silica gel to give 18.39 g product in 41% yield. HRMS (ESI): *m*/*z* [M + H]⁺ calcd for C₁₆H₂₂NO₄: 292.1549; found: 292.1542.

### Step 3: Synthesis of ((2R,4S)-1-benzylazetidine-2,4-diyl)dimethanol (1c-3)

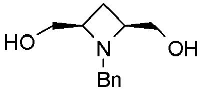

To a solution of (2R,4S)-diethyl 1-benzylazetidine-2,4-dicarboxylate (0.8 g, 2.75 mmol) in EtOH/MeOH (9:1; 10 mL) was added CaCl₂ (0.92 g, 8.25 mmol) at r.t. To the resulting stirred mixture was then added NaBH₄ (0.63 g, 16.5 mmol) in portions. The reaction mixture was stirred for overnight at r.t. Subsequently H₂O (5 mL) was added, and the mixture was stirred for 30 min. The mixture was then concentrated in vacuum, and partitioned between H₂O (10 mL) and CH₂Cl₂ (10 mL). The aqueous layer was extracted with CH₂Cl₂ (2 × 10 mL), and the organic layers were combined, washed with brine (10 mL), dried over Na₂SO₄, and concentrated in vacuo to give 0.25 g product as yellow oil. This product was used in the next step without further purification. MS (ESI): *m*/*z* [M + H]⁺: 208.1477.

### Step 4: Synthesis of (2R,4S)-benzyl 2,4-bis(hydroxymethyl)azetidine-1-carboxylate (1c-4)

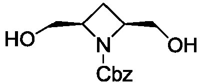

To a solution of ((2*R*,4*S*)-1-benzylazetidine-2,4-diyl)dimethanol (0.52 g, 2.9 mmol) in MeOH (10 mL) was added Pd(OH)₂ (0.13 g), and the mixture was stirred for 2 h under H₂ at r.t. The suspension was filtered through a short pad of Celite and eluted with additional MeOH. The solvent was removed in vacuo. The residue was dissolved in anhydrous CH₂Cl₂ (30 mL). To the resulting solution was added DIPEA (0.37 g, 2.9 mmol), and then CbzCl (0.44 g, 2.56 mmol) dropwise. The mixture was stirred for 2 h at r.t., and then quenched with H₂O (50 mL). The organic layer was washed with brine, dried over Na₂SO₄, and concentrated. The crude product was purified by chromatography on silica gel (5% MeOH in CH₂Cl₂) to afford 0.33 g product as a yellow oil in 45% yield.

### Step 5: Synthesis of (2R,4S)-benzyl-2,4-bis(((methylsulfonyl)oxy)methyl)azetidine-1-carboxylate (1c-5)

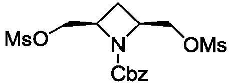

To a solution of (2*R*,4*S*)-benzyl 2,4-bis(hydroxymethyl)azetidine-1-carboxylate (51 mg, 0.2 mmol) in CH₂Cl₂(15 mL) was added Et₃N (61 mg, 0.6 mmol), and then MsCl (70 mg, 0.6 mmol) dropwise. The mixture was stirred for 5 h at r.t., and the reaction mixture was washed with 1N HCl and brine, dried over Na₂SO₄, and concentrated. The crude product was purified by chromatography on silica gel (5% MeOH in CH₂Cl₂) to afford 65 mg product as a yellow oil in 80% yield.

HRMS (ESI): *m*/*z* [M + H]⁺ calcd for C₁₅H₂₂NO₈S₂: 408.0787; found: 408.0780.

### Step 6: Synthesis of (2R,4S)-benzyl-2,4-bis(bromomethyl)azetidine-1-carboxylate (1c-6)

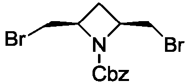

A mixture of (2*R*,4*S*)-benzyl-2,4-bis(((methylsulfonyl)oxy)methyl)azetidine-1-carboxylate (65 mg, 0.16 mmol) and LiBr (139 mg, 1.6 mmol) in acetone (15mL) was refluxed for 10h. The reaction mixture was evaporated, the residue was added H₂O (20 mL) and Et₂O (20 mL). The aqueous layer was extracted with Et₂O (2 × 20 mL), and the combined organic layer was washed with brine (10 mL), dried over Na₂SO₄ and concentrated under vacuum to give 51 mg product as yellow oil in 85% yield. HRMS (ESI): *m*/*z* [M + H]⁺ calcd for C₁₃H₁₅Br₂NO₂: 375.9548; found: 375.9558.

### Step 7: Synthesis of (1R,5S)-benzyl 3-thia-6-azabicyclo[3.1.1]heptane-6-carboxylate (1c-7)

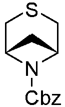

To a solution of (2*R*,4*S*)-benzyl 2,4-bis(bromomethyl)azetidine-1-carboxylate (0.77 g, 2.05 mmol) in DMF (5 mL) was added Na₂S·9H₂O (0.59 g, 2.46 mmol). The mixture was stirred at rt for 45min. To the solution was added H₂O (20 mL) and EtOAc (25 mL). The aqueous layer was extracted with EtOAc (2 × 20 mL), and the combined organic layer was washed with brine (10 mL), dried over Na₂SO₄ and concentrated under vacuum. The crude product was purified by chromatography on silica gel (20-30% EtOAc in PE) to give 0.15 g product as a colorless oil in 28.7% yield. HRMS (ESI): *m*/*z* [M + H]⁺ calcd for C₁₃H₁₇NO₂S: 250.0902; found: 250.0900.

### Step 8: Synthesis of (1R,5S)-3-thia-6-azabicyclo[3.1.1]heptane iodate (1c)

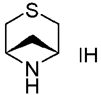

To a solution of (1*R,* 5*S*)-benzyl-3-thia-6-azabicyclo[3.1.1]heptane-6-carboxylate (0.19 g, 0.8 mmol) in anhydrous CH₂Cl₂ (20 mL) was added TMSI (0.39 g, 1.9 mmol) under Ar at 0 °C. The resulting mixture was stirred at rt for 2 h, and MeOH (5 mL) was added to the reaction dropwise. The result solution was stirred for additional 0.5 h, and then evaporated to remove the solvent. The residue was washed with PE/EtOAc (2:1) to give 0.24 g crude product as a brown solid and was used without purification.

### Example 4

### Preparation of (1R, 5S)-3-Thia-8-azabicyclo[3.2.1]octane iodate (1d)

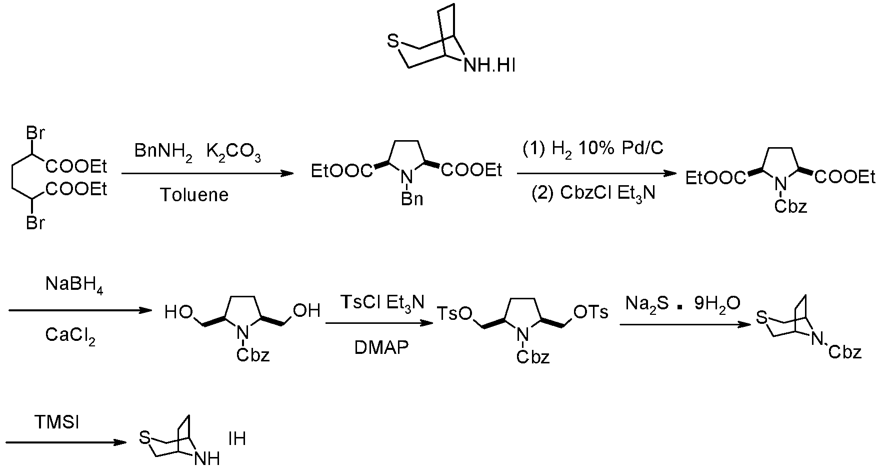

### Step 1: Synthesis of cis-Diethyl 1-benzylpyrrolidine-2,5-dicarboxylate (1d-1)

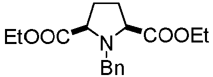

To a stirred solution of diethyl 2,5-dibromohexanedioate (10.8 g, 30 mmol) and benzylamine (3.2 g, 30 mmol) in toluene (45 mL) and H₂O (9 mL) was added K₂CO₃ (5 g, 36 mmol) at r.t. The mixture was refluxed for 20 h under Ar, and then poured into H₂O (30 mL). The aqueous layer was extracted with EtOAc (2 × 20 mL), the combined organic layers were washed with brine (20 mL), dried over Na₂SO₄, and purified by chromatography on silica gel (10-20% EtOAc in PE) to afford 6.1 g product as a yellow oil in 67% yield.

¹H-NMR (400 MHz, CDCl₃) δ: 7.35-7.22 (m, 5 H), 4.06-4.00 (m, 4 H), 3.97 (s, 2 H), 3.46 (brs, 2 H), 2.08-2.04 (m, 4 H), 1.19 (t, *J =* 7.1 Hz, 6 H). HRMS (ESI): *m*/*z* [M + H]⁺ calcd for C₁₇H₂₄NO₄: 306.1705; found: 306.1695.

### Step 2: Synthesis of cis-1-Benzyl 2,5-diethyl pyrrolidine-1,2,5-tricarboxylate (1d-2)

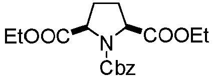

To a solution of cis-diethyl 1-benzylpyrrolidine-2,5-dicarboxylate (5.4 g, 17.7 mmol) in MeOH (100 mL) was added 10% Pd/C (0.54 g), and the mixture was shaken in a Parr Shaker for 4 h at 50 psi under H₂ at r.t. The suspension was filtered through a short pad of Celite and eluted with additional MeOH. The solvent was removed in vacuo. The residue was dissolved in anhydrous CH₂Cl₂ (50 mL), and cooled to 0 °C. To the resulting solution was added Et₃N (2.2 g, 21.6 mmol), and then CbzCl (3.7 g, 21.6 mmol) dropwise. The mixture was stirred overnight at r.t., and then quenched with H₂O (50 mL). The organic layer was washed with brine, dried over Na₂SO₄, and concentrated. The crude product was purified by chromatography on silica gel (5-20% EtOAc in PE) to afford 5.22 g product as a yellow oil in 84% yield. ¹H-NMR (400 MHz, CDCl₃) δ: 7.33-7.29 (m, 5 H), 5.19-5.10 (m, 2 H), 4.47 (m, 1 H), 4.40 (m, 1 H), 4.22 (q, *J* = 7.2 Hz, 2 H), 4.09 (q, *J =* 6.8 Hz, 2 H), 2.25-2.14 (m, 4 H), 1.28 (t, *J* = 6.8 Hz, 3 H), 1.17 (t, *J =* 6.8 Hz, 3 H). HRMS (ESI): *m*/*z* [M + H]⁺ calcd for C₁₈H₂₄NO₆: 350.1604; found: 350.1649.

### Step 3: Synthesis of cis-Benzyl 2,5-bis(hydroxymethyl)pyrrolidine-1-carboxylate (1d-3)

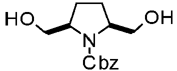

To a solution of *cis*-1-benzyl 2,5-diethyl pyrrolidine-1,2,5-tricarboxylate (5.75 g, 16.4 mmol) in EtOH/MeOH (10:1; 300 mL) was added CaCl₂ (5.5 g, 49.2 mmol) at r.t. To the resulting stirred mixture was then added NaBH₄ (3.75 g, 98.4 mmol) in portions. The reaction mixture was stirred for overnight at r.t. Subsequently H₂O (50 mL) was added, and the mixture was stirred for 30 min. The mixture was then concentrated in vacuo, and partitioned between H₂O (100 mL) and EtOAc (100 mL). The aqueous layer was extracted with EtOAc (2 × 50 mL), and the organic layers were combined, washed with brine (10 mL), dried over Na₂SO₄, and concentrated in vacuo to give 4.57 g product as colorless oil. This product was used in the next step without further purification.

¹H-NMR (400 MHz, CDCl₃) δ: 7.39-7.33(m, 5 H), 5.16 (s, 2 H), 4.09-3.82 (m, 4 H), 3.56 (d, *J* = 8.1 Hz, 2 H), 2.91 (brs, 2 H), 2.04-1.97 (m, 4 H). HRMS (ESI): *m*/*z* [M + Na]⁺ calcd for C₁₄H₁₉NNaO₄: 288.1206; found: 288.1196.

### Step 4: Synthesis of cis-Benzyl 2,5-bis(tosyloxymethyl)pyrrolidine-1-carboxylate (1d-4)

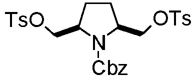

A solution of compound cis-benzyl 2,5-bis(hydroxymethyl)pyrrolidine-1-carboxylate (4.35 g, 16.4 mmol), Et₃N (3.65 g, 36.1 mmol), and DMAP (4.01 g, 32.8 mmol) in CH₂Cl₂ (50 mL) was cooled to 0 °C. To this mixture was added p-toluenesulfonyl chloride (6.88 g, 36.1 mmol) in one portion and the resulting mixture was stirred overnight at r.t. The mixture was then washed with water and brine, dried over Na₂SO₄ and concentrated under vacuum. The residue was purified by flash column chromatography (30-40% EtOAc in PE) to give 8.97 g product as a semi-solid in 95% yield.

¹H-NMR (400 MHz, CDCl₃) δ: 7.73 (brs, 4 H), 7.36-7.29 (m, 9 H), 5.03-4.96 (m, 2 H), 4.15-3.89 (m, 6 H), 2.44 (s, 6 H), 1.87-1.83 (m, 4 H). ¹³C-NMR (100 MHz, CDCl₃) δ (rotamers): 165.30, 154.49, 144.90, 135.88, 132.61, 129.90, 128.55, 128.19, 127.89, 69.16, 68.87, 67.28, 57.30, 56.56, 26.61, 25.44, 21.63. HRMS (ESI): *m*/*z* [M + H]⁺ calcd for C₂₈H₃₂NO₈S₂: 574.1564; found: 574.1547.

### Step 5: Synthesis of (1R, 5S)-Benzyl-3-thia-8-azabicyclo[3.2.1]octane-8-carboxylate (1d-5)

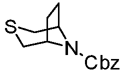

To a solution of *cis*-benzyl 2,5-bis(tosyloxymethyl)pyrrolidine-1-carboxylate (4.1 g, 7.1 mmol) in ethanol (25 mL) and H₂O (25 mL) was added Na₂S·9H₂O (5.12 g, 21.3 mmol). The mixture was refluxed for 5 h. The mixture was then cooled to r.t., and the solvent was removed in vacuo. To the residue was added H₂O (20 mL) and EtOAc (25 mL). The aqueous layer was extracted with EtOAc (2 × 20 mL), and the combined organic layer was washed with brine (10 mL), dried over Na₂SO₄ and concentrated under vacuum. The crude product was purified by chromatography on silica gel (20-30% EtOAc in PE) to give 1.38 g product as a colorless oil in 73% yield.

¹H-NMR (400 MHz, CDCl₃) δ: 7.37-7.30 (m, 5 H), 5.16 (s, 2 H), 4.52-4.47 (m, 2 H), 3.22 (d, *J =* 11.6 Hz, 1 H), 3.11 (d, *J* = 10.8 Hz, 1 H), 2.12 (d, *J =* 12.8 Hz, 2 H), 2.06 (d, *J =* 1.2 Hz, 4 H). ¹³C-NMR (100 MHz, CDCl₃) δ: 152.95, 136.59, 128.40, 127.93, 127.80, 66.73, 53.94, 32.72, 32.08, 28.82, 27.99. HRMS (ESI): *m*/*z* [M + H]⁺ calcd for C₁₄H₁₈NO₂S: 264.1058; found: 264.1113.

### Step 6: Synthesis of (1R, 5S)-3-Thia-8-azabicyclo[3.2.1]octane iodate (1d)

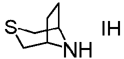

To a solution of (1*R,* 5*S*)-benzyl-3-thia-8-azabicyclo[3.2.1]octane-8-carboxylate (0.24 g, 0.91 mmol) in anhydrous CH₂Cl₂ (20 mL) was added TMSI (0.44 g, 2.18 mmol) under Ar at 0 °C. The resulting mixture was stirred at rt for 0.5 h, and MeOH (0.26 mL) was added to the reaction dropwise. The result solution was stirred for additional 0.5 h, and then evaporated to remove the solvent. The residue was washed with PE/EtOAc (1:2) to give 0.21 g product as a yellow solid in 91% yield. Mp: 208-210□. ¹H-NMR (400 MHz, CDCl₃) δ: 8.92 (brs, 1 H), 8.72 (brs, 1 H), 4.38 (s, 2 H), 3.78 (d, *J =* 14.0 Hz, 2 H), 2.41-2.35 (m, 4 H), 2.24-2.22 (d, *J =* 7.6 Hz, 2 H). ¹³C-NMR (100 MHz, CDCl₃) δ: 55.67, 31.42, 27.23. HRMS (ESI): *m*/*z* [M + H]⁺ calcd for C₆H₁₂NS: 130.0685; found: 130.0686.

### Example 5

### Preparation of 2-Thia-6-aza-spiro[3.3]heptane (1e)

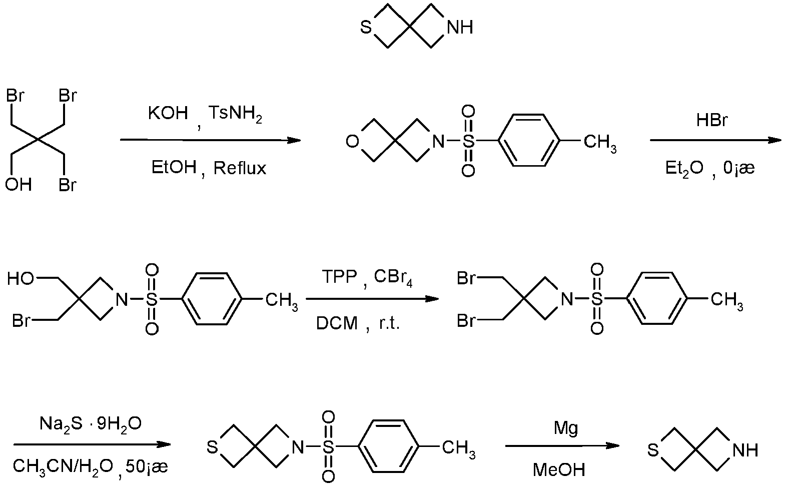

### Step 1: Synthesis of 6-Tosyl-2-oxa-6-azaspiro[3.3]heptane (1e-1)

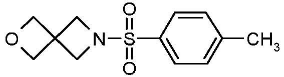

To a solution of KOH (9.04 g, 161 mmol) and 3-bromo-2,2-bis(bromomethyl)propan-1-ol (15.3 g, 47.0 mmol) in 500 mL ethanol was added p-tosylamide (17.9 g, 104 mmol) at room temperature and the reaction mixture was refluxed for 20 h. The solvent was removed by evaporation, 100 mL 8% NaOH solution was added and the suspension was stirred for another 2 h. The mixture was filtered and the white filter cake was rinsed with water until the washing water was neutral. The filter cake was dried to give the title product. Yield: 6.1 g (40.2 %). ¹H-NMR (400 MHz, CDCl₃) δ: 7.71 (d, *J* = 8.0 Hz, 2 H), 7.37 (d, *J* = 8.0 Hz, 2 H), 4.59 (s, 4 H), 3.91 (s, 4 H), 2.46 (s, 3 H). HRMS (ESI-TOF⁺): *m*/*z* [M + H]⁺ calcd for C₁₂H₁₆NO₃S: 254.0825; found: 254.0851.

### Step 2: Synthesis of (3-(bromomethyl)-1-tosylazetidin-3-yl)methanol (1e-2)

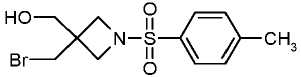

To a suspension of 6-(*p*-toluenesulfonyl)-2-oxa-6-azaspiro[3.3]heptane (1e-1) (9.79 g, 38.7 mmol) in Et₂O (200 mL) at 0 °C was added a solution of hydrobromic acid (ca. 33% in AcOH) in dropwise. The resulting solution was stirred at room temperature for 30 min, it was adjusted to pH=8 with 1mol/L NaOH. The phases were separated and the aqueous phase was extracted with Et₂O (3×100 mL). The combined organic layers were dried (Na₂SO₄), filtered, and concentrated *in vacuo* to afford the title compound as a colorless solid. Yield: 10.0 g (77.4%). ¹H-NMR (400 MHz, CDCl₃) δ: 7.74 (d, *J =* 8.0 Hz, 2 H), 7.39 (d, *J =* 8.0 Hz, 2 H), 3.68 (s, 2 H), 3.68 (s, 2 H), 3.62 (d, *J =* 8.4 Hz, 2 H), 3.55 (d, *J =* 8.4 Hz, 2 H), 3.45 (s, 2 H), 2.47 (s, 3 H).

### Step 3: Synthesis of 3,3-bis(bromomethyl)-1-tosylazetidine (1e-3)

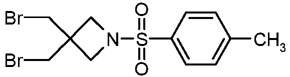

(3-(Bromomethyl)-1-tosylazetidin-3-yl)methanol (1e-2) (10.0 g, 30.0 mmol) was dissolved in CH₂Cl₂ and CBr₄ (16.4 g, 49.4 mmol) was added. The resulting solution was cooled to 0 °C and PPh₃ (17.9 g, 104 mmol) was added. The reaction mixture was stirred at room temperature overnight. The mixture was concentrated under reduced pressure and the residue was purified by chromatography on silica gel (5 - 10% EtOAc in PE) to give the pure title compound. Yield: 8.85 g (74.8%). ¹H-NMR (400 MHz, CDCl₃) δ: 7.73 (d, *J =* 8.0 Hz, 2 H), 7.39 (d, *J =* 8.0 Hz, 2 H), 3.59 (s, 4 H), 3.53 (s, 4 H), 2.47 (s, 3 H).

### Step 4: Synthesis of 6-tosyl-2-thia-6-azaspiro[3.3]heptane (1e-4)

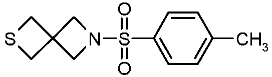

To a solution of 3,3-bis(bromomethyl)-1-tosylazetidine (1e-3) (8.82 g, 7.9 mmol) in a mixture of CH₃CN (90 mL) and H₂O (9 mL) was added Na₂S·9H₂O (10.7 g, 44.7 mmol) and the reaction mixture was stirred at 50 °C for 4 h, then it was concentrated to dryness. EtOAc (100 mL) and NaHCO₃ solution (100 mL) were added, and the phases were separated. The aqueous phase was extracted with EtOAc (2×100 mL). The organic phase was washed with brine, dried (Na₂SO₄), filtered, and concentrated *in vacuo* to give the title compound. Yield: 5.46 g (90.1%). ¹H-NMR (400 MHz, CDCl₃) δ: 7.71 (d *J* = 8.0 Hz, 2 H), 7.37 (d *J =* 8.0 Hz, 2 H), 3.78 (s, 4 H), 3.14 (s, 4 H), 2.46(s, 3 H). HRMS (ESI-TOF⁺): *m*/*z* [M + H]⁺ calcd for C₁₂H₁₆NO₂S₂: 270.0622; found: 270.0621.

### Step 5: Synthesis of 2-thia-6-azaspiro[3.3]heptane (1e)

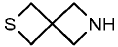

6-Tosyl-2-thia-6-azaspiro[3.3]heptane (1e-4) (2.0 g, 7.9 mmol) was dissolved in MeOH (40 mL). To the resulting solution was added magnesium powder (1.0g), and the reaction mixture was sonicated at RT for about 3 hrs. The reaction mixture was concentrated *in vacuo,* the crude product was used in next step without purification.

### Example 6

### Preparation of 2-Oxa-6-aza-spiro[3.3]heptane (1f)

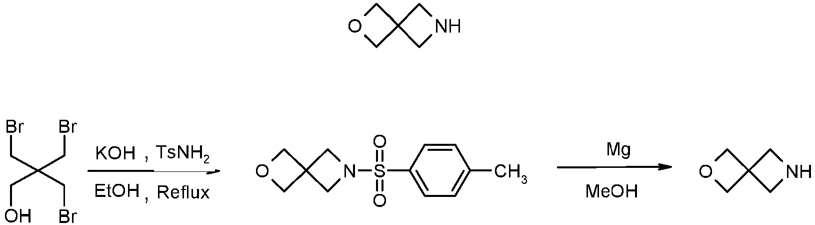

### Step 1: Synthesis of 6-Tosyl-2-oxa-6-azaspiro[3.3]heptane

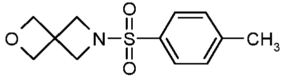

This product was synthesized as described in Example 5, Step 1.

### Step 2: Synthesis of 2-oxa-6-azaspiro[3.3]heptane (1f)

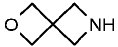

6-Tosyl-2-oxa-6-azaspiro[3.3]heptane (6.3 g, 25.0 mmol) was dissolved in MeOH (50 mL). To the resulting solution was added magnesium powder (6.0g), and the reaction mixture was sonicated at RT for about 3 hrs. The reaction mixture was concentrated *in vacuo,* the crude product was used in next step without purification.

### Example 7

### Preparation of N-Boc protected 2, 6-Diaza-spiro[3.3]heptane (1g)

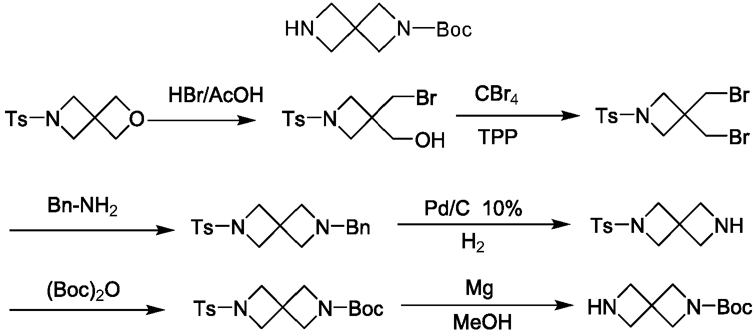

### Step 1: Synthesis of (3-(Bromomethyl)-1-(p-toluenesulfonyl)azetidin-3-yl)methanol (1g-1)

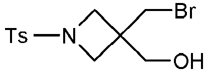

To a suspension of 6-(p-toluenesulfonyl)-2-oxa-6-azaspiro[3.3]heptane (6.25 g, 24.7 mmol) (obtained according to Example 5 step 1) in Et₂O (100 mL) at 0 °C was dropwise added over a period of 15 min a solution of hydrobromic acid (ca. 33% in AcOH; 4.1 mL, 24.7 mmol) in Et₂O (5 mL). The resulting mixture was warmed to room temperature and stirred for 45 min. The resulting colorless solution was poured into a saturated aqueous solution of NaHCO₃ (100 mL). The organic phase was separated and the aqueous phase was extracted with Et₂O (100 mL). The combined organic layers were dried (MgSO₄), filtered, and concentrated *in vacuo* to afford the title compound 7.74g as a colorless solid. The crude product was pure enough for further transformations.

### Step 2: Synthesis of 3,3-Bis(bromomethyl)-1-(p-toluenesulfonyl)azetidine (1g-2)

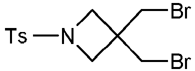

The above crude product 1g-1 (7.74 g, 23.1 mmol) was dissolved in CH₂Cl₂ (100 mL) and CBr₄ (13.7 g, 41.2 mmol) was added in one portion. The resulting solution was cooled to 0 °C and PPh₃ (26.26 g, 41.2 mmol) was added in one portion. The reaction mixture turned to a dark orange solution, which was stirred at 0 °C for 1.5 h, then warmed to room temperature and stirred for further 8 h. The mixture was concentrated under reduced pressure to afford a dark orange oil, which was purified by chromatography (hexanes : EtOAc 4:1) to give the title compound 7.61g. ¹H-NMR (400 MHz, CDCl₃) δ: 7.73 (d, *J* = 8.4 Hz, 2 H), 7.40 (d, *J* = 7.6 Hz, 2 H), 3.60 (s, 4 H), 3.53 (s, 4 H), 2.48 (s, 3 H).

### Step 3: Synthesis of 2-Benzyl-6-(p-toluenesulfonyl)-2,6-diazaspiro[3.3]heptane (1g-3)

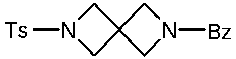

Dibromide 1g-2 (7.61 g, 19.1 mmol) was dissolved in CH₃CN (100 mL). Benzylamine (4.1 g, 38.3 mmol) and DIPEA (12.4 g, 95.5 mmol) were added to the above mixture and the reaction mixture was heated to reflux for 3 d. Then the yellowish solution was cooled to room temperature and concentrated to about 1/6 of the initial volume. The residue was partitioned between CH₂Cl₂ (100 mL) and 1 mol/L NaOH (100 mL). The organic phase was separated and the aqueous layer was extracted with CH₂Cl₂ (50 mL). The combined organic layers were dried (MgSO₄), filtered, and concentrated *in vacuo.* The residue was purified by chromatography (hexanes : EtOAc : Et3N 1:1:1% to 1:2:1% gradient) to afford the title compound 4.0 g. ¹H-NMR (400 MHz, CDCl₃) δ: 7.69 (d, *J =* 8.2 Hz, 2 H), 7.34 (d, *J =* 8.2 Hz, 2 H), 7.32-7.11 (m, 5 H), 3.82 (s, 4 H), 3.47 (s, 2 H), 3.13 (s, 4 H), 2.44 (s, 3 H).

### Step 4: Synthesis of tert-Butyl 6-(p-toluenesulfonyl)-2,6-diazaspiro[3.3]heptane-2-carboxylate (1g-5)

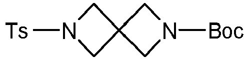

Benzyl azetidine 1g-3 (2.70 g, 7.88 mmol) was dissolved in MeOH (40 mL), and Pd/C (10% on charcoal; 0.54 g) was added to the above mixture. A hydrogen atmosphere (50 PSI) was built up and the mixture was heated to 45 °C and stirred at this temperature for 48 h. Then the reaction mixture was cooled to room temperature and filtered over celite. The filter cake was washed thoroughly with MeOH (2 × 20 mL). To the above solution of the intermediate Ts-protected azetidine (1g-4) in MeOH (ca. 80 mL) was added Boc₂O (1.77 g, 7.88 mmol). The resulting solution was stirred at room temperature for 1 h and concentrated *in vacuo.* The residue was purified by chromatography (hexanes : EtOAc 1: 1 to 1:2 gradient) to furnish the pure title compound. ¹H-NMR (400 MHz, CDCl₃) δ: 7.71 (d, *J* = 7.6 Hz, 2 H), 7.37 (d, *J* = 8.0 Hz, 2 H), 3.85 (s, 4 H), 3.84 (s, 4 H), 2.46 (s, 3 H), 1.39 (s, 9 H).

### Step 5: Synthesis of tert-Butyl 2,6-diazaspiro[3.3]heptane-2-carboxylate (1g)

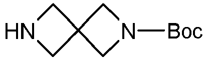

The above product 1g-5 (3.50 g, 10.0 mmol) was dissolved in MeOH (30 mL). Mg powder (1.92 g, 80.0 mmol) was added, and the mixture was sonicated for 6 h. The reaction mixture was concentrated *in vacuo* to afford a dark gray solid, which can be . used for the further reaction without purification.

### Example 8

### General Synthetic Methods: preparation of oxazolidinone compounds

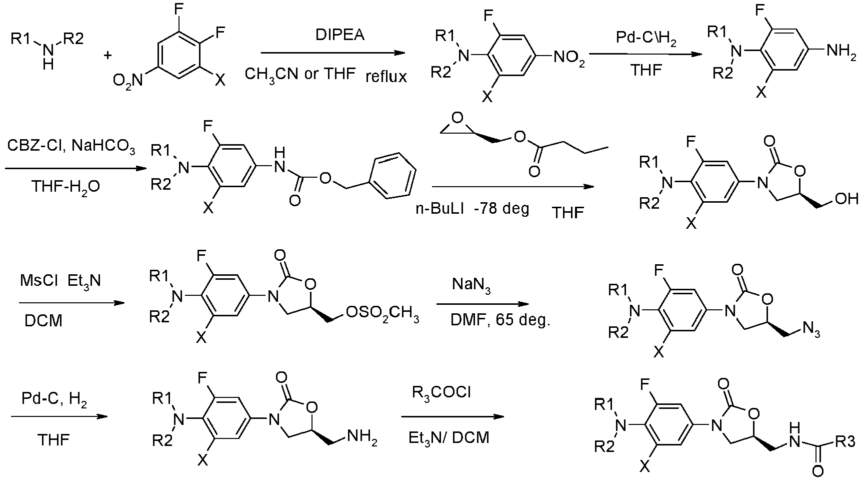

wherein, 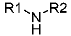 represents ring A as previously defined in Formula I; X = H, or F.

General procedures for the preparation of (*S*)-N-((3-(3-fluoro-4-(1,5-thiazocan-5-yl) phenyl)-2-oxooxazolidin-5-yl)methyl)acetamide (OTB-114) and its sulfoxide (OTB-124), are provided below.

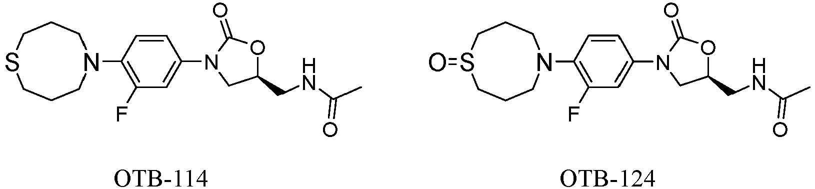

### Step A: 5-(2-Fluoro-4-nitrophenyl)-1,5-thiazocane

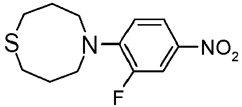

A solution of 3,4-difluoronitrobenzene (4.92 g, 31 mmol) and N,N-diisopropylethylamine (8.81 g, 68 mmol) in CH₃CN (30 mL) was treated with 1,5-thiazocane hydrochloride (5.2 g, 31 mol) at ambient temperature and the reaction mixture heated to reflux for 24 h. The reaction mixture was cooled to rt and concentrated. The residue was diluted with H₂O and CH₂Cl₂, aqueous layer was extracted with CH₂Cl₂ (50mL*3). The organic layer was dried over Na₂SO₄, filtered, and concentrated. The crude product was purified by chromatography on silica gel (PE/CH₂Cl₂=10:1) to give 3.36 g (40%) of the title compound as a yellow solid.¹H-NMR (400 MHz, CDCl₃) δ: 7.92 (m, 2H), 6.80 (t, *J =* 9.2 Hz, 8.8 Hz, 1H), 3.67 (t, *J* = 6.0 Hz, 5.6 Hz, 4H), 2.72 (t, *J* = 5.6 Hz, 6.0 Hz, 4H), 2.08 (m, 4H).

### Step B: Benzyl(3-fluoro-4-(1,5-thiazocan-5-yl)phenyl)carbamate

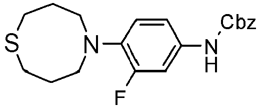

To a solution of 5-(2-fluoro-4-nitrophenyl)-1,5-thiazocane (3.0 g, 12.5 mmol) in MeOH/THF was added 10% Pd/C (0.3 g), and the mixture was shaken 4 h under H₂ at r.t. The suspension was filtered through a short pad of Celite and eluted with additional MeOH. The solvent was removed in vacuo. The residue was dissolved in THF/H₂O (50 mL). To the resulting solution was added NaHCO₃ (2.12 g, 25.2 mmol), and then CbzCl (2.58 g, 15.1 mmol) dropwise. The mixture was stirred overnight at r.t., and concentrated. The residue was added H₂O (50 mL) and extracted with CH₂Cl₂. The organic layer was washed with brine, dried over Na₂SO₄, and concentrated. The crude product was purified by chromatography on silica gel (PE/EtOAc=5:1) to afford 4.6 g product as a colorless solid in 98% yield. ¹H-NMR (400 MHz, CDCl₃) δ: 7.30 (m, 5H), 6.96 (m, 2H), 6.76 (s, 1H), 4.11 (m, 2H), 3.30 (m, 4H), 2.74 (m, 4H), 1.93 (m, 4H).

### Step C: (R)-[3-[3-Fluoro-4-(1,5-thiazocane-5-yl)phenyl]-2-oxo-5-oxazolidinyl]methanol

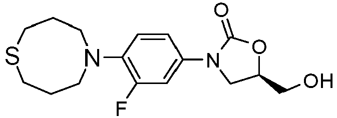

A solution of benzyl(3-fluoro-4-(1,5-thiazocan-5-yl)phenyl)carbamate (2.44 g, 6.5 mmol) in dry THF (20 mL) was cooled to -78 °C (dry ice/acetone bath) under N₂. *n*-Butyllithium (2.5 M solution in hexanes, 2.9 mL, 7.2 mmol) was added to the reaction mixture over 10 min. The resultant light yellow solution was stirred at -78 °C for 50 min and then treated with (*R*)-(-)-glycidyl butyrate (0.95 mL, 6.9 mmol) dropwise. The reaction mixture was stirred for an additional 30 min at -78 °C, and then the cooling bath was removed. The reaction mixture was allowed to warm to ambient temperature overnight. Saturated aqueous NH₄Cl (50 mL) was added to the reaction mixture. The reaction mixture was extracted with EtOAc. The combined organic extracts were washed with brine, dried over Na₂SO₄, filtered, and concentrated. The residue was purified by by chromatography on silica gel (PE/EtOAc=1:2) to afford 1.33 g product as a colorless solid in 59% yield. ¹H-NMR (400 MHz, CDCl₃) δ: 7.37 (dd, *J* = 14.8 Hz, 2.4 Hz, 1H), 7.09 (dd, *J* = 8.8 Hz, 2.4 Hz, 1H), 7.00 (m, 1H), 4.73 (m, 1H), 3.95 (m, 3H), 3.76 (m, 1H), 3.35 (m, 4H), 2.74 (m, 4H), 1.97 (m, 4H).

### Step D: (R)-[3-[3-Fluoro-4-(1,5-thiazocane-5-yl)phenyl]-2-oxo-5-oxazolidinyl] methyl methanesulfonate

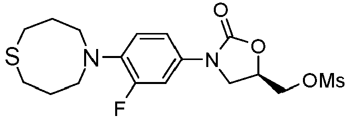

A solution of (*R*)-[3-[3-Fluoro-4-(1,5-thiazocane-5-yl)phenyl]-2-oxo-5-oxazolidinyl] methanol (1.0 g, 2.94 mmol) in dry CH₂Cl₂ was cooled with an ice bath and treated with Et₃N (446 mg, 4.41 mmol) and methanesulfonyl chloride (404 mg, 3.53 mmol). The mixture was stirred for 2h at rt, and was washed with H₂O, saturated aqueous NaHCO₃, and brine. The organic layer was then dried over Na₂SO₄, filtered, and concentrated. The product was used in next step without purification.

### Step E: (R)-[3-[3-Fluoro-4-(1,5-thiazocane-5-yl)phenyl]-2-oxo-5-oxazolidinyl] methyl azide

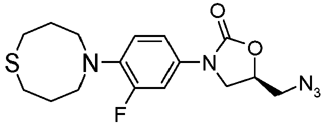

A solution of (*R*)-[3-[3-Fluoro-4-(1,5-thiazocane-5-yl)phenyl]-2-oxo-5-oxazolidinyl] methyl methanesulfonate (859 mg, 2.1 mmol) in dry DMF was treated with solid NaN₃ (683 mg, 10.5 mmol) at rt. The mixture was then heated to 65 °C for 8 h, after cooling to rt; the reaction mixture was quenched with H₂O and was extracted with EtOAc. The combined organic layer was washed with H₂O and brine, dried over Na₂SO₄, filtered, and concentrated. The product was used in next step without purification.

### Step F: (S)-N-((3-(3-fluoro-4-(1,5-thiazocan-5-yl)phenyl)-2-oxooxazolidin-5-yl) methyl)acetamide

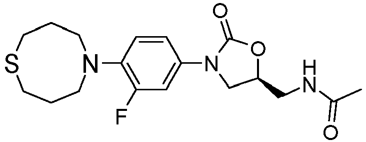

To a solution of (*R*)-[3-[3-fluoro-4-(1,5-thiazocane-5-yl)phenyl]-2-oxo-5-oxazolidinyl]methyl azide (216 mg, 0.59 mmol) in MeOH/THF was added 10% Pd/C (22 mg), and the mixture was shaken 4 h under H₂ at r.t. The suspension was filtered through a short pad of Celite and eluted with additional MeOH. The solvent was removed in vacuo. The residue was dissolved in CH₂Cl₂ and was treated with Et₃N (121 mg, 1.2 mmol) and AcCl (56 mg, 1.2 mmol). The reaction mixture was quenched with H₂O and extracted with CH₂Cl₂. The combined organic extracts were washed with brine, dried over Na₂SO₄, filtered, and concentrated. The residue was purified by chromatography on silica gel (CH₂Cl₂/MeOH=100:1) to afford 0.1 g product as a colorless solid in 44% yield. mp 78-80°C. ${\left[α\right]}_{D}^{20}$ -15.4 (c 0.25, CHCl₃). ¹H-NMR (400 MHz, CDCl₃) δ: 7.34 (m, 1H), 7.02 (m, 2H), 6.23 (m, 1H), 4.75 (m, 1H), 4.00 (t, *J* = 8.8 Hz, 8.8 Hz, 1H), 3.73 (m, 2H), 3.64 (m, 1H), 3.36 (t, *J* = 6.4 Hz, 6.0 Hz, 4H), 2.73 (m, 4H), 2.04 (s, 3H), 1.97 (m, 4H). ¹³C-NMR (125 MHz, CDCl₃) δ: 171.4, 155.2 (d, *J* = 243.5 Hz), 154.4, 134.5, 130.2, 119.9, 114.3, 108.3 (d, *J* = 26.8 Hz), 71.9 , 48.1, 47.8, 42.0, 31.9, 29.7, 23.1. HR-MS (ESI-TOF): m/z [M+H]⁺ calcd for C₁₈H₂₅O₃N₃FS: 382.1595; found: 382.1620.

### Step G: (S)-N-[[3-(3-fluoro-4-(1-oxido-1,5-thiazocan-5-yl)phenyl)-2-oxo-oxazolidin-5-yl]methyl]acetamide

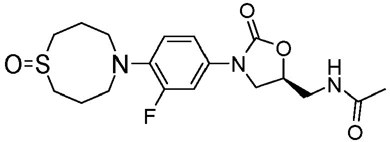

A solution of sodium metaperiodate (30 mg, 0.14 mmol) in H₂O (2 mL) was cooled to 0 °C. (S)-N-((3-(3-fluoro-4-(1,5-thiazocan-5-yl)phenyl)-2-oxooxazolidin-5-yl) methyl)acetamide (50 mg, 0.13 mmol) was added and then MeOH (3 mL). The reaction mixture was stirred at 0 °C for 2 h, and was concentrated. H₂O was added to the residue and then extracted with CH₂Cl₂ The combined organic layer was washed with brine, dried over anhydrous Na₂SO₄, filtered, and concentrated. The residue was purified by chromatography on silica gel (CH₂Cl₂/MeOH=100:1) to afford 39 mg product as a colorless solid in 75% yield. mp 69-70 °C. ¹H-NMR (400 MHz, CDCl₃) δ: 7.46 (dd, *J* = 2.8 Hz, 14.8 Hz, 1H), 7.14 (t, *J* = 9.2 Hz, 8.8 Hz, 1H), 7.07 (m, 1H), 6.15 (m, 1H), 4.78 (m, 1H), 4.03 (t, *J* = 9.2 Hz, 8.8 Hz, 1H), 3.74 (m, 3H), 3.31 (m, 1H), 3.18 (m, 4H), 2.98 (m, 2H), 2.17 (m, 4H), 2.03 (s, 3H). ¹³C-NMR (125 MHz, CDCl₃) δ: 171.0, 154.2, 134.0, 128.5, 127.3, 122.6, 113.9, 108.1 (d, *J* = 26.9 Hz), 71.9, 53.1, 51.7, 47.7, 42.0, 29.7, 25.0, 23.2. HR-MS (ESI): m/z [M+H]⁺ calcd for C₁₈H₂₅O₄N₃FS: 398.1544; found: 398.1540.

### Example 9

### In Vitro Assay for Antimicrobial Susceptibility

Antimicrobial susceptibility testing was performed in 96-well microplates. Initial drug dilutions (6.4 mg/ml) were prepared in dimethyl sulfoxide, and subsequent two-fold dilutions were performed in 0.1 ml of 7H9 broth media (BD) in the microplates. The final drug concentrations were about 0.008 µg/ml. Every concentration of test compounds was added to two wells. Control wells consisted of bacteria and positive drug (Linezolid). Plates were incubated at 37°C. The final bacterial titers were 1×10⁶ CFU/ml for H₃₇Rv. Starting at day 7 of incubation, 20µl of 10× Alamar blue solution (Life Technologies) and 12.5µl of 20% Tween 80 (Sigma-Aldrich) were added to each well and the plates were reincubated at 37°C. Wells were observed at 24h and the colors of all were recorded. Visual MICs were defined as the lowest amount of drug that prevented a color change from blue to pink. Fluorescence was measured in a microplate fluorometer in bottom-reading mode with excitation at 530 nm and emission at 590 nm. For fluorometric MICs, the lowest drug concentration effecting an inhibition of ≥90% was considered the MIC. The MIC results are provided in Table 1 above.

### Example 10

### In Vitro Assay for MPS Inhibition

H9C2 cells were incubated in DMEM (Hyclone, GE LifeSciences) with 10% FBS (Gibco, Life Technologies) and 1× Glutamine (Gibco, Life Technologies) and NEAA (Gibco, Life Technologies) at 37°C, 5% CO2 at 1500 cells/well in a 384-well plate. Test compound was added after 18 hr incubation, and then incubated for 5 days. COX-1 protein (cyclooxygenase I) and SDH-A (succinate dehydrogenase-A) formation reduction were measured by ELISA assay (MitoBiogenesisTM In-Cell ELISA Kit (Colorimetric, Abcam). MPS assay results are provided in Table 1 above.

### Example 11

### Specific Compounds Synthesized According to General Methods

### OTB-107 (R)-5-[(1H-1,2,3-Triazol-1-yl)methyl]-3-[3-fluoro-4-(1,4-thiazepan-4-yl)phenyl]oxa zolidin-2-one

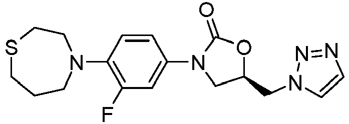

¹H-NMR (400 MHz, CDCl₃) δ: 7.94 (s, 1 H), 7.75 (s, 1 H), 7.17 (dd, *J =* 15.2 Hz, 1.2 Hz, 1 H), 6.89 (dd, *J* = 8.8 Hz, 1.6 Hz, 1 H), 6.82 (t, *J* = 9.6 Hz, 8.8 Hz, 1 H), 5.03 (m, 1 H), 4.78 (s, 2 H), 4.10 (t, *J* = 9.2 Hz, 8.8 Hz, 1 H), 3.87 (m, 1 H), 3.68 (m, 4 H), 2.87 (m, 2 H), 2.68 (t, *J* = 6.4 Hz, 6.0 Hz, 2 H), 2.06 (m, 2 H). ¹³C-NMR (100 MHz, CDCl₃) δ: 153.4, 151.0, 134.3, 124.9, 116.8, 115.1, 108.7 (d, *J* = 27.3 Hz), 70.2, 56.1, 51.9, 51.3, 47.4, 34.0, 31.5, 30.3. HR-MS (ESI): m/z [M + H]⁺ calcd for C₁₇H₂₁O₂N₅FS: 378.1395; found: 378.1396.

### OTB-106 (R)-5-[(2H-1,2,3-Triazol-2-yl)methyl]-3-[3-fluoro-4-(1,4-thiazepan-4-yl)phenyl]oxa zolidin-2-one

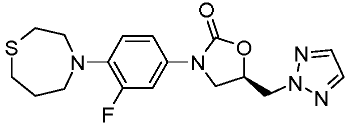

¹H-NMR (400 MHz, CDCl₃) δ: 7.65 (s, 2 H), 7.27 (m, 1 H), 7.01 (dd, *J* = 8.8 Hz, 1.6 Hz, 1 H), 6.84 (t, *J* = 9.2 Hz, 9.2 Hz, 1 H), 5.11 (m. 1 H), 4.85 (dd, *J* = 14 Hz, 4.8 Hz, 1 H), 4.74 (dd, *J* = 14 Hz, 6.8 Hz, 1 H), 4.05 (t, *J* = 9.2 Hz, 8.8 Hz, 1 H), 3.95 (m, 1 H), 3.67 (m, 4 H), 2.87 (m, 2 H), 2.69 (t, *J* = 6.4 Hz, 6.0 Hz, 2 H), 2.06 (m, 2 H). ¹³C-NMR (125 MHz, CDCl₃) δ: 153.7, 152.4 (d, *J* = 241.8 Hz), 135.0, 134.7, 129.1, 116.9 (d, *J* = 5.1 Hz), 114.7 (d, *J* = 2.9 Hz), 108.4 (d, *J* = 27.4 Hz), 69.9, 56.2, 56.1, 51.4, 48.2, 34.1, 31.6, 30.4. HR-MS (ESI): m/z [M + H]⁺ calcd for C₁₇H₂₁O₂N₅FS: 378.1395; found: 378.1403.

### OTB-109 (S)-3-[3-Fluoro-4-(1,4-thiazepan-4-yl)phenyl]-5-[(methylamino)methyl]oxazolidin-2 -one

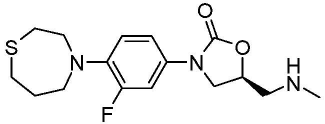

¹H-NMR (400 MHz, CDCl₃) δ: 7.30 (dd, *J* = 15.6 Hz, 2.4 Hz, 1 H), 7.06 (d, *J* = 8.8 Hz, 1 H), 6.82 (t, *J* = 9.6 Hz, 9.6 Hz, 1 H), 4.83 (m, 1 H), 4.02 (t, *J* = 8.8 Hz, 8.4 Hz, 1 H), 3.79 (t, *J* = 8.0 Hz, 7.2 Hz, 1 H), 3.67 (m, 4 H), 2.97 (m, 2 H), 2.87 (m, 2 H), 2.68 (m, 2 H), 2.54 (s, 3 H), 2.05 (m, 2 H). HR-MS (ESI): m/z [M + H]⁺ calcd for C₁₆H₂₃O₂N₃FS: 340.1490; found: 340.1484.

### OTB-108 (R)-[3-[3-Fluoro-4-(1,4-thiazepan-4-yl)phenyl]-2-oxo-5-oxazolidinyl]methyl butyrate

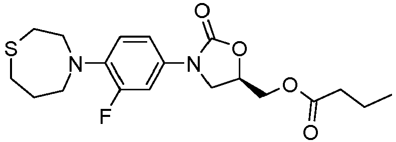

¹H-NMR (400 MHz, CDCl₃) δ: 7.34 (dd, *J* = 15.6 Hz, 2.4 Hz, 1 H), 7.05 (dd, *J* = 9.2 Hz, 2.0 Hz, 1 H), 6.89 (m, 1 H), 4.84 (m, 1 H), 4.37 (dd, *J* = 16.0 Hz, 4.0 Hz, 1 H), 4.31 (dd, *J* = 12.0 Hz, 4.8 Hz, 1 H), 4.06 (t, *J* = 9.2 Hz, 8.8 Hz, 1 H), 3.76 (m, 1 H), 3.68 (m, 4 H), 2.89 (m, 2 H), 2.70 (t, *J* = 6.4 Hz, 6.0 Hz, 2 H), 2.34 (t, *J* = 7.6 Hz, 7.2 Hz, 2 H), 2.08 (m, 2 H), 1.65 (m, 2 H), 0.94 (t, *J* = 7.6 Hz, 7.2 Hz, 3 H). ¹³C-NMR (125 MHz, CDCl₃) δ: 173.3, 154.4, 152.7 (d, *J* = 241.6 Hz), 134.9 (d, *J* = 8.3 Hz), 129.5 (d, *J* = 10.1 Hz), 117.2 (d, *J* = 5.4 Hz), 114.7 (d, *J* = 2.9 Hz), 108.5 (d, *J* = 27.5 Hz), 70.2, 64.1, 56.3, 51.7, 47.5, 36.0, 34.4, 31.9, 30.7, 18.4, 13.7. HR-MS (ESI-TOF): m/z [M + H]⁺ calcd for C₁₉H₂₆O₄N₂FS: 397.1592; found: 397.1613.

### OTB-111 (S)-N-[(3-[3-Fluoro-4-(1,4-thiazepan-4-yl)phenyl]-2-oxo-oxazolidin-5-yl)methyl]fur an-2-carboxamide

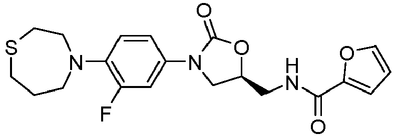

¹H-NMR (400 MHz, CDCl₃) δ: 7.47 (s, 1 H), 7.32 (dd, *J* = 16.0 Hz, 2.0 Hz, 1 H), 7.14 (d, *J* = 3.2 Hz, 1 H), 7.01 (dd, *J* = 8.8 Hz, 1.6 Hz, 1 H), 6.87 (m, 1 H), 6.81 (m, 1 H), 6.52 (s, 1 H), 4.82 (m, 1 H), 4.04 (t, *J* = 9.2 Hz, 8.8 Hz, 1 H), 3.89 (m, 1 H), 3.76 (m, 2 H), 3.67 (m, 4 H), 2.88 (m, 2 H), 2.69 (t, *J* = 6.4 Hz, 6.0 Hz, 2 H), 2.07 (m, 2 H). ¹³C-NMR (125 MHz, CDCl₃) δ: 159.0, 154.4, 152.6 (d, *J* = 241.9 Hz), 147.1, 144.5, 134.5, 129.3, 117.2, 115.1, 114.7, 112.3, 108.5 (d, *J* = 27.4 Hz), 71.9, 56.3, 51.7, 47.9, 41.6, 34.1, 31.7, 30.5. HR-MS (ESI-TOF): m/z [M + H]⁺ calcd for C₂₀H₂₃O₄N₃FS: 420.1388; found: 420.1400.

### OTB-112 (S)-N-[(3-[3-Fluoro-4-(1,4-thiazepan-4-yl)phenyl]-2-oxo-oxazolidin-5-yl)methyl] thiophene-2-carboxamide

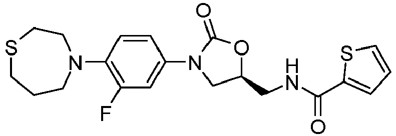

¹H-NMR (400 MHz, CDCl₃) δ: 7.34 (m, 1 H), 7.02 (m, 2 H), 6.23 (m, 1 H), 4.75 (m, 1 H), 4.00 (t, *J* = 8.8 Hz, 8.8 Hz, 1 H), 3.73 (m, 2 H), 3.64 (m, 1 H), 3.36 (t, *J* = 6.4 Hz, 6.0 Hz, 4 H), 2.73 (m, 4 H), 2.04 (s, 3 H), 1.97 (m, 4 H). ¹³C-NMR (125 MHz, CDCl₃) δ: 171.4, 155.2 (d, *J* = 243.5 Hz), 154.4, 134.5, 130.2, 119.9, 114.3, 108.3 (d, *J* = 26.8 Hz), 71.9 , 48.1, 47.8, 42.0, 31.9, 29.7, 23.1. HR-MS (ESI-TOF): m/z [M + H]⁺ calcd for C₁₈H₂₅O₃N₃FS: 382.1595; found: 382.1620.

### OTB-115 (S)-N-[[3-(3-Fluoro-4-(1,4-thiazepan-4-yl)phenyl)-2-oxo-oxazolidin-5-yl]methyl]piv alamide

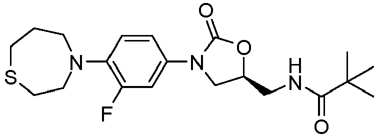

¹H-NMR (400 MHz, CDCl₃) δ: 7.34 (dd, *J* = 15.6 Hz, 2.0 Hz, 1 H), 7.01 (dd, *J* = 8.8 Hz, 2.4 Hz, 1 H), 6.89 (m, 1 H), 6.12 (m, 1 H), 4.74 (m, 1 H), 3.99 (t, *J =* 9.2 Hz, 8.8 Hz, 1 H), 3.74 (m, 1 H), 3.67 (m, 6 H), 2.89 (t, *J* = 5.6 Hz, 5.2 Hz, 2 H), 2.70 (t, *J =* 6.4 Hz, 6.4 Hz, 2 H), 2.08 (m, 2 H), 1.17 (s, 9H). ¹³C-NMR (125 MHz, CDCl₃) δ: 179.7, 154.6, 152.7 (d, *J* = 241.6 Hz), 134.8, 129.5, 117.2 (d, *J* = 5.4 Hz), 114.7 (d, *J* = 2.9 Hz), 108.5 (d, *J* = 27.6 Hz), 72.1, 56.3, 51.7, 48.0, 42.4, 39.0, 34.4, 31.9, 30.7, 27.7. HR-MS (ESI-TOF): m/z [M + H]⁺ calcd for C₂₀H₂₉O₃N₃FS: 410.1908; found: 410.1942.

### OBD-005 (S)-N-((3-(3-Fluoro-4-(1,4-thiazepan-4-yl)phenyl)-2-oxooxazolidin-5-yl)methyl)buty ramide

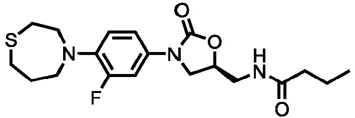

¹H-NMR (300 MHz, CDCl₃) δ: 7.42-7.23 (m, 2 H), 7.01 (dd, *J* = 8.9, 2.3 Hz, 1 H), 6.04 (s, 1 H), 4.75 (ddd, *J* = 9.0, 7.9, 4.6 Hz, 1 H), 4.00 (t, *J* = 9.0 Hz, 1 H), 3.79-3.05 (m, 7 H), 2.91 (dd, *J* = 16.2, 10.1 Hz, 2 H), 2.70 (t, *J* = 6.3 Hz, 2 H), 2.28-2.13 (m, 2 H), 2.13-1.97 (m, 2 H), 1.82-1.25 (m, 3 H), 0.92 (t, *J* = 7.4 Hz, 3 H), 0.01 (s, 1 H).

LC-MS (ESI): *m*/*z* = 395.9 [M+H]⁺.

### OTB-116 (R)-N-[[3-(3-Fluoro-4-(1,4-thiazepan-4-yl)phenyl)-2-oxo-oxazolidin-5-yl]methyl]but ane-1-sulfonamide

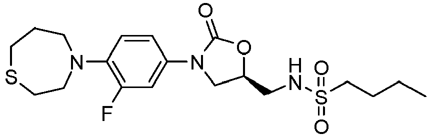

¹H-NMR (400 MHz, CDCl₃) δ: 7.33 (d, *J* = 15.6 Hz, 1 H), 7.05 (d, *J* = 8.8 Hz, 1 H), 6.88 (m, 1 H), 4.92 (t, *J* = 6.8 Hz, 6.4 Hz, 1 H), 4.78 (m, 1 H ), 4.02 *(t, J* = 9.2 Hz, 8.8 Hz, 1 H), 3.90 (m, 1 H), 3.69 (m, 4 H), 3.54 (m, 1 H), 3.43 (m, 1 H), 3.07 (m, 2 H), 2.94 (m, 2 H), 2.69 (m, 2 H), 2.08 (m, 2 H), 1.79 (m, 2 H), 1.46 (m, 2 H), 0.95 (t, *J* = 7.2 Hz, 7.2 Hz, 3 H). ¹³C-NMR (125 MHz, CDCl₃) δ: 154.2, 152.5 (d, *J* = 241.8 Hz), 134.8 (d, *J* = 8.3 Hz), 129.2 (d, *J* = 10.5 Hz), 117.2, 115.0, 108.6 (d, *J* = 27.5 Hz), 71.5, 56.3, 53.2, 51.6, 47.5, 45.5, 34.1, 31.7, 30.5, 25.6, 21.5, 13.5. HR-MS (ESI-TOF): m/z [M + H]⁺ calcd for C₁₉H₂₉O₄N₃FS₂: 446.1578; found: 446.1623.

### OTB-119 (R)-5-[(1H-1,2,4-Triazol-1-yl)methyl]-3-[3-fluoro-4-(1,4-thiazepan-4-yl)phenyl]oxa zolidin-2-one

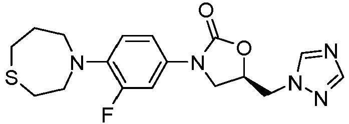

¹H-NMR (400 MHz, CDCl₃) δ: 8.24 (s, 1 H), 7.96 (s, 1 H), 7.22 (m, 1 H), 6.97 (m, 1 H), 6.89 (m, 1 H), 5.02 (m, 1 H), 4.54 (d, *J* = 4.8 Hz, 2 H), 4.10 (t, *J* = 9.2 Hz, 9.2 Hz, 1 H), 3.94 (m, 1 H), 3.68 (m, 4 H), 2.89 (m, 2 H), 2.70 (m, 2 H), 2.08 (m, 2 H). HR-MS (ESI-TOF): *m*/*z* [M + H]⁺ calcd for C₁₇H₂₁O₂N₅FS: 378.1395; found: 378.1421.

### OTB-412 Methyl (S)-((3-(3-fluoro-4-(1,4-thiazepan-4-yl)phenyl)-2-oxo-oxazolidin-5-yl) methyl) carbamate

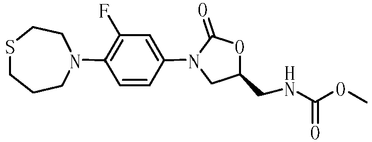

¹H-NMR (400 MHz, CDCl₃) *δ*: 7.29-7.33 (m, 1 H), 7.03-7.05 (m, 1 H), 6.82 (t, *J* = 8.8 Hz, 1 H), 5.10 (m, 1 H), 4.73 (m, 1 H), 3.99 (t, *J* = 9.9 Hz, 1 H), 3.69-3.74 (m, 1 H), 3.67 (s, 3 H), 3.66-3.67 (m, 4 H), 3.61 (m, 1 H), 3.50-3.55 (m, 1 H), 2.87 (m, 1 H), 2.68 (m, 2 H), 2.04-2.07 (m, 2 H). ¹³C-NMR (150 MHz, CDCl₃) *δ*: 157.5, 154.3, 153.3, 151.7, 134.8, 134.7, 129.3, 129.2, 117.0, 117.0, 114.7, 114.7, 108.5, 108.4, 71.7, 56.2, 56.2, 51.5, 47.7, 43.7, 34.3, 31.8, 30.6. HR-MS (ESI-TOF): m/z [M + H]⁺ calcd for C₁₇H₂₃O₄N₃FS: 384.1388; found: 384.1371.

### OTB-413 (S)-N-((3-(3-Fluoro-4-(1,4-thiazepan-4-yl)phenyl)-2-oxo-oxazolidin-5-yl)methyl)cyc lopropanecarboxamide

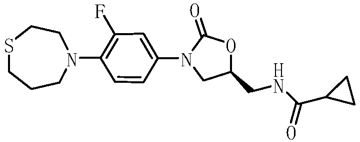

¹H-NMR (400 MHz, CDCl₃) *δ*: 7.29-7.33 (m, 1 H), 7.03-7.00 (m, 1 H), 6.81 (t, *J* = 9.6 Hz, 1 H), 6.09 (m, 1 H), 4.74 (m, 1 H), 3.98 (t, *J* = 8.8 Hz, 1 H), 3.73-3.74 (m, 1 H), 3.67-3.71 (m, 4 H), 3.62-3.66 (m, 1 H), 2.87 (t, *J* = 4.8 Hz, 2 H), 2.68 (t, *J* = 10.0 Hz, 2 H), 2.04-2.07 (m, 2 H), 1.36-1.43 (m, 1 H), 1.05-1.07 (m, 1 H), 0.93-0.97 (m, 2 H), 0.77-0.78 (m, 1 H). ¹³C-NMR (150 MHz, CDCl₃) *δ*: 174.5, 154.5, 153.3, 151.7, 134.8, 134.7, 129.2, 129.2, 117.0, 114.7, 108.6, 108.4, 72.0, 56.2, 51.5, 47.8, 42.1, 34.3, 31.8, 30.6, 14.7, 7.7. HR-MS (ESI-TOF): m/z [M + H]⁺ calcd for C₁₉H₂₅O₃N₃FS: 394.1595; found: 394.1580.

### OTB-414 (S)-N-((3-(3-Fluoro-4-(1,4-thiazepan-4-yl)phenyl)-2-oxooxazolidin-5-yl)methyl)cycl obutanecarboxamide

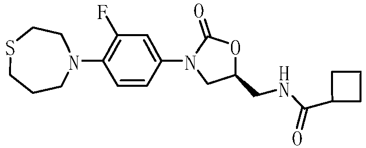

¹H-NMR (400 MHz, CDCl₃) *δ*: 7.29-7.34 (m, 1 H), 7.03-7.00 (m, 1 H), 6.82 (t, *J* = 9.6 Hz, 1 H), 5.84 (m, 1 H), 4.75 (m, 1 H), 3.99 (t, *J* = 8.8 Hz, 1 H), 3.72-3.76 (m, 1 H), 3.67-3.71 (m, 4H), 3.63-3.66 (m, 1 H), 3.14-3.23 (m, 2 H), 2.68 (m, 1 H), 1.89-2.35 (m, 10 H). ¹³C-NMR (150 MHz, CDCl₃) *δ*: 180.2, 176.0, 154.5, 134.8, 134.8, 129.2, 129.1, 117.0, 117.0, 114.7, 114.7, 108.5, 108.4, 72.0, 56.2, 56.2, 51.5, 51.5, 47.9, 42.0, 39.7, 37.8, 34.3, 31.8, 30.6, 25.2, 18.4. HR-MS (ESI-TOF): m/z [M + H]⁺ calcd for C₂₀H₂₇O₃N₃FS: 408.1752; found: 408.1736.

### OTB-407 (S)-N-((3-(3,5-Difluoro-4-(1,4-thiazepan-4-yl)phenyl)-2-oxo-oxazolidin-5-yl)methyl )acetamide

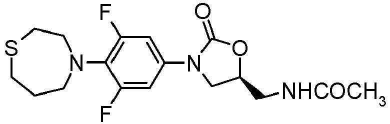

¹H-NMR (400 MHz, CDCl₃) *δ*: 7.07 (d, *J* = 10.4 Hz, 2 H), 6.08 (m, 1 H), 4.76-4.77 (m, 1 H), 3.98 (t, *J* = 9.2 Hz, 1 H), 3.59-3.70 (m, 3 H), 3.45-3.48 (m, 4 H), 2.89 (t, *J* = 6.0 Hz, 2 H), 2.77-2.80 (m, 2 H), 2.02 (s, 3 H). ¹³C-NMR (150 MHz, CDCl₃) *δ*: 171.1, 159.8, 159.7, 158.1, 158.1, 154.0, 133.3, 126.0, 102.5, 102.3, 71.9, 58.8, 54.1, 47.5, 41.9, 36.1, 31.8, 31.6, 23.1. HR-MS (ESI-TOF): m/z [M + H]⁺ calcd for C₁₇H₂₂O₃N₃F₂S: 386.1344; found: 386.1330.

### OTB-410 Methyl (S)-((3-(3,5-difluoro-4-(1,4-thiazepan-4-yl)phenyl)-2-oxo-oxazolidin-5-yl) methyl)carbamate

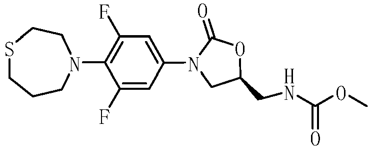

¹H-NMR (500 MHz, CDCl₃) *δ*: 7.09 (d, *J* = 10.0 Hz, 2 H), 5.09 (m, 1 H), 4.76 (m, 1 H), 3.99 (t, *J* = 9.0 Hz, 1 H), 3.74-3.76 (m, 1 H), 3.73 (s, 3 H), 3.61 (m, 1 H), 3.52-3.55 (m, 1 H), 3.46-3.47 (m, 4 H), 2.90 (t, *J* = 6.0 Hz, 1 H), 2.78-2.80 (m, 2 H), 1.94-1.98 (m, 2 H). ¹³C-NMR (125 MHz, CDCl₃) *δ*: 157.5, 154.3, 153.3, 151.7, 134.8, 134.7, 129.3, 129.2, 117.0, 117.0, 114.7, 114.7, 108.4, 108.4, 71.3, 56.2, 56.2, 51.5, 47.8, 43.7, 34.3, 31.8, 30.6. HR-MS (ESI-TOF): m/z [M + H]⁺ calcd for C₁₇H₂₂O₄N₃F₂S: 402.1297; found: 402.1287.

### OTB-408 (S)-5-((Cyclopropylamino)methyl)-3-(3,5-difluoro-4-(1,4-thiazepan-4-yl)phenyl)oxa zolidin-2-one

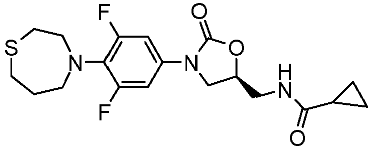

¹H-NMR (400 MHz, CDCl₃) *δ*: 7.07 (d, *J* = 10.8 Hz, 2 H), 6.06 (m, 1 H), 4.76 (m, 1 H), 3.96 (t, *J* = 8.8 Hz, 1 H), 3.66-3.75 (m, 3 H), 3.43-3.47 (m, 3 H), 2.89 (t, *J* = 9.2 Hz, 2 H), 2.78-2.80 (m, 3 H), 1.94-1.97 (m, 2 H), 1.37-1.39 (m, 1 H), 0.97 (m, 1 H), 0.93 (m, 1 H), 0.77-0.79 (m, 2 H). ¹³C-NMR (150 MHz, CDCl₃) *δ*: 174.7, 159.8, 159.7, 158.2, 158.1, 154.0, 133.4, 126.0, 102.6, 102.4, 72.0, 58.8, 54.1, 47.5, 42.0, 36.1, 31.8, 31.6, 14.7, 7.8, 7.7. HR-MS (ESI-TOF): m/z [M + H]⁺ calcd for C₁₉H₂₄O₃N₃F₂S: 412.1501; found: 412.1485.

### OTB-409 (S)-5-((Cyclobutylamino)methyl)-3-(3,5-difluoro-4-(1,4-thiazepan-4-yl)phenyl)oxaz olidin-2-one

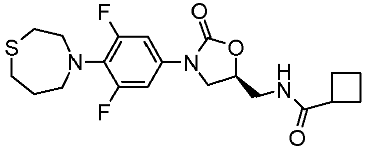

¹H-NMR (400 MHz, CDCl₃) *δ*: 7.07 (d, *J* = 10.8 Hz, 2 H), 5.81 (m, 1 H), 4.75 (m, 1 H), 3.98 (t, *J* = 8.8 Hz, 1 H), 3.72-3.76 (m, 1 H), 3.64-3.66 (m, 2 H), 3.45-3.46 (m, 3 H), 3.01 (t, *J* = 8.8 Hz, 1 H), 2.90 (t, *J* = 6.4 Hz, 2 H), 2.77-2.80 (m, 2 H), 2.13-2.26 (m, 4 H), 1.92-1.96 (m, 3 H). ¹³C-NMR (150 MHz, CDCl₃) *δ*: 176.0, 159.8, 159.8, 158.2, 158.1, 154.0, 133.3, 126.0, 102.5, 102.3, 72.0, 58.8, 54.1, 47.5, 41.9, 39.7, 36.1, 31.8, 31.6, 25.4, 25.3, 18.1. HR-MS (ESI-TOF): m/z [M + H]⁺ calcd for C₂₀H₂₆O₃N₃F₂S: 426.1658; found: 426.1643.

### OTB-411 (R)-5-((1H-1,2,3-triazol-1-yl)methyl)-3-(3,5-difluoro-4-(1,4-thiazepan-4-yl)phenyl)o xazolidin-2-one

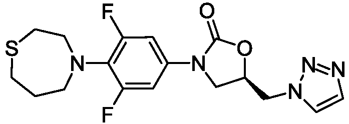

¹H-NMR (400 MHz, CDCl₃) *δ*: 7.76 (d, *J* = 11.2 Hz, 2 H), 6.95 (d, *J* = 10.4 Hz, 2 H), 5.04-5.07 (m, 1 H), 4.78 (d, *J* = 4.0 Hz, 2 H), 4.10 (t, *J* = 9.2 Hz, 1 H), 3.86-3.90 (m, 1 H), 3.44-3.46 (m, 4 H), 2.88 (t, *J* = 6.4 Hz, 2 H), 2.76-2.79 (m, 2 H), 1.91-1.97 (m, 2H). ¹³C-NMR (125 MHz, CDCl₃) *δ*: 159.8, 159.8, 157.9, 157.8, 153.0, 134.6, 125.1, 102.8, 102.5, 70.3, 58.7, 54.0, 51.9, 47.1, 36.1, 31.8, 31.6. HR-MS (ESI-TOF): m/z [M + H]⁺ calcd for C₁₇H₂₀O₂N₅F₂S: 396.1300; found: 396.1296.

### OTB-126 [(5R)-3-[3-Fluoro-4-(1-oxido-1,4-thiazepan-4-yl)phenyl)-2-oxo-oxazolidin-5-yl]met hyl butyrate

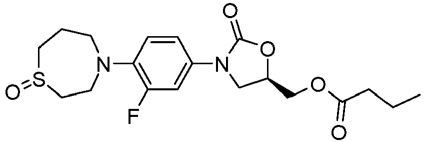

¹H-NMR (400 MHz, CDCl₃) δ: 7.45 (dd, *J* = 14.8 Hz, 1.6 Hz, 1 H), 7.07 (dd, *J* = 8.8 Hz, 2.4 Hz, 1 H), 6.96 (t, *J* = 9.2 Hz, 9.2 Hz, 1 H), 4.85 (m, 1 H), 4.37 (dd, *J* = 12.0 Hz, 4.0 Hz, 1 H), 4.30 (dd, *J* = 12.4 Hz, 4.8 Hz, 1 H), 4.07 (m, 1 H), 3.78 (m, 2 H), 3.40 (m, 2 H), 3.19 (m, 4 H), 2.98 (m, 1 H), 2.72 (m, 1 H), 2.33 (t, *J* = 7.6 Hz, 7.2 Hz, 2 H), 2.04 (m, 1 H), 1.63 (m, 2 H), 0.92 (t, *J* = 7.6 Hz, 7.2 Hz, 3 H). ¹³C-NMR (125 MHz, CDCl₃) δ: 173.2, 154.1, 154.0 (d, *J* = 241.8 Hz), 136.6, 131.4, 118.1 (d, *J* = 4.3 Hz), 114.0, (d, *J* = 2.9 Hz), 107.8 (d, *J* = 26.9 Hz), 70.1, 63.9, 52.8, 49.6, 47.2, 46.4, 43.7, 35.8, 18.3, 16.2, 13.6. HR-MS (ESI-TOF): *m*/*z* [M + H]⁺ calcd for C₁₉H₂₆O₅N₂FS: 413.1541; found: 413.1573.

### OTB-127 N-[[(5S)-3-(3-Fluoro-4-(1-oxido-1,4-thiazepan-4-yl)phenyl)-2-oxo-oxazolidin-5-yl] methyl]furan-2-carboxamide

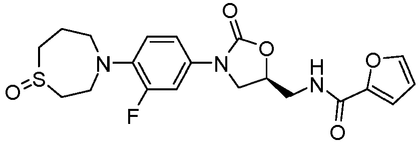

¹H-NMR (400 MHz, CDCl₃) δ: 7.47 (s, 1 H), 7.42 (m, 1 H), 7.14 (d, *J* = 3.2 Hz, 1 H), 7.04 (m, 1 H), 6.87 (m, 2 H), 6.51 (m, 1 H), 4.85 (m, 1 H), 4.05 (m, 1 H), 3.81 (m, 4 H), 3.38 (m, 2 H), 3.15 (m, 2 H), 3.04 (m, 3 H), 2.70 (m, 1 H), 2.03 (m, 1 H). HR-MS (ESI-TOF): *m*/*z* [M + H]⁺ calcd for C₂₀H₂₃O₅N₃FS: 436.1337; found: 436.1371.

### OTB-137 (5R)-5-((1H-1,2,3-Triazol-1-yl)methyl)-3-(3-fluoro-4-(1-oxido-1,4-thiazepan-4-yl)p henyl)oxazolidin-2-one

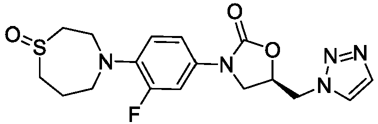

¹H-NMR (400 MHz, CDCl₃) δ: 7.79 (s, 1 H), 7.75 (s, 1 H), 7.30-7.25 (m, 1 H), 6.91-6.89 (m, 2 H), 5.07-5.02 (m, 1 H), 4.78 (d, *J* = 4.0 Hz, 2 H), 4.11 (t, *J* = 9.2 Hz, 1 H), 3.93-3.88 (m, 1 H), 3.82-3.76 (m, 1 H), 3.43-3.36 (m, 2 H), 3.24-2.91 (m, 4 H), 2.75-2.69 (m, 1 H), 2.04-2.02 (m, 2 H). HR-MS (ESI): m/z [M + H]⁺ calcd for C₁₇H₂₁O₃N₅FS: 394.1344; found: 394.1328.

### OTB-138 (5R)-5-((2H-1,2,3-Triazol-2-yl)methyl)-3-(3-fluoro-4-(1-oxido-1,4-thiazepan-4-yl)p henyl)oxazolidin-2-one

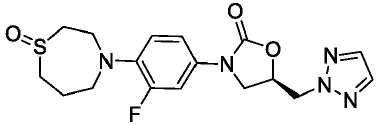

¹H-NMR (400 MHz, CDCl₃) δ: 7.65 (s, 2 H), 7.39 (d, *J* = 14.4 Hz, 1 H), 7.04-6.98 (m, 2 H), 5.15-5.09 (m, 1 H), 4.86 (dd, *J* = 14.0, 4.4 Hz, 1 H), 4.75 (dd, *J* = 14.0, 6.8 Hz, 1 H), 4.06 (dt, *J* = 9.2, 2.4 Hz, 1 H), 4.00-3.96 (m, 1 H), 3.86-3.82 (m, 1 H), 3.46-3.38 (m, 2 H), 3.29-3.08 (m, 3 H), 3.02-2.96 (m, 1 H), 2.76-2.71 (m, 1 H), 2.04-2.02 (m, 2 H). HR-MS (ESI): m/z [M + H]⁺ calcd for C₁₇H₂₁O₃N₅FS: 394.1344; found: 394.1338.

### OTB-140 (5R)-5-((1H-1,2,4-Triazol-1-yl)methyl)-3-(3-fluoro-4-(1-oxido-1,4-thiazepan-4-yl)p henyl)oxazolidin-2-one

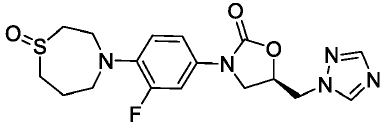

¹H-NMR (400 MHz, CDCl₃) δ: 8.24 (s, 1 H), 7.94 (s, 1 H), 7.35 (d, *J* = 14.4 Hz, 1 H), 7.0-6.96 (m, 2 H), 5.02-4.99 (m, 1 H), 4.55 (d, *J* = 4.4 Hz, 2 H), 4.10 (dt, *J* = 8.8, 2.0 Hz, 1 H), 3.99-3.95 (m, 1 H), 3.86-3.82 (m, 1 H), 3.45-3.38 (m, 2 H), 3.29-3.09 (m, 3 H), 3.00-2.94 (m, 1 H), 2.77-2.71 (m, 1 H), 2.07-2.03 (m, 2 H). HR-MS (ESI): m/z [M + H]⁺ calcd for C₁₇H₂₁O₃N₅FS: 394.1344; found: 394.1339.

### OBD-006 N-(((5S)-3-(3-Fluoro-4-(1-oxido-1,4-thiazepan-4-yl)phenyl)-2-oxooxazolidin-5-yl)m ethyl)butyramide

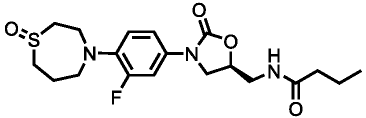

¹H-NMR (300 MHz, CDCl₃) δ: 7.52 (d, *J =* 15.0 Hz, 1 H), 7.16 (s, 1 H), 7.03 (d, *J* = 8.7 Hz, 1 H), 5.99 (s, 1 H), 4.78 (s, 1 H), 4.02 (t, *J* = 8.8 Hz, 2 H), 3.88-3.56 (m, 3 H), 3.55-2.92 (m, 7H), 2.77 (s, 1 H), 2.20 (t, *J* = 7.1 Hz, 3 H), 1.64 (dd, *J* = 14.9, 7.4 Hz, 2 H), 0.91 (t, *J =* 7.4 Hz, 3 H).

LC-MS (ESI): *m*/*z* = 411.8 [M+H]⁺.

### OBD-007 (S)-N-((3-(4-(1,1-Dioxido-1,4-thiazepan-4-yl)-3-fluorophenyl)-2-oxooxazolidin-5-yl )methyl)butyramide

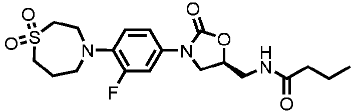

¹H-NMR (300 MHz, CDCl₃) δ: 7.51 (d, *J* = 14.7 Hz, 1 H), 7.10 (d, *J* = 9.9 Hz, 2 H), 5.92 (s, 1 H), 4.78 (s, 1 H), 4.03 (t, *J* = 9.0 Hz, 1 H), 3.87-3.39 (m, 7 H), 3.27 (d, *J* = 5.7 Hz, 2 H), 2.39 (d, *J* = 6.2 Hz, 2 H), 2.20 (t, *J* = 7.2 Hz, 2 H), 1.64 (dd, *J* = 14.8, 7.4 Hz, 2 H), 0.92 (t, *J* = 7.3 Hz, 3 H).

LC-MS (ESI): *m*/*z* = 427.8 [M+H]⁺.

### OTB-110 (R)-[3-[3-Fluoro-4-(1,5-thiazocan-5-yl)phenyl]-2-oxo-5-oxazolidinyl]methyl butyrate

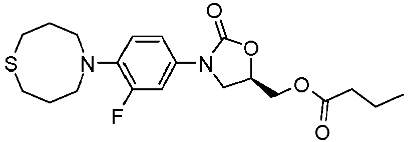

¹H-NMR (400 MHz, CDCl₃) δ: 7.36 (dd, *J* = 14.8 Hz, 4.0 Hz, 1 H), 7.09 (dd, *J* = 8.8 Hz, 2.4 Hz, 1 H), 7.04 (t, *J* = 10.4 Hz, 9.2 Hz, 1 H), 4.85 (m, 1 H), 4.37 (m, 1 H), 4.31 (m, 1 H), 4.08 (m, 1 H), 3.78 (m, 1 H), 3.37 (m, 4 H), 2.75 (m, 4 H), 2.34 (t, *J* = 7.6 Hz, 7.2 Hz, 2 H), 1.97 (m, 4 H), 1.64 (m, 2 H), 0.93 (t, 7.6 Hz, *J* = 7.2 Hz, 3 H). ¹³C-NMR (125 MHz, CDCl₃) δ: 173.3, 155.4 (d, *J* = 243.5 Hz), 154.3, 134.5 (d, *J* = 8.3 Hz), 131.1 (d, *J* = 10.1 Hz), 128.2, 127.1, 120.0 (d, *J* = 4.9 Hz), 114.3 (d, *J* = 3.0 Hz), 108.4 (d, *J* = 26.8 Hz), 70.2, 64.0, 48.1, 47.4, 36.0, 32.1, 29.8, 18.4, 13.7. HR-MS (ESI-TOF): m/z [M + H]⁺ calcd for C₂₀H₂₈O₄N₂FS: 411.1748; found: 411.1786.

### OTB-113 (R)-5-[(2H-1,2,3-triazol-2-yl)methyl]-3-[3-fluoro-4-(1,5-thiazocan-5-yl)phenyl]oxaz olidin-2-one

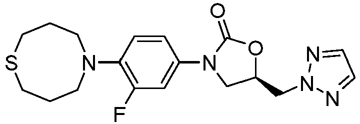

¹H-NMR (400 MHz, CDCl₃) δ: 7.65 (s, 2 H), 7.30 (d, *J* = 14.8 Hz, 1 H), 7.03 (m, 2 H), 5.10 (m, 1 H), 4.85 (dd, *J* = 14.0 Hz, 4.8 Hz, 1 H), 4.74 (dd, *J* = 14.0 Hz, 7.2 Hz, 1 H), 4.06 (t, *J* = 9.2 Hz, 8.8 Hz, 1 H), 3.97 (m, 1 H), 3.37 (t, *J* = 6.0 Hz, 6.0 Hz, 4 H), 2.73 (m, 4 H), 1.97 (m, 4 H). ¹³C-NMR (125 MHz, CDCl₃) δ: 155.2 (d, *J* = 243.5 Hz), 135.2, 134.4 (d, *J* = 8.1 Hz), 130.8 (d, *J* = 10.3 Hz), 119.8 (d, *J* = 4.9 Hz), 114.5 (d, *J* = 3.1 Hz), 108.4 (d, *J* = 26.6 Hz), 70.0, 56.3, 48.3, 47.9, 31.9, 29.6. HR-MS (ESI-TOF): m/z [M + H]⁺ calcd for C₁₈H₂₃O₂N₅FS: 392.1551; found: 392.1590.

### OTB-114 (R)-[3-[3-Fluoro-4-(1,5-thiazocan-5-yl)phenyl]-2-oxo-5-oxazolidinyl]methyl acetamide

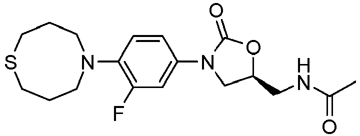

¹H-NMR (400 MHz, CDCl₃) δ: 7.34 (m, 1 H), 7.02 (m, 2 H), 6.23 (m, 1 H), 4.75 (m, 1 H), 4.00 (t, *J* = 8.8 Hz, 8.8 Hz, 1 H), 3.73 (m, 2 H), 3.64 (m, 1 H), 3.36 (t, *J* = 6.4 Hz, 6.0 Hz, 4 H), 2.73 (m, 4 H), 2.04 (s, 3 H), 1.97 (m, 4 H). ¹³C-NMR (125 MHz, CDCl₃) δ: 171.4, 155.2 (d, *J* = 243.5 Hz), 154.4, 134.5, 130.2, 119.9, 114.3, 108.3 (d, *J* = 26.8 Hz), 71.9 , 48.1, 47.8, 42.0, 31.9, 29.7, 23.1. HR-MS (ESI-TOF): m/z [M + H]⁺ calcd for C₁₈H₂₅O₃N₃FS: 382.1595; found: 382.1620.

### OTB-117 (S)-N-[[3-(3-Fluoro-4-(1,5-thiazocan-5-yl)phenyl)-2-oxo-oxazolidin-5-yl]methyl]fur an-2-carboxamide

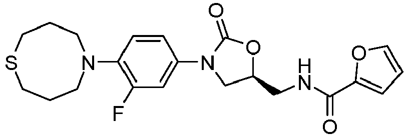

¹H-NMR (400 MHz, CDCl₃) δ: 7.47 (s, 1 H), 7.36 (d, *J* = 14.4 Hz, 1 H), 7.14 (d, *J =* 3.2 Hz, 1 H), 7.01 (m, 2 H), 6.78 (m, 1 H), 6.51 (m, 1 H), 4.84 (m, 1 H), 4.05 (t, *J =* 9.2 Hz, 8.8 Hz, 1 H), 3.88 (m, 1 H), 3.80 (m, 2 H), 3.36 (t, *J* = 6.0 Hz, 6.0 Hz, 4 H), 2.73 (m, 4 H), 1.96 (m, 4 H). ¹³C-NMR (125 MHz, CDCl₃) δ: 159.0, 155.2 (d, *J =* 243.6 Hz), 154.3, 147.1, 144.5, 134.3, 130.9, 119.8, 115.1, 114.3 (d, *J* = 3.0 Hz), 112.3, 108.4 (d, *J =* 26.8 Hz), 71.9, 47.9, 41.5, 31.9, 29.6. HR-MS (ESI-TOF): m/z [M + H]⁺ calcd for C₂₁H₂₅O₄N₃FS: 434.1544; found: 434.1581.

### OTB-118 (S)-N-[[3-(3-Fluoro-4-(1,5-thiazocan-5-yl)phenyl)-2-oxo-oxazolidin-5-yl]methyl]thi ophene-2-carboxamide

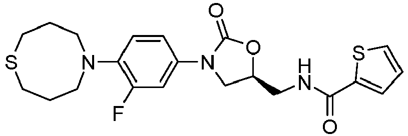

¹H-NMR (400 MHz, CDCl₃) δ: 7.54 (m, 1 H), 7.52 (m, 1 H), 7.34 (m, 1 H), 7.10 (m, 1 H), 7.04 (m, 1 H), 7.00 (m, 1 H), 6.57 (t, *J* = 6.0 Hz, 6.0 Hz, 1 H), 4.86 (m, 1 H), 4.07 *(t, J* = 9.2 Hz, 8.8 Hz, 1 H), 4.07 (m, 1 H), 3.82 (m, 2 H), 3.36 (t, *J* = 6.0 Hz, 6.0 Hz, 4 H), 2.74 (m, 4 H), 1.96 (m, 4 H). ¹³C-NMR (125 MHz, CDCl₃) δ: 162.7, 155.2 (d, *J* = 243.5 Hz), 154.5, 137.9, 130.8, 128.7, 127.8, 119.8, 114.5 (d, *J* = 3.0 Hz), 108.5 (d, *J* = 26.8 Hz), 72.1, 48.0, 42.5, 31.9, 29.6. HR-MS (ESI-TOF): m/z [M + H]⁺ calcd for C₂₁H₂₅O₄N₄FS₂: 450.1316; found: 450.1356.

### OTB-120 (R)-N-[[3-(3-Fluoro-4-(1,5-thiazocan-5-yl)phenyl)-2-oxo-oxazolidin-5-yl]methyl]but ane-1-sulfonamide

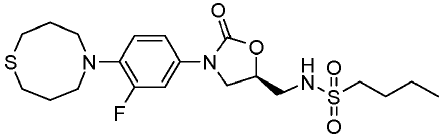

¹H-NMR (400 MHz, CDCl₃) δ: 7.42 (d, *J* = 14.4 Hz, 1 H), 7.08 (m, 2 H), 4.94 (m, 1 H), 4.79 (m, 1 H), 4.04 (t, *J* = 8.8 Hz, 8.8 Hz, 1 H), 3.93 (m, 1 H), 3.55 (m, 1 H), 3.43 (m, 5 H), 3.07 (m, 2 H), 2.76 (m, 4 H), 2.01 (m, 4 H), 1.80 (m, 2 H), 1.45 (m, 2 H), 0.95 (t, *J* = 7.6 Hz, 7.2 Hz, 3 H). ¹³C-NMR (125 MHz, CDCl₃) δ: 155.2 (d, *J* = 243.6 Hz), 154.2, 134.5, 130.7, 119.8 (d, *J* = 4.9 Hz), 114.5 (d, *J* = 3.0 Hz), 108.5 (d, *J* = 26.8 Hz), 71.5, 53.1, 47.9, 47.4, 45.5, 31.9, 29.6, 25.6, 21.5, 13.5. HR-MS (ESI-TOF): m/z [M + H]⁺ calcd for C₂₀H₃₁O₄N₃FS₂: 460.1735; found: 460.1778.

### OTB-121 (S)-N-[[3-(3-Fluoro-4-(1,5-thiazocan-5-yl)phenyl)-2-oxo-oxazolidin-5-yl]methyl]piv alamide

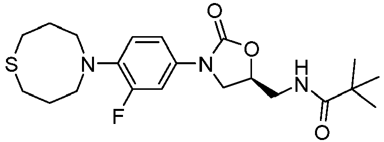

¹H-NMR (400 MHz, CDCl₃) δ: 7.39 (d, *J* = 14.4 Hz, 1 H), 7.04 (m, 2 H), 6.11 (m, 1 H), 4.74 (m, 1 H), 4.00 (t, *J* = 9.2 Hz, 8.8 Hz, 1 H), 3.76 (m, 1 H), 3.67 (m, 2 H), 3.39 (m, 4 H), 2.74 (m, 4 H), 1.98 (m, 4 H), 1.17 (s, 9 H). ¹³C-NMR (125 MHz, CDCl₃) δ: 179.6, 155.2 (d, *J* = 243.6 Hz), 154.4, 134.3 (d, *J* = 8.0 Hz), 130.9 (d, *J* = 5.4 Hz), 128.8, 119.8 (d, *J* = 4.9 Hz), 114.2 (d, *J* = 2.9 Hz), 108.2 (d, *J* = 26.9 Hz), 72.0, 47.9, 47.8, 42.2, 38.9, 31.9, 29.6, 27.5. HR-MS (ESI-TOF): m/z [M + H]⁺ calcd for C₂₁H₃₁O₃N₃FS: 424.2065; found: 424.2096.

### OBD-001 (R)-5-((1H-1,2,3-Triazol-1-yl)methyl)-3-(3-fluoro-4-(1,5-thiazocan-5-yl)phenyl)oxa zolidin-2-one

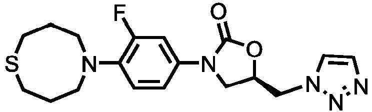

¹H-NMR (300 MHz, CDCl₃) δ: 7.76 (d, *J* = 17.6 Hz, 2 H), 7.41-7.09 (m, 1 H), 7.11-6.73 (m, 2 H), 5.04 (d, *J =* 3.0 Hz, 1 H), 4.78 (d, *J =* 3.4 Hz, 2 H), 4.12 (t, *J=* 9.2 Hz, 1 H), 3.88 (dd, *J* = 9.2, 6.1 Hz, 1 H), 3.36 (t, *J* = 6.0 Hz, 3 H), 2.92-2.59 (m, 4 H), 2.01 (dd, *J =* 27.9, 7.3 Hz, 4 H).

LC-MS (ESI): *m*/*z* = 391.9 [M+H]⁺.

### OBD-003 (S)-N-((3-(3-Fluoro-4-(1,5-thiazocan-5-yl)phenyl)-2-oxooxazolidin-5-yl)methyl)buty ramide

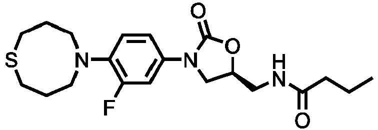

¹H-NMR (300 MHz, CDCl₃) δ: 7.50 (s, 2 H), 7.03 (d, *J* = 6.1 Hz, 1 H), 5.99 (s, 1 H), 4.77 (d, *J* = 5.7 Hz, 1 H), 4.02 (t, *J* = 9.0 Hz, 2 H), 3.70 (ddd, *J* = 20.7, 15.2, 7.7 Hz, 4 H), 3.48 (s, 4 H), 2.95-2.68 (m, 4 H), 2.20 (t, *J* = 7.2 Hz, 3 H), 2.06 (d, *J* = 6.1 Hz, 4H), 1.64 (dd, *J* = 14.8, 7.4 Hz, 4 H), 0.91 (t, *J* = 7.4 Hz, 4 H).

LC-MS (ESI): *m*/*z* = 409.9 [M+H]⁺.

### OBD-008 (R)-5-((1H-1,2,4-Triazol-1-yl)methyl)-3-(3-fluoro-4-(1,5-thiazocan-5-yl)phenyl)oxa zolidin-2-one

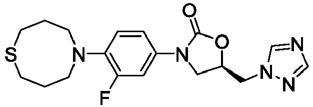

¹H-NMR (300 MHz, CDCl₃) δ: 8.24 (s, 1 H), 7.97 (s, 1 H), 7.00 (s, 2 H), 5.12-4.91 (m, 1 H), 4.56 (d, *J* = 4.7 Hz, 2 H), 4.24-3.83 (m, 2 H), 3.38 (t, *J* = 6.0 Hz, 4 H), 2.95-2.59 (m, 4 H), 1.98 (s, 5 H).

LC-MS (ESI): *m*/*z* = 391.9 [M+H]⁺.

### OTB-124 (S)-N-[[3-(3-Fluoro-4-(1-oxido-1,5-thiazocan-5-yl)phenyl)-2-oxo-oxazolidin-5-yl]m ethyl]acetamide

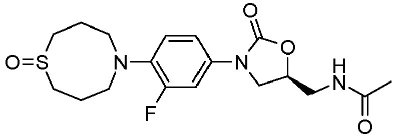

¹H-NMR (400 MHz, CDCl₃) δ: 7.46 (dd, *J* = 2.8 Hz, 14.8 Hz, 1 H), 7.14 (t, *J* = 9.2 Hz, 8.8 Hz, 1 H), 7.07 (m, 1 H), 6.15 (m, 1 H), 4.78 (m, 1 H), 4.03 (t, *J* = 9.2 Hz, 8.8 Hz, 1 H), 3.74 (m, 3 H), 3.31 (m, 1 H), 3.18 (m, 4 H), 2.98 (m, 2 H), 2.17 (m, 4 H), 2.03 (s, 3 H). ¹³C-NMR (125 MHz, CDCl₃) δ: 171.0, 154.2, 134.0, 128.5, 127.3, 122.6, 113.9, 108.1 (d, *J* = 26.9 Hz), 71.9, 53.1, 51.7, 47.7, 42.0, 29.7, 25.0, 23.2. HR-MS (ESI): *m*/*z* [M + H]⁺ calcd for C₁₈H₂₅O₄N₃FS: 398.1544; found: 398.1540.

### OBD-002 (R)-5-((1H-1,2,3-Triazol-1-yl)methyl)-3-(3-fluoro-4-(1-oxido-1,5-thiazocan-5-yl)phe nyl)oxazolidin-2-one

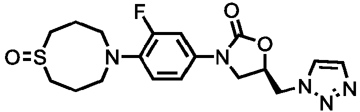

¹H-NMR (300 MHz, CDCl₃) δ: 7.92-7.67 (m, 2 H), 7.32 (d, *J* = 16.8 Hz, 1 H), 7.12 (t, *J* = 9.0 Hz, 1 H), 6.96 (d, *J* = 8.1 Hz, 1 H), 5.07 (s, 1 H), 4.81 (d, *J* = 4.0 Hz, 2 H), 4.15 (t, *J =* 9.0 Hz, 1 H), 4.01-3.83 (m, 1 H), 3.32 (d, *J* = 14.2 Hz, 5 H), 3.11-2.87 (m, 2 H), 2.59 (s, 2 H), 2.19 (s, 4 H).

LC-MS (ESI): *m*/*z* = 407.8 [M+H]⁺.

### OBD-004 (S)-N-((3-(3-Fluoro-4-(1-oxido-1,5-thiazocan-5-yl)phenyl)-2-oxooxazolidin-5-yl)me thyl)butyramide

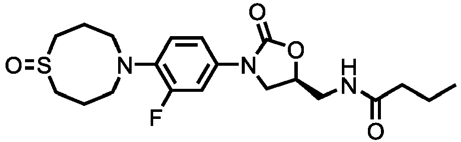

¹H-NMR (300 MHz, CDCl₃) δ: 7.44 (dd, *J* = 14.7, 2.4 Hz, 1 H), 7.19-6.99 (m, 2 H), 6.44 (s, 1 H), 4.84-4.71 (m, 1 H), 4.02 (t, *J =* 8.9 Hz, 1 H), 3.78 (dd, *J =* 9.0, 6.6 Hz, 1 H), 3.66 (t, *J* = 4.6 Hz, 2 H), 3.38-3.09 (m, 6 H), 2.99 (dd, *J* = 12.6, 6.3 Hz, 2 H), 2.20 (dd, *J* = 9.4, 5.3 Hz, 6 H), 1.71-1.56 (m, 2H), 0.91 (dd, *J* = 9.6, 5.1 Hz, 3 H). LC-MS (ESI): *m*/*z* = 425.8 [M+H]⁺.

### OBD-009 (R)-5-((1H-1,2,4-Triazol-1-yl)methyl)-3-(3-fluoro-4-(1-oxido-1,5-thiazocan-5-yl)phe nyl)oxazolidin-2-one

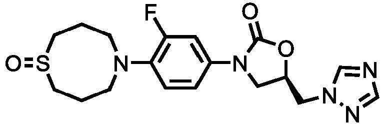

¹H-NMR (300 MHz, CDCl₃) δ: 8.24 (s, 1 H), 7.93 (s, 1 H), 7.41-7.20 (m, 1 H), 7.19-6.92 (m, 2 H), 5.10-4.92 (m, 1 H), 4.56 (d, *J* = 4.7 Hz, 2 H), 4.12 (t, *J* = 9.0 Hz, 1 H), 3.97 (dd, *J* = 9.2, 6.2 Hz, 1 H), 3.28 (dd, *J* = 13.0, 6.9 Hz, 2 H), 3.12 (dd, *J* = 12.5, 5.9 Hz, 4 H), 3.02-2.83 (m, 2 H), 2.22-1.99 (m, 5 H), 1.26 (d, *J* = 9.4 Hz, 4 H). LC-MS (ESI): *m*/*z* = 407.8 [M+H]⁺.

### OTB-227 N-(((5S)-3-(4-(3-Thia-6-azabicyclo[3.1.1]heptan-6-yl)-3-fluorophenyl)-2-oxo-oxazol idin-5-yl)methyl)acetamide

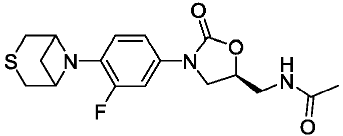

¹H-NMR (400 MHz, CDCl₃) δ: 7.36 (d, *J* = 14.4 Hz, 1 H), 7.05 (d, *J* = 8.4 Hz, 1 H), 6.60 (t, *J* = 9.2 Hz, 1 H), 6.35 (brs, 1 H), 4.76-4.74 (m, 1 H), 4.56-4.54 (m, 2 H), 4.00 (t, *J =* 9.2 Hz, 1 H), 3.76-3.65 (m, 2 H), 3.62-3.57 (m, 1 H), 3.43 (d, *J =* 12.0 Hz, 2 H), 2.93-2.87 (m, 1 H), 2.74 (d, *J* = 12.0 Hz, 2 H), 2.09 (s, 1 H), 2.03 (s, 3 H). ¹³C-NMR (100 MHz, CDCl₃) δ: 171.1, 154.5, 151.8 (d, *J* = 238.5 Hz), 131.9 (d, *J* = 11.8 Hz), 129.2 (d, *J* = 9.5 Hz), 115.2 (d, *J* = 6.2 Hz), 114.7 (d, *J* = 2.8 Hz), 108.0 (d, *J =* 24.9 Hz), 71.9, 60.8, 47.8, 41.9, 29.4, 25.3, 23.2. HRMS (ESI-TOF+): m/z [M + H]⁺ calcd for C₁₇H₂₁FN₃O₃S: 366.1288; found: 366.1277.

### OTB-501 (R)-3-(4-((1R,5S)-3-Thia-8-azabicyclo[3.2.1]octan-8-yl)-3-fluorophenyl)-5-(hydroxy methyl)oxazolidin-2-one

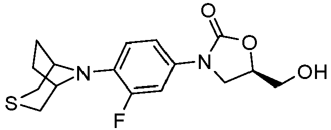

¹H-NMR (400 MHz, CDCl₃) δ: 7.39 (d, *J* = 12.8 Hz, 1 H), 7.16 (d, *J* = 8.8 Hz, 1 H), 6.90 (t, *J* = 9.2 Hz, 1 H), 4.74 (m, 1 H), 4.43 (s, 2 H), 3.99-3.96 (m, 3 H), 3.79-3.75 (m, 1 H), 3.48 (d, *J* = 13.2 Hz, 2 H), 2.21-2.10 (m, 6 H). HRMS (ESI): *m*/*z* [M + H]⁺ calcd for C₁₆H₂₀N₂O₃FS: 339.1179; found: 339.1169.

### OTB-502 N-(((S)-3-(4-((1R,5S)-3-Thia-8-azabicyclo[3.2.1]octan-8-yl)-3-fluorophenyl)-2-oxoo xazolidin-5-yl)methyl)acetamide

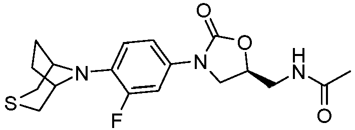

¹H-NMR (400 MHz, CDCl₃) δ: 7.36 (dd, *J* = 15.2, 2.4 Hz, 1 H), 7.06 (dd, *J* = 8.8, 1.8 Hz, 1 H), 6.83 (t, *J* = 9.2 Hz, 1 H), 6.18 (s, 1 H), 4.77-4.75 (m, 1 H), 4.40 (s, 2 H), 4.00 (t, *J* = 8.8 Hz, 1 H), 3.76-3.72 (m, 2 H), 3.68-3.62 (m, 1 H), 3.37 (d, *J* = 12.8 Hz, 2 H), 2.26-2.04 (m, 6 H), 2.02 (s, 3 H). ¹³C-NMR (100 MHz, CDCl₃) δ: 171.1, 154.5, 152.8 (d, *J* = 241.7 Hz), 132.2 (d, *J* = 8.6 Hz), 130.2 (d, *J* = 10.4 Hz), 118.1 (d, *J* = 5.0 Hz), 114.7, 108.4 (d, *J* = 27.2 Hz), 71.9 , 57.4, 47.8, 42.0, 30.2, 28.4, 23.1. HR-MS (ESI-TOF): m/z [M + H]⁺ calcd for C₁₈H₂₃N₃O₃FS: 380.1444; found: 380.1435.

### OTB-504 (R)-5-((1H-1,2,3-Triazol-1-yl)methyl)-3-(4-((1R,5S)-3-thia-8-azabicyclo[3.2.1]octan -8-yl)-3-fluorophenyl)oxazolidin-2-one

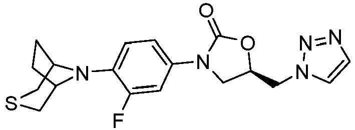

¹H-NMR (400 MHz, CDCl₃) δ: 7.80 (s, 1 H), 7.76 (s, 1 H), 7.24 (m, 1 H), 6.96 (d, *J* = 8.8 Hz, 1 H), 6.83 (t, *J* = 8.8 Hz, 1 H), 5.07-5.04 (m, 1 H), 4.79 (s, 2 H), 4.40 (s, 2 H), 4.11 (t, *J* = 8.0 Hz, 1 H), 3.90-3.87 (m, 1 H), 3.42 (d, *J* = 12.8 Hz, 2 H), 2.20-2.07 (m, 6 H). HR-MS (ESI-TOF): m/z [M + H]⁺ calcd for C₁₈H₂₁N₅O₂FS: 390.1400; found: 390.1385.

### OTB-236 N-(((R)-3-(4-((1R,5S)-3-Thia-8-azabicyclo[3.2.1]octan-8-yl)-3-fluorophenyl)-2-oxo-oxazolidin-5-yl)methyl)methanesulfonamide

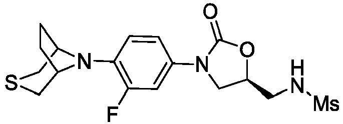

¹H-NMR (400 MHz, CDCl₃) δ: 7.47-7.41 (m, 1 H), 7.13 (d, *J* = 9.6 Hz, 1 H), 7.02 (t, *J* = 9.6 Hz, 1 H), 4.76-4.70 (m, 1 H), 4.34 (s, 2 H), 4.08 (t, *J* = 9.2 Hz, 1 H), 3.76 (t, *J* = 8.8 Hz, 1 H), 3.29-3.26 (m, 2 H), 3.11 (d, *J* = 12.8 Hz, 2 H), 2.93 (s, 3 H), 2.11 (d, *J* = 12.4 Hz, 2 H), 2.02 (s, 4 H). HR-MS (ESI-TOF): m/z [M + H]⁺ calcd for C₁₇H₂₃N₃O₄FS₂: 416.1114; found: 416.1097.

### OTB-237 Methyl (((S)-3-(4-((1R,5S)-3-thia-8-azabicyclo[3.2.1]octan-8-yl)-3-fluorophenyl) -2-Oxo-oxazolidin-5-yl)methyl)carbamate

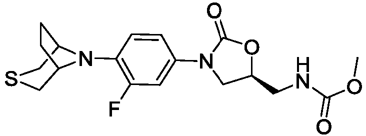

¹H-NMR (400 MHz, CDCl₃) δ: 7.36 (d, *J* = 15.2 Hz, 1 H), 7.07 (d, *J* = 8.8 Hz, 1 H), 6.81 (t, *J =* 9.2 Hz, 1 H), 5.18 (brs, 1 H), 4.74 (brs, 1 H), 4.39 (s, 2 H), 4.01 (t, *J=* 8.8 Hz, 1 H), 3.76 (t, *J =* 7.6 Hz, 1 H), 3.69 (s, 3 H), 3.56-3.51 (m, 1 H), 3.33 (d, *J* = 12.8 Hz, 2 H), 2.18-2.07 (m, 6 H). HR-MS (ESI-TOF): m/z [M + H]⁺ calcd for C₁₈H₂₃N₃O₄FS: 396.1393; found: 396.1388.

### OBD-016 N-(((S)-3-(4-((1R,5S)-3-Thia-8-azabicyclo[3.2.1]octan-8-yl)-3-fluorophenyl)-2-oxoo xazolidin-5-yl)methyl)butyramide

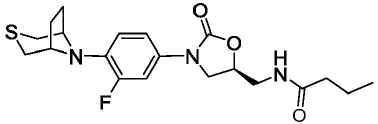

¹H-NMR (300 MHz, DMSO-*d*₆) δ: 8.18 (s, 1 H), 7.42 (d, *J* = 16.0 Hz, 1 H), 7.35-6.88 (m, 2 H), 4.71 (s, 1 H), 4.35 (s, 2 H), 4.07 (t, *J* = 8.7 Hz, 1 H), 3.77-3.57 (m, 1 H), 3.51-3.27 (m, 2 H), 3.12 (d, *J =* 12.4 Hz, 2 H), 2.09 (dd, *J* = 20.9, 12.2 Hz, 8 H), 1.47 (dd, *J =* 14.0, 7.1 Hz, 2 H), 0.80 (dd, *J* = 8.0, 6.7 Hz, 3 H).

LC-MS (ESI): *m*/*z* = 407.9 [M+H]⁺.

### OBD-021 (R)-5-((1H-1,2,4-Triazol-1-yl)methyl)-3-(4-((1R,5S)-3-thia-8-azabicyclo[3.2.1]octan -8-yl)-3-fluorophenyl)oxazolidin-2-one

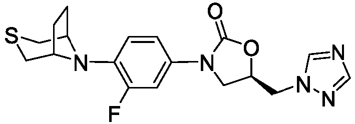

¹H-NMR (300 MHz, DMSO-*d*₆) δ: 8.57 (s, 1 H), 8.01 (s, 1 H), 7.36 (dd, *J =* 15.8, 2.1 Hz, 1 H), 7.18-6.92 (m, 2 H), 5.06 (dd, *J* = 8.9, 4.8 Hz, 1 H), 4.72-4.52 (m, 2 H), 4.36 (s, 2 H), 4.17 (t, *J* = 9.1 Hz, 1 H), 3.84 (dt, *J =* 49.3, 24.7 Hz, 1 H), 3.12 (d, *J=* 12.8 Hz, 2 H), 2.16 (s, 1 H), 2.11 (s, 1 H), 2.04 (s, 4 H).

LC-MS (ESI): *m*/*z* = 389.9 [M+H]⁺.

### OTB-506 N-(((S)-3-(4-((1R,5S)-3-Thia-8-azabicyclo[3.2.1]octan-8-yl)-3-fluorophenyl)-2-oxo-oxazolidin-5-yl)methyl)cyclopropanecarboxamide

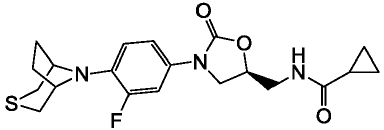

¹H-NMR (400 MHz, CDCl₃) δ: 7.37 (d, *J* = 13.6 Hz, 1 H), 7.07 (d, *J* = 7.6 Hz, 1 H), 6.85 (brs, 1 H), 6.22 (t, *J =* 6.0 Hz, 1 H), 4.79-4.73 (m, 1 H), 4.41 (brs, 2 H), 3.99 (t, *J* = 7.2 Hz, 1 H), 3.77-3.64 (m, 3 H), 3.39 (d, *J* = 12.8 Hz, 2 H), 2.20-2.09 (m, 6 H), 1.43-1.37 (m, 1 H), 0.98-0.90 (m, 2 H), 0.82-0.75 (m, 2 H). HR-MS (ESI-TOF): m/z [M + H]⁺ calcd for C₂₀H₂₅N₃O₃FS: 406.1595; found: 406.1527.

### OTB-507 N-(((S)-3-(4-((1R,5S)-3-Thia-8-aza-bicyclo[3.2.1]octan-8-yl)-3-fluorophenyl)-2-oxo-oxazolidin-5-yl)methyl)cyclobutanecarboxamide

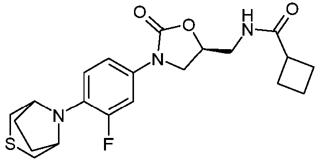

¹H-NMR (400 MHz, CDCl₃) δ: 7.45 (d, *J =* 12.4 Hz, 1 H), 7.17 (d, *J* = 8.8 Hz, 1 H), 7.05 (t, *J =* 8.8 Hz, 1 H) 5.95 (m, 1 H), 4.78 (m, 1 H), 4.51 (brs, 2 H), 4.00 (t, *J =* 9.2 Hz, 1 H), 3.84-3.74 (m, 3 H), 3.66 (m, 2 H), 3.02 (m, 1 H), 2.28-2.15 (m, 10 H), 1.95 (m, 1 H), 1.85 (m, 1 H). ¹³C-NMR (100 MHz, CDCl₃) δ: 175.9, 154.3, 153.0 (d, *J* = 242.9 Hz), 131.2, 127.0, 118.6 (d, *J* = 4.4 Hz), 114.5 (d, *J* = 2.9 Hz), 108.3 (d, *J* = 27.1 Hz), 71.9, 58.2, 47.7, 41.9, 39.6, 30.3, 28.3, 25.4, 25.3, 18.1. HRMS (ESI): *m*/*z* [M + H]⁺ calcd for C₂₁H₂₇N₃O₃SF: 420.1757; found: 420.1736.

### OTB-510 N-(((S)-3-(4-((1R,5S)-3-Thia-8-aza-bicyclo[3.2.1]octan-8-yl)-3,5-difluorophenyl)-2-oxo-oxazolidin-5-yl)methyl)acetamide

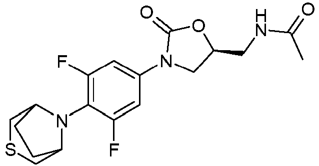

¹H-NMR (400 MHz, CDCl₃) δ: 7.08 (m, 2 H), 6.00 (m, 1 H), 4.76 (m, 1 H), 4.24 (brs, 2 H), 3.97 (t, *J =* 8.8 Hz, 1 H), 3.75-3.60 (m, 3 H), 3.38 (m, 2 H), 2.21 (m, 2 H), 2.14 (s, 4 H), 2.03 (s, 3 H). ¹³C-NMR (100 MHz, CDCl₃) δ: 171.2, 155.3 (dd, *J* = 241.5, 9.5 Hz), 154.1, 130.6 (t, *J* = 13.6 Hz), 122.9 (t, *J* = 12.4 Hz), 102.9 (dd, *J =* 20.7, 11.3 Hz), 71.9, 60.4, 47.4, 41.9, 33.7, 29.2, 23.1. HRMS (ESI): *m*/*z* [M + H]⁺ calcd for C₁₈H₂₂N₃O₃SF₂: 398.1350; found: 398.1329.

### OTB-512 Methyl (((S)-3-(4-((1R,5S)-3-thia-8-azabicyclo[3.2.1]octan-8-yl)-3,5-difluoro phenyl)-2-oxo-oxazolidin-5-yl)methyl)carbamate

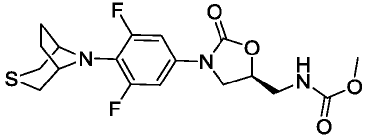

¹H-NMR (400 MHz, CDCl₃) δ: 7.09 (m, 2 H), 5.08 (m, 1 H), 4.76 (m, 1 H), 4.24 (brs, 2 H), 3.98 (t, *J* = 8.8 Hz, 1 H), 3.75-3.50 (m, 6 H), 3.38 (m, 2 H), 2.20 (m, 2 H), 2.14 (s, 4 H). ¹³C-NMR (100 MHz, CDCl₃) δ: 157.5, 155.3 (dd, *J* = 241.6, 9.4 Hz), 153.9, 130.7 (t, *J* = 13.6 Hz), 122.9 (t, *J* = 12.3 Hz), 102.9 (dd, *J* = 20.7, 11.3 Hz), 71.8, 60.4, 52.6, 47.3, 43.6, 33.7, 29.2. HRMS (ESI): *m*/*z* [M + H]⁺ calcd for C₁₈H₂₂N₃O₄SF₂: 414.1294; found: 414.1278.

### OTB-511 (R)-5-((1H-1,2,3-Triazol-1-yl)methyl)-3-(4-((1R,5S)-3-thia-8-azabicyclo[3.2.1]octan -8-yl)-3,5-difluorophenyl)oxazolidin-2-one

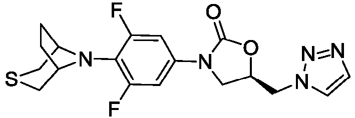

¹H-NMR (400 MHz, CDCl₃) δ: 7.78 (s, 1 H), 7.76 (s, 1 H), 6.94 (d, *J* = 11.6 Hz, 2 H), 5.07-5.04 (m, 1 H), 4.79 (d, *J* = 3.6 Hz, 2 H), 4.20 (brs, 2 H), 4.09 (t, *J* = 8.8 Hz, 1 H), 3.88-3.83 (m, 1 H), 3.32 (d, *J* = 12.8 Hz, 2 H), 2.19-2.11 (m, 6 H). ¹³C-NMR (100 MHz, CDCl₃) δ: 155.3 (d, *J* = 242.1 Hz), 155.2 (d, *J* = 242.1 Hz), 153.1, 134.5, 129.9 (t, *J* = 13.6 Hz), 125.1, 123.3 (t, *J* = 12.3 Hz), 103.3 (dd, *J* = 20.7, 11.2 Hz), 70.3, 60.4, 52.0, 47.2, 33.8, 29.2. HRMS (ESI): *m*/*z* [M + H]⁺ calcd for C₁₈H₂₀N₅O₂SF₂: 408.1300; found: 408.1295.

### OTB-508 N-(((S)-3-(4-((1R,5S)-3-Thia-8-aza-bicyclo[3.2.1]octan-8-yl)-3,5-difluorophenyl)-2-o xo-oxazolidin-5-yl)methyl)cyclopropanecarboxamide

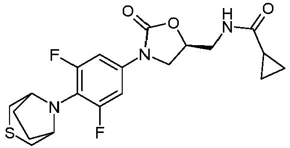

¹H-NMR (400 MHz, CDCl₃) δ: 7.05 (m, 2 H), 6.12 (m, 1 H), 4.76 (m, 1 H), 4.21 (brs, 2 H), 3.95 (t, *J* = 9.2 Hz, 1 H), 3.72-3.65 (m, 3 H), 3.34 (m, 2 H), 2.21-2.12 (m, 6 H), 1.37 (m, 1 H), 0.99-0.75 (m, 4 H). ¹³C-NMR (100 MHz, CDCl₃) δ: 174.8, 155.3 (dd, *J* = 241.7, 9.5 Hz), 154.2, 130.5 (t, *J* = 13.6 Hz), 123.0 (t, *J* = 12.5 Hz), 103.0 (dd, *J* = 20.8, 11.4 Hz), 72.1, 60.4, 47.5, 41.9, 33.8, 29.2, 14.6, 7.8, 7.7. HRMS (ESI): *m*/*z* [M + H]⁺ calcd for C₂₀H₂₄N₃O₃SF₂: 420.1506; found: 424.1484.

### OTB-509 N-(((S)-3-(4-((1R,5S)-3-Thia-8-aza-bicyclo[3.2.1]octan-8-yl)-3,5-difluorophenyl)-2-o xo-oxazolidin-5-yl)methyl)cyclobutanecarboxamide

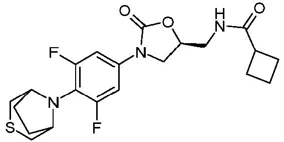

¹H-NMR (400 MHz, CDCl₃) δ: 7.09 (m, 2 H), 5.78 (m, 1 H), 4.75 (m, 1 H), 4.23 (brs, 2 H), 3.97 (t, *J =* 8.8 Hz, 1 H), 3.73 (m, 1 H), 3.65 (m, 2 H), 3.36 (m, 2 H), 2.99 (m, 1 H), 2.27-2.14 (m, 9 H), 1.97 (m, 1 H), 1.85 (m, 2 H). ¹³C-NMR (100 MHz, CDCl₃) δ: 176.0, 155.3 (dd, *J* = 243.4, 9.5 Hz), 154.1, 130.6 (t, *J* = 13.5 Hz), 122.9 (t, *J* = 12.2 Hz), 102.9 (dd, *J =* 20.7, 11.4 Hz), 72.0, 60.4, 47.5, 41.8, 33.7, 29.2, 25.4, 25.3, 18.1. HRMS (ESI): *m*/*z* [M + H]⁺ calcd for C₂₁H₂₆N₃O₃SF₂: 438.1663; found: 438.1642.

### OTB-503 N-(((5S)-3-(3-Fluoro-4-((1R,5S)-3-oxido-3-thia-8-azabicyclo[3.2.1]octan-8-yl)pheny l)-2-oxo-oxazolidin-5-yl)methyl)acetamide

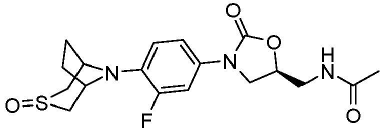

¹H-NMR (400 MHz, CDCl₃) δ: 7.45 (dd, *J* = 16.0, 2.8 Hz, 1 H), 7.12 (dd, *J* = 8.8, 2.0 Hz, 1 H), 6.83 (t, *J* = 9.2 Hz, 1 H), 6.14 (s, 1 H), 4.78-4.77 (m, 1 H), 4.61 (s, 2 H), 4.00 (t, *J =* 8.8 Hz, 1 H), 3.77-3.64 (m, 3 H), 3.45 (d, *J* = 9.6 Hz, 2 H), 2.85 (d, *J* = 12.4 Hz, 2 H), 2.22-2.20 (m, 2 H), 2.03 (s, 3 H), 1.92-1.88 (m, 2 H). HR-MS (ESI-TOF): m/z [M + H]⁺ calcd for C₁₈H₂₃N₃O₄FS: 396.1388; found: 396.1379.

### OTB-505 (5R)-5-((1H-1,2,3-Triazol-1-yl)methyl)-3-(3-fluoro-4-((1R,5S)-3-oxido-3-thia-8-aza bicyclo[3.2.1]octan-8-yl)phenyl)oxazolidin-2-one

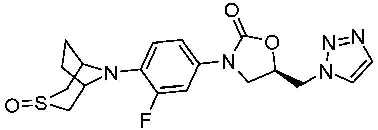

¹H-NMR (400 MHz, CDCl₃) δ: 7.79 (s, 1 H), 7.75 (s, 1 H), 7.30 (dd, *J* = 15.2, 2.0 Hz, 1 H), 6.98 (d, *J =* 8.4 Hz, 1 H), 6.79 (t, *J* = 9.2 Hz, 1 H), 5.07-5.05 (m, 1 H), 4.79 (s, 2 H), 4.58 (s, 2 H), 4.12 (t, *J =* 9.2 Hz, 1 H), 3.91-3.89 (m, 1 H), 3.43 (d, *J* = 11.6 Hz, 2 H), 2.88-2.81 (m, 2 H), 2.21-2.18 (m, 2 H), 1.89-1.87 (m, 2 H). HR-MS (ESI-TOF): m/z [M + H]⁺ calcd for C₁₈H₂₁N₅O₃FS: 406.1349; found: 406.1339.

### OTB-513 N-(((5S)-3-(3,5-Difluoro-4-((1R,5S)-3-oxido-3-thia-8-azabicyclo[3.2.1]octan-8-yl)ph enyl)-2-oxo-oxazolidin-5-yl)methyl)cyclobutanecarboxamide

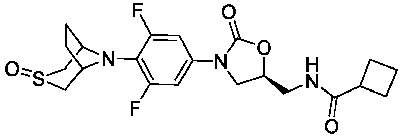

¹H-NMR (400 MHz, CDCl₃) δ: 7.12 (d, *J* = 12.0 Hz, 2 H), 5.97 (brs, 1 H), 4.77-4.75 (m, 1 H), 4.45 (s, 2 H), 3.97 (t, *J* = 8.8 Hz, 1 H), 3.74 (t, *J* = 8.4 Hz, 1 H), 3.66 (t, *J* = 5.2 Hz, 2 H), 3.54 (d, *J* = 9.2 Hz, 2 H), 3.02 (m, 1 H), 2.91 (d, *J* = 12.0 Hz, 2 H), 2.26-2.11 (m, 6 H), 1.99-1.92 (m, 1 H), 1.87-1.85 (m, 3 H). HRMS (ESI): *m*/*z* [M + H]⁺ calcd for C₂₁H₂₆N₃O₄SF₂: 454.1606; found: 454.1588.

### OTB-514 N-(((5S)-3-(3,5-Difluoro-4-((1R,5S)-3-oxido-3-thia-8-azabicyclo[3.2.1]octan-8-yl)ph enyl)-2-oxo-oxazolidin-5-yl)methyl) acetamide

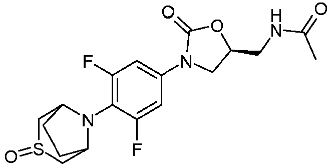

¹H-NMR (400 MHz, CDCl₃) δ: 7.12 (d, *J* = 12.0 Hz, 2 H), 6.15 (brs, 1 H), 4.81-4.75 (m, 1 H), 4.45 (s, 2 H), 3.98 (t, *J* = 8.8 Hz, 1 H), 3.74-3.63 (m, 3 H), 3.56 (d, *J* = 9.2 Hz, 2 H), 2.92 (d, *J =* 12.4 Hz, 2 H), 2.20-2.17 (m, 2 H), 2.03 (s, 3 H), 1.89-1.83 (m, 2 H). HRMS (ESI): *m*/*z* [M + H]⁺ calcd for C₁₈H₂₂N₃O₄SF₂: 414.1293; found: 414.1275.

### OBD-017 N-(((5S)-3-(3-Fluoro-4-((1R,5S)-3-oxido-3-thia-8-azabicyclo[3.2.1]octan-8-yl)pheny l)-2-oxooxazolidin-5-yl)methyl)butyramide

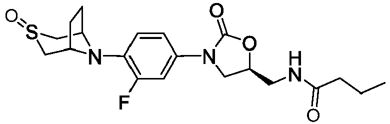

¹H-NMR (300 MHz, CDCl₃) δ: 7.39 (dd, *J* = 15.8, 2.3 Hz, 1 H), 7.03 (d, *J* = 6.1 Hz, 2 H), 6.78 (t, *J* = 9.3 Hz, 1 H), 4.72 (s, 1 H), 4.55 (s, 2 H), 3.94 (t, *J* = 8.9 Hz, 1 H), 3.81-3.66 (m, 1 H), 3.58 (s, 2 H), 3.42 (d, *J =* 10.3 Hz, 2 H), 2.77 (d, *J =* 11.9 Hz, 2 H), 2.17 (dd, *J* = 25.1, 17.8 Hz, 4 H), 1.84 (d, *J* = 7.9 Hz, 2 H), 1.56 (dd, *J* = 14.5, 7.2 Hz, 2 H), 0.83 (t, *J* = 7.4 Hz, 3 H).

LC-MS (ESI): *m*/*z* = 423.8 [M+H]⁺.

### OBD-018 (5R)-5-((1H-1,2,4-Triazol-1-yl)methyl)-3-(3-fluoro-4-((1R,5S)-3-oxido-3-thia-8-aza bicyclo[3.2.1]octan-8-yl)phenyl)oxazolidin-2-one

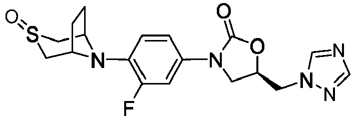

¹H-NMR (300 MHz, DMSO-*d*₆) δ: 12.17 (s, 1 H), 8.69 (d, *J* = 2.9 Hz, 1 H), 8.20 - 8.03 (m, 1 H), 7.44 (d, *J* = 16.2 Hz, 1 H), 7.28-7.02 (m, 2 H), 5.08 (dd, *J* = 8.5, 5.1 Hz, 1 H), 4.68-4.52 (m, 4 H), 4.20 (t, *J* = 9.1 Hz, 1 H), 3.91 (dd, *J* = 8.7, 6.0 Hz, 1 H), 3.56 (d, *J* = 11.1 Hz, 2 H), 2.48 (d, *J =* 12.3 Hz, 2 H), 2.06 (d, *J* = 5.1 Hz, 2 H), 1.79 (d, *J =* 7.6 Hz, 2 H).

LC-MS (ESI): *m*/*z* = 405.8 [M+H]⁺.

### OTB-260 (R)-3-(3-Fluoro-4-(2-thia-6-azaspiro[3.3]heptan-6-yl)phenyl)-5-(hydroxymethyl)oxa zolidin-2-one

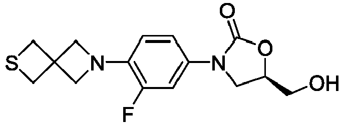

¹H-NMR (400 MHz, CDCl₃) δ:7.38 (d, *J* = 14.0 Hz, 1 H), 7.04 (d, *J* = 8.0 Hz, 1 H), 6.55 (t, *J* = 8.8 Hz, 1 H), 4.72 (brs, 1 H), 4.02 (s, 4 H), 3.99-3.89 (m, 3 H), 3.76-3.73 (m, 1 H), 3.42 (s, 4 H). ¹³C-NMR (100 MHz, CDCl₃) δ: 154.8, 152.2 (d, *J* = 240.8 Hz), 135.9 (d, *J* = 11.7 Hz), 130.2 (d, *J* = 9.3 Hz), 114.6 (d, *J* = 5.2 Hz), 114.5 (d, *J* = 3.1 Hz), 107.6 (d, *J* = 23.8 Hz), 72.8, 66.8, 62.8, 46.7, 44.3, 36.8. HRMS (ESI): *m*/*z* [M + H]⁺ calcd for C₁₅H₁₈FN₂O₃S: 325.1022; found: 325.1010.

### OTB-261 (S)-N-((3-(3-Fluoro-4-(2-thia-6-azaspiro[3.3]heptan-6-yl)phenyl)-2-oxo-oxazolidin-5 -yl)methyl)acetamide

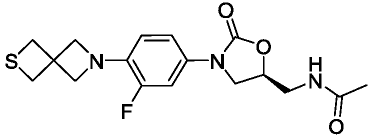

¹H-NMR (400 MHz, CDCl₃) δ: 7.33 (d, *J* = 14.0 Hz, 1 H), 6.99 (d, *J* = 8.4 Hz, 1 H), 6.48 (t, *J =* 8.8 Hz, 1 H), 6.20 (brs, 1 H), 4.75-4.73 (m, 1 H), 3.98-3.96 (m, 5 H), 3.73-3.66 (m, 2 H), 3.62-3.57 (m, 1 H), 3.41 (s, 4 H), 2.01 (s, 3 H). HRMS (ESI): *m*/*z* [M + H]⁺ calcd for C₁₇H₂₁FN₃O₃S: 366.1288; found: 366.1274.

### OTB-241 (R)-5-((1H-1,2,3-Triazol-1-yl)methyl)-3-(3-fluoro-4-(2-thia-6-azaspiro[3.3]heptan-6-yl)phenyl)oxazolidin-2-one

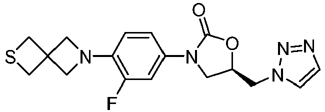

¹H-NMR (400 MHz, CDCl₃) δ: 7.79 (s, 1 H), 7.75 (s, 1 H), 7.22 (dd, *J* = 13.6, 2.0 Hz, 1 H), 6.88 (d, *J* = 8.8 Hz, 1 H), 6.51 (t, *J* = 9.2 Hz, 1 H), 5.06-5.00 (m, 1 H), 4.78-4.77 (m, 2 H), 4.08 (t, *J =* 9.2 Hz, 1 H), 4.01 (s, 4 H), 3.89-3.85 (m, 1 H), 3.40 (s, 4 H). ¹³C-NMR (100 MHz, CDCl₃) δ: 153.6, 152.0 (d, *J* = 243.1 Hz), 136.3 (d, *J* = 11.1 Hz), 134.5, 129.1 (d, *J* = 9.0 Hz), 125.1, 115.0 (d, *J* = 3.2 Hz), 114.5 (d, *J* = 5.2 Hz), 108.1 (d, *J* = 23.6 Hz), 70.4, 66.7, 52.1, 47.7, 44.3, 36.8. HRMS (ESI): *m*/*z* [M + H]⁺ calcd for C₁₇H₁₉FN₅O₂S: 376.1244; found: 376.1231.

### OTB-516 (R)-3-(3,5-Difluoro-4-(2-thia-6-azaspiro[3.3]heptan-6-yl)phenyl)-5-(hydroxymethyl) oxazolidin-2-one

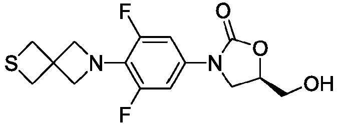

¹H-NMR (400 MHz, CDCl₃) δ: 7.03 (d, *J* = 12.0 Hz, 2 H), 4.74-4.70 (m, 1 H), 4.18 (s, 4 H), 3.98 (dd, *J* = 12.8, 3.2 Hz, 1 H), 3.93-3.85 (m, 2 H), 3.75 (dd, *J* = 12.4, 4.0 Hz, 1 H), 3.41 (s, 4 H). HRMS (ESI): *m*/*z* [M + H]⁺ calcd for C₁₅H₁₇N₂O₃SF₂: 343.0922; found: 343.0912.

### OTB-515 (S)-N-((3-(3,5-Difluoro-4-(2-thia-6-azaspiro[3.3]heptan-6-yl)phenyl)-2-oxo-oxazolidi n-5-yl)methyl)acetamide

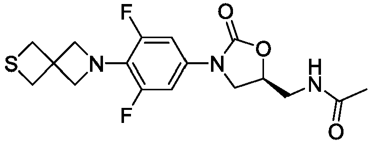

¹H-NMR (400 MHz, CDCl₃) δ: 6.98 (d, *J* = 11.6 Hz, 2 H), 5.99 (brs, 1 H), 4.74-4.72 (m, 1 H), 4.16 (s, 4 H), 3.94 (t, *J* = 8.8 Hz, 1 H), 3.71-3.65 (m, 2 H), 3.61-3.55 (m, 1 H), 3.40 (s, 4 H), 2.01 (s, 3 H). ¹³C-NMR (100 MHz, CDCl₃) δ: 171.1, 154.2, 152.6 (dd, *J* = 240.6, 11.1 Hz), 153.1, 134.5, 128.5 (t, *J* = 12.6 Hz), 124.7 (t, *J* = 13.3 Hz), 102.8 (dd, *J* = 18.2, 10.7 Hz), 71.9, 68.7, 47.6, 45.2, 42.0, 36.5, 23.1. HRMS (ESI): *m*/*z* [M + H]⁺ calcd for C₁₇H₂₀N₃O₃SF₂: 384.1188; found: 384.1168.

### OTB-242 Methyl (S)-((3-(3,5-difluoro-4-(2-thia-6-azaspiro[3.3]heptan-6-yl)phenyl)-2-oxo-oxazolidin-5-yl)methyl)carbamate

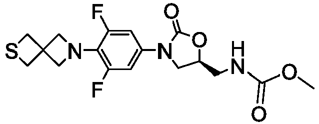

¹H NMR (400 MHz, CDCl₃) δ: 7.00 (d, *J* = 11.6 Hz, 2 H), 5.16 (brs, 1 H), 4.81-4.67 (m, 1 H), 4.16 (s, 4 H), 3.94 (t, *J* = 8.8 Hz, 1 H), 3.68 (s, 3 H), 3.56-3.50 (m, 3 H), 3.40 (s, 4 H). HRMS (ESI): *m*/*z* [M + H]⁺ calcd for C₁₇H₂₀F₂N₃O₄S: 400.1143; found: 400.1125.

### OTB-245 (R)-5-((1H-1,2,3-Triazol-1-yl)methyl)-3-(3,5-difluoro-4-(2-thia-6-azaspiro[3.3]heptan -6-yl)phenyl)oxazolidin-2-one

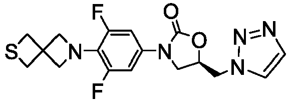

¹H NMR (400 MHz, CDCl₃) δ: 7.77 (s, 1 H), 7.74 (s, 1 H), 6.87 (d, *J* = 11.6 Hz, 2 H), 5.03-5.01 (m, 1 H), 4.77-4.76 (m, 2 H), 4.15 (s, 4 H), 4.06 (t, *J* = 9.2 Hz, 1 H), 3.86-3.82 (m, 1 H), 3.41 (s, 4 H). HRMS (ESI): *m*/*z* [M + H]⁺ calcd for C₁₇H₁₈F₂N₅O₂S: 394.1149; found: 394.1129.

### OTB-243 (S)-N-((3-(3,5-Difluoro-4-(2-thia-6-azaspiro[3.3]heptan-6-yl)phenyl)-2-oxo-oxazolidi n-5-yl)methyl)cyclopropanecarboxamide

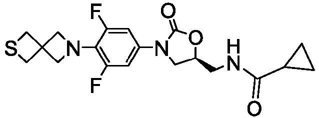

¹H NMR (400 MHz, CDCl₃) δ: 6.98 (d, *J* = 11.6 Hz, 2 H), 6.25-6.24 (m, 1 H), 4.77-4.71 (m, 1 H), 4.16 (s, 4 H), 3.92 (t, *J* = 8.8 Hz, 1 H), 3.71 - 3.56 (m, 3 H), 3.40 (s, 4 H), 1.40-1.38 (m, 1 H), 1.04-0.87 (m, 2 H), 0.82-0.73 (m, 2 H). HRMS (ESI): *m*/*z* [M + H]⁺ calcd for C₁₉H₂₂F₂N₃O₃S: 410.1350; found: 410.1331.

### OTB-244 (S)-N-((3-(3,5-Difluoro-4-(2-thia-6-azaspiro[3.3]heptan-6-yl)phenyl)-2-oxo-oxazolidi n-5-yl)methyl)cyclobutanecarboxamide

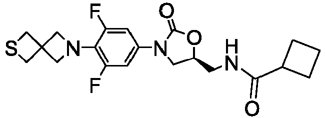

¹H NMR (400 MHz, CDCl₃) δ: 6.99 (d, *J* = 11.6 Hz, 2 H), 5.83 (brs, 1 H), 4.82-4.68 (m, 1 H), 4.17 (s, 4 H), 3.93 (t, *J* = 8.8 Hz, 1 H), 3.72-3.64 (m, 3 H), 3.40 (s, 4 H), 3.03-2.96 (m, 1 H), 2.26-1.85 (m, 6 H). HRMS (ESI): *m*/*z* [M + H]⁺ calcd for C₂₀H₂₄F₂N₃O₃S: 424.1506; found:.424.1483

### OTB-201 (R)-3-(3-Fluoro-4-(2-oxa-6-azaspiro[3.3]heptan-6-yl)phenyl)-5-(hydroxymethyl)oxaz olidin-2-one

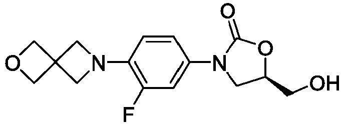

¹H-NMR (400 MHz, CDCl₃) δ:7.42 (dd, *J* = 14.0, 2.0 Hz, 1 H), 7.03 (dd, *J* = 8.8, 2.0 Hz, 1 H), 6.62 (t, *J* = 8.8 Hz, 1 H), 4.85 (s, 4 H), 4.73 (m, 1 H), 4.17 (s, 4 H), 4.00-3.95 (m, 2 H), 3.93-3.89 (m, 1 H), 3.77-3.74 (m, 1 H). ¹³C-NMR (100 MHz, CDCl₃) δ: 155.2, 152.3 (d, *J =* 240.7 Hz), 135.9 (d, *J* = 11.3 Hz), 130.4 (d, *J =* 8.9 Hz), 114.7 (d, *J* = 3.0 Hz), 114.6 (d, *J =* 5.6 Hz), 107.8 (d, *J* = 23.8 Hz), 81.3, 73.2, 63.1, 62.9, 46.9, 40.1. HRMS (ESI): *m*/*z* [M + H]⁺ calcd for C₁₅H₁₈FN₂O₄: 309.1251; found: 309.1269.

### OTB-202 (S)-N-((3-(3-Fluoro-4-(2-oxa-6-azaspiro[3.3]heptan-6-yl)phenyl)-2-oxo-oxazolidin-5-yl)methyl)acetamide

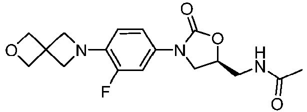

¹H-NMR (300 MHz, CDCl₃) δ: 7.35 (d, *J* = 14.1 Hz, 1 H), 7.00 (d, *J* = 8.7 Hz, 1 H), 6.51 (t, *J* =9.0 Hz, 1 H), 6.03(s, 1 H), 4.84 (s, 4 H), 4.74 (m, 1 H), 4.12 (s, 4 H), 3.99 (t, *J* =8.7 Hz, 1 H), 3.73-3.69 (m, 2 H), 3.61 (m, 1 H), 2.02 (s, 3 H). ¹³C-NMR (100 MHz, CDCl₃) δ: 171.4, 154.8, 152.4 (d, *J* = 240.8 Hz), 136.1 (d, *J* = 11.9 Hz), 130.1 (d, *J* = 9.2 Hz), 114.7 (d, *J* = 3.1 Hz), 114.6 (d, *J* = 5.0 Hz), 107.9 (d, *J* = 23.6 Hz), 81.2, 72.1, 63.1, 48.1, 42.2, 40.1, 23.4. HRMS (ESI): *m*/*z* [M + H]⁺ calcd for C₁₇H₂₁FN₃O₄: 350.1516; found: 350.1497.

### OTB-203 (R)-5-((1H-1,2,3-Triazol-1-yl)methyl)-3-(3-fluoro-4-(2-oxa-6-azaspiro[3.3]heptan-6-y l)phenyl)oxazolidin-2-one

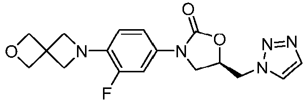

¹H-NMR (400 MHz, CDCl₃) δ: 7.78 (s, 1 H), 7.75 (s, 1 H), 7.18 (d, *J* = 13.6 Hz, 1 H), 6.88 (d, *J* = 8.8 Hz, 1 H), 6.46 (t, *J* = 9.2 Hz, 1 H), 5.03 (m, 1 H), 4.83 (s, 4 H), 4.78 (m, 2 H), 4.10-4.07 (m, 5 H), 3.91-3.85 (m, 1 H). ¹³C-NMR (100 MHz, CDCl₃) δ: 153.6, 152.1 (d, *J* = 241.4 Hz), 136.1 (d, *J* = 10.6 Hz), 134.5, 129.2 (d, *J* = 9.3 Hz), 125.1, 115.0 (d, *J* = 2.9 Hz), 114.5 (d, *J* = 4.7 Hz), 108.1 (d, *J* = 23.7 Hz), 81.0, 70.4, 62.9, 52.1, 47.7, 39.8. HRMS (ESI): *m*/*z* [M + H]⁺ calcd for C₁₇H₁₉FN₅O₃: 360.1472; found: 360.1451.

### OTB-204 (R)-5-((2H-1,2,3-Triazol-2-yl)methyl)-3-(3-fluoro-4-(2-oxa-6-azaspiro[3.3]heptan-6-y l)phenyl)oxazolidin-2-one

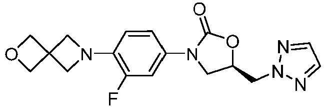

¹H-NMR (400 MHz, CDCl₃) δ: 7.65 (s, 2 H), 7.31 (d, *J* = 14.4, 2.4 Hz,1 H), 6.99 (d, *J* = 8.4 Hz, 1 H), 6.59 (t, *J* = 9.2 Hz, 1 H), 5.14-5.07 (m, 1 H), 4.88-4.83 (m, 5 H), 4.77-4.71 (m, 1 H), 4.16 (s, 4 H), 4.07-4.02 (m, 1 H), 3.98-3.92 (m, 1 H). ¹³C-NMR (100 MHz, CDCl₃) δ: 158.99, 154.38, 147.08, 144.53, 135.18, 115.11, 114.43, 112.30, 107.91, 107.87, 81.04, 71.85, 70.04, 62.91, 48.38, 48.02, 41.57, 39.85. HRMS (ESI): *m*/*z* [M + H]⁺ calcd for C₁₇H₁₉FN₅O₃: 360.1472; found: 360.1451.

### OTB-205 (S)-N-((3-(3-Fluoro-4-(2-oxa-6-azaspiro[3.3]heptan-6-yl)phenyl)-2-oxo-oxazolidin-5-yl)methyl)furan-2-carboxamide

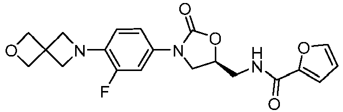

¹H-NMR (400 MHz, CDCl₃) δ: 7.46 (s, 1 H), 7.32 (dd, *J* = 14.0, 2.4 Hz, 1 H), 7.14 (d, *J* = 3.2 Hz, 1 H), 7.00 (d, *J* = 8.4 Hz, 1 H), 6.81 (t, *J* = 6.4 Hz, 1 H), 6.51 (d, *J* = 3.2 Hz, 1 H), 6.45 (t, *J=* 9.2 Hz, 1 H), 4.83 (brs, 5 H), 4.08 (s, 4 H), 4.03 (t, *J* = 8.8 Hz, 1 H), 3.92-3.85 (m, 1 H), 3.79-3.73 (m, 2 H). ¹³C-NMR (100 MHz, CDCl₃) δ: 159.2, 154.6, 152.4 (d, *J=* 240.8 Hz), 147.3, 144.7, 136.5, 130.5 (d, *J* = 9.4 Hz), 115.3, 114.7 (d, *J* = 3.1 Hz), 112.5, 109.9, 107.9 (d, *J* = 23.9 Hz), 81.2, 72.1, 63.1, 48.2, 41.8, 40.1. HRMS (ESI): *m*/*z* [M + H]⁺ calcd for C₂₀H₂₁FN₃O₅: 402.1465; found: 402.1561.

### OTB-206 (S)-N-((3-(3-Fluoro-4-(2-oxa-6-azaspiro[3.3]heptan-6-yl)phenyl)-2-oxo-oxazolidin-5-yl)methyl)thiophene-2-carboxamide

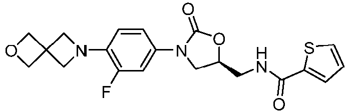

¹H-NMR (400 MHz, CDCl₃) δ: 7.53 (d, *J* = 3.2 Hz, 1 H), 7.50 (d, *J* = 4.8 Hz, 1 H), 7.32 (d, *J* = 14.0 Hz, 1 H), 7.09-6.96 (m, 2 H), 6.66 (m, 1 H), 6.45 (t, *J* = 9.2 Hz, 1 H), 4.82 (s, 4 H), 4.09-4.01 (m, 6 H), 3.81-3.79 (m, 1 H), 3.78-3.73 (m, 2 H). ¹³C-NMR (100 MHz, CDCl₃) δ: 162.9, 154.7, 152.5 (d, *J* = 241.1 Hz), 150.1, 140.8, 138.1, 130.9, 128.9, 128.0, 114.9 (d, *J* = 2.8 Hz), 114.2 (d, *J* = 3.2 Hz), 108.1 (d, *J* = 23.9 Hz), 81.20 72.2, 63.2, 48.3, 42.7, 40.0. HRMS (ESI): *m*/*z* [M + H]⁺ calcd for C₂₀H₂₁FN₃O₄S: 418.1237; found: 418.1331.

### OTB-222 (R)-N-((3-(3-Fluoro-4-(2-oxa-6-azaspiro[3.3]heptan-6-yl)phenyl)-2-oxo-oxazolidin-5-yl)methyl)methanesulfonamide

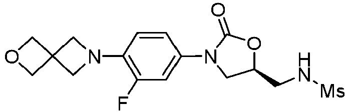

¹H-NMR (400 MHz, CDCl₃) δ: 7.44-7.35 (m, 1 H), 7.07- 6.97 (m, 1 H), 6.69-6.59 (m, 1 H), 4.98-4.91 (m, 1 H), 4.85 (s, 4 H), 4.81-4.75 (m, 1 H), 4.24-4.14 (m, 4 H), 4.13-4.09 (m, 1 H), 4.06-3.98 (m, 1 H), 3.92-3.86 (m, 1 H), 3.63-3.53 (m, 1 H), 3.47-3.36 (m, 1 H), 3.03 (s, 3 H). ¹³C-NMR (100 MHz, DMSO-*d*₆) δ: 154.5, 151.7 (d, *J* = 237.7 Hz), 135.9 (d, *J* = 11.0 Hz), 130.5 (d, *J* = 9.4 Hz), 115.2 (d, *J* = 5.4 Hz), 115.1 (d, *J* = 2.8 Hz), 107.3 (d, *J* = 23.8 Hz), 80.2, 71.7, 62.7, 47.6, 45.5. HRMS (ESI): *m*/*z* [M + H]⁺ calcd for C₁₆H₂₁FN₃O₅S: 386.1186; found: 386.1185.

### OTB-223 Methyl (S)-((3-(3-fluoro-4-(2-oxa-6-azaspiro[3.3]heptan-6-yl)phenyl)-2-oxo-oxazolidin-5-yl)methyl)carbamate

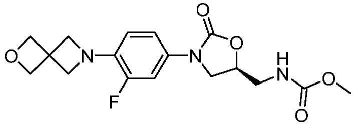

¹H-NMR (400 MHz, CDCl₃) δ: 7.39 (d, *J* = 14.0 Hz, 1 H), 7.01 (d, *J* = 8.8 Hz, 1 H), 6.61 (t, *J* = 9.2 Hz, 1 H), 5.11 (brs, 1 H), 4.84 (s, 4 H), 4.73 (brs, 1 H), 4.16 (s, 4 H), 3.99 (t, *J* = 8.8 Hz, 1 H), 3.77-3.75 (m, 1 H), 3.68 (s, 3 H), 3.64-3.60 (m, 1 H), 3.55-3.50 (m, 1 H). HRMS (ESI): *m*/*z* [M + H]⁺ calcd for C₁₇H₂₁FN₃O₅: 366.1465; found: 366.1466.

### OTB-238 (S)-N-((3-(3-Fluoro-4-(2-oxa-6-azaspiro[3.3]heptan-6-yl)phenyl)-2-oxo-oxazolidin-5-yl)methyl)cyclopropanecarboxamide

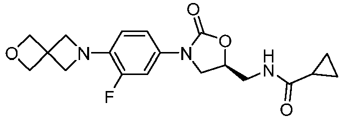

¹H-NMR (400 MHz, CDCl₃) δ: 7.35 (dd, *J* = 14.0, 2.4 Hz, 1 H), 7.00 (d, *J* = 8.4 Hz, 1 H), 6.51 (t, *J* = 9.2 Hz, 1 H), 6.13 (brs, 1 H), 4.84 (s, 4 H), 4.75-4.73 (m, 1 H), 4.12 (s, 4 H), 3.97 (t, *J* = 8.8 Hz, 1 H), 3.75-3.65 (m, 3 H), 1.39-1.36 (m, 1 H), 0.97-0.91 (m, 2 H), 0.79-0.75 (m, 2 H). ¹³C-NMR (100 MHz, CDCl₃) δ: 174.8, 154.6, 151.6 (d, *J* = 240.8 Hz), 136.0 (d, *J* = 11.3 Hz), 129.8 (d, *J* = 9.2 Hz), 114.5 (d, *J* = 3.1 Hz), 114.6 (d, *J* = 3.1 Hz), 114.3 (d, *J* = 5.2 Hz), 107.8 (d, *J* = 23.8 Hz), 81.1, 72.1, 62.9, 47.9, 42.0, 39.9, 14.6, 7.7. HRMS (ESI): *m*/*z* [M + H]⁺ calcd for C₁₉H₂₃FN₃O₄: 376.1673; found: 376.1652.

### OTB-239 (S)-N-((3-(3-Fluoro-4-(2-oxa-6-azaspiro[3.3]heptan-6-yl)phenyl)-2-oxo-oxazolidin-5-yl)methyl)cyclobutanecarboxamide

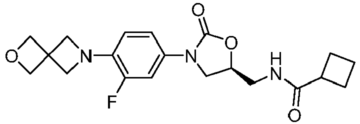

¹H-NMR (400 MHz, CDCl₃) δ: 7.49 (d, *J* = 14.4 Hz, 1 H), 7.01 (d, *J* = 8.4 Hz, 1 H), 6.85 (t, *J* = 9.2 Hz, 1 H), 5.82 (brs, 1 H), 4.86 (s, 4 H), 4.76 (brs, 1 H), 4.27 (s, 4 H), 4.00 (t, *J* = 9.2 Hz, 1 H), 3.78-3.65 (m, 3 H), 3.03-2.99 (m, 1 H), 2.26-2.13 (m, 4 H), 1.99-1.85 (m, 2 H). ¹³C-NMR (100 MHz, CDCl₃) δ: 176.0, 154.5, 152.1 (d, *J* = 240.9 Hz), 136.0 (d, *J* = 11.2 Hz), 129.8 (d, *J* = 9.2 Hz), 114.4 (d, *J* = 3.1 Hz), 114.3 (d, *J* = 5.2 Hz), 107.7 (d, *J* = 23.9 Hz), 81.1, 71.9, 62.8, 47.9, 41.9, 39.7, 25.4, 25.3, 18.2. HRMS (ESI): *m*/*z* [M + H]⁺ calcd for C₂₀H₂₅FN₃O₄: 390.1829; found: 390.1808.

### OTB-229 (R)-3-(3,5-Difluoro-4-(2-oxa-6-azaspiro[3.3]heptan-6-yl)phenyl)-5-(hydroxymethyl)o xazolidin-2-one

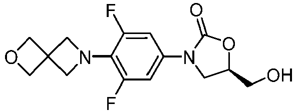

¹H-NMR (400 MHz, CDCl₃) δ: 7.03 (d, *J* = 10.8 Hz, 2 H), 4.82 (s, 4 H), 4.72 (brs, 1 H), 4.28 (s, 4 H), 3.99-3.85 (m, 3 H), 3.75-3.72 (m, 1 H). ¹³C-NMR (100 MHz, CDCl₃) δ: 154.6, 152.6 (dd, *J* = 240.2, 10.8 Hz), 128.9 (t, *J* = 12.8 Hz), 124.3 (t, *J* = 13.3 Hz), 102.8 (dd, *J* = 18.2, 10.7 Hz), 81.0, 72.9, 64.9, 62.6, 46.3, 40.8. HRMS (ESI): *m*/*z* [M + H]⁺ calcd for C₁₅H₁₇F₂N₂O₄: 327.1156; found: 327.1135.

### OTB-230 (S)-N-((3-(3,5-Difluoro-4-(2-oxa-6-azaspiro[3.3]heptan-6-yl)phenyl)-2-oxo-oxazolidi n-5-yl)methyl)acetamide

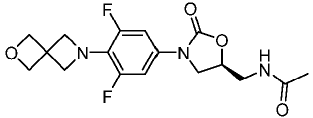

¹H-NMR (400 MHz, CDCl₃) δ: 6.97 (d, *J* = 10.0 Hz, 2 H), 6.49 (brs, 1 H), 4.81 (s, 4 H), 4.75 (brs, 1 H), 4.27 (s, 4 H), 3.94 (t, *J* = 8.8 Hz, 1 H), 3.70-3.63 (m, 3 H), 2.02 (s, 3 H). ¹³C-NMR (100 MHz, CDCl₃) δ: 171.2, 154.2, 152.5 (dd, *J* = 240.5, 10.9 Hz), 128.6 (t, *J* = 12.6 Hz), 124.5 (t, *J* = 13.4 Hz), 102.8 (dd, *J* = 18.2, 10.6 Hz), 80.9, 71.9, 64.9, 47.5, 41.9, 40.8, 23.1. HRMS (ESI): *m*/*z* [M + H]⁺ calcd for C₁₇H₂₀F₂N₃O₄: 368.1422; found: 368.1418.

### OTB-231 Methyl (S)-((3-(3,5-difluoro-4-(2-oxa-6-azaspiro[3.3]heptan-6-yl)phenyl)-2-oxo-oxazolidin-5-yl)methyl)carbamate

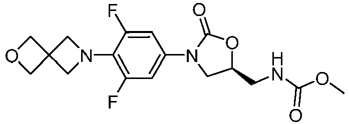

¹H-NMR (400 MHz, CDCl₃) δ: 7.04 (d, *J* = 10.8 Hz, 2 H), 5.08 (brs, 1 H), 4.83 (s, 4 H), 4.74 (brs, 1 H), 4.34 (s, 4 H), 3.96 (t, *J* = 9.2 Hz, 1 H), 3.72-3.51 (m, 6 H). ¹³C-NMR (100 MHz, CDCl₃) δ: 157.5, 154.0, 152.6 (dd, *J* = 240.4, 10.9 Hz), 128.7 (t, *J* = 12.8 Hz), 124.5 (t, *J* = 13.5 Hz), 102.8 (dd, *J* = 23.3, 15.7 Hz), 80.9, 71.7, 64.8, 52.6, 47.4, 43.6, 40.8. HRMS (ESI): *m*/*z* [M + H]⁺ calcd for C₁₇H₂₀F₂N₃O₅: 384.1371; found: 384.1367.

### OTB-232 (R)-N-((3-(3,5-Difluoro-4-(2-oxa-6-azaspiro[3.3]heptan-6-yl)phenyl)-2-oxo-oxazolidi n-5-yl)methyl)methanesulfonamide

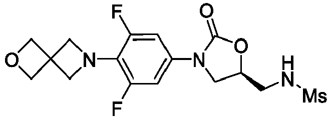

¹H-NMR (400 MHz, DMSO-*d*₆) δ: 7.8 (d, *J* = 12.4 Hz, 2 H), 4.74-4.69 (m, 5 H), 4.23 (s, 4 H), 4.06 (t, *J* = 8.8 Hz, 1 H), 3.74-3.70 (m, 1 H), 3.31-3.27 (m, 2 H), 2.94 (s, 3 H). ¹³C-NMR (100 MHz, DMSO-*d*₆) δ: 154.3, 152.2 (dd, *J* = 237.8, 11.3 Hz), 129.5 (t, *J* = 13.0 Hz), 124.4 (t, *J* = 13.6 Hz), 102.9 (dd, *J* = 18.2, 10.5 Hz), 80.0, 71.8, 64.7, 47.4, 45.5, 40.7. HRMS (ESI): *m*/*z* [M + H]⁺ calcd for C₁₆H₂₀F₂N₃O₅S: 404.1092; found: 404.1087.

### OTB-233 (S)-N-((3-(3,5-Difluoro-4-(2-oxa-6-azaspiro[3.3]heptan-6-yl)phenyl)-2-oxo-oxazolidi n-5-yl)methyl)cyclopropanecarboxamide

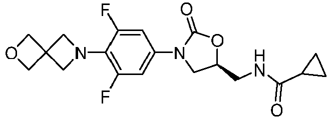

¹H-NMR (400 MHz, CDCl₃) δ: 6.98 (d, *J* = 10.0 Hz, 2 H), 6.36 (brs, 1 H), 4.82 (s, 4 H), 4.75 (brs, 1 H), 4.28 (s, 4 H), 3.93 (t, *J* = 8.8 Hz, 1 H), 3.72-3.65 (m, 3 H), 1.42-1.40 (m, 1 H), 0.96-0.88 (m, 2 H), 0.77-0.75 (m, 2 H). ¹³C-NMR (100 MHz, CDCl₃) δ: 174.9, 154.4, 152.5 (dd, *J* = 240.4, 10.3 Hz), 128.6 (t, *J* = 12.7 Hz), 124.4 (t, *J* = 13.4 Hz), 102.8 (dd, *J* = 18.2, 10.6 Hz), 80.9, 72.2, 64.8, 47.6, 41.9, 40.8, 14.5, 7.7. HRMS (ESI): *m*/*z* [M + H]⁺ calcd for C₁₉H₂₂F₂N₃O₄: 394.1578; found: 394.1575.

### OTB-234 (R)-5-((1H-1,2,3-Triazol-1-yl)methyl)-3-(3,5-difluoro-4-(2-oxa-6-azaspiro[3.3]heptan -6-yl)phenyl)oxazolidin-2-one

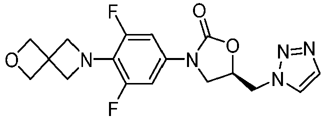

¹H-NMR (400 MHz, CDCl₃) δ: 7.77 (s, 1 H), 7.75 (s, 1 H), 6.86 (d, *J* = 11.6 Hz, 2 H), 5.03-5.02 (m, 1 H), 4.81 (s, 4 H), 4.77-4.76 (m, 2 H), 4.27 (s, 4 H), 4.06 (t, *J* = 9.2 Hz, 1 H), 3.86-3.82 (m, 1 H). ¹³C-NMR (100 MHz, CDCl₃) δ: 153.2, 152.4 (dd, *J* = 240.7, 11.0 Hz), 134.5, 127.8 (t, *J =* 12.8 Hz), 125.1, 124.8 (t, *J =* 13.3 Hz), 103.1 (dd, *J =* 18.1, 10.7 Hz), 80.9, 70.4, 64.8, 52.0, 47.3, 40.8. HRMS (ESI): *m*/*z* [M + H]⁺ calcd for C₁₇H₁₈F₂N₅O₃: 378.1378; found: 378.1365.

### OTB-240 (S)-N-((3-(3,5-Difluoro-4-(2-oxa-6-azaspiro[3.3]heptan-6-yl)phenyl)-2-oxo-oxazolidi n-5-yl)methyl)cyclobutanecarboxamide

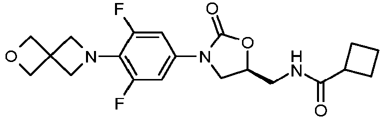

¹H-NMR (400 MHz, CDCl₃) δ: 7.00 (d, *J* = 12.0 Hz, 2 H), 5.87 (t, *J* = 6.0 Hz, 1 H), 4.82 (s, 4 H), 4.77-4.71 (m, 1 H), 4.28 (s, 4 H), 3.94 (t, *J* = 8.8 Hz, 1 H), 3.72-3.64 (m, 3 H), 3.03-2.99 (m, 1 H), 2.26-2.13 (m, 4 H), 1.99-1.82 (m, 2 H). HRMS (ESI): *m*/*z* [M + H]⁺ calcd for C₂₀H₂₄F₂N₃O₄: 408.1735; found: 408.1716.

### OTB-701 (R)-N-((3-(3-Fluoro-4-(2,6-diazaspiro[3.3]heptan-2-yl)phenyl)-2-oxo-oxazolidin-5-y l)methyl)acetamide

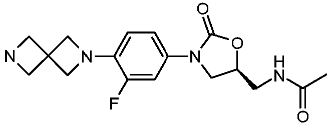

¹H-NMR (400 MHz, CDCl₃) δ: 7.38 (d, *J* = 14.4 Hz, 1 H), 7.09 (d, *J* = 8.4 Hz, 1 H), 6.58 (t, *J* = 9.2 Hz, 1 H), 4.76 (m, 2 H), 4.30 (s, 4 H), 4.10 (s, 4 H), 3.76 (m, 1 H), 3.54 (m, 2 H), 1.96 (s, 3 H). ¹³C-NMR (100 MHz, CDCl₃) δ: 172.6, 155.4, 153.2, 150.8, 135.6, 130.5, 114.4, 107.3, 72.0, 62.2, 55.0, 48.0, 41.7, 37.2, 21.0. HRMS (ESI): *m*/*z* [M+H]⁺ calcd for C₁₇H₂₂FN₄O₃:349.1671; found: 349.1662.

### OTB-702 (R)-N-((3-(3-Fluoro-4-(6-methyl-2,6-diazaspiro[3.3]heptan-2-yl)phenyl)-2-oxo-oxaz olidin-5-yl)methyl)acetamide

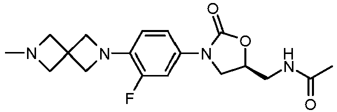

¹H-NMR (400 MHz, CDCl₃) δ: 7.36 (d, *J* = 14.4 Hz, 1 H), 7.08 (d, *J* = 8.8 Hz, 1 H), 6.57 (t, *J* = 9.2 Hz, 1 H), 4.75 (m, 1 H), 4.10 (t, *J* = 9.2 Hz, 1 H), 4.01 (s, 4 H), 3.74 (m, 1 H), 3,73 (s, 4 H), 3.53 (m, 2 H), 2.52 (s, 3 H), 1.96 (s, 3 H). ¹³C NMR (100 MHz, CDCl₃) δ: 172.6, 155.4, 153.2, 150.8, 136.1, 130.3, 114.4, 107.3, 72.0, 64.9, 62.6, 48.0, 43.0, 41.7, 34.8, 21.0. HRMS (ESI): *m*/*z* [M+H]⁺ calcd for C₁₈H₂₄FN₄O₃: 363.1827, found: 363.1819.

### OTB-704 (R)-N-((3-(3,5-Difluoro-4-(2,6-diazaspiro[3.3]heptan-2-yl)phenyl)-2-oxo-oxazolidin-5-yl)methyl)acetamide

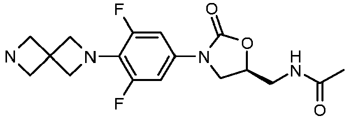

¹H-NMR (400 MHz, CD₃OD) δ: 7.12 (d, *J* = 9.6 Hz, 2 H), 4.76, (m, 1 H), 4.30 (s, 4 H), 4.24 (s, 4 H), 4.06 (m, 1 H), 3.73 (m, 1 H), 3.53 (m, 2 H), 1.96 (s, 3 H).

¹³C NMR (100 MHz, CD₃OD) δ: 172.6, 155.0, 153.7, 151.3, 129.6, 129.4, 102.7, 102.5, 72.0, 64.3, 55.0, 48.0, 41.6, 38.3, 21.0. HRMS (ESI): *m*/*z* [M+H]⁺calcd for C₁₇H₂₁F₂N₄O₃: 367.1583 , found: 367.1576.

### OTB-705 (R)-N-((3-(3,5-Difluoro-4-(6-methyl-2,6-diazaspiro[3.3]heptan-2-yl)phenyl)-2-oxo-o xazolidin-5-yl)methyl)acetamide

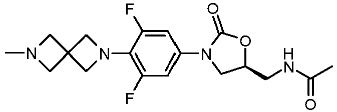

¹H-NMR (400 MHz, CD₃OD) δ: 7.12 (d, *J* = 10.0 Hz, 2 H), 4.75 (m, 1 H), 4.22 (s, 4 H), 4.05 (m, 1 H), 3.78 (s, 4 H), 3.73 (m, 1 H), 3.53 (m, 2 H), 2.55 (s, 3 H), 1.96 (s, 3 H). ¹³C-NMR (100 MHz, CD₃OD) δ: 172.6, 155.0, 153.7, 151.3, 129.4, 102.8, 72.0, 64.7, 64.6, 48.0, 42.7, 41.7, 22.4, 21.0. HRMS (ESI): *m*/*z* [M+H]⁺ calcd for C₁₈H₂₃F₂N₄O₃: 381.1733, found: 381.1725.

### Example 12

### Synthesis of Additional Embodiments of the Invention

### Procedures for preparation of (6):

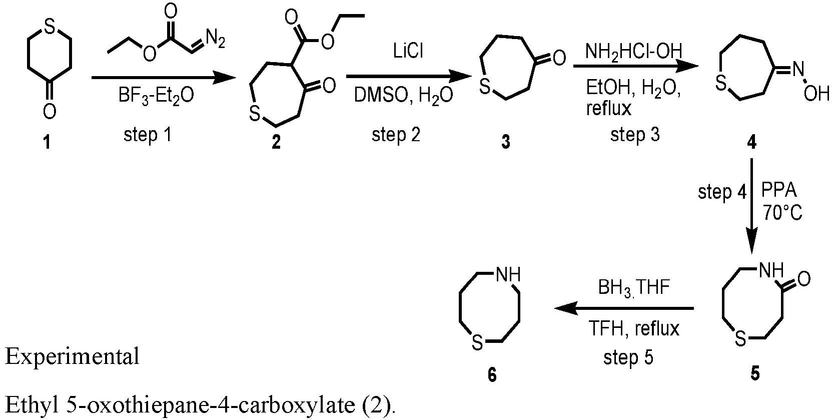

To a solution of tetrahydrothiopyran-4-one (100 g, 862 mmol) in Et₂O (150 mL) was added BF₃-Et₂O (120 mL, 948 mmol) at -30 °C, then the reaction mixture was stirred at -30 °C for 2 h under a nitrogen gas atmosphere, after that the solution of ethyl 2-diazoacetate (147 g, 1293 mmol) in Et₂O (100 mL) was added to the mixture at -30 °C, then the mixture was warmed to room temperature and stirred for overnight. Quenched with K₂CO₃, the solvent was concentrated and dried to give ethyl 5-oxothiepane-4-carboxylate (2) (80 g, 46%) as brown oil.

### Thiepan-4-one (3).

A mixture of ethyl 5-oxothiepane-4-carboxylate (2) (80 g, 396 mmol) and lithium chloride (16.6 g, 396 mmol) in DMSO (100 mL) and H₂O (5 drop) was stirred at 180°C for 2 h. The reaction mixture was cooled to room temperature and poured into ice water, extracted with EA, the organic layer was concentrated under reduced pressure to afford thiepan-4-one (3) (15.9 g crude, 31%) as brown solid.

### (Z)-Thiepan-4-one oxime (4).

To a solution of thiepan-4-one (3) (15.9 g, 122 mmol) in EtOH (150 mL) and H₂O (50 mL) was added with NH₂OH-HCl (8.47 g, 122 mmol), then the reaction mixture was stirred at 75 °C for 4 h under a nitrogen gas atmosphere, then mixture was concentrated and dried to give (Z)-thiepan-4-one oxime (4) (9.97 g, 56%) as brown solid.

### 1,5-Thiazocan-4-one (5).

A mixture of (Z)-thiepan-4-one oxime (4) (9.97 g, 68.7 mmol) and polyphosphoric acid (50 g) was stirred at 70 °C for 2 h. The reaction mixture was cooled to room temperature and poured into ice water, adjusted pH = 8 using potassium carbonate solution, extracted with EA, the organic layer was concentrated under reduced pressure to afford 1,5-thiazocan-4-one (5) (3 g crude, 30%) as brown solid.

### 1,5-Thiazocane (6).

To a solution of 1,5-thiazocan-4-one (5) (3 g, 20.7 mmol) in THF (100 mL) was added BH₃ (31 mL, 31.1 mmol) in THF at 0 °C, followed by refluxing for 12 h. The reaction was quenched with CH₃OH (50 mL). The solvent was evaporated to afford 1,5-thiazocane (6) as a white oil (1.7 g, 63%), and the crude material was used for next reaction without further purification.

LC-MS (ESI) *m*/*z* = 132 [M+H]⁺.

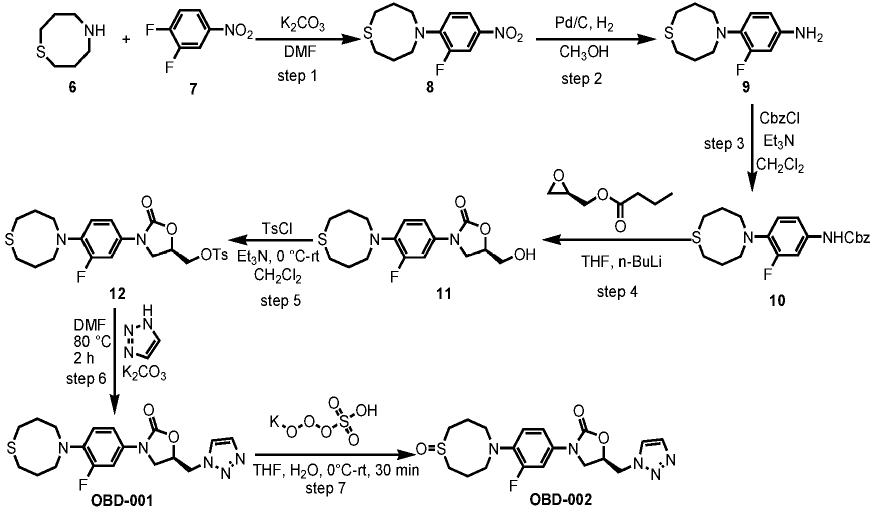

### Step 1: Preparation of 5-(2-fluoro-4-nitrophenyl)-1,5-thiazocane (8):

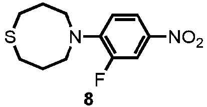

To a solution of 1,5-thiazocane (6) (1 g, 7.6 mmol) and 1,2-difluoro-4-nitrobenzene (1.2 g, 7.6 mmol) in DMF (10 mL) was added K₂CO₃ (1.05 g, 7.6 mmol) at 25°C then the reaction mixture was stirred at 80°C for 2 h under a nitrogen gas atmosphere, monitored by TLC. The mixture was concentrated under reduced pressure, and the crude material was purified by silica gel column chromatography (EA: PE = 3: 1) to afford 5-(2-fluoro-4-nitrophenyl)-1,5-thiazocane (8) (1.5 g, 74%) as a yellow solid.

LC-MS (ESI) *m*/*z* = 271 [M+H]⁺.

### Step 2: 3-Fluoro-4-(1,5-thiazocan-5-yl)benzenamine (9):

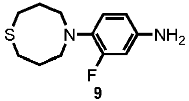

To a solution of 5-(2-fluoro-4-nitrophenyl)-1,5-thiazocane (8) (1.5 g, 5.7 mmol) and Palladium carbon (200 mg) in MeOH (15 mL), then under a hydrogen gas atmosphere and the reaction mixture was stirred at room temperature for overnight, monitored by TLC. The filtrate was concentrated under reduced pressure to afford 3-fluoro-4-(1,5-thiazocan-5-yl)benzenamine (9) (1.2 g, 91%) as a white oil., and the crude material was used for next reaction without further purification.

LC-MS (ESI) *m*/*z* = 241 [M+H]⁺.

### Step 3: Benzyl 3-fluoro-4-(1,5-thiazocan-5-yl)phenylcarbamate (10):

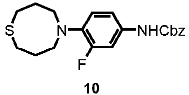

Benzyl carbonochloridate (1.76 g, 10.4 mmol) was added to a suspension of 3-fluoro-4-(1,5-thiazocan-5-yl)benzenamine (9) (1.2 g, 5.2 mmol) and triethylamine (1.05 g, 10.4 mmol) in DCM (200 mL) at -20 °C under a nitrogen gas atmosphere, then reaction mixture was stirred at 0 °C for 30 min, monitored by TLC. Quenched with ammonium chloride, extracted with DCM, the organic layer was concentrated under reduced pressure, and the crude material was purified by silica gel column chromatography (EA: PE = 10: 1) to afford benzyl 3-fluoro-4-(1,5-thiazocan-5-yl)phenylcarbamate (10) (740 mg, 38%) as a white solid.

LC-MS (ESI) *m*/*z* = 375 [M+H]⁺.

### Step 4:

### (R)-3-(3-Fluoro-4-(1,5-thiazocan-5-yl)phenyl)-5-(hydroxymethyl)oxazolidin-2-one (11):

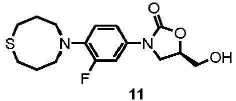

To a solution of benzyl 3-fluoro-4-(1,5-thiazocan-5-yl)phenylcarbamate (10) (740 mg, 1.97 mmol) in THF (10 mL) at - 78 °C under a nitrogen gas atmosphere was added n-BuLi (1.3 ml, 2.96 mmol), then the mixture was stirred at -78°C for 30 min, after that the solution of (R)-oxiran-2-ylmethyl butyrate (427 mg, 2.96 mmol) in THF was added to the mixture at -78 °C, then warmed to room temperature and stirred for overnight, monitored by TLC. Quenched with ammonium chloride, extracted with EA, the organic layer was concentrated under reduced pressure, and the crude material was purified by silica gel column chromatography (DCM: MeOH = 70: 1) to afford (*R*)-3-(3-fluoro-4-(1,5-thiazocan-5-yl)phenyl)-5-(hydroxymethyl)oxazolidin-2-one (11) (382 mg, 57%) as a white solid.

LC-MS (ESI) *m*/*z* = 341 [M+H]⁺.

### Step 5: (R)-(3-(3-fluoro-4-(1,5-thiazocan-5-yl)phenyl)-2-oxooxazolidin-5-yl)methyl 4-methylbenzenesulfonate (12):

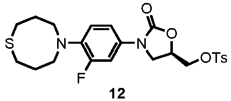

4-methylbenzene-1-sulfonyl chloride (418 mg, 2.2 mmol) was added to a suspension of (*R*)-3-(3-fluoro-4-(1,5-thiazocan-5-yl)phenyl)-5-(hydroxymethyl)oxazolidin-2-one (11) (382 mg, 1.1 mmol) and Et₃N (222 mg, 2.2 mmol) in DCM (10 mL) at 0 °C under a nitrogen gas atmosphere and the reaction mixture was stirred at room temperature for overnight, monitored by TLC. Quenched with ammonium chloride, extracted with DCM, the organic layer was concentrated under reduced pressure, and the crude material was purified by silica gel column chromatography (DCM: MeOH = 50: 1) to afford (*R*)-(3-(3-fluoro-4-(1,5-thiazocan-5-yl)phenyl)-2-oxooxazolidin-5-yl)methyl 4-methylbenzenesulfonate (12) (407 mg, 75%) as a white solid.

LC-MS (ESI) *m*/*z* = 495 [M+H]⁺.

### Step 6:

### (R)-5-((1H-1,2,3-triazol-1-yl)methyl)-3-(3-fluoro-4-(1,5-thiazocan-5-yl)phenyl)oxaz olidin-2-one (OBD-001):

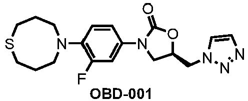

To a solution of (*R*)-(3-(3-fluoro-4-(1, 5-thiazocan-5-yl)phenyl)-2-oxooxazolidin-5-yl)methyl 4-methylbenzenesulfonate (12) (200 mg, 0.4 mmol) and 1H-1,2,3-triazole (56 mg, 0.8 mmol) in DMF (5 mL) was added K₂CO₃ (110 mg, 0.8 mmol) at 25°C, then the reaction mixture was stirred at 80°C for 2 h under a nitrogen gas atmosphere, monitored by TLC. Quenched with ammonium chloride, extracted with EA, the organic layer was concentrated under reduced pressure, and the crude material was purified by silica gel column chromatography (EA: PE = 2: 1) to afford (*R*)-5-((1H-1,2,3-triazol-1-yl)methyl)-3-(3-fluoro-4-(1,5-thiazocan-5-yl)phenyl)oxaz olidin-2-one (OBD-001) (70 mg, 45%) as a white solid.

¹H NMR (301 MHz, CDCl₃) δ 7.76 (d, J = 17.6 Hz, 2H), 7.41 - 7.09 (m, 1H), 7.11 - 6.73 (m, 2H), 5.04 (d, J = 3.0 Hz, 1H), 4.78 (d, J = 3.4 Hz, 2H), 4.12 (t, J = 9.2 Hz, 1H), 3.88 (dd, J = 9.2, 6.1 Hz, 1H), 3.36 (t, J = 6.0 Hz, 3H), 2.92 - 2.59 (m, 4H), 2.01 (dd, J = 27.9, 7.3 Hz, 4H).

LC-MS (ESI) *m*/*z* = 391.9 [M+H]⁺.

### Step 7: Preparation of (OBD-002):

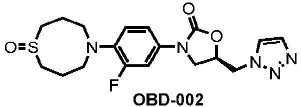

To a solution of (*R*)-5-((1H-1,2,3-triazol-1-yl)methyl)-3-(3-fluoro-4-(1,5-thiazocan-5-yl)phenyl)oxaz olidin-2-one (OBD-001) (50 mg, 0.13 mmol) in THF (10 mL) and 10 drops water was added potassium peroxomonosulfate (80 mg, 0.13 mmol) at 0 °C, then the reaction mixture was stirred at 0 °C for 2 h, monitored by TLC. Quenched with sodium thiosulfate, and the crude material was purified by silica gel column chromatography (DCM: MeOH = 80: 1) to afford (OBD-002) (22 mg, 41%) as a white solid.

¹H NMR (301 MHz, CDCl₃) δ 7.92 - 7.67 (m, 2H), 7.32 (d, J = 16.8 Hz, 1H), 7.12 (t, J = 9.0 Hz, 1H), 6.96 (d, J = 8.1 Hz, 1H), 5.07 (s, 1H), 4.81 (d, J = 4.0 Hz, 2H), 4.15 (t, J = 9.0 Hz, 1H), 4.01 - 3.83 (m, 1H), 3.32 (d, J = 14.2 Hz, 5H), 3.11 - 2.87 (m, 2H), 2.59 (s, 2H), 2.19 (s, 4H).

LC-MS (ESI) *m*/*z* = 407.8 [M+H]⁺.

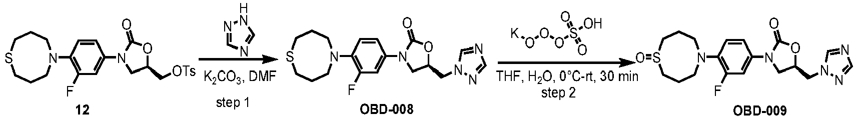

### Step 1:

### (R)-5-((1H-1,2,4-triazol-1-yl)methyl)-3-(3-fluoro-4-(1,5-thiazocan-5-yl)phenyl)oxaz olidin-2-one (OBD-008):

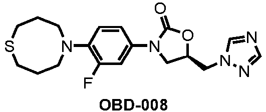

To a solution of (*R*)-(3-(3-fluoro-4-(1, 5-thiazocan-5-yl)phenyl)-2-oxooxazolidin-5-yl)methyl 4-methylbenzenesulfonate (12) (200 mg, 0.4 mmol) and 1H-1,2,4-triazole (56 mg, 0.8 mmol) in DMF (5 mL) was added K₂CO₃ (110 mg, 0.8 mmol) at 25°C, then the reaction mixture was stirred at 80°C for 2 h under a nitrogen gas atmosphere, monitored by TLC. Quenched with ammonium chloride, extracted with EA, the organic layer was concentrated under reduced pressure, and the crude material was purified by silica gel column chromatography (EA: PE = 2: 1) to afford (*R*)-5-((1H-1,2,4-triazol-1-yl)methyl)-3-(3-fluoro-4-(1,5-thiazocan-5-yl)phenyl)oxaz olidin-2-one (OBD-008) (84 mg, 54%) as a white solid.

¹H NMR (301 MHz, CDCl₃) δ 8.24 (s, 1H), 7.97 (s, 1H), 7.00 (s, 2H), 5.12 - 4.91 (m, 1H), 4.56 (d, J = 4.7 Hz, 2H), 4.24 - 3.83 (m, 2H), 3.38 (t, J = 6.0 Hz, 4H), 2.95 - 2.59 (m, 4H), 1.98 (s, 5H).

LC-MS (ESI) *m*/*z* = 391.9 [M+H]⁺.

### Step 2: Preparation of (OBD-009):

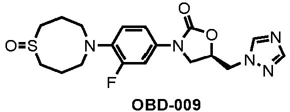

To a solution of (*R*)-5-((1H-1,2,3-triazol-1-yl)methyl)-3-(3-fluoro-4-(1,5-thiazocan-5-yl)phenyl)oxaz olidin-2-one (OBD-008) (50 mg, 0.13 mmol) in THF (10 mL) and 10 drops water was added potassium peroxomonosulfate (80 mg, 0.13 mmol) at 0 °C, then the reaction mixture was stirred at 0 °C for 2 h, monitored by TLC. Quenched with sodium thiosulfate, and the crude material was purified by silica gel column chromatography (DCM: MeOH = 80: 1) to afford (OBD-009) (22 mg, 41%) as a white solid.

¹H NMR (301 MHz, CDCl₃) δ 8.24 (s, 1H), 7.93 (s, 1H), 7.41 - 7.20 (m, 1H), 7.19 - 6.92 (m, 2H), 5.10 - 4.92 (m, 1H), 4.56 (d, J = 4.7 Hz, 2H), 4.12 (t, J = 9.0 Hz, 1H), 3.97 (dd, J = 9.2, 6.2 Hz, 1H), 3.28 (dd, J = 13.0, 6.9 Hz, 2H), 3.12 (dd, J = 12.5, 5.9 Hz, 4H), 3.02 - 2.83 (m, 2H), 2.22 - 1.99 (m, 5H), 1.26 (d, J = 9.4 Hz, 4H).

LC-MS (ESI) *m*/*z* = 407.8 [M+H]⁺.

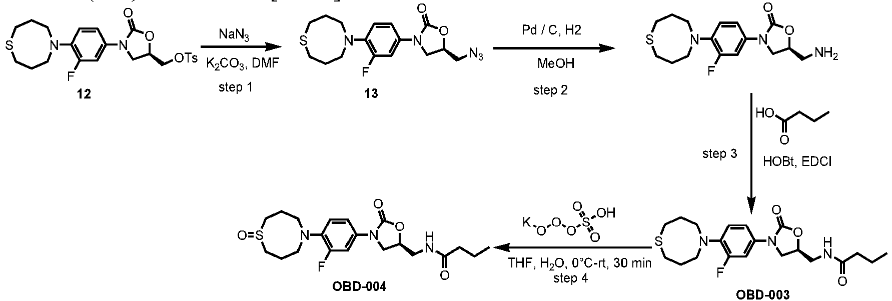

### Step 1:

### (R)-5-(azidomethyl)-3-(3-fluoro-4-(1,5-thiazocan-5-yl)phenyl)oxazolidin-2-one (13):

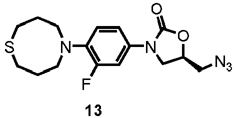

To a solution of (*R*)-(3-(3-fluoro-4-(1,5-thiazocan-5-yl)phenyl)-2-oxooxazolidin-5-yl)methyl 4-methylbenzenesulfonate (12) (800 mg, 1.62 mmol) and sodium azide (105 mg, 1.62 mmol) in DMF (10 mL) was added K₂CO₃ (447 g, 3.24 mmol) at 25°C, then the reaction mixture was stirred at 80°C for 1 h under a nitrogen gas atmosphere, monitored by TLC. Quenched with ammonium chloride, extracted with EA, the organic layer was concentrated under reduced pressure, and the crude material was purified by silica gel column chromatography (PE: EA = 2: 1) to afford (*R*)-5-(azidomethyl)-3-(3-fluoro-4-(1,5-thiazocan-5-yl)phenyl)oxazolidin-2-one (13) (470 mg, 80%) as a white solid.

LC-MS (ESI) *m*/*z* = 366 [M+H]⁺.

### Step 2:

### (S)-5-(aminomethyl)-3-(3-fluoro-4-(1,5-thiazocan-5-yl)phenyl)oxazolidin-2-one (14):

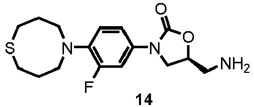

To a solution of (*R*)-5-(azidomethyl)-3-(3-fluoro-4-(1,5-thiazocan-5-yl)phenyl)oxazolidin-2-one (13) (470 mg, 1.3 mmol) in MeOH (10 mL) was added palladium carbon (100 mg) at 25°C, then the reaction mixture was stirred at room temperature for overnight under a hydrogen gas atmosphere, monitored by TLC. The filtrate was concentrated under reduced pressure, and the crude material was purified by silica gel column chromatography (DCM: MeOH = 50: 1) to afford (*S*)-5-(aminomethyl)-3-(3-fluoro-4-(1,5-thiazocan-5-yl)phenyl)oxazolidin-2-one (14) (374 mg, 85%) as a white solid.

LC-MS (ESI) *m*/*z* = 340 [M+H]⁺.

### Step 3:

### (S)-N-((3-(3-fluoro-4-(1,4-thiazepan-4-yl)phenyl)-2-oxooxazolidin-5-yl)methyl)buty ramide (OBD-005):

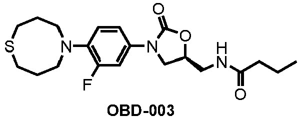

To a solution of (*S*)-5-(aminomethyl)-3-(3-fluoro-4-(1,5-thiazocan-5-yl)phenyl)oxazolidin-2-one (14) (374 mg, 1.1 mmol) and butyric acid (97 mg, 1.1 mmol) in DCM (10 mL) were added HOBt (223 mg, 1.65 mmol), EDCI (420 mg, 2.2 mmol) and DIPEA (284 mg, 2.2 mmol) at 25 °C, then the reaction mixture was stirred at 25 °C for 2 h under a nitrogen gas atmosphere, monitored by TLC. Quenched with ammonium chloride, extracted with DCM, the organic layer was concentrated under reduced pressure, and the crude material was purified by silica gel column chromatography (DCM: MeOH = 80: 1) to afford (*S*)-N-((3 -(3-fluoro-4-(1,5-thiazocan-5-yl)phenyl)-2-oxooxazolidin-5-yl)methyl)buty ramide (OBD-003) (252 mg, 56%) as a white solid.

¹H NMR (300 MHz, CDCl₃) δ 7.50 (s, 2H), 7.03 (d, J = 6.1 Hz, 1H), 5.99 (s, 1H), 4.77 (d, J = 5.7 Hz, 1H), 4.02 (t, J = 9.0 Hz, 2H), 3.70 (ddd, J = 20.7, 15.2, 7.7 Hz, 4H), 3.48 (s, 4H), 2.95 - 2.68 (m, 4H), 2.20 (t, J = 7.2 Hz, 3H), 2.06 (d, J = 6.1 Hz, 4H), 1.64 (dd, J = 14.8, 7.4 Hz, 4H), 0.91 (t, J = 7.4 Hz, 4H).

LC-MS (ESI) *m*/*z* = 409.9 [M+H]⁺.

### Step 4: Preparation of (OBD-004):

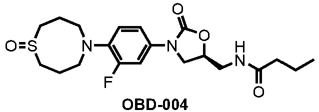

To a solution of (*S*)-N-((3-(3-fluoro-4-(1,5-thiazocan-5-yl)phenyl)-2-oxooxazolidin-5-yl)methyl)buty ramide (OBD-003) (150 mg, 0.37 mmol) in THF (10 mL) and 10 drops water was added potassium peroxomonosulfate (225 mg, 0.37 mmol) at 0 °C, then the reaction mixture was stirred at 0 °C for 2 h, monitored by TLC. Quenched with sodium thiosulfate, and the crude material was purified by prep-HPLC to afford (OBD-004) (48 mg, 31%) as a white solid.

¹H NMR (301 MHz, CDCl₃) δ 7.44 (dd, J = 14.7, 2.4 Hz, 1H), 7.19 - 6.99 (m, 2H), 6.44 (s, 1H), 4.84 - 4.71 (m, 1H), 4.02 (t, J = 8.9 Hz, 1H), 3.78 (dd, J = 9.0, 6.6 Hz, 1H), 3.66 (t, J = 4.6 Hz, 2H), 3.38 - 3.09 (m, 6H), 2.99 (dd, J = 12.6, 6.3 Hz, 2H), 2.20 (dd, J = 9.4, 5.3 Hz, 6H), 1.71 - 1.56 (m, 2H), 0.91 (dd, J = 9.6, 5.1 Hz, 3H).

LC-MS (ESI) *m*/*z* = 425.8 [M+H]⁺.

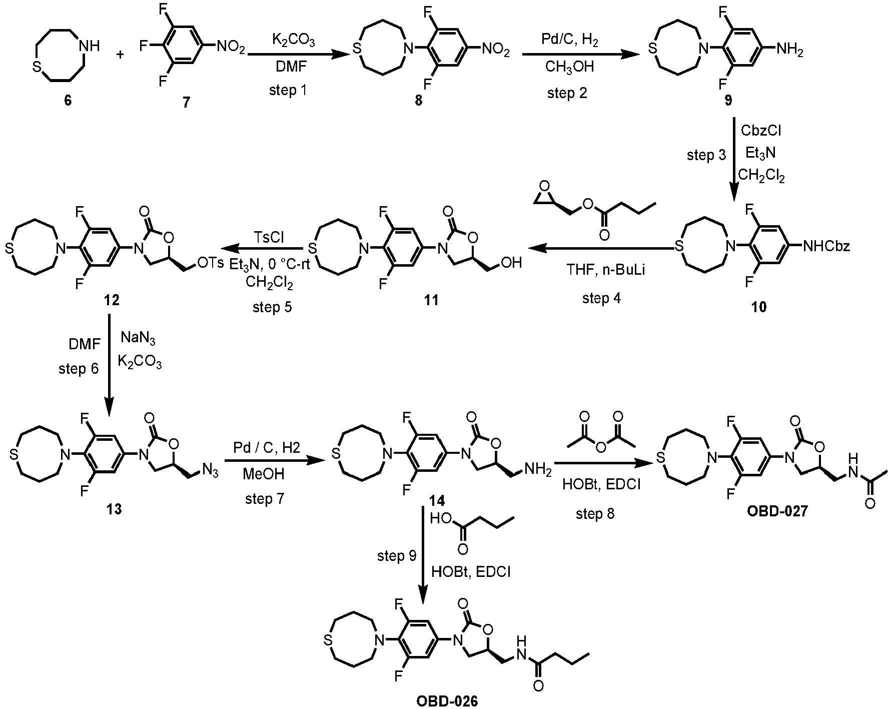

### Step 1: Preparation of 5-(2,6-difluoro-4-nitrophenyl)-1,5-thiazocane (8):

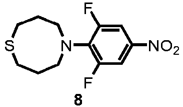

To a solution of 1,5-thiazocane (6) (1 g, 7.6 mmol) and 1,2,3-trifluoro-5-nitrobenzene (1.35 g, 7.6 mmol) in DMF (10 mL) was added K₂CO₃ (2.1 g, 15.2 mmol) at 25°C then the reaction mixture was stirred at 80°C for 2 h under a nitrogen gas atmosphere, monitored by TLC. The mixture was concentrated under reduced pressure, and the crude material was purified by silica gel column chromatography (EA: PE = 3: 1) to afford 5-(2,6-difluoro-4-nitrophenyl)-1,5-thiazocane (8) (1.64 g, 75%) as a yellow solid.

LC-MS (ESI) *m*/*z* = 289 [M+H]⁺.

### Step 2: 3,5-Difluoro-4-(1,5-thiazocan-5-yl)benzenamine (9):

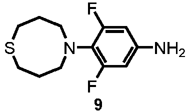

To a solution of 5-(2,6-difluoro-4-nitrophenyl)-1,5-thiazocane (8) (1.64 g, 5.7 mmol) and Palladium carbon (200 mg) in MeOH (15 mL), then under a hydrogen gas atmosphere and the reaction mixture was stirred at room temperature for overnight, monitored by TLC. The filtrate was concentrated under reduced pressure to afford 3,5-difluoro-4-(1,5-thiazocan-5-yl)benzenamine (9) (1.4 g, 94%) as a white oil, and the crude material was used for next reaction without further purification.

LC-MS (ESI) *m*/*z* = 259 [M+H]⁺.

### Step 3: Benzyl 3,5-difluoro-4-(1,5-thiazocan-5-yl)phenylcarbamate (10):

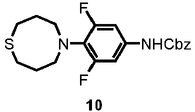

Benzyl carbonochloridate (1.87 g, 10.6 mmol) was added to a suspension of 3,5-difluoro-4-(1,5-thiazocan-5-yl)benzenamine (9) (1.4 g, 5.3 mmol) and triethylamine (1.07 g, 10.6 mmol) in DCM (200 mL) at -20 °C under a nitrogen gas atmosphere, then reaction mixture was stirred at 0 °C for 30 min, monitored by TLC. Quenched with ammonium chloride, extracted with DCM, the organic layer was concentrated under reduced pressure, and the crude material was purified by silica gel column chromatography (EA: PE = 10: 1) to afford benzyl 3,5-difluoro-4-(1,5-thiazocan-5-yl)phenylcarbamate (10) (872 mg, 42%) as a white solid.

LC-MS (ESI) *m*/*z* = 393 [M+H]⁺.

### Step 4:

### (R)-3-(3,5-difluoro-4-(1,5-thiazocan-5-yl)phenyl)-5-(hydroxymethyl)oxazolidin-2-o ne (11):

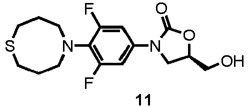

To a solution of benzyl 3,5-difluoro-4-(1,5-thiazocan-5-yl)phenylcarbamate (10) (872 mg, 2.23 mmol) in THF (10 mL) at - 78 °C under a nitrogen gas atmosphere was added n-BuLi (1.4 ml, 3.34 mmol), then the mixture was stirred at -78°C for 30 min, after that the solution of (R)-oxiran-2-ylmethyl butyrate (480 mg, 3.34 mmol) in THF was added to the mixture at -78°C, then warmed to room temperature and stirred for overnight, monitored by TLC. Quenched with ammonium chloride, extracted with EA, the organic layer was concentrated under reduced pressure, and the crude material was purified by silica gel column chromatography (DCM: MeOH = 70: 1) to afford (*R*)-3-(3,5-difluoro-4-(1,5-thiazocan-5-yl)phenyl)-5-(hydroxymethyl)oxazolidin-2-o ne (11) (519 mg, 65%) as a white solid.

LC-MS (ESI) *m*/*z* = 359 [M+H]⁺.

### Step 5:

### (R)-(3-(3,5-difluoro-4-(1,5-thiazocan-5-yl)phenyl)-2-oxooxazolidin-5-yl)methyl 4-methylbenzenesulfonate (12):

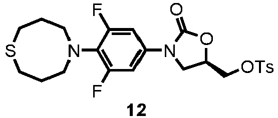

4-methylbenzene-1-sulfonyl chloride (550 mg, 2.9 mmol) was added to a suspension of (*R*)-3-(3,5-difluoro-4-(1,5-thiazocan-5-yl)phenyl)-5-(hydroxymethyl)oxazolidin-2-o ne (11) (519 mg, 1.45 mmol) and Et₃N (292 mg, 2.9 mmol) in DCM (10 mL) at 0 °C under a nitrogen gas atmosphere and the reaction mixture was stirred at room temperature for overnight, monitored by TLC. Quenched with ammonium chloride, extracted with DCM, the organic layer was concentrated under reduced pressure, and the crude material was purified by silica gel column chromatography (DCM: MeOH = 50: 1) to afford (*R*)-(3-(3,5-difluoro-4-(1,5-thiazocan-5-yl)phenyl)-2-oxooxazolidin-5-yl)methyl 4-methylbenzenesulfonate (12) (594 mg, 80%) as a white solid.

LC-MS (ESI) *m*/*z* = 513 [M+H]⁺.

### Step 6:

### (R)-5-(azidomethyl)-3-(3,5-difluoro-4-(1,5-thiazocan-5-yl)phenyl)oxazolidin-2-one (13):

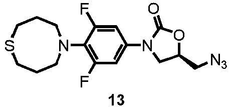

To a solution of (*R*)-(3-(3,5-difluoro-4-(1,5-thiazocan-5-yl)phenyl)-2-oxooxazolidin-5-yl)methyl 4-methylbenzenesulfonate (12) (594 g, 1.2 mmol) and sodium azide (75 mg, 1.2 mmol) in DMF (10 mL) was added K₂CO₃ (160 mg, 2.4 mmol) at 25°C, then the reaction mixture was stirred at 80°C for 1 h under a nitrogen gas atmosphere, monitored by TLC. Quenched with ammonium chloride, extracted with EA, the organic layer was concentrated under reduced pressure, and the crude material was purified by silica gel column chromatography (PE: EA = 2: 1) to afford (*R*)-5-(azidomethyl)-3-(3,5-difluoro-4-(1,5-thiazocan-5-yl)phenyl)oxazolidin-2-one (13) (400 mg, 87%) as a white solid.

LC-MS (ESI) *m*/*z* = 384 [M+H]⁺.

### Step 7:

### (S)-5-(aminomethyl)-3-(3,5-difluoro-4-(1,5-thiazocan-5-yl)phenyl)oxazolidin-2-one (14):

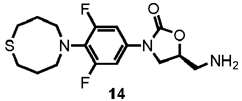

To a solution of (*R*)-5-(azidomethyl)-3-(3,5-difluoro-4-(1,5-thiazocan-5-yl)phenyl)oxazolidin-2-one (13) (400 g, 1.04 mmol) in MeOH (10 mL) was added palladium carbon (100 mg) at 25°C, then the reaction mixture was stirred at room temperature for overnight under a hydrogen gas atmosphere, monitored by TLC. The filtrate was concentrated under reduced pressure, and the crude material was purified by silica gel column chromatography (DCM: MeOH = 50: 1) to afford (*S*)-5-(aminomethyl)-3-(3,5-difluoro-4-(1,5-thiazocan-5-yl)phenyl)oxazolidin-2-one (14) (331 g, 89 %) as a white solid.

LC-MS (ESI) *m*/*z* = 358 [M+H]⁺.

### Step 8:

### (S)-N-((3-(3,5-difluoro-4-(1,5-thiazocan-5-yl)phenyl)-2-oxooxazolidin-5-yl)methyl) butyramide (OBD-026):

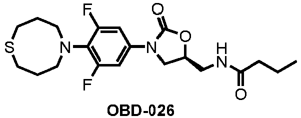

To a solution of (*S*)-5-(aminomethyl)-3-(3,5-difluoro-4-(1,5-thiazocan-5-yl)phenyl)oxazolidin-2-one (14) (165 mg, 0.46 mmol) and butyric acid (52 mg, 0.46 mmol) in DCM (10 mL) were added HOBt (95 mg, 0.7 mmol), EDCI (175 mg, 0.92 mmol) and DIPEA (118 mg, 0.92 mmol) at 25 °C, then the reaction mixture was stirred at 25 °C for 2 h under a nitrogen gas atmosphere, monitored by TLC. Quenched with ammonium chloride, extracted with DCM, the organic layer was concentrated under reduced pressure, and the crude material was purified by silica gel column chromatography (DCM: MeOH = 80: 1) to afford (*S*)-N-((3-(3,5-difluoro-4-(1,5-thiazocan-5-yl)phenyl)-2-oxooxazolidin-5-yl)methyl) butyramide (OBD-026) (82 mg, 42%) as a white solid.

¹H NMR (400 MHz, CDCl₃) δ 8.02 (s, 1H), 7.14 (d, *J* = 11.1 Hz, 1H), 5.93 (s, 1H), 4.81 (s, 1H), 4.02 (t, *J* = 8.9 Hz, 1H), 3.70 (s, 2H), 3.31 (s, 3H), 2.87 (s, 2H), 2.22 (s, 2H), 1.90 (s, 5H), 1.66 (d, *J* = 7.3 Hz, 2H), 0.93 (t, *J* = 7.4 Hz, 2H).

LC-MS (ESI) m/z = 428 [M+H]⁺.

### Step 9:

### N-(((S)-3-(4-(3-thia-8-aza-bicyclo[3.2.1]octan-8-yl)-3,5-difluorophenyl)-2-oxooxazo lidin-5-yl)methyl)butyramide (OBD-027):

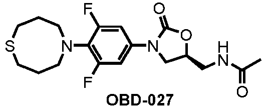

To a solution of (*S*)-5-(aminomethyl)-3-(3,5-difluoro-4-(1,5-thiazocan-5-yl)phenyl)oxazolidin-2-one (14) (165 mg, 0.46 mmol) and butyric acid (52 mg, 0.46 mmol) in DCM (10 mL) were added HOBt (95 mg, 0.7 mmol), EDCI (175 mg, 0.92 mmol) and DIPEA (118 mg, 0.92 mmol) at 25 °C, then the reaction mixture was stirred at 25 °C for 2 h under a nitrogen gas atmosphere, monitored by TLC. Quenched with ammonium chloride, extracted with DCM, the organic layer was concentrated under reduced pressure, and the crude material was purified by silica gel column chromatography (DCM: MeOH = 80: 1) to afford N-(((*S*)-3-(4-(3-thia-8-aza-bicyclo[3.2.1]octan-8-yl)-3,5-difluorophenyl)-2-oxooxazo lidin-5-yl)methyl)butyramide (OBD-027) (82 mg, 45%) as a white solid.

¹H NMR (301 MHz, CDCl₃) δ 7.23 - 6.85 (m, 2H), 6.00 (s, 1H), 4.90 - 4.66 (m, 1H), 4.17 - 3.86 (m, 1H), 3.71 (s, 2H), 3.28 (s, 2H), 2.84 (s, 3H), 2.04 (s, 3H), 1.88 (s, 5H).

LC-MS (ESI) m/z = 400 [M+H]⁺.

### The synthesis route:

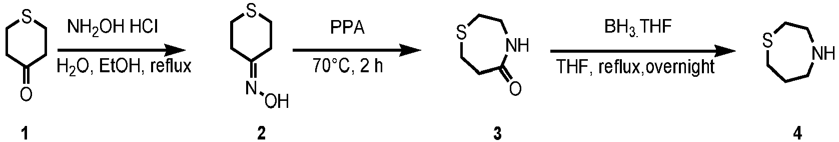

### Experimental

Tetrahydrothiopyran-4-one oxime (2).

To a solution of tetrahydrothiopyran-4-one (20 g, 172 mmol) in EtOH (150 mL) and H₂O (50 mL) was added with NH₂OH-HCl (11.9 g, 172 mmol), then the reaction mixture was stirred at 75 °C for 4 h under a nitrogen gas atmosphere, then mixture was concentrated and dried to give tetrahydrothiopyran-4-one oxime (2) (14.7 g, 66%) as brown solid.

1,4-Thiazepan-5-one (3).

A mixture of tetrahydrothiopyran-4-one oxime (2) (14.7 g, 112 mmol) and polyphosphoric acid (50 g) was stirred at 70 °C for 2 h. The reaction mixture was cooled to room temperature and poured into ice water, adjusted pH = 8 using potassium carbonate solution, extracted with EA, the organic layer was concentrated under reduced pressure to afford 1,4-thiazepan-5-one (3) (11.9 g crude, 81%) as brown solid.

1,4-Thiazepane (4).

To a solution of 1,4-thiazepan-5-one (3) (11.9 g, 105 mmol) in THF (100 mL) was added BH₃(158 mL, 158 mol) in THF at 0 °C, followed by refluxing for 12 h. The reaction was quenched with CH₃OH (50 mL). The solvent was evaporated to afford 1,4-thiazepane (4) as a white oil (10.7 g, 87%), and the crude material was used for next reaction without further purification.

LC-MS (ESI) *m*/*z* = 118 [M+H]⁺.

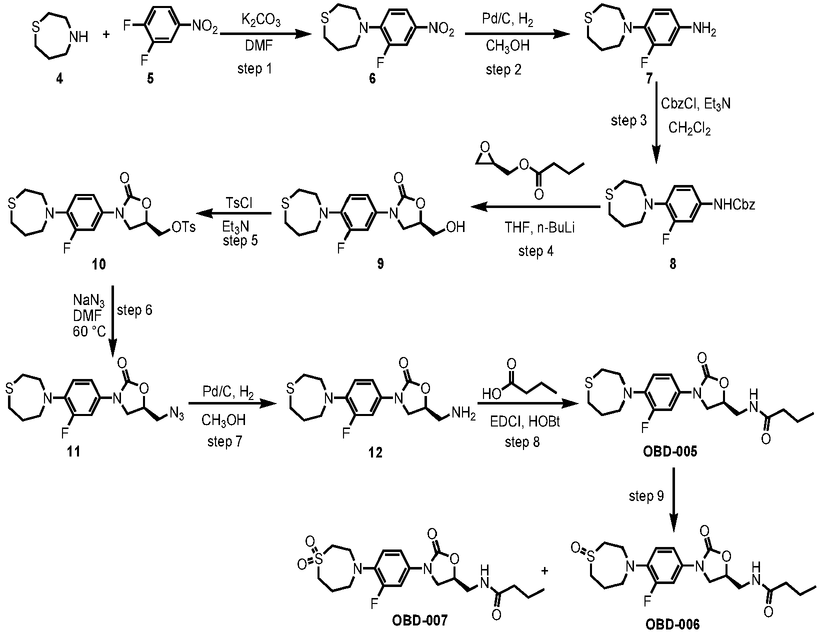

### Step 1: Preparation of 4-(2-fluoro-4-nitrophenyl)-1,4-thiazepane (6):

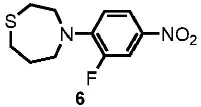

To a solution of 1,4-thiazepane (4) (7 g, 59.8 mmol) and 1,2-difluoro-4-nitrobenzene (10.4 g, 65.8 mmol) in DMF (10 mL) was added K₂CO₃ (16.5 g, 119.6 mmol) at 25°C and then reaction mixture was stirred at 80°C for 2 h under a nitrogen gas atmosphere, monitored by TLC. The mixture was concentrated under reduced pressure, and the crude material was purified by silica gel column chromatography (EA: PE = 3: 1) to afford 4-(2-fluoro-4-nitrophenyl)-1,4-thiazepane (6) (10 g, 65%) as a yellow solid.

LC-MS (ESI) *m*/*z* = 257 [M+H]⁺.

### Step 2: 3-Fluoro-4-(1,4-thiazepan-4-yl)benzenamine (7):

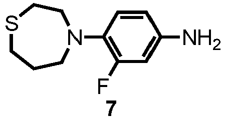

To a solution of 4-(2-fluoro-4-nitrophenyl)-1,4-thiazepane (6) (8 g, 31.2 mmol) and Palladium carbon (500 mg) in MeOH (15 mL), then the reaction mixture was stirred at room temperature for overnight under a hydrogen gas atmosphere, monitored by TLC. The filtrate was concentrated under reduced pressure to afford 3-fluoro-4-(1,4-thiazepan-4-yl)benzenamine (7) (6.4 g, 93%) as a white oil, and the crude material was used for next reaction without further purification.

LC-MS (ESI) *m*/*z* = 227 [M+H]⁺.

### Step 3: Benzyl 3-fluoro-4-(1,4-thiazepan-4-yl)phenylcarbamate (8):

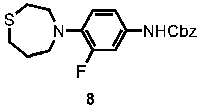

Benzyl carbonochloridate (9.6 g, 56.6 mmol) was added to a suspension of 3-fluoro-4-(1,4-thiazepan-4-yl)benzenamine (7) (6.4 g, 28.3 mmol) and triethylamine (5.7 g, 56.6 mmol) in DCM (200 mL) at -20 °C under a nitrogen gas atmosphere, then reaction mixture was stirred at 0 °C for 30 min, monitored by TLC. Quenched with ammonium chloride, extracted with DCM, the organic layer was concentrated under reduced pressure, and the crude material was purified by silica gel column chromatography (EA: PE = 10: 1) to afford benzyl 3-fluoro-4-(1,4-thiazepan-4-yl)phenylcarbamate (8) (2.34 g, 23%) as a white oil.

LC-MS (ESI) *m*/*z* = 361 [M+H]⁺.

### Step 4:

### (R)-3-(3-Fluoro-4-(1,4-thiazepan-4-yl)phenyl)-5-(hydroxymethyl)oxazolidin-2-one (9):

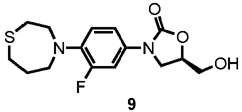

To a solution of benzyl 3-fluoro-4-(1,4-thiazepan-4-yl)phenylcarbamate (8) (2.34 g, 6.5 mmol) in THF (10 mL) at - 78 °C under a nitrogen gas atmosphere was added n-BuLi (4 ml, 9.7 mmol), then the mixture was stirred at -78°C for 30 min, after that the solution of (R)-oxiran-2-ylmethyl butyrate (1.4 g, 9.7 mmol) in THF was added to the mixture at -78°C, then warmed to room temperature and stirred for overnight, monitored by TLC. Quenched with ammonium chloride, extracted with EA, the organic layer was concentrated under reduced pressure, and the crude material was purified by silica gel column chromatography (DCM: MeOH = 70: 1) to afford (*R*)-3-(3-fluoro-4-(1,4-thiazepan-4-yl)phenyl)-5-(hydroxymethyl)oxazolidin-2-one (9) (1.16 g, 55%) as a white solid.

LC-MS (ESI) *m*/*z* = 327 [M+H]⁺.

### Step 5: (R)-(3-(3-Fluoro-4-(1,4-thiazepan-4-yl)phenyl)-2-oxooxazolidin-5-yl)methyl 4-methylbenzenesulfonate (10):

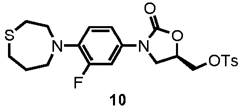

4-Methylbenzene-1-sulfonyl chloride (1.4 g, 7.2 mmol) was added to a suspension of (*R*)-3-(3-fluoro-4-(1,4-thiazepan-4-yl)phenyl)-5-(hydroxymethyl)oxazolidin-2-one (9) (1.16 g, 3.6 mmol) and Et₃N (727 mg, 7.2 mmol) in DCM (10 mL) at 0 °C under a nitrogen gas atmosphere and the reaction mixture was stirred at room temperature for overnight, monitored by TLC. Quenched with ammonium chloride, extracted with DCM, the organic layer was concentrated under reduced pressure, and the crude material was purified by silica gel column chromatography (DCM: MeOH = 50: 1) to afford (*R*)-(3-(3-fluoro-4-(1,4-thiazepan-4-yl)phenyl)-2-oxooxazolidin-5-yl)methyl 4-methylbenzenesulfonate (10) (1.41 g, 41%) as a white solid.

LC-MS (ESI) *m*/*z* = 481 [M+H]⁺.

### Step 1:

### (R)-5-(azidomethyl)-3-(3-fluoro-4-(1,4-thiazepan-4-yl)phenyl)oxazolidin-2-one (11):

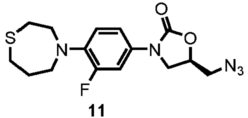

To a solution of (*R*)-(3-(3-fluoro-4-(1,4-thiazepan-4-yl)phenyl)-2-oxooxazolidin-5-yl)methyl 4-methylbenzenesulfonate (10) (1.41 g, 2.95 mmol) and sodium azide (190 mg, 2.95 mmol) in DMF (10 mL) was added K₂CO₃ (814 g, 5.9 mmol) at 25°C, then the reaction mixture was stirred at 80°C for 1 h under a nitrogen gas atmosphere, monitored by TLC. Quenched with ammonium chloride, extracted with EA, the organic layer was concentrated under reduced pressure, and the crude material was purified by silica gel column chromatography (PE: EA = 2: 1) to afford (*R*)-5-(azidomethyl)-3-(3-fluoro-4-(1,4-thiazepan-4-yl)phenyl)oxazolidin-2-one (11) (830 mg, 80%) as a white solid.

LC-MS (ESI) *m*/*z* = 352 [M+H]⁺.

### Step 2:

### (S)-5-(aminomethyl)-3-(3-fluoro-4-(1,4-thiazepan-4-yl)phenyl)oxazolidin-2-one (12):

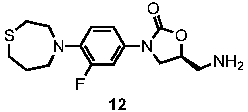

To a solution of (*R*)-5-(azidomethyl)-3-(3-fluoro-4-(1,4-thiazepan-4-yl)phenyl)oxazolidin-2-one (11) (830 mg, 2.4 mmol) in MeOH (10 mL) was added palladium carbon (100 mg) at 25°C, then the reaction mixture was stirred at room temperature for overnight under a hydrogen gas atmosphere, monitored by TLC. The filtrate was concentrated under reduced pressure, and the crude material was purified by silica gel column chromatography (DCM: MeOH = 50: 1) to afford (*S*)-5-(aminomethyl)-3-(3-fluoro-4-(1,4-thiazepan-4-yl)phenyl)oxazolidin-2-one (12) (654 mg, 85%) as a white solid.

LC-MS (ESI) *m*/*z* = 326 [M+H]⁺.

### Step 3:

### (S)-N-((3-(3-fluoro-4-(1,4-thiazepan-4-yl)phenyl)-2-oxooxazolidin-5-yl)methyl)buty ramide (OBD-005):

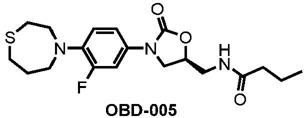

To a solution of (*S*)-5-(aminomethyl)-3-(3-fluoro-4-(1,4-thiazepan-4-yl)phenyl)oxazolidin-2-one (12) (654 mg, 2 mmol) and butyric acid (177 mg, 2 mmol) in DCM (10 mL) were added HOBt (405 mg, 3 mmol), EDCI (764 mg, 4 mmol) and DIPEA (516 mg, 4 mmol) at 25 °C, then the reaction mixture was stirred at 25 °C for 2 h under a nitrogen gas atmosphere, monitored by TLC. Quenched with ammonium chloride, extracted with DCM, the organic layer was concentrated under reduced pressure, and the crude material was purified by silica gel column chromatography (DCM: MeOH = 80: 1) to afford (*S*)-N-((3-(3-fluoro-4-(1,4-thiazepan-4-yl)phenyl)-2-oxooxazolidin-5-yl)methyl)buty ramide (OBD-005) (286 mg, 34%) as a white solid.

¹H NMR (300 MHz, CDCl₃) δ 7.42 - 7.23 (m, 2H), 7.01 (dd, J = 8.9, 2.3 Hz, 1H), 6.04 (s, 1H), 4.75 (ddd, J = 9.0, 7.9, 4.6 Hz, 1H), 4.00 (t, J = 9.0 Hz, 1H), 3.79 - 3.05 (m, 7H), 2.91 (dd, J = 16.2, 10.1 Hz, 2H), 2.70 (t, J = 6.3 Hz, 2H), 2.28 - 2.13 (m, 2H), 2.13 - 1.97 (m, 2H), 1.82 - 1.25 (m, 3H), 0.92 (t, J = 7.4 Hz, 3H), 0.01 (s, 1H).

LC-MS (ESI) *m*/*z* = 395.9 [M+H]⁺.

### Step 4: Preparation of (OBD-006 and OBD-007):

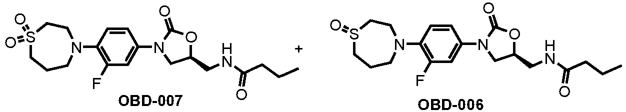

To a solution of (*S*)-N-((3-(3-fluoro-4-(1,4-thiazepan-4-yl)phenyl)-2-oxooxazolidin-5-yl)methyl)buty ramide (OBD-005) (150 mg, 0.38 mmol) in THF (10 mL) and 10 drops water was added potassium peroxomonosulfate (233 mg, 0.38 mmol) at 0 °C, then the reaction mixture was stirred at 0 °C for 2 h, monitored by TLC. Quenched with sodium thiosulfate, and the crude material was purified by prep-HPLC to afford (OBD-006) (50 mg, 32%) as a white solid and (OBD-007) (24 mg, 15%) as a white solid.

### (OBD-006)

¹H NMR (301 MHz, CDCl₃) δ 7.52 (d, J = 15.0 Hz, 1H), 7.16 (s, 1H), 7.03 (d, J = 8.7 Hz, 1H), 5.99 (s, 1H), 4.78 (s, 1H), 4.02 (t, J = 8.8 Hz, 2H), 3.88 - 3.56 (m, 3H), 3.55 - 2.92 (m, 7H), 2.77 (s, 1H), 2.20 (t, J = 7.1 Hz, 3H), 1.64 (dd, J = 14.9, 7.4 Hz, 2H), 0.91 (t, J = 7.4 Hz, 3H).

LC-MS (ESI) *m*/*z* = 411.8 [M+H]⁺.

### (OBD-007)

¹H NMR (301 MHz, CDCl₃) δ 7.51 (d, J = 14.7 Hz, 1H), 7.10 (d, J = 9.9 Hz, 2H), 5.92 (s, 1H), 4.78 (s, 1H), 4.03 (t, J = 9.0 Hz, 1H), 3.87 - 3.39 (m, 7H), 3.27 (d, J = 5.7 Hz, 2H), 2.39 (d, J = 6.2 Hz, 2H), 2.20 (t, J = 7.2 Hz, 2H), 1.64 (dd, J = 14.8, 7.4 Hz, 2H), 0.92 (t, J = 7.3 Hz, 3H).

LC-MS (ESI) *m*/*z* = 427.8 [M+H]⁺.

### Procedures for preparation of R:

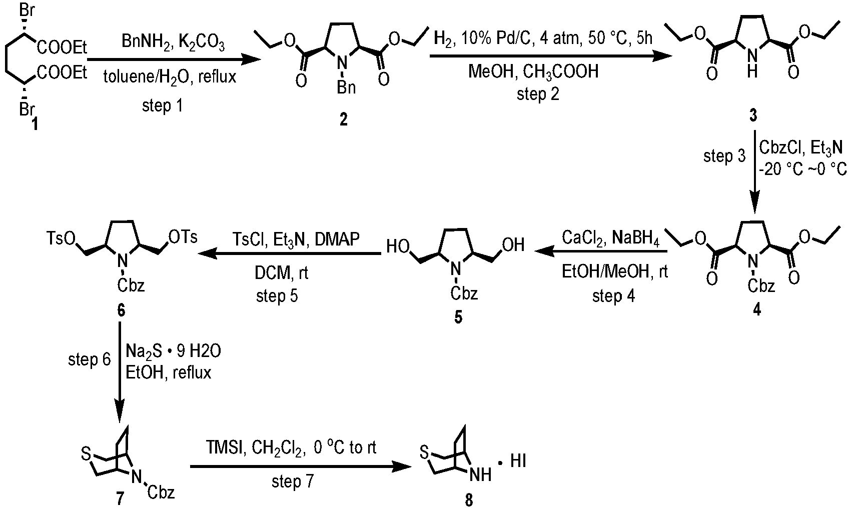

### Step 1: Preparation of (2S,5R)-diethyl 1-benzylpyrrolidine-2,5-dicarboxylate (2):

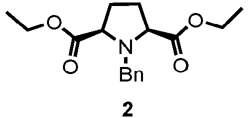

The mixture of (2*R*,5*S*)-diethyl 2,5-dibromohexanedioate (1) (100 g, 278 mmol), BnNH₂ (44.6 g, 416 mmol) and K₂CO₃ (76.84 g, 556 mmol) in toluene/H₂O was stirred at 110°C for overnight, monitored by TLC. The mixture was extracted with EtOAc, washed with water and brine and then dried over anhydrous sodium sulfate, filtered, and concentrated under reduced pressure to afford (2S,5R)-diethyl 1-benzylpyrrolidine-2,5-dicarboxylate (2) (63.75 g, 75%) as a white oil.

LC-MS (ESI) *m*/*z* = 306 [M+H]⁺.

### Step 2: (2S,5R)-diethyl pyrrolidine-2,5-dicarboxylate (3):

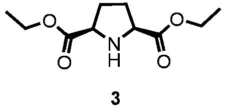

To a solution of (2*S*,5*R*)-diethyl 1-benzylpyrrolidine-2,5-dicarboxylate (2) (63.75 g, 209 mmol) and Palladium carbon (2 g) in MeOH (15 mL) was added CH₃COOH (5 mL), then the reaction mixture was stirred at 50°C under a hydrogen gas atmosphere, 4atm for 5 h, monitored by TLC. The filter was concentrated under reduced pressure, and the crude material was used for next reaction without further purification.

LC-MS (ESI) *m*/*z* = 216 [M+H]⁺.

### Step 3: (2S,5R)-1-benzyl 2,5-diethyl pyrrolidine-1,2,5-tricarboxylate (4):

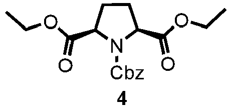

To a solution of (2*S*,5*R*)-diethyl pyrrolidine-2,5-dicarboxylate (3) (62 g, 288 mmol) and Et₃N (58 g, 577 mmol) in DCM (100 mL) was added benzyl carbonochloridate (98 g, 577 mmol) at -20 °C under a nitrogen gas atmosphere, then the reaction mixture was stirred at rt for 5 h, monitored by TLC. The mixture was extracted with DCM, washed with water and brine and then dried over anhydrous sodium sulfate, filtered, and concentrated under reduced pressure to afford (2S,5R)-1-benzyl 2,5-diethyl pyrrolidine-1,2,5-tricarboxylate (4) (80 g, 80%) as a white oil.

LC-MS (ESI) *m*/*z* = 350 [M+H]⁺.

### Step 4: (2S,5R)-benzyl 2,5-bis(hydroxymethyl)pyrrolidine-1-carboxylate (5):

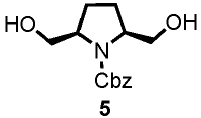

CaCl₂ (76 g, 688 mmol) and NaBH₄ (43 g, 1146 mmol) were added to a stirred solution of (2*S*,5*R*)-1-benzyl 2,5-diethyl pyrrolidine-1,2,5-tricarboxylate (4) (80 g, 229 mmol) in EtOH-MeOH (9:1; 200 mL) at rt under a nitrogen gas atmosphere, then the reaction mixture was stirred for 5 h, monitored by TLC. H₂O (5 mL) was added, and the mixture was stirred tor a turther 15 min. The mixture was then concentrated in vacuo. The mixture was extracted with EtOAc, then dried over anhydrous sodium sulfate, filtered, and concentrated under reduced pressure to afford (2*S*,5*R*)-benzyl 2,5-bis(hydroxymethyl)pyrrolidine-1-carboxylate (5) (32 g, 52%) as a white oil.

LC-MS (ESI) *m*/*z* = 266 [M+H]⁺.

### Step 5: (2S,5R)-benzyl 2,5-bis(tosyloxymethyl)pyrrolidine-1-carboxylate (6):

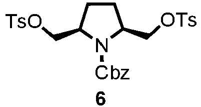

4-methylbenzene-1-sulfonyl chloride (92 g, 483 mmol) was added to a stirred solution of (2S,5R)-benzyl 2,5-bis(hydroxymethyl)pyrrolidine-1-carboxylate (5) (32 g, 121 mmol) and Et₃N in DCM(200 mL) at 0 °C under a nitrogen gas atmosphere, then the reaction mixture was allowed to warm to room temperature and stirred for 5 h, monitored by TLC. The mixture was extracted with DCM, then dried over anhydrous sodium sulfate, filtered, and concentrated under reduced pressure, and the crude material was purified by silica gel column chromatography (EA: PE = 2: 1) to afford (2*S*,5*R*)-benzyl 2,5-bis(tosyloxymethyl)pyrrolidine-1-carboxylate (6) (30 g, 43%) as a white solid.

LC-MS (ESI) *m*/*z* = 574 [M+H]⁺.

### Step 6: 8-benzyl-3-thia-8-aza-bicyclo[3.2.1]octane-1-carboxylate (7):

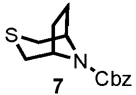

Sodium sulfide hydrate (38 g, 157 mmol) was added to a stirred solution of (2*S*,5*R*)-benzyl 2,5-bis(tosyloxymethyl)pyrrolidine-1-carboxylate (6) (30 g, 50 mmol) in EtOH (50 mL) and water (50 mL) at room temperature, then the reaction mixture was stirred at 90 °C for 2 h, monitored by TLC. The mixture was extracted with DCM, then dried over anhydrous sodium sulfate, filtered, and concentrated under reduced pressure, and the crude material was purified by silica gel column chromatography (EA: PE = 10: 1) to afford 8-benzyl-3-thia-8-aza-bicyclo[3.2.1]octane-1-carboxylate (7) (10 g, 73%) as a white solid.

LC-MS (ESI) *m*/*z* = 264 [M+H]⁺.

### Step 7: 3-thia-8-aza-bicyclo[3.2.1]octane hydrogen iodide (8):

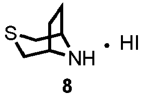

Iodotrimethylsilane (15 g, 75 mmol) was added to a stirred solution of 8-benzyl-3-thia-8-aza-bicyclo[3.2.1]octane-1-carboxylate (7) (10 g, 38 mmol) in DCM (200 mL) at 0 °C under a nitrogen gas atmosphere, then the reaction mixture was allowed to warm to room temperature and stirred for 30 min, monitored by TLC.

The mixture was extracted with DCM, then dried over anhydrous sodium sulfate, filtered, and concentrated under reduced pressure, and the crude material was purified by silica gel column chromatography (DCM: MeOH = 15: 1) to afford 3-thia-8-aza-bicyclo[3.2.1]octane hydrogen iodide (8) (8.13 g, 84%) as a brown solid.

LC-MS (ESI) *m*/*z* = 130 [M+H]⁺

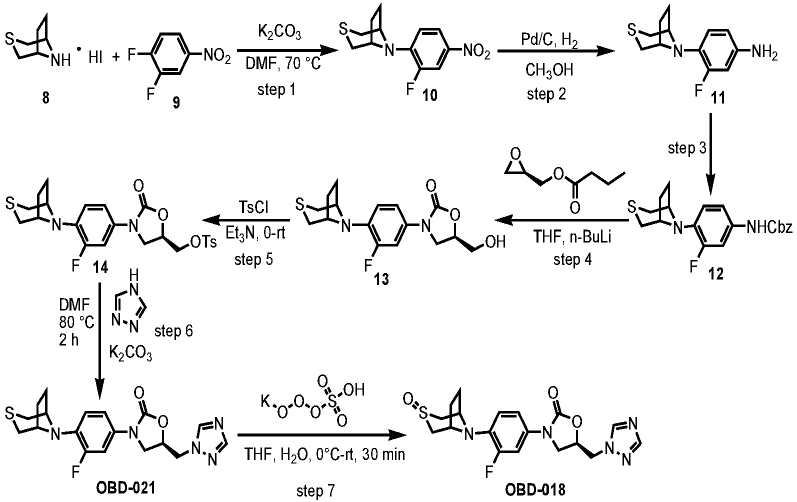

### Step 1: Preparation of 8-(2-fluoro-4-nitrophenyl)-3-thia-8-aza-bicyclo[3.2.1]octane (10):

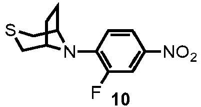

To a solution of 3-thia-8-aza-bicyclo[3.2.1]octane hydrogen iodide (8) (5.58 g, 21.7 mmol) and 1,2-difluoro-4-nitrobenzene (3.8 g, 23.8 mmol) in DMF (10 mL) was added K₂CO₃ (6 g, 43.4 mmol) at 25°C under a nitrogen gas atmosphere and the reaction mixture was stirred at 80°C for 2 h, monitored by TLC. The mixture was concentrated under reduced pressure, and the crude material was purified by silica gel column chromatography (EA: PE = 3: 1) to afford 8-(2-fluoro-4-nitrophenyl)-3-thia-8-aza-bicyclo[3.2.1]octane (10) (4.89 g, 84%) as a yellow solid.

LC-MS (ESI) *m*/*z* = 269 [M+H]⁺.

### Step 2: 4-(3-thia-8-aza-bicyclo[3.2.1]octan-8-yl)-3-fluorobenzenamine (11):

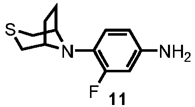

To a solution of 8-(2-fluoro-4-nitrophenyl)-3-thia-8-aza-bicyclo[3.2.1]octane (10) (4.89 g, 18.2 mmol) and Palladium carbon (200 mg) in MeOH (15 mL), then under a hydrogen gas atmosphere and the reaction mixture was stirred at room temperature for overnight, monitored by TLC. The filtrate was concentrated under reduced pressure to afford 4-(3-thia-8-aza-bicyclo[3.2.1]octan-8-yl)-3-fluorobenzenamine (11) (4.08 g, 94%) as a white oil, and the crude material was used for next reaction without further purification.

LC-MS (ESI) *m*/*z* = 239 [M+H]⁺.

### Step 3: Benzyl 4-(3-thia-8-aza-bicyclo[3.2.1]octan-8-yl)-3-fluorophenylcarbamate (12):

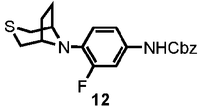

Carbonic acid, 2,5-dioxo-1-pyrrolidinyl phenylmethyl ester (6.38 g, 25.6 mmol) was added to a suspension of 4-(3-thia-8-aza-bicyclo[3.2.1]octan-8-yl)-3-fluorobenzenamine (11) (4.08 g, 17.1 mmol) in THF (30 mL) at 0 °C under a nitrogen gas atmosphere and the reaction mixture was stirred at 50°C for 5 h, monitored by TLC. The mixture was concentrated under reduced pressure, and the crude material was purified by silica gel column chromatography (EA: PE = 10: 1) to afford benzyl 4-(3-thia-8-aza-bicyclo[3.2.1]octan-8-yl)-3-fluorophenylcarbamate (12) (4.34 g, 68%) as a white solid.

LC-MS (ESI) *m*/*z* = 373 [M+H]⁺.

### Step 4: (5R)-3-(4-(3-thia-8-aza-bicyclo[3.2.1]octan-8-yl)-3-fluorophenyl)-5-(hydroxymethyl)oxazolidin-2-one (13):

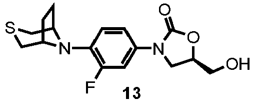

To a solution of benzyl 4-(3-thia-8-aza-bicyclo[3.2.1]octan-8-yl)-3-fluorophenylcarbamate (12) (4.34 g, 11.6 mmol) in THF (10 mL) at - 78 °C under a nitrogen gas atmosphere was added n-BuLi (7.3 ml, 17.5 mmol), then the mixture was stirred at -78°C for 30 min, after that the solution of (*R*)-oxiran-2-ylmethyl butyrate (2.5 g, 17.4 mmol) in THF was added to the mixture at -78°C, then warmed to room temperature and stirred for overnight, monitored by TLC. Quenched with ammonium chloride, extracted with EA, the organic layer was concentrated under reduced pressure, and the crude material was purified by silica gel column chromatography (DCM: MeOH = 70: 1) to afford (5*R*)-3-(4-(3-thia-8-aza-bicyclo[3.2.1] Octan-8-yl)-3-fluorophenyl)-5-(hydroxymethyl)oxazolidin-2-one (13) (3.03 g, 77%) as a white solid.

LC-MS (ESI) *m*/*z* = 339 [M+H]⁺.

### Step 5:

### ((R)-3-(4-(3-thia-8-aza-bicyclo[3.2.1]octan-8-yl)-3-fluorophenyl)-2-oxooxazolidin -5-yl)methyl 4-methylbenzenesulfonate (14):

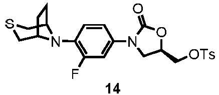

4-methylbenzene-1-sulfonyl chloride (3.41 g, 17.9 mmol) was added to a suspension of (5*R*)-3-(4-(3-thia-8-aza-bicyclo[3.2.1]octan-8-yl)-3-fluorophenyl)-5-(hydroxymethyl ) oxazolidin-2-one (13) (3.03 g, 8.9 mmol) and Et₃N (1.8 g, 17.9 mmol) in DCM (10 mL) at 0 °C under a nitrogen gas atmosphere and the reaction mixture was stirred at room temperature for overnight, monitored by TLC. Quenched with ammonium chloride, extracted with DCM, the organic layer was concentrated under reduced pressure, and the crude material was purified by silica gel column chromatography (DCM: MeOH = 50: 1) to afford ((R)-3-(4-(3-thia-8-aza-bicyclo[3.2.1]octan-8-yl)-3-fluorophenyl)-2-oxooxazolidin -5-yl)methyl 4-methylbenzenesulfonate (14) (3.74 g, 85%) as a white solid.

LC-MS (ESI) *m*/*z* = 493 [M+H]⁺.

### Step 6:

### (5R)-5-((1H-1,2,4-triazol-1-yl)methyl)-3-(4-(3-thia-8-aza-bicyclo[3.2.1]octan-8-yl) -3-fluorophenyl)oxazolidin-2-one (OBD-021):

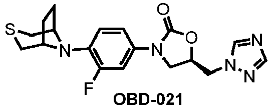

To a solution of ((*R*)-3-(4-(3-thia-8-aza-bicyclo[3.2.1]octan-8-yl)-3-fluorophenyl)-2-oxooxazolidin-5-yl)methyl 4-methylbenzenesulfonate (14) (500 mg, 1 mmol) and 1H-1,2,4-triazole (140 mg, 2 mmol) in DMF (10 mL) was added K₂CO₃ (280 mg, 2 mmol) at 25°C, then the reaction mixture was stirred at 80°C for 2 h under a nitrogen gas atmosphere, monitored by TLC. Quenched with ammonium chloride, extracted with EA, the organic layer was concentrated under reduced pressure, and the crude material was purified by silica gel column chromatography (EA: PE = 2: 1) to afford (5*R*)-5-((1H-1,2,4-triazol-1-yl)methyl)-3-(4-(3-thia-8-aza-bicyclo[3.2.1]octan-8-yl)-3-fluorophenyl)oxazolidin-2-one (OBD-021) (177 mg, 45%) as a white solid.

¹H NMR (300 MHz, DMSO-d₆) δ 8.57 (s, 1H), 8.01 (s, 1H), 7.36 (dd, J = 15.8, 2.1 Hz, 1H), 7.18 - 6.92 (m, 2H), 5.06 (dd, J = 8.9, 4.8 Hz, 1H), 4.72 - 4.52 (m, 2H), 4.36 (s, 2H), 4.17 (t, J = 9.1 Hz, 1H), 3.84 (dt, J = 49.3, 24.7 Hz, 1H), 3.12 (d, J = 12.8 Hz, 2H), 2.16 (s, 1H), 2.11 (s, 1H), 2.04 (s, 4H).

LC-MS (ESI) *m*/*z* = 390 [M+H]⁺.

### Step 7: Preparation of (OBD-018):

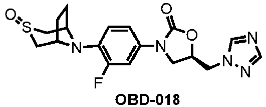

To a solution of (5*R*)-5-((1H-1,2,4-triazol-1-yl)methyl)-3-(4-(3-thia-8-aza-bicyclo[3.2.1] octan-8-yl)-3-fluorophenyl)oxazolidin-2-one (OBD-021) (100 mg, 0.26 mmol) in THF (10 mL) and 10 drops water was added potassium peroxomonosulfate (157 mg, 0.26 mmol) at 0 °C, then the reaction mixture was stirred at 0 °C for 2 h, monitored by TLC. Quenched with sodium thiosulfate, and the crude material was purified by silica gel column chromatography (DCM: MeOH = 80: 1) to afford (OBD-018) (52 mg, 50%) as a white solid.

¹H NMR (300 MHz, DMSO-d₆) δ 12.17 (s, 1H), 8.69 (d, *J* = 2.9 Hz, 1H), 8.20 - 8.03 (m, 1H), 7.44 (d, *J* = 16.2 Hz, 1H), 7.28 - 7.02 (m, 2H), 5.08 (dd, *J* = 8.5, 5.1 Hz, 1H), 4.68 - 4.52 (m, 4H), 4.20 (t, *J* = 9.1 Hz, 1H), 3.91 (dd, *J* = 8.7, 6.0 Hz, 1H), 3.56 (d, *J* = 11.1 Hz, 2H), 2.48 (d, *J* = 12.3 Hz, 2H), 2.06 (d, *J* = 5.1 Hz, 2H), 1.79 (d, *J* = 7.6 Hz, 2H).

LC-MS (ESI) *m*/*z* = 405.8 [M+H]⁺.

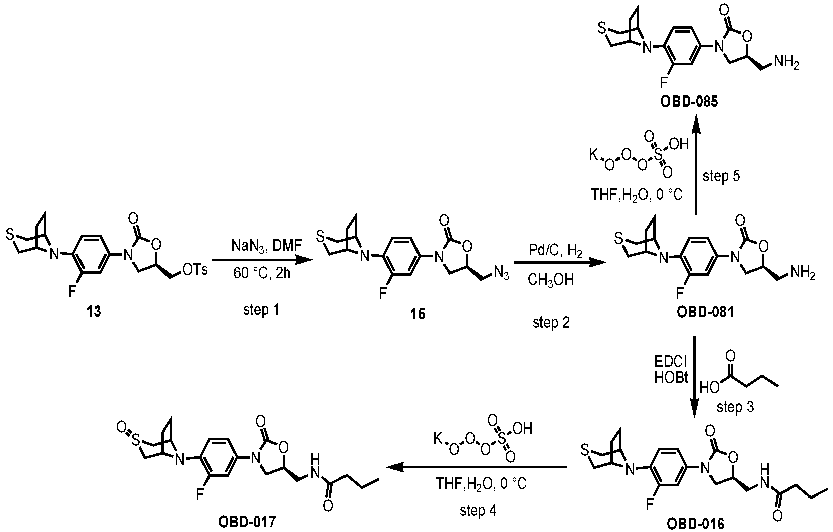

### Step 1:

### (5R)-3-(4-(3-thia-8-aza-bicyclo[3.2.1]octan-8-yl)-3-fluorophenyl)-5-(azidomethyl)ox azolidin-2-one (15):

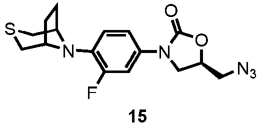

To a solution of ((*R*)-3-(4-(3-thia-8-aza-bicyclo[3.2.1]octan-8-yl)-3-fluorophenyl)-2-oxooxazolidin-5-yl)methyl 4-methylbenzenesulfonate (14) (2 g, 4 mmol) and sodium azide (265 mg, 4 mmol) in DMF (10 mL) was added K₂CO₃ (1.1 g, 8 mmol) at 25°C, then the reaction mixture was stirred at 80°C for 1 h under a nitrogen gas atmosphere, monitored by TLC. Quenched with ammonium chloride, extracted with EA, the organic layer was concentrated under reduced pressure, and the crude material was purified by silica gel column chromatography (PE: EA = 2: 1) to afford (5*R*)-3-(4-(3-thia-8-aza-bicyclo[3.2.1]octan-8-yl) -3-fluorophenyl)-5-(azidomethyl)oxazolidin-2-one (15) (1.01 g, 70%) as a white solid.

LC-MS (ESI) *m*/*z* = 364 [M+H]⁺.

### Step 2:

### (5S)-3-(4-(3-thia-8-aza-bicyclo[3.2.1]octan-8-yl)-3-fluorophenyl)-5-(aminomethyl)o xazolidin-2-one (OBD-081):

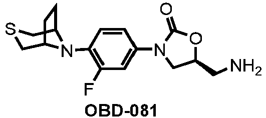

To a solution of (SR)-3-(4-(3-thia-8-aza-bicyclo[3.2.1]octan-8-yl) -3-fluorophenyl)-5-(azidomethyl)oxazolidin-2-one (15) (1.01 g, 2.8 mmol) in MeOH (10 mL) was added palladium carbon (100 mg) at 25°C, then the reaction mixture was stirred at room temperature for overnight under a hydrogen gas atmosphere, monitored by TLC. The filtrate was concentrated under reduced pressure, and the crude material was purified by silica gel column chromatography (DCM: MeOH = 50: 1) to afford (5*S*)-3-(4-(3-thia-8-aza-bicyclo[3.2.1]octan-8-yl)-3-fluorophenyl)-5-(aminomethyl)o xazolidin-2-one (OBD-081) (800 mg, 85%) as a white solid.

LC-MS (ESI) *m*/*z* = 338 [M+H]⁺.

### Step 3:

### N-(((S)-3-(4-(3-thia-8-aza-bicyclo[3.2.1]octan-8-yl)-3-fluorophenyl)-2-oxooxazolidi n-5-yl)methyl)butyramide (OBD-016):

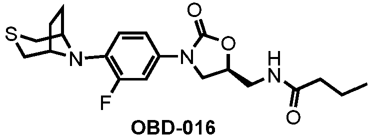

To a solution of (5S)-3-(4-(3-thia-8-aza-bicyclo[3.2.1]octan-8-yl)-3-fluorophenyl)-5-(aminomethyl)o xazolidin-2-one (OBD-081) (200 mg, 0.59 mmol) and butyric acid (52 mg, 0.59 mmol) in DCM (10 mL) were added HOBt (95 mg, 0.7 mmol), EDCI (170 mg, 0.88 mmol) and DIPEA (115 mg, 0.88 mmol) at 25 °C, then the reaction mixture was stirred at 25 °C for 2 h under a nitrogen gas atmosphere, monitored by TLC. Quenched with ammonium chloride, extracted with DCM, the organic layer was concentrated under reduced pressure, and the crude material was purified by silica gel column chromatography (DCM: MeOH = 80: 1) to afford N-(((*S*)-3-(4-(3-thia-8-aza-bicyclo[3.2.1]octan-8-yl)-3-fluorophenyl)-2-oxooxazolidi n-5-yl)methyl)butyramide (OBD-016) (156 mg, 65%) as a white solid.

¹H NMR (300 MHz, DMSO-d₆) δ 8.18 (s, 1H), 7.42 (d, J = 16.0 Hz, 1H), 7.35 - 6.88 (m, 2H), 4.71 (s, 1H), 4.35 (s, 2H), 4.07 (t, J = 8.7 Hz, 1H), 3.77 - 3.57 (m, 1H), 3.51 - 3.27 (m, 2H), 3.12 (d, J = 12.4 Hz, 2H), 2.09 (dd, J = 20.9, 12.2 Hz, 8H), 1.47 (dd, J = 14.0, 7.1 Hz, 2H), 0.80 (dd, J = 8.0, 6.7 Hz, 3H).

LC-MS (ESI) m/z = 407.9 [M+H]⁺.

### Step 4: Preparation of (OBD-017):

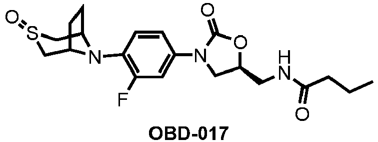

To a solution of N-(((*S*)-3-(4-(3-thia-8-aza-bicyclo [3.2.1]octan-8-yl)-3-fluorophenyl)-2-oxooxazolidin-5-yl)methyl)butyramide (OBD-016) (100 mg, 0.25 mmol) in THF (10 mL) and 10 drops water was added potassium peroxomonosulfate (157 mg, 0.26 mmol) at 0 °C, then the reaction mixture was stirred at 0 °C for 2 h, monitored by TLC. Quenched with sodium thiosulfate, and the crude material was purified by prep-HPLC to afford (OBD-017) (16 mg, 15%) as a white solid.

¹H NMR (301 MHz, CDCl₃) δ 7.39 (dd, J = 15.8, 2.3 Hz, 1H), 7.03 (d, J = 6.1 Hz, 2H), 6.78 (t, J = 9.3 Hz, 1H), 4.72 (s, 1H), 4.55 (s, 2H), 3.94 (t, J = 8.9 Hz, 1H), 3.81 - 3.66 (m, 1H), 3.58 (s, 2H), 3.42 (d, J = 10.3 Hz, 2H), 2.77 (d, J = 11.9 Hz, 2H), 2.17 (dd, J = 25.1, 17.8 Hz, 4H), 1.84 (d, J = 7.9 Hz, 2H), 1.56 (dq, J = 14.5, 7.2 Hz, 2H), 0.83 (t, J = 7.4 Hz, 3H).

LC-MS (ESI) m/z = 423.8 [M+H]⁺.

### Step 4: Preparation of (OBD-085):

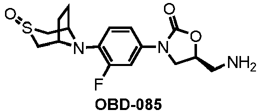

To a solution of (5S)-3-(4-(3-thia-8-aza-bicyclo[3.2.1]octan-8-yl)-3-fluorophenyl)-5-(aminomethyl)o xazolidin-2-one (OBD-081) (100 mg, 0.29 mmol) in THF (10 mL) and 10 drops water was added potassium peroxomonosulfate (182 mg, 0.29 mmol) at 0 °C, then the reaction mixture was stirred at 0 °C for 2 h, monitored by TLC. Quenched with sodium thiosulfate, and the crude material was purified by prep-HPLC to afford (OBD-085) (28 mg, 28%) as a white solid.

¹H NMR (400 MHz, DMSO-d₆) δ 7.52 (dd, J = 16.4, 2.2 Hz, 1H), 7.24 (dd, J = 8.9, 2.0 Hz, 1H), 7.13 (t, J = 9.6 Hz, 1H), 4.58 (dd, J = 14.1, 4.9 Hz, 3H), 4.04 (t, J = 8.9 Hz, 1H), 3.84 (dd, J = 8.7, 6.5 Hz, 1H), 3.56 (d, J = 10.0 Hz, 2H), 2.81 (dd, J = 9.3, 4.9 Hz, 2H), 2.46 (s, 2H), 2.17 - 1.99 (m, 2H), 1.79 (dd, J = 17.3, 9.5 Hz, 4H).

LC-MS (ESI) m/z = 354 [M+H]⁺.

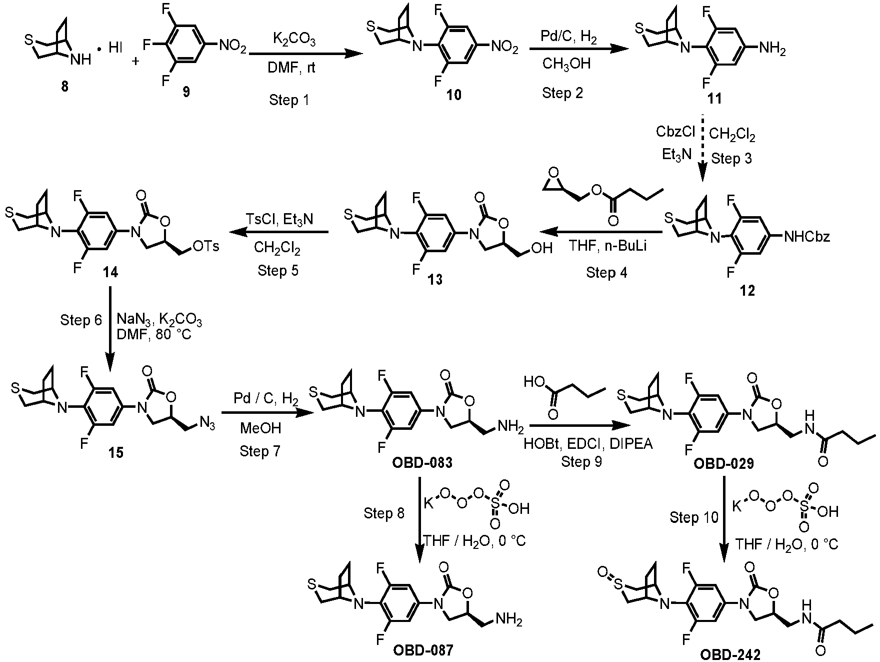

### Step 1: Preparation of

### 8-(2,6-difluoro-4-nitrophenyl)-3-thia-8-aza-bicyclo[3.2.1]octane (10):

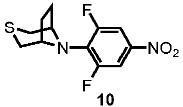

To a solution of 3-thia-8-aza-bicyclo[3.2.1]octane hydrogen iodide (8) (5.0 g, 19.4 mmol) and 1,2,3-trifluoro-5-nitrobenzene (4.13 g, 23.3 mmol) in DMF (10 mL) was added K₂CO₃ (5.35 g, 38.8 mmol) at 25°C then the reaction mixture was stirred at 80°C for 2 h under a nitrogen gas atmosphere, monitored by TLC. The mixture was concentrated under reduced pressure, and the crude material was purified by silica gel column chromatography (EA: PE = 3: 1) to afford 8-(2,6-difluoro-4-nitrophenyl)-3-thia-8-aza-bicyclo[3.2.1]octane (10) (3.6 g, 65%) as a yellow solid.

LC-MS (ESI) *m*/*z* = 287 [M+H]⁺.

### Step 2: 4-(3-thia-8-aza-bicyclo[3.2.1]octan-8-yl)-3,5-difluorobenzenamine (11):

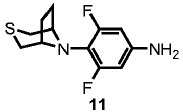

To a solution of 8-(2-fluoro-4-nitrophenyl)-3-thia-8-aza-bicyclo[3.2.1]octane (10) (3.6 g, 12.5 mmol) and Palladium carbon (200 mg) in MeOH (15 mL), then under a hydrogen gas atmosphere and the reaction mixture was stirred at room temperature for overnight, monitored by TLC. The filtrate was concentrated under reduced pressure to afford 4-(3-thia-8-aza-bicyclo[3.2.1]octan-8-yl)-3,5-difluorobenzenamine (11) (2.9 g, 90%) as a white oil, and the crude material was used for next reaction without further purification.

LC-MS (ESI) *m*/*z* = 257 [M+H]⁺.

### Step 3: benzyl

### 4-(3-thia-8-aza-bicyclo[3.2.1]octan-8-yl)-3,5-difluorophenylcarbamate (12):

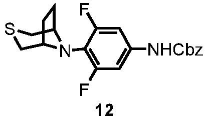

Carbonic acid, 2,5-dioxo-1-pyrrolidinyl phenylmethyl ester (5.57 g, 22.4 mmol) was added to a suspension of 4-(3-thia-8-aza-bicyclo[3.2.1]octan-8-yl)-3,5-difluorobenzenamine (11) (2.9 g, 11.2 mmol) in THF (30 mL) at 0 °C under a nitrogen gas atmosphere and the reaction mixture was stirred at 50°C for 5 h, monitored by TLC. The mixture was concentrated under reduced pressure, and the crude material was purified by silica gel column chromatography (EA: PE = 10: 1) to afford benzyl 4-(3-thia-8-aza-bicyclo[3.2.1]octan-8-yl)-3,5-difluorophenylcarbamate (12) (3.0 g, 68%) as a white solid.

LC-MS (ESI) *m*/*z* = 391 [M+H]⁺.

### Step 4:

### (5R)-3-(4-(3-thia-8-aza-bicyclo[3.2.1]octan-8-yl)-3,5-difluorophenyl)-5-(hydroxyme thyl)oxazolidin-2-one (13):

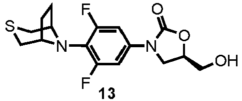

To a solution of benzyl 4-(3-thia-8-aza-bicyclo[3.2.1]octan-8-yl)-3,5-difluorophenylcarbamate (12) (3.0 g, 7.7 mmol) in THF (10 mL) at - 78 °C under a nitrogen gas atmosphere was added n-BuLi (4.8 ml, 11.5 mmol), then the mixture was stirred at -78°C for 30 min, after that the solution of (R)-oxiran-2-ylmethyl butyrate (1.66 g, 11.5 mmol) in THF was added to the mixture at -78°C, then warmed to room temperature and stirred for overnight, monitored by TLC. Quenched with ammonium chloride, extracted with EA, the organic layer was concentrated under reduced pressure, and the crude material was purified by silica gel column chromatography (DCM: MeOH = 70: 1) to afford (5R)-3-(4-(3-thia-8-aza-bicyclo[3.2.1]octan-8-yl)-3,5-difluorophenyl)-5-(hydroxymethyl)oxazolidin-2-one (13) (2.3 g, 84%) as a white solid.

LC-MS (ESI) *m*/*z* = 357 [M+H]⁺.

### Step 5:

### ((R)-3-(4-(3-thia-8-aza-bicyclo[3.2.1]octan-8-yl)-3,5-difluorophenyl)-2-oxooxazolidi n-5-yl)methyl 4-methylbenzenesulfonate (14):

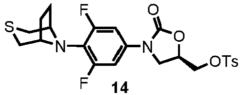

4-methylbenzene-1-sulfonyl chloride (2.45 g, 13 mmol) was added to a suspension of (5R)-3-(4-(3-thia-8-aza-bicyclo[3.2.1]octan-8-yl)-3,5-difluorophenyl)-5-(hydroxymethyl)oxazolidin-2-one (13) (2.3 g, 6.5 mmol) and Et₃N (1.3 g, 13 mmol) in DCM (10 mL) at 0 °C under a nitrogen gas atmosphere and the reaction mixture was stirred at room temperature for overnight, monitored by TLC. Quenched with ammonium chloride, extracted with DCM, the organic layer was concentrated under reduced pressure, and the crude material was purified by silica gel column chromatography (DCM: MeOH = 50: 1) to afford ((R)-3-(4-(3-thia-8-aza-bicyclo[3.2.1]octan-8-yl)-3,5-difluorophenyl)-2-oxooxazolidi n-5-yl)methyl 4-methylbenzenesulfonate (14) (2.81 g, 85%) as a white solid.

LC-MS (ESI) *m*/*z* = 511 [M+H]⁺.

### Step 6:

### (5R)-3-(4-(3-thia-8-aza-bicyclo[3.2.1]octan-8-yl)-3,5-difluorophenyl)-5-(azidomethy l)oxazolidin-2-one (15):

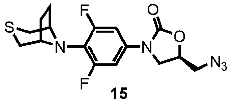

To a solution of ((R)-3-(4-(3-thia-8-aza-bicyclo[3.2.1]octan-8-yl)-3,5-difluorophenyl)-2-oxooxazolidi n-5-yl)methyl 4-methylbenzenesulfonate (14) (2.81 g, 5.52 mmol) and sodium azide (360 mg, 5.52 mmol) in DMF (10 mL) was added K₂CO₃ (1.52 mg, 11.04 mmol) at 25°C, then the reaction mixture was stirred at 80°C for 1 h under a nitrogen gas atmosphere, monitored by TLC. Quenched with ammonium chloride, extracted with EA, the organic layer was concentrated under reduced pressure, and the crude material was purified by silica gel column chromatography (PE: EA = 2: 1) to afford (5R)-3-(4-(3-thia-8-aza-bicyclo[3.2.1]octan-8-yl)-3,5-difluorophenyl)-5-(azidomethy l)oxazolidin-2-one (15) (1.85 g, 88%) as a white solid.

LC-MS (ESI) *m*/*z* = 382 [M+H]⁺.

### Step 7:

### (5S)-3-(4-(3-thia-8-aza-bicyclo[3.2.1]octan-8-yl)-3,5-difluorophenyl)-5-(aminometh yl)oxazolidin-2-one (OBD-083):

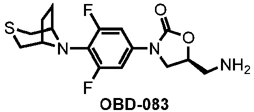

To a solution of (5R)-3-(4-(3-thia-8-aza-bicyclo[3.2.1]octan-8-yl)-3,5-difluorophenyl)-5-(azidomethy l)oxazolidin-2-one (15) (1.85 g, 4.87 mmol) in MeOH (10 mL) was added palladium carbon (100 mg) at 25°C, then the reaction mixture was stirred at room temperature for overnight under a hydrogen gas atmosphere, monitored by TLC. The filtrate was concentrated under reduced pressure, and the crude material was purified by silica gel column chromatography (DCM: MeOH = 50: 1) to afford (5S)-3-(4-(3-thia-8-aza-bicyclo[3.2.1]octan-8-yl)-3,5-difluorophenyl)-5-(aminometh yl)oxazolidin-2-one (OBD-083) (1.6 g, 90 %) as a white solid.

¹H NMR (400 MHz, DMSO) δ 7.34 - 7.15 (m, 2H), 4.59 (td, J = 11.0, 5.0 Hz, 1H), 4.10 (s, 2H), 3.99 (t, J = 8.9 Hz, 1H), 3.79 (dd, J = 8.9, 6.4 Hz, 1H), 3.11 (dd, J = 12.6, 1.6 Hz, 2H), 2.79 (qd, J = 13.7, 4.9 Hz, 2H), 2.26 (dd, J = 12.4, 3.3 Hz, 2H), 2.01 (s, 4H), 1.72 (d, J = 59.8 Hz, 2H).

LC-MS (ESI) *m*/*z* = 356 [M+H]⁺.

### Step 8: Preparation of (OBD-087):

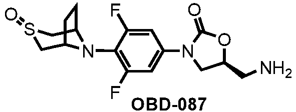

To a solution of (5S)-3-(4-(3-thia-8-aza-bicyclo[3.2.1]octan-8-yl)-3,5-difluorophenyl)-5-(aminometh yl)oxazolidin-2-one (OBD-083) (100 mg, 0.28 mmol) in THF (10 mL) and 10 drops water was added potassium peroxomonosulfate (173 mg, 0.28 mmol) at 0 °C, then the reaction mixture was stirred at 0 °C for 2 h, monitored by TLC. Quenched with sodium thiosulfate, and the crude material was purified by prep-HPLC to afford (OBD-087) (31 mg, 30 %) as a white solid.

¹H NMR (400 MHz, DMSO) δ 7.32 (t, J = 9.4 Hz, 2H), 4.61 (dd, J = 8.8, 6.0 Hz, 0H), 4.34 (s, 1H), 4.02 (t, J = 8.9 Hz, 1H), 3.82 (dd, J = 8.9, 6.4 Hz, 1H), 3.68 (dd, J = 12.4, 3.7 Hz, 1H), 2.81 (qd, J = 13.7, 4.9 Hz, 1H), 2.58 (d, J = 11.6 Hz, 1H), 2.12 - 1.98 (m, 1H), 1.78 (q, J = 6.9 Hz, 2H).

LC-MS (ESI) m/z = 372 [M+H]⁺.

### Step 9:

### N-(((S)-3-(4-(3-thia-8-aza-bicyclo[3.2.1]octan-8-yl)-3,5-difluorophenyl)-2-oxooxazo lidin-5-yl)methyl)butyramide (OBD-029):

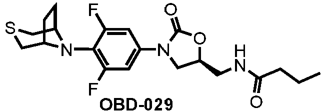

To a solution of (5S)-3-(4-(3-thia-8-aza-bicyclo[3.2.1]octan-8-yl)-3,5-difluorophenyl)-5-(aminometh yl)oxazolidin-2-one (OBD-083) (200 mg, 0.56 mmol) and butyric acid (52 mg, 0.59 mmol) in DCM (10 mL) were added HOBt (95 mg, 0.7 mmol), EDCI (170 mg, 0.88 mmol) and DIPEA (115 mg, 0.88 mmol) at 25 °C, then the reaction mixture was stirred at 25 °C for 2 h under a nitrogen gas atmosphere, monitored by TLC. Quenched with ammonium chloride, extracted with DCM, the organic layer was concentrated under reduced pressure, and the crude material was purified by silica gel column chromatography (DCM: MeOH = 80: 1) to afford N-(((S)-3-(4-(3-thia-8-aza-bicyclo[3.2.1]octan-8-yl)-3,5-difluorophenyl)-2-oxooxazo lidin-5-yl)methyl)butyramide (OBD-029) (119 mg, 50%) as a white solid.

¹H NMR (400 MHz, DMSO-d₆) δ 8.18 (s, 1H), 7.22 (d, J = 12.2 Hz, 2H), 4.73 (d, J = 3.6 Hz, 1H), 4.07 (dd, J = 19.1, 10.0 Hz, 3H), 3.68 (dd, J = 9.1, 6.2 Hz, 1H), 3.41 (s, 2H), 3.12 (d, J = 11.3 Hz, 2H), 2.26 (dd, J = 12.4, 3.0 Hz, 2H), 2.05 (dd, J = 16.8, 9.5 Hz, 6H), 1.47 (dd, J = 14.7, 7.3 Hz, 2H), 0.79 (t, J = 7.4 Hz, 3H).

LC-MS (ESI) m/z = 426 [M+H]⁺.

### Step 10: Preparation of (OBD-242):

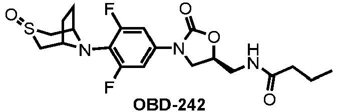

To a solution of N-(((S)-3-(4-(3-thia-8-aza-bicyclo[3.2.1]octan-8-yl)-3,5-difluorophenyl)-2-oxooxazo lidin-5-yl)methyl)butyramide (OBD-029) (100 mg, 0.23 mmol) in THF (10 mL) and 10 drops water was added potassium peroxomonosulfate (144 mg, 0.23 mmol) at 0 °C, then the reaction mixture was stirred at 0 °C for 2 h, monitored by TLC. Quenched with sodium thiosulfate, and the crude material was purified by prep-HPLC to afford (OBD-242) (30 mg, 15%) as a white solid.

¹H NMR (400 MHz, DMSO-d₆) δ 8.19 (d, J = 5.6 Hz, 1H), 7.28 (d, J = 12.7 Hz, 3H), 4.77 - 4.69 (m, 1H), 4.34 (s, 2H), 4.07 (t, J = 9.0 Hz, 1H), 3.67 (d, J = 9.0 Hz, 3H), 3.40 (dd, J = 11.0, 5.5 Hz, 1H), 2.56 (d, J = 11.9 Hz, 1H), 2.06 (t, J = 7.3 Hz, 4H), 1.82 - 1.72 (m, 2H), 1.51 - 1.40 (m, 2H), 0.78 (t, J = 7.4 Hz, 3H).

LC-MS (ESI) m/z = 442 [M+H]⁺.

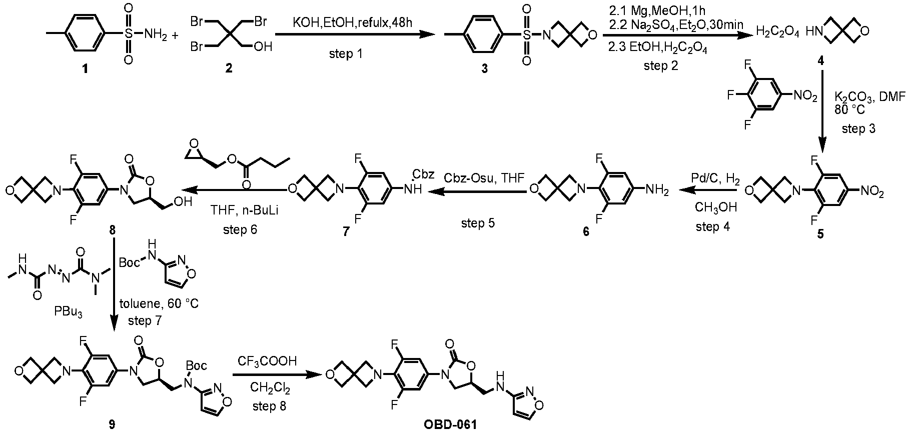

### Step 1: Preparation of (3):

To a solution of p-toluenesulfonamide (57 g, 330 mmol) and potassium hydroxide (49.8 g, 890 mmol) in ethanol (1000 mL) was added 3-bromo-2,2-bis(bromomethyl)propan-1-ol (90 g, 270 mmol) at 25°C then the reaction mixture was stirred at 100°C for 48 h. The mixture was concentrated under reduced pressure, and the crude material was poured into solution of potassium hydroxide (75 mL) and stirred for 2 h, to afford filter cake (3) (10 g, 59%) as a white solid.

LC-MS (ESI) *m*/*z* = 254 [M+H]⁺.

### Step 2: Preparation of (4):

A mixture of (3) (10 g, 39.5 mmol) and magnesium (6.7 g) in methanol (15 mL) was sonicated for 1 h at 40 °C, after that the solvent was removed under reduced pressure to afford a viscous grey residue, Et2O and sodium sulfate were added and the resulting grey mixture was stirred vigorously for 30 min before filtration. A solution of oxalic acid in ethanol was added to the filtrate. A think white precipitate formed instantly, which was target product (4) (3.7 g, 50%), and the crude material was used for next reaction without further purification.

### Step 3: Preparation of (5):

To a solution of (4) (3.7 g, 19.5 mmol) and 1,2,3-trifluoro-5-nitrobenzene (3.81 g, 21.5 mmol) in DMF (10 mL) was added K₂CO₃ (5.38 g, 39 mmol) at 25°C then the reaction mixture was stirred at 80°C for 2 h under a nitrogen gas atmosphere, monitored by TLC. The mixture was concentrated under reduced pressure, and the crude material was purified by silica gel column chromatography (EA: PE = 3: 1) to afford (5) (1.9 g, 38%) as a yellow solid.

LC-MS (ESI) *m*/*z* = 257 [M+H]⁺.

### Step 4: Preparation of (6):

To a solution of (5) (1.9 g, 7.4 mmol) and Palladium carbon (200 mg) in methanol (15 mL), then under a hydrogen gas atmosphere and the reaction mixture was stirred at room temperature for overnight, monitored by TLC. The filtrate was concentrated under reduced pressure to afford (6) (1.5 g, 90%) as a white oil, and the crude material was used for next reaction without further purification.

LC-MS (ESI) *m*/*z* = 227 [M+H]⁺.

### Step 5: Preparation of (7):

Carbonic acid, 2,5-dioxo-1-pyrrolidinyl phenylmethyl ester (3.3 g, 13.3 mmol) was added to a suspension of (6) (1.5 g, 6.7 mmol) in THF (30 mL) at 0 °C under a nitrogen gas atmosphere and the reaction mixture was stirred at 50°C for 5 h, monitored by TLC. The mixture was concentrated under reduced pressure, and the crude material was purified by silica gel column chromatography (EA: PE = 10: 1) to afford (7) (1.6 g, 68%) as a white solid.

LC-MS (ESI) *m*/*z* = 361 [M+H]⁺.

### Step 6: Preparation of (8):

To a solution of (7) (1.6 g, 4.6 mmol) in THF (10 mL) at - 78 °C under a nitrogen gas atmosphere was added n-BuLi (2.8 ml, 6.8 mmol), then the mixture was stirred at -78°C for 30 min, after that the solution of (R)-oxiran-2-ylmethyl butyrate (980 mg, 6.8 mmol) in THF was added to the mixture at -78°C, then warmed to room temperature and stirred for overnight, monitored by TLC. Quenched with ammonium chloride, extracted with EA, the organic layer was concentrated under reduced pressure, and the crude material was purified by silica gel column chromatography (DCM: MeOH = 70: 1) to afford (8) (1.2 g, 84%) as a white solid.

LC-MS (ESI) *m*/*z* = 327 [M+H]⁺.

### Step 7: Preparation of (9):

(E)-N1,N1,N2-trimethyldiazene-1,2-dicarboxamide (443 mg, 2.6 mmol) was added to a suspension of (8) (560 mg, 1.7 mmol), tert-butyl isoxazol-3-ylcarbamate (380 mg, 2.1 mmol) and tributylphosphine (521 mg, 2.6 mmol) in toluene (30 mL) at 0 °C under a nitrogen gas atmosphere and then the reaction mixture was stirred at 60 °C for overnight, monitored by TLC. The mixture was concentrated under reduced pressure, and the crude material was purified by silica gel column chromatography (EA: PE = 10: 1) to afford (9) (309 mg, 37%) as a white solid.

LC-MS (ESI) *m*/*z* = 493 [M+H]⁺.

### Step 8: Preparation of (OBD-061):

To a solution of (9) (309 g, 0.6 mmol) in CH₂Cl₂ (10 mL) at 0 °C was added trifluoroacetic acid (1 ml), then the mixture was stirred at 0 °C for 30 min, monitored by TLC. Quenched with ammonium chloride, extracted with CH₂Cl₂, the organic layer was concentrated under reduced pressure, and the crude material was purified by prep-HPLC to afford (OBD-061) (93 mg, 38 %) as a white solid.

¹H NMR (300 MHz, CDCl₃) δ 8.07 (s, 1H), 7.01 (d, J = 12.1 Hz, 2H), 5.85 (d, J = 1.7 Hz, 1H), 4.92 (s, 1H), 4.82 (s, 4H), 4.29 (s, 4H), 3.99 (s, 2H), 3.75 (s, 2H), 3.60 (s, 2H)

LC-MS (ESI) m/z = 392.9 [M+H]⁺.

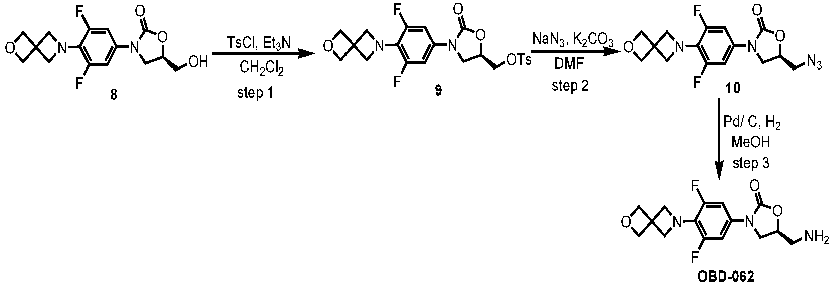

### Step 1: Preparation of (9):

4-methylbenzene-1-sulfonyl chloride (2.45 g, 13 mmol) was added to a suspension of (8) (2.3 g, 6.5 mmol) and Et₃N (1.3 g, 13 mmol) in DCM (10 mL) at 0 °C and then the reaction mixture was stirred at room temperature for overnight under a nitrogen gas atmosphere, monitored by TLC. Quenched with ammonium chloride, extracted with DCM, the organic layer was concentrated under reduced pressure, and the crude material was purified by silica gel column chromatography (DCM: MeOH = 50: 1) to afford (9) (2.65 g, 85%) as a white solid.

LC-MS (ESI) *m*/*z* = 481 [M+H]⁺.

### Step 2: Preparation of (10):

To a solution of (9) (2.65 g, 5.52 mmol) and sodium azide (360 mg, 5.52 mmol) in DMF (10 mL) was added K₂CO₃ (1.52 mg, 11.04 mmol) at 25°C, then the reaction mixture was stirred at 80°C for 1 h under a nitrogen gas atmosphere, monitored by TLC. Quenched with ammonium chloride, extracted with EA, the organic layer was concentrated under reduced pressure, and the crude material was purified by silica gel column chromatography (PE: EA = 2: 1) to afford (10) (1.7 g, 88%) as a white solid.

LC-MS (ESI) *m*/*z* = 352 [M+H]⁺.

### Step 3: Preparation of (OBD-062):

To a solution of (10) (1.7 g, 4.86 mmol) in MeOH (10 mL) was added palladium carbon (100 mg) at 25°C, then the reaction mixture was stirred at room temperature for overnight under a hydrogen gas atmosphere, monitored by TLC. The filtrate was concentrated under reduced pressure, and the crude material was purified by silica gel column chromatography (DCM: MeOH = 50: 1) to afford (OBD-062) (1.3 g, 85 %) as a white solid.

¹H NMR (400 MHz, DMSO-d₆) δ 7.34 - 6.98 (m, 2H), 4.70 (s, 4H), 4.59 (dt, J = 11.3, 5.1 Hz, 1H), 4.23 (d, J = 2.2 Hz, 4H), 3.99 (dd, J = 20.9, 12.0 Hz, 1H), 3.78 (dd, J = 8.9, 6.4 Hz, 1H), 2.80 (ddd, J = 24.5, 13.6, 4.9 Hz, 2H), 1.99 (s, 2H).

LC-MS (ESI) m/z = 326.1 [M+H]⁺.

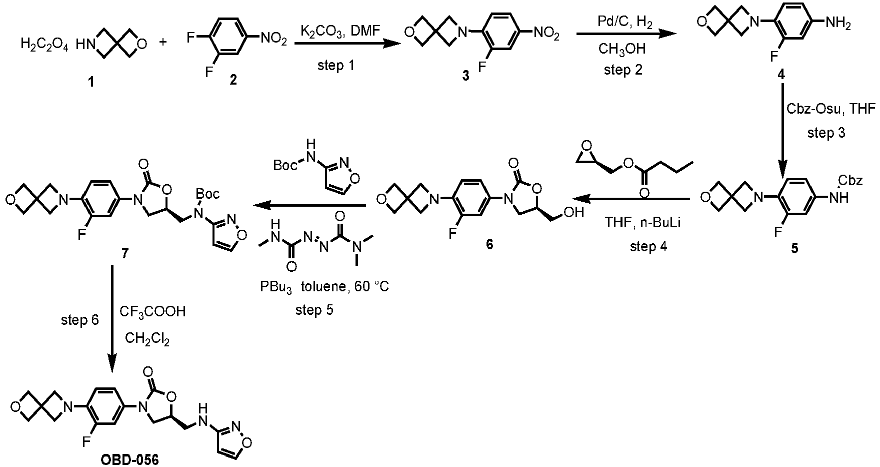

### Step 1: Preparation of (3):

To a solution of (1) (3.7 g, 19.5 mmol) and 1,2-difluoro-4-nitrobenzene (3.41 g, 21.5 mmol) in DMF (10 mL) was added K₂CO₃ (5.38 g, 39 mmol) at 25°C then the reaction mixture was stirred at 80°C for 2 h under a nitrogen gas atmosphere, monitored by TLC. The mixture was concentrated under reduced pressure, and the crude material was purified by silica gel column chromatography (EA: PE = 3: 1) to afford (3) (1.7 g, 38%) as a yellow solid.

LC-MS (ESI) *m*/*z* = 239 [M+H]⁺.

### Step 2: Preparation of (4):

To a solution of (3) (1.7 g, 7.4 mmol) and Palladium carbon (200 mg) in methanol (15 mL), then under a hydrogen gas atmosphere and the reaction mixture was stirred at room temperature for overnight, monitored by TLC. The filtrate was concentrated under reduced pressure to afford (4) (1.4 g, 90%) as a white oil, and the crude material was used for next reaction without further purification.

LC-MS (ESI) *m*/*z* = 209 [M+H]⁺.

### Step 3: Preparation of (7):

Carbonic acid, 2,5-dioxo-1-pyrrolidinyl phenylmethyl ester (3.3 g, 13.3 mmol) was added to a suspension of (6) (1.4 g, 6.7 mmol) in THF (30 mL) at 0 °C under a nitrogen gas atmosphere and the reaction mixture was stirred at 50°C for 5 h, monitored by TLC. The mixture was concentrated under reduced pressure, and the crude material was purified by silica gel column chromatography (EA: PE = 10: 1) to afford (7) (1.6 g, 70%) as a white solid.

LC-MS (ESI) *m*/*z* = 343 [M+H]⁺.

### Step 4: Preparation of (8):

To a solution of (7) (1.6 g, 4.7 mmol) in THF (10 mL) at - 78 °C under a nitrogen gas atmosphere was added n-BuLi (2.9 ml, 7.0 mmol), then the mixture was stirred at -78°C for 30 min, after that the solution of (R)-oxiran-2-ylmethyl butyrate (1 g, 7.0 mmol) in THF was added to the mixture at -78°C, then warmed to room temperature and stirred for overnight, monitored by TLC. Quenched with ammonium chloride, extracted with EA, the organic layer was concentrated under reduced pressure, and the crude material was purified by silica gel column chromatography (DCM: MeOH = 70: 1) to afford (8) (1.2 g, 84%) as a white solid.

LC-MS (ESI) *m*/*z* = 309 [M+H]⁺.

### Step 5: Preparation of (9):

(E)-N₁,N₁,N₂-trimethyldiazene-1,2-dicarboxamide (443 mg, 2.6 mmol) was added to a suspension of (8) (523 mg, 1.7 mmol), tert-butyl isoxazol-3-ylcarbamate (380 mg, 2.1 mmol) and tributylphosphine (521 mg, 2.6 mmol) in toluene (30 mL) at 0 °C under a nitrogen gas atmosphere and then the reaction mixture was stirred at 60 °C for overnight, monitored by TLC. The mixture was concentrated under reduced pressure, and the crude material was purified by silica gel column chromatography (EA: PE = 10: 1) to afford (9) (298 mg, 37%) as a white solid.

LC-MS (ESI) *m*/*z* = 475 [M+H]⁺.

### Step 6: Preparation of (OBD-056):

To a solution of (9) (298 mg, 0.6 mmol) in CH₂Cl₂ (10 mL) at 0 °C was added trifluoroacetic acid (1 ml), then the mixture was stirred at 0 °C for 30 min, monitored by TLC. Quenched with ammonium chloride, extracted with CH₂Cl₂, the organic layer was concentrated under reduced pressure, and the crude material was purified by prep-HPLC to afford (OBD-056) (84 mg, 38 %) as a white solid.

¹H NMR (301 MHz, CDCl₃) δ 7.39 (d, J = 14.4 Hz, 1H), 7.25 (s, 1H), 7.04 (d, J = 8.6 Hz, 1H), 6.83 (t, J = 8.9 Hz, 1H), 5.14 (s, 1H), 4.73 (s, 1H), 4.39 (s, 2H), 4.00 (t, J = 8.8 Hz, 1H), 3.84 - 3.42 (m, 8H), 3.05 (dd, J = 23.2, 11.2 Hz, 5H), 2.13 - 1.88 (m, 5H).

LC-MS (ESI) m/z = 375 [M+H]⁺.

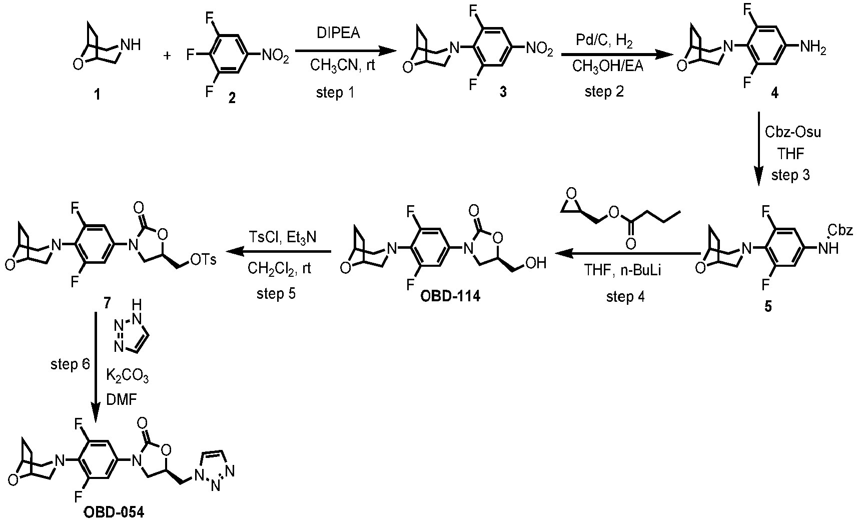

### Step 1: Preparation of 3-(2,6-difluoro-4-nitrophenyl)-8-oxa-3-aza-bicyclo[3.2.1]octane (3):

To a solution of 8-oxa-3-aza-bicyclo[3.2.1]octane (1) (5.0 g, 44.2 mmol) and 1,2,3-trifluoro-5-nitrobenzene (8.6 g, 48.6 mmol) in DMF (10 mL) was added K₂CO₃ (12.2 g, 88.4 mmol) at 25°C then the reaction mixture was stirred at 80°C for 2 h under a nitrogen gas atmosphere, monitored by TLC. The mixture was concentrated under reduced pressure, and the crude material was purified by silica gel column chromatography (EA: PE = 3: 1) to afford 3-(2,6-difluoro-4-nitrophenyl)-8-oxa-3-aza-bicyclo[3.2.1]octane (3) (9.3 g, 78%) as a yellow solid.

LC-MS (ESI) *m*/*z* = 271 [M+H]⁺.

### Step 2: Preparation of

### 4-(8-oxa-3-aza-bicyclo[3.2.1]octan-3-yl)-3,5-difluorobenzenamine (4):

To a solution of 3-(2,6-difluoro-4-nitrophenyl)-8-oxa-3-aza-bicyclo[3.2.1]octane (3) (9.3 g, 34.4 mmol) and Palladium carbon (1 g) in MeOH (15 mL), then under a hydrogen gas atmosphere and the reaction mixture was stirred at room temperature for overnight, monitored by TLC. The filtrate was concentrated under reduced pressure to afford 4-(8-oxa-3-aza-bicyclo[3.2.1]octan-3-yl)-3,5-difluorobenzenamine (4) (7.8 g, 95%) as a white oil, and the crude material was used for next reaction without further purification.

LC-MS (ESI) *m*/*z* = 241 [M+H]⁺.

### Step 3: Preparation of benzyl

### 4-(8-oxa-3-aza-bicyclo[3.2.1]octan-3-yl)-3,5-difluorophenylcarbamate (5):

Carbonic acid, 2,5-dioxo-1-pyrrolidinyl phenylmethyl ester (12 g, 48.7 mmol) was added to a suspension of 4-(8-oxa-3-aza-bicyclo[3.2.1]octan-3-yl)-3,5-difluorobenzenamine (4) (7.8 g, 32.5 mmol) in THF (100 mL) at 0 °C under a nitrogen gas atmosphere and the reaction mixture was stirred at 50°C for 5 h, monitored by TLC. The mixture was concentrated under reduced pressure, and the crude material was purified by silica gel column chromatography (EA: PE = 10: 1) to afford benzyl 4-(8-oxa-3-aza-bicyclo[3.2.1]octan-3-yl)-3,5-difluorophenylcarbamate (5) (8.2 g, 68%) as a white solid.

LC-MS (ESI) *m*/*z* = 375 [M+H]⁺.

### Step 4: Preparation of

### 3-(4-(8-oxa-3-aza-bicyclo[3.2.1]octan-3-yl)-3,5-difluorophenyl)-5-(hydroxymethyl)o xazolidin-2-one (OBD-114):

To a solution of benzyl 4-(8-oxa-3-aza-bicyclo[3.2.1]octan-3-yl)-3,5-difluorophenylcarbamate (5) (8.2 g, 22.1 mmol) in THF (10 mL) at - 78 °C under a nitrogen gas atmosphere was added n-BuLi (13.8 ml, 33.1 mmol), then the mixture was stirred at -78°C for 30 min, after that the solution of (R)-oxiran-2-ylmethyl butyrate (4.7 g, 33.1 mmol) in THF was added to the mixture at -78°C, then warmed to room temperature and stirred for overnight, monitored by TLC. Quenched with ammonium chloride, extracted with EA, the organic layer was concentrated under reduced pressure, and the crude material was purified by silica gel column chromatography (DCM: MeOH = 70: 1) to afford 3-(4-(8-oxa-3-aza-bicyclo[3.2.1]octan-3-yl)-3,5-difluorophenyl)-5-(hydroxymethyl)o xazolidin-2-one (OBD-114) (4.5 g, 60%) as a white solid.

LC-MS (ESI) *m*/*z* = 341 [M+H]⁺.

### Step 5: Preparation of (3-(4-(8-oxa-3-aza-bicyclo[3.2.1]octan-3-yl)-3,5-difluorophenyl)-2-oxooxazolidin-5-yl)methyl 4-methylbenzenesulfonate (7):

4-methylbenzene-1-sulfonyl chloride (5 g, 26.6 mmol) was added to a suspension of 3-(4-(8-oxa-3-aza-bicyclo[3.2.1]octan-3-yl)-3,5-difluorophenyl)-5-(hydroxymethyl)o xazolidin-2-one (OBD-114) (4.5 g, 13.3 mmol) and Et₃N (2.7 g, 26.6 mmol) in DCM (10 mL) at 0 °C under a nitrogen gas atmosphere and the reaction mixture was stirred at room temperature for overnight, monitored by TLC. Quenched with ammonium chloride, extracted with DCM, the organic layer was concentrated under reduced pressure, and the crude material was purified by silica gel column chromatography (DCM: MeOH = 50: 1) to afford (3-(4-(8-oxa-3-aza-bicyclo[3.2.1]octan-3-yl)-3,5-difluorophenyl)-2-oxooxazolidin-5-yl)methyl 4-methylbenzenesulfonate (7) (5.58 g, 85%) as a white solid.

LC-MS (ESI) *m*/*z* = 495 [M+H]⁺.

### Step 6: Preparation of 5-((1H-1,2,3-triazol-1-yl)methyl)-3-(4-(8-oxa-3-aza-bicyclo[3.2.1]octan-3-yl)-3,5-di fluorophenyl)oxazolidin-2-one (OBD-054):

To a solution of (3-(4-(8-oxa-3-aza-bicyclo[3.2.1]octan-3-yl)-3,5-difluorophenyl)-2-oxooxazolidin-5-yl)methyl 4-methylbenzenesulfonate (7) (300 mg, 0.6 mmol) and 1H-1,2,3-triazole (42 mg, 0.6 mmol) in DMF (10 mL) was added K₂CO₃ (166 mg, 1.2 mmol) at 25°C, then the reaction mixture was stirred at 80°C for 1 h under a nitrogen gas atmosphere, monitored by TLC. Quenched with ammonium chloride, extracted with EA, the organic layer was concentrated under reduced pressure, and the crude material was purified by prep-HPLC to afford 5-((1H-1,2,3-triazol-1-yl)methyl)-3-(4-(8-oxa-3-aza-bicyclo[3.2.1]octan-3-yl)-3,5-di fluorophenyl)oxazolidin-2-one (OBD-054) (82 mg, 35%) as a white solid.

¹H NMR (300 MHz, DMSO-d₆) δ 8.14 (d, *J =* 1.0 Hz, 1H), 7.73 (d, *J =* 1.0 Hz, 1H), 7.17 (d, *J =* 11.7 Hz, 2H), 5.11 (d, *J* = 3.5 Hz, 1H), 4.79 (d, *J =* 5.0 Hz, 2H), 4.18 (dd, *J =* 23.1, 13.7 Hz, 3H), 3.91 - 3.70 (m, 1H), 3.23 (d, *J =* 10.9 Hz, 3H), 2.72 (d, *J* = 10.7 Hz, 3H), 2.07 - 1.61 (m, 7H).

LC-MS (ESI) *m*/*z* = 392 [M+H]⁺.

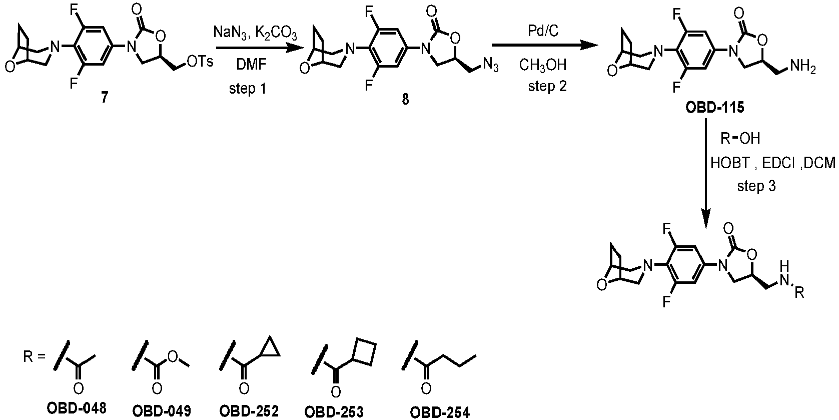

### Step 1: Preparation of 3-(4-(8-oxa-3-aza-bicyclo[3.2.1]octan-3-yl)-3,5-difluorophenyl)-5-(azidomethyl)oxa zolidin-2-one (8):

To a solution of (3-(4-(8-oxa-3-aza-bicyclo[3.2.1]octan-3-yl)-3,5-difluorophenyl)-2-oxooxazolidin-5-yl)methyl 4-methylbenzenesulfonate (7) (4 g, 8 mmol) and sodium azide (526 mg, 8 mmol) in DMF (10 mL) was added K₂CO₃ (2.2 mg, 16 mmol) at 25°C, then the reaction mixture was stirred at 80°C for 1 h under a nitrogen gas atmosphere, monitored by TLC. Quenched with ammonium chloride, extracted with EA, the organic layer was concentrated under reduced pressure, and the crude material was purified by silica gel column chromatography (PE: EA = 2: 1) to afford 3-(4-(8-oxa-3-aza-bicyclo[3.2.1]octan-3-yl)-3,5-difluorophenyl)-5-(azidomethyl)oxa zolidin-2-one (8) (2.57 g, 88%) as a white solid.

LC-MS (ESI) *m*/*z* = 366 [M+H]⁺.

### Step 2: Preparation of 3-(4-(8-oxa-3-aza-bicyclo[3.2.1]octan-3-yl)-3,5-difluorophenyl)-5-(aminomethyl)ox azolidin-2-one (OBD-115):

To a solution of 3-(4-(8-oxa-3-aza-bicyclo[3.2.1]octan-3-yl)-3,5-difluorophenyl)-5-(azidomethyl)oxa zolidin-2-one (8) (2.57 g, 7 mmol) in MeOH (10 mL) was added palladium carbon (300 mg) at 25°C, then the reaction mixture was stirred at room temperature for overnight under a hydrogen gas atmosphere, monitored by TLC. The filtrate was concentrated under reduced pressure, and the crude material was purified by silica gel column chromatography (DCM: MeOH = 50: 1) to afford 3-(4-(8-oxa-3-aza-bicyclo[3.2.1]octan-3-yl)-3,5-difluorophenyl)-5-(aminomethyl)ox azolidin-2-one (OBD-115) (2.1 g, 85 %) as a white solid.

¹H NMR (400 MHz, CDCl₃) δ 7.40 - 7.20 (m, 2H), 4.62 (td, *J* = 10.9, 4.9 Hz, 1H), 4.28 (s, 2H), 4.02 (t, *J =* 8.9 Hz, 1H), 3.81 (dd, *J* = 8.9, 6.3 Hz, 1H), 3.27 (d, *J =* 10.4 Hz, 2H), 2.81 (ddd, *J =* 28.3, 18.6, 7.7 Hz, 4H), 2.21 (s, 2H), 2.04 - 1.94 (m, 2H), 1.88 - 1.71 (m, 2H).

LC-MS (ESI) *m*/*z* = 340 [M+H]⁺.

### Step 3: Preparation of (OBD-048, 049, 252, 253, 254):

To a solution of 3-(4-(8-oxa-3-aza-bicyclo[3.2.1]octan-3-yl)-3,5-difluorophenyl)-5-(aminomethyl)ox azolidin-2-one (OBD-115) (200 mg, 0.59 mmol) and R-OH (0.59 mmol) in DCM (10 mL) were added HOBt (119 mg, 0.88 mmol), EDCI (225 mg, 1.18 mmol) and DIPEA (152 mg, 1.18 mmol) at 25 °C, then the reaction mixture was stirred at 25 °C for 2 h under a nitrogen gas atmosphere, monitored by TLC. Quenched with ammonium chloride, extracted with DCM, the organic layer was concentrated under reduced pressure, and the crude material was purified by silica gel column chromatography (DCM: MeOH = 80: 1) to afford (OBD-048, 049, 252, 253, 254) as a white solid.

### OBD-048

¹H NMR (300 MHz, CDCl₃) δ 7.06 (d, *J =* 10.9 Hz, 2H), 5.98 (s, 1H), 4.75 (s, 1H), 4.33 (s, 2H), 3.98 (t, *J* = 8.8 Hz, 1H), 3.68 (dd, *J* = 19.8, 11.0 Hz, 3H), 3.45 (d, *J* = 10.9 Hz, 3H), 2.78 (d, *J =* 11.1 Hz, 2H), 2.11 (d, *J =* 6.6 Hz, 3H), 1.98 (d, *J =* 24.1 Hz, 6H).

LC-MS (ESI) m/z = 426 [M+H]⁺.

### OBD-049

¹H NMR (301 MHz, CDCl₃) δ 7.06 (d, J = 11.0 Hz, 2H), 5.15 (s, 1H), 4.75 (s, 1H), 4.32 (s, 2H), 3.97 (t, J = 9.0 Hz, 2H), 3.80 - 3.70 (m, 4H), 3.56 (d, J = 5.9 Hz, 2H), 3.42 (s, 2H), 2.77 (d, J = 11.1 Hz, 2H), 2.10 (d, J = 6.4 Hz, 2H), 1.92 (d, J = 5.0 Hz, 2H).

LC-MS (ESI) m/z = 397.7 [M+H]⁺.

### OBD-252

¹H NMR (301 MHz, CDCl₃) δ 7.07 (d, *J =* 11.0 Hz, 2H), 6.19 (s, 1H), 4.77 (s, 1H), 4.35 (s, 2H), 3.97 (t, *J =* 8.9 Hz, 1H), 3.78 - 3.62 (m, 3H), 3.46 (d, *J* = 10.1 Hz, 2H), 2.79 (d, *J* = 11.1 Hz, 2H), 2.12 (d, *J =* 6.5 Hz, 2H), 1.94 (d, *J =* 4.5 Hz, 2H), 1.43 - 1.33 (m, 1H), 0.95 (dd, *J =* 9.5, 4.4 Hz, 2H), 0.78 (d, *J =* 6.4 Hz, 2H).

LC-MS (ESI) m/z = 408.1 [M+H]⁺.

### OBD-253

¹H NMR (301 MHz, CDCl₃) δ 7.07 (d, *J =* 11.0 Hz, 2H), 5.88 (s, 1H), 4.76 (s, 1H), 4.34 (s,2H), 3.98 (t, *J =* 9.0 Hz, 1H), 3.79 - 3.59 (m, 3H), 3.45 (d, *J =* 10.8 Hz, 2H), 3.12 - 2.97 (m, 1H), 2.79 (d, *J =* 11.3 Hz, 2H), 2.38 - 2.10 (m, 6H), 2.00 - 1.79 (m, 4H).

LC-MS (ESI) m/z = 422.1 [M+H]⁺.

### OBD-254

¹H NMR (301 MHz, CDCl₃) δ 7.17 - 6.96 (m, 2H), 6.07 (s, 1H), 4.78 (s, 1H), 4.34 (s, 2H), 3.98 (t, *J =* 8.9 Hz, 1H), 3.82 - 3.64 (m, 3H), 3.45 (d, *J =* 10.0 Hz, 2H), 2.79 (d, *J* = 11.2 Hz, 2H), 2.38 - 2.06 (m, 4H), 1.98 - 1.78 (m, 2H), 1.63 (dq, *J =* 14.7, 7.3 Hz, 2H), 1.26 (s, 1H), 0.90 (t, *J =* 7.4 Hz, 3H).

LC-MS (ESI) m/z = 410.1 [M+H]⁺.

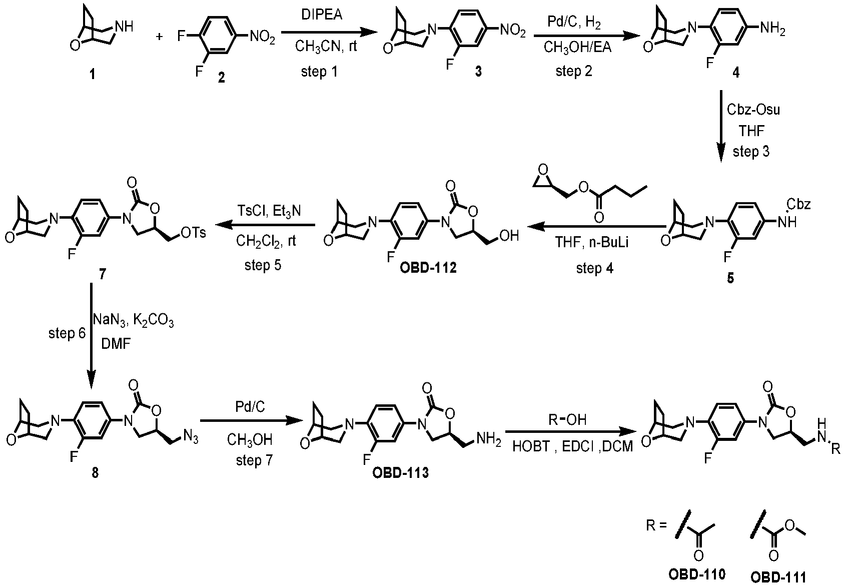

### Step 1: Preparation of 3-(2-fluoro-4-nitrophenyl)-8-oxa-3-aza-bicyclo[3.2.1]octane (3):

To a solution of 8-oxa-3-aza-bicyclo[3.2.1]octane (1) (5.0 g, 44.2 mmol) and 1,2-difluoro-4-nitrobenzene (7.7 g, 48.6 mmol) in DMF (10 mL) was added K₂CO₃ (12.2 g, 88.4 mmol) at 25°C then the reaction mixture was stirred at 80°C for 2 h under a nitrogen gas atmosphere, monitored by TLC. The mixture was concentrated under reduced pressure, and the crude material was purified by silica gel column chromatography (EA: PE = 5: 1) to afford 3-(2-fluoro-4-nitrophenyl)-8-oxa-3-aza-bicyclo[3.2.1]octane (3) (9.1 g, 82%) as a yellow solid.

LC-MS (ESI) *m*/*z* = 253 [M+H]⁺.

### Step 2: Preparation of 4-(8-oxa-3-aza-bicyclo[3.2.1]octan-3-yl)-3-fluorobenzenamine (4):

To a solution of 3-(2-fluoro-4-nitrophenyl)-8-oxa-3-aza-bicyclo[3.2.1]octane (3) (3) (9.1 g, 36.2 mmol) and Palladium carbon (1 g) in MeOH (15 mL), then under a hydrogen gas atmosphere and the reaction mixture was stirred at room temperature for overnight, monitored by TLC. The filtrate was concentrated under reduced pressure to afford 4-(8-oxa-3-aza-bicyclo[3.2.1]octan-3-yl)-3-fluorobenzenamine (4) (7.3 g, 91%) as a white oil, and the crude material was used for next reaction without further purification.

LC-MS (ESI) *m*/*z* = 223 [M+H]⁺.

### Step 3: Preparation of benzyl 4-(8-oxa-3-aza-bicyclo[3.2.1]octan-3-yl)-3-fluorophenylcarbamate (5):

Carbonic acid, 2,5-dioxo-1-pyrrolidinyl phenylmethyl ester (16.4 g, 65.88 mmol) was added to a suspension of 4-(8-oxa-3-aza-bicyclo[3.2.1]octan-3-yl)-3-fluorobenzenamine (4) (7.3 g, 32.9 mmol) in THF (100 mL) at 0 °C under a nitrogen gas atmosphere and the reaction mixture was stirred at 50°C for 5 h, monitored by TLC. The mixture was concentrated under reduced pressure, and the crude material was purified by silica gel column chromatography (PE: EA = 10: 1) to afford benzyl 4-(8-oxa-3-aza-bicyclo[3.2.1]octan-3-yl)-3-fluorophenylcarbamate (5) (7.1 g, 61%) as a white solid.

LC-MS (ESI) *m*/*z* = 357 [M+H]⁺.

### Step 4: Preparation of 3-(4-(8-oxa-3-aza-bicyclo[3.2.1]octan-3-yl)-3-fluorophenyl)-5-(hydroxymethyl)oxaz olidin-2-one (OBD-112):

To a solution of benzyl 4-(8-oxa-3-aza-bicyclo[3.2.1]octan-3-yl)-3-fluorophenylcarbamate (5) (7.1 g, 20.1 mmol) in THF (10 mL) at - 78 °C under a nitrogen gas atmosphere was added n-BuLi (12.5 ml, 30.1 mmol), then the mixture was stirred at -78°C for 30 min, after that the solution of (R)-oxiran-2-ylmethyl butyrate (4.3 g, 30.1 mmol) in THF was added to the mixture at -78°C, then warmed to room temperature and stirred for overnight, monitored by TLC. Quenched with ammonium chloride, extracted with EA, the organic layer was concentrated under reduced pressure, and the crude material was purified by silica gel column chromatography (DCM: MeOH = 70: 1) to afford 3-(4-(8-oxa-3-aza-bicyclo[3.2.1]octan-3-yl)-3-fluorophenyl)-5-(hydroxymethyl)oxaz olidin-2-one (OBD-112) (3.9 g, 60%) as a white solid.

¹H NMR (301 MHz, DMSO-d₆) δ 7.46 (dd, *J =* 15.4, 2.5 Hz, 1H), 7.14 (d, *J =* 6.5 Hz, 1H), 7.03 - 6.84 (m, 1H), 5.18 (s, 1H), 4.64 (d, *J =* 3.3 Hz, 1H), 4.31 (s, 2H), 4.00 (t, *J* = 9.0 Hz, 1H), 3.81 - 3.72 (m, 1H), 3.57 (d, *J* = 24.9 Hz, 2H), 3.00 (d, *J* = 11.2 Hz, 3H), 2.85 (d, *J =* 10.9 Hz, 2H), 2.08 - 1.63 (m, 5H).

LC-MS (ESI) *m*/*z* = 323 [M+H]⁺.

### Step 5: Preparation of (3-(4-(8-oxa-3-aza-bicyclo[3.2.1]octan-3-yl)-3-fluorophenyl)-2-oxooxazolidin-5-yl) methyl 4-methylbenzenesulfonate (7):

4-methylbenzene-1-sulfonyl chloride (2.3 g, 24 mmol) was added to a suspension of 3-(4-(8-oxa-3-aza-bicyclo[3.2.1]octan-3-yl)-3-fluorophenyl)-5-(hydroxymethyl)oxaz olidin-2-one (OBD-112) (3.9 g, 12 mmol) and Et₃N (1.2 g, 24 mmol) in DCM (10 mL) at 0 °C under a nitrogen gas atmosphere and the reaction mixture was stirred at room temperature for overnight, monitored by TLC. Quenched with ammonium chloride, extracted with DCM, the organic layer was concentrated under reduced pressure, and the crude material was purified by silica gel column chromatography (DCM: MeOH = 50: 1) to afford (3-(4-(8-oxa-3-aza-bicyclo[3.2.1]octan-3-yl)-3-fluorophenyl)-2-oxooxazolidin-5-yl) methyl 4-methylbenzenesulfonate (7) (4.86 g, 85%) as a white solid.

LC-MS (ESI) *m*/*z* = 477 [M+H]⁺.

### Step 6: Preparation of 3-(4-(8-oxa-3-aza-bicyclo[3.2.1]octan-3-yl)-3-fluorophenyl)-5-(azidomethyl)oxazoli din-2-one (8):

To a solution of (3-(4-(8-oxa-3-aza-bicyclo[3.2.1]octan-3-yl)-3-fluorophenyl)-2-oxooxazolidin-5-yl) methyl 4-methylbenzenesulfonate (7) (4.86 g, 10.2 mmol) and sodium azide (663 mg, 10.2 mmol) in DMF (10 mL) was added K₂CO₃ (1.4 g, 20.4 mmol) at 25°C, then the reaction mixture was stirred at 80°C for 1 h under a nitrogen gas atmosphere, monitored by TLC. Quenched with ammonium chloride, extracted with EA, the organic layer was concentrated under reduced pressure, and the crude material was purified by silica gel column chromatography (PE: EA = 2: 1) to afford 3-(4-(8-oxa-3-aza-bicyclo[3.2.1]octan-3-yl)-3-fluorophenyl)-5-(azidomethyl)oxazoli din-2-one (8) (2.97 g, 84%) as a white solid.

LC-MS (ESI) *m*/*z* = 348 [M+H]⁺.

### Step 7: Preparation of 3-(4-(8-oxa-3-aza-bicyclo[3.2.1]octan-3-yl)-3-fluorophenyl)-5-(aminomethyl)oxazol idin-2-one (OBD-113):

To a solution of 3-(4-(8-oxa-3-aza-bicyclo[3.2.1]octan-3-yl)-3-fluorophenyl)-5-(azidomethyl)oxazoli din-2-one (8) (2.97 g, 8.5 mmol) in MeOH (10 mL) was added palladium carbon (300 mg) at 25°C, then the reaction mixture was stirred at room temperature for overnight under a hydrogen gas atmosphere, monitored by TLC. The filtrate was concentrated under reduced pressure, and the crude material was purified by silica gel column chromatography (DCM: MeOH = 50: 1) to afford 3-(4-(8-oxa-3-aza-bicyclo[3.2.1]octan-3-yl)-3-fluorophenyl)-5-(aminomethyl)oxazol idin-2-one (OBD-113) (2.2 g, 81 %) as a white solid.

¹H NMR (301 MHz, CDCl₃) δ 7.53 - 7.35 (m, 1H), 7.09 (d, *J =* 8.9 Hz, 1H), 6.96 - 6.73 (m, 1H), 4.66 (s, 1H), 4.40 (s, 1H), 4.00 (t, *J =* 8.7 Hz, 1H), 3.89 - 3.74 (m, 1H), 3.06 (dd, *J* = 21.9, 11.0 Hz, 6H), 2.27 - 1.85 (m, 4H).

LC-MS (ESI) *m*/*z* = 322 [M+H]⁺.

### Step 8: Preparation of (OBD-110, 111):

To a solution of 3-(4-(8-oxa-3-aza-bicyclo[3.2.1]octan-3-yl)-3-fluorophenyl)-5-(aminomethyl)oxazol idin-2-one (OBD-113) (200 mg, 0.62 mmol) and R-OH (0.62 mmol) in DCM (10 mL) were added HOBt (126 mg, 0.96 mmol), EDCI (237 mg, 1.24 mmol) and DIPEA (160 mg, 1.24 mmol) at 25 °C, then the reaction mixture was stirred at 25 °C for 2 h under a nitrogen gas atmosphere, monitored by TLC. Quenched with ammonium chloride, extracted with DCM, the organic layer was concentrated under reduced pressure, and the crude material was purified by silica gel column chromatography (DCM: MeOH = 80: 1) to afford (OBD-110, 111) as a white solid.

### OBD-110

¹H NMR (301 MHz, CDCl₃) δ 7.51 - 7.32 (m, 1H), 7.02 (d, *J =* 8.5 Hz, 1H), 6.83 (t, *J* = 9.1 Hz, 1H), 6.07 (s, 1H), 4.74 (s, 1H), 4.39 (s, 2H), 4.00 (t, J = 8.9 Hz, 1H), 3.85 - 3.49 (m, 3H), 3.05 (dd, *J =* 23.2, 10.6 Hz, 4H), 2.32 - 1.67 (m, 8H).

LC-MS (ESI) m/z = 426 [M+H]⁺.

### OBD-111

¹H NMR (301 MHz, CDCl₃) δ 7.41 (s, 1H), 7.03 (s, 1H), 6.17 (s, 0H), 5.20 - 5.02 (m, 1H), 4.85 - 4.62 (m, 1H), 4.41 (s, 1H), 4.01 (s, 1H), 3.68 (s, 3H), 3.09 (d, *J* = 7.9 Hz, 2H), 2.05 (d, *J =* 42.7 Hz, 3H), 1.83 - 1.35 (m, 3H).

LC-MS (ESI) m/z = 397.7 [M+H]⁺.

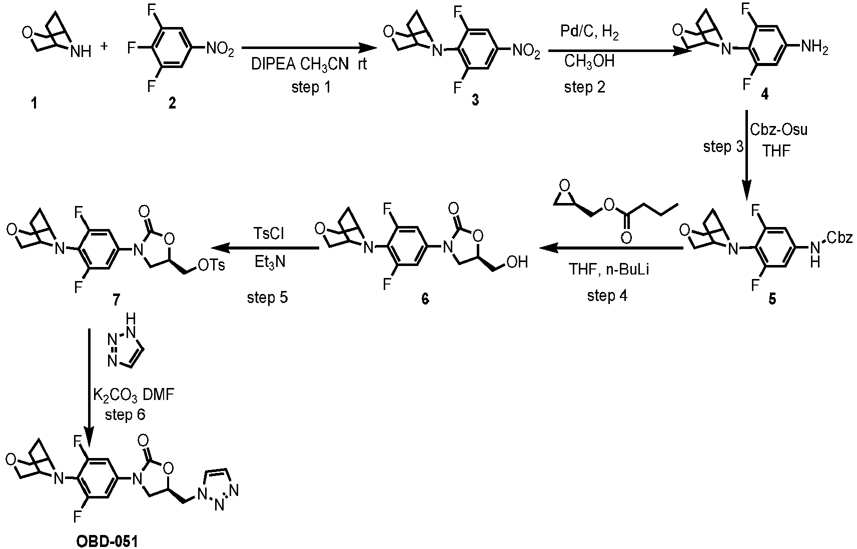

### Step 1: Preparation of 8-(2,6-difluoro-4-nitrophenyl)-3-oxa-8-aza-bicyclo[3.2.1]octane (3):

To a solution of 3-oxa-8-aza-bicyclo[3.2.1]octane (1) (5.0 g, 44.2 mmol) and 1,2,3-trifluoro-5-nitrobenzene (8.6 g, 48.6 mmol) in DMF (10 mL) was added K₂CO₃ (12.2 g, 88.4 mmol) at 25°C then the reaction mixture was stirred at 80°C for 2 h under a nitrogen gas atmosphere, monitored by TLC. The mixture was concentrated under reduced pressure, and the crude material was purified by silica gel column chromatography (EA: PE = 3: 1) to afford 8-(2,6-difluoro-4-nitrophenyl)-3-oxa-8-aza-bicyclo[3.2.1]octane (3) (9.3 g, 78%) as a yellow solid.

LC-MS (ESI) *m*/*z* = 271 [M+H]⁺.

### Step 2: Preparation of 4-(3-oxa-8-aza-bicyclo[3.2.1]octan-8-yl)-3,5-difluorobenzenamine (4):

To a solution of 3-(2,6-difluoro-4-nitrophenyl)-8-oxa-3-aza-bicyclo[3.2.1]octane (3) (9.3 g, 34.4 mmol) and Palladium carbon (1 g) in MeOH (15 mL), then under a hydrogen gas atmosphere and the reaction mixture was stirred at room temperature for overnight, monitored by TLC. The filtrate was concentrated under reduced pressure to afford 4-(3-oxa-8-aza-bicyclo[3.2.1]octan-8-yl)-3,5-difluorobenzenamine (4) (7.8 g, 95%) as a white oil, and the crude material was used for next reaction without further purification.

LC-MS (ESI) *m*/*z* = 241 [M+H]⁺.

### Step 3: Preparation of benzyl 4-(3-oxa-8-aza-bicyclo[3.2.1]octan-8-yl)-3,5-difluorophenylcarbamate (5):

Carbonic acid, 2,5-dioxo-1-pyrrolidinyl phenylmethyl ester (12 g, 48.7 mmol) was added to a suspension of 4-(3-oxa-8-aza-bicyclo[3.2.1]octan-3-yl)-3,5-difluorobenzenamine (4) (7.8 g, 32.5 mmol) in THF (100 mL) at 0 °C under a nitrogen gas atmosphere and the reaction mixture was stirred at 50°C for 5 h, monitored by TLC. The mixture was concentrated under reduced pressure, and the crude material was purified by silica gel column chromatography (PE: EA = 10: 1) to afford benzyl 4-(3-oxa-8-aza-bicyclo[3.2.1]octan-8-yl)-3,5-difluorophenylcarbamate (5) (8.2 g, 68%) as a white solid.

LC-MS (ESI) *m*/*z* = 375 [M+H]⁺.

### Step 4: Preparation of (5R)-3-(4-(3-oxa-8-aza-bicyclo[3.2.1]octan-8-yl)-3,5-difluorophenyl)-5-(hydroxyme thyl)oxazolidin-2-one (6):

To a solution of benzyl 4-(3-oxa-8-aza-bicyclo[3.2.1]octan-8-yl)-3,5-difluorophenylcarbamate (5) (8.2 g, 22.1 mmol) in THF (10 mL) at - 78 °C under a nitrogen gas atmosphere was added n-BuLi (13.8 ml, 33.1 mmol), then the mixture was stirred at -78°C for 30 min, after that the solution of (R)-oxiran-2-ylmethyl butyrate (4.7 g, 33.1 mmol) in THF was added to the mixture at -78°C, then warmed to room temperature and stirred for overnight, monitored by TLC. Quenched with ammonium chloride, extracted with EA, the organic layer was concentrated under reduced pressure, and the crude material was purified by silica gel column chromatography (DCM: MeOH = 70: 1) to afford (5R)-3-(4-(3-oxa-8-aza-bicyclo[3.2.1]octan-8-yl)-3,5-difluorophenyl)-5-(hydroxyme thyl)oxazolidin-2-one (6) (4.5 g, 60%) as a white solid.

LC-MS (ESI) *m*/*z* = 341 [M+H]⁺.

### Step 5: Preparation of ((R)-3-(4-(3-oxa-8-aza-bicyclo[3.2.1]octan-8-yl)-3,5-difluorophenyl)-2-oxooxazolidi n-5-yl)methyl 4-methylbenzenesulfonate (7):

4-methylbenzene-1-sulfonyl chloride (5 g, 26.6 mmol) was added to a suspension of (5R)-3-(4-(3-oxa-8-aza-bicyclo[3.2.1]octan-8-yl)-3,5-difluorophenyl)-5-(hydroxyme thyl)oxazolidin-2-one (6) (4.5 g, 13.3 mmol) and Et₃N (2.7 g, 26.6 mmol) in DCM (10 mL) at 0 °C under a nitrogen gas atmosphere and the reaction mixture was stirred at room temperature for overnight, monitored by TLC. Quenched with ammonium chloride, extracted with DCM, the organic layer was concentrated under reduced pressure, and the crude material was purified by silica gel column chromatography (DCM: MeOH = 50: 1) to afford ((R)-3-(4-(3-oxa-8-aza-bicyclo[3.2.1]octan-8-yl)-3,5-difluorophenyl)-2-oxooxazolidi n-5-yl)methyl 4-methylbenzenesulfonate (7) (5.58 g, 85%) as a white solid.

LC-MS (ESI) *m*/*z* = 495 [M+H]⁺.

### Step 6: Preparation of (5R)-5-((1H-1,2,3-triazol-1-yl)methyl)-3-(4-(3-oxa-8-aza-bicyclo[3.2.1]octan-8-yl)-3,5-difluorophenyl)oxazolidin-2-one (OBD-055):

To a solution of ((R)-3-(4-(3-oxa-8-aza-bicyclo[3.2.1]octan-8-yl)-3,5-difluorophenyl)-2-oxooxazolidi n-5-yl)methyl 4-methylbenzenesulfonate (7) (300 mg, 0.6 mmol) and 1H-1,2,3-triazole (42 mg, 0.6 mmol) in DMF (10 mL) was added K₂CO₃ (166 mg, 1.2 mmol) at 25°C, then the reaction mixture was stirred at 80°C for 1 h under a nitrogen gas atmosphere, monitored by TLC. Quenched with ammonium chloride, extracted with EA, the organic layer was concentrated under reduced pressure, and the crude material was purified by prep-HPLC to afford (5R)-5-((1H-1,2,3-triazol-1-yl)methyl)-3-(4-(3-oxa-8-aza-bicyclo[3.2.1]octan-8-yl)-3,5-difluorophenyl)oxazolidin-2-one (OBD-055) (82 mg, 35%) as a white solid.

¹H NMR (301 MHz, CDCl₃) δ 7.76 (d, J = 5.7 Hz, 2H), 6.94 (d, J = 12.1 Hz, 2H), 5.06 (s, 1H), 4.78 (d, J = 4.1 Hz, 1H), 4.08 (t, J = 9.0 Hz, 1H), 3.90 (t, J = 8.9 Hz, 4H), 3.58 (d, J = 10.4 Hz, 2H), 2.04 (t, J = 8.0 Hz, 4H), 1.76 (s, 2H).

LC-MS (ESI) m/z = 391.8 [M+H]⁺.

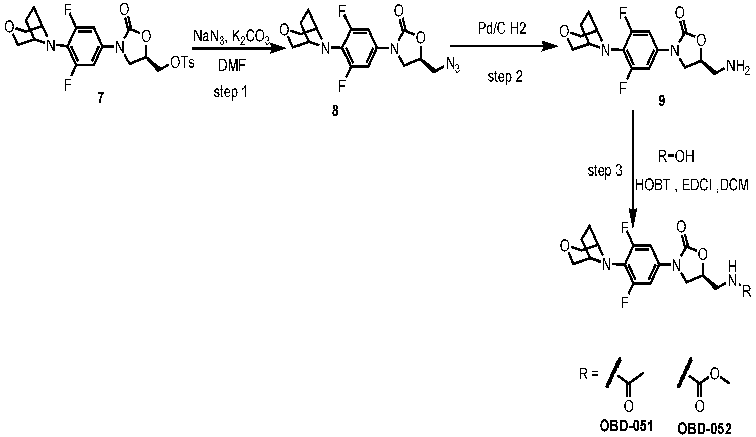

### Step 1: Preparation of (5R)-3-(4-(3-oxa-8-aza-bicyclo[3.2.1]octan-8-yl)-3,5-difluorophenyl)-5-(azidomethy l)oxazolidin-2-one (8):

To a solution of ((R)-3-(4-(3-oxa-8-aza-bicyclo[3.2.1]octan-8-yl)-3,5-difluorophenyl)-2-oxooxazolidi n-5-yl)methyl 4-methylbenzenesulfonate (7) (4 g, 8 mmol) and sodium azide (526 mg, 8 mmol) in DMF (10 mL) was added K₂CO₃ (2.2 g, 16 mmol) at 25°C, then the reaction mixture was stirred at 80°C for 1 h under a nitrogen gas atmosphere, monitored by TLC. Quenched with ammonium chloride, extracted with EA, the organic layer was concentrated under reduced pressure, and the crude material was purified by silica gel column chromatography (PE: EA = 2: 1) to afford (5R)-3-(4-(3-oxa-8-aza-bicyclo[3.2.1]octan-8-yl)-3,5-difluorophenyl)-5-(azidomethy l)oxazolidin-2-one (8) (2.4 g, 82%) as a white solid.

LC-MS (ESI) *m*/*z* = 366 [M+H]⁺.

### Step 2: Preparation of (5S)-3-(4-(3-oxa-8-aza-bicyclo[3.2.1]octan-8-yl)-3,5-difluorophenyl)-5-(aminometh yl)oxazolidin-2-one (9):

To a solution of (5R)-3-(4-(3-oxa-8-aza-bicyclo[3.2.1]octan-8-yl)-3,5-difluorophenyl)-5-(azidomethy l)oxazolidin-2-one (8) (2.4 g, 6.5 mmol) in MeOH (10 mL) was added palladium carbon (300 mg) at 25°C, then the reaction mixture was stirred at room temperature for overnight under a hydrogen gas atmosphere, monitored by TLC. The filtrate was concentrated under reduced pressure, and the crude material was purified by silica gel column chromatography (DCM: MeOH = 50: 1) to afford (5S)-3-(4-(3-oxa-8-aza-bicyclo[3.2.1]octan-8-yl)-3,5-difluorophenyl)-5-(aminometh yl)oxazolidin-2-one (9) (1.9 g, 86 %) as a white solid.

LC-MS (ESI) *m*/*z* = 340 [M+H]⁺.

### Step 3: Preparation of (OBD-051, 052):

To a solution of (5S)-3-(4-(3-oxa-8-aza-bicyclo[3.2.1]octan-8-yl)-3,5-difluorophenyl)-5-(aminometh yl)oxazolidin-2-one (9) (200 mg, 0.59 mmol) and R-OH (0.59 mmol) in DCM (10 mL) were added HOBt (119 mg, 0.88 mmol), EDCI (224 mg, 1.18 mmol) and DIPEA (152 mg, 1.18 mmol) at 25 °C, then the reaction mixture was stirred at 25 °C for 2 h under a nitrogen gas atmosphere, monitored by TLC. Quenched with ammonium chloride, extracted with DCM, the organic layer was concentrated under reduced pressure, and the crude material was purified by silica gel column chromatography (DCM: MeOH = 80: 1) to afford (OBD-051, 052) as a white solid.

### OBD-051

¹H NMR (301 MHz, CDCl₃) δ 6.99 (t, *J =* 9.3 Hz, 2H), 5.38 (s, 1H), 4.84 - 4.69 (m, 1H), 3.98 - 3.86 (m, 4H), 3.75 - 3.60 (m, 4H), 2.24 - 1.97 (m, 8H).

LC-MS (ESI) m/z = 381.9 [M+H]⁺.

### OBD-052

¹H NMR (301 MHz, CDCl₃) δ 7.06 (d, *J =* 12.4 Hz, 2H), 5.10 (s, 1H), 4.75 (s, 1H), 4.02 - 3.86 (m, 4H), 3.68 (s, 2H), 3.58 (d, *J* = 9.6 Hz, 2H), 2.02 (d, *J=* 7.7 Hz, 2H), 1.80 (s, 4H), 0.98 (d, *J =* 6.7 Hz, 3H).

LC-MS (ESI) m/z = 398.0 [M+H]⁺.

### Example 13

### Synthesis of Additional Embodiments of the Invention

In a manner similar to those disclosed in Examples 8 and 12 above, the following compounds were made:

### OTB-518

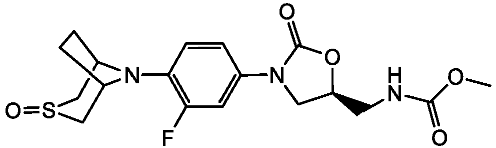

### Methyl (((5S)-3-(3-fluoro-4-((1R,5S)-3-oxido-3-thia-8-azabicyclo[3.2.1]octan-8-yl)phenyl)-2-oxooxazolidin-5-yl) methyl)carbamate

1H-NMR (400 MHz, CDCl3) δ: 7.45 (d, J = 16.4 Hz, 1 H), 7.12 (d, J = 7.6 Hz, 1 H), 6.83 (t, J = 9.2 Hz, 1 H), 5.17-5.12 (m, 1 H), 4.80-4.74 (m, 1 H), 4.62 (brs, 2 H), 4.02 (t, J = 8.4 Hz, 1 H), 3.77 (t, J = 8.0 Hz, 1 H), 3.69-3.53 (m, 4 H), 3.45 (d, J = 10.4 Hz, 2 H), 2.86 (d, J = 12.0 Hz, 2 H), 2.22 (m, 2 H), 1.90-1.88 (m, 2 H).

HRMS (ESI): m/z [M + H]+ calcd for C₁₈H₂₃FN₃O₃S: 412.1344; found: 412.1359

### OTB-519

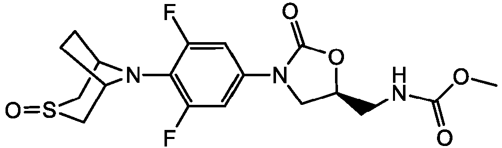

### Methyl (((5S)-3-(3,5-difluoro-4-((1R,5S)-3-oxido-3-thia-8-azabicyclo[3.2.1]octan-8-yl)phenyl)-2-oxooxazolidin-5-yl) methyl)carbamate

1H-NMR (400 MHz, CDCl3) δ: 7.13 (d, *J =* 12.4 Hz, 2 H), 5.10 (m, 1 H), 4.80-4.74 (m, 1 H), 4.46 (brs, 2 H), 3.98 (t, J = 8.8 Hz, 1 H), 3.76-3.69 (m, 4 H), 3.60-3.49 (m, 3 H), 2.94 (d, J = 12.0 Hz, 2 H), 2.20-2.18 (m, 2 H), 1.87-1.85 (m, 2 H).

HRMS (ESI): m/z [M + H]+ calcd for C₁₈H₂₂F₂N₃O₅S: 430.1248; found: 430.1259

### OTB-517

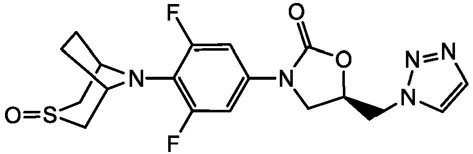

### (5R)-5-((1H-1,2,3-Triazol-1-yl)methyl)-3-(3,5-difluoro-4-((1R,5S)-3-oxido-3-thia-8-azabicyclo[3.2.1] octan-8-yl)phenyl)oxazolidin-2-one

1H-NMR (400 MHz, CDCl3) δ: 7.79 (s, 1 H), 7.77 (s, 1 H), 6.98 (d, *J* = 12.0 Hz, 2 H), 5.10-5.04 (m, 1 H), 4.79 (d, J = 4.0 Hz, 2 H), 4.43 (brs, 2 H), 4.10 (t, J = 9.2 Hz, 1 H), 3.91-3.87 (m, 1 H), 3.55 (d, J = 12.4 Hz, 2 H), 2.92 (d, J = 10.4 Hz, 2 H), 2.19-2.16 (m, 2 H), 1.88-1.83 (m, 2 H).

HRMS (ESI): m/z [M + H]+ calcd for C₁₈H₂₀F₂N₃O₃S: 424.1249; found: 424.1271

### OTB-523

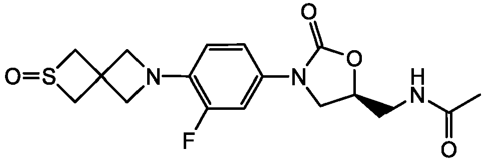

### (S)-N-((3-(3-Fluoro-4-(2-oxido-2-thia-6-azaspiro[3.3]heptan-6-yl)phenyl)-2-oxooxazolidin-5-yl) methyl)acetamide

1H-NMR (400 MHz, CDCl3) δ: 7.34 (d, *J =* 14.0 Hz, 1 H), 7.01 (d, J = 8.8 Hz, 1 H), 6.41 (t, J = 8.8 Hz, 1 H), 6.12 (t, J = 6.0 Hz, 1 H), 4.76-4.73 (m, 1 H), 4.01-3.90 (m, 7 H), 3.74-3.66 (m, 2 H), 3.62-3.56 (m, 1 H), 3.45-3.40 (m, 2 H), 2.02 (s, 3 H).

HRMS (ESI): m/z [M + H]+ calcd for C17H21FN304S: 382.1237; found: 382.1217

### OTB-515

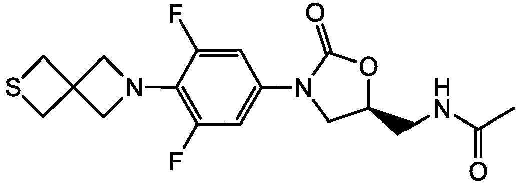

### (S)-N-((3-(3,5-Difluoro-4-(2-thia-6-azaspiro[3.3]heptan-6-yl)phenyl)-2-oxooxazolidin-5-yl) methyl)acetamide

HNMR (400 MHz, CDCl3) δ: 7.03-6.94 (m, 2 H), 6.09 (t, J = 5.6 Hz, 1 H), 4.75 (q, J = 3.2 Hz, J = 2.8 Hz, 1 H), 4.16 (s, 4 H), 3.95 (t, J = 8.8 Hz, 1 H), 3.72-3.61 (m, 3 H), 3.40 (s, 4 H), 2.03 (s, 3 H).

m/z [M + Na]+ calcd for C₁₇H₁₉F₂N₃O₃S:383.1115; found: 384.0

### OTB-248

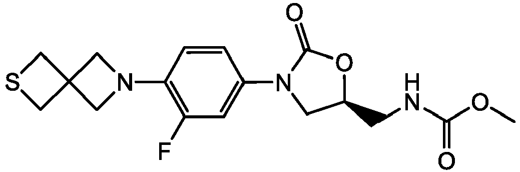

### (S)-Methyl ((3-(3-fluoro-4-(2-thia-6-azaspiro[3.3]heptan-6-yl)phenyl)-2-oxooxazolidin-5-yl)methyl)carbamate

HNMR (400 MHz, CDCl3) δ: 7.33 (q, J = 2.0 Hz, J = 11.6 Hz, 2 H), 7.01 (d, J = 2.0 Hz, 1 H), 6.44 (t, J = 9.2 Hz, 1 H), 5.15 (bs, 1 H), 4.77-4.73 (m, 1 H), 4.01-3.97 (m, 4 H), 3.76-3.52 (m, 6 H), 3.42 (s, 4 H).

m/z [M + Na]+ calcd for C₁₇H₂₀FN₃O₄S:381.1159; found: 404.1

### OTB-256

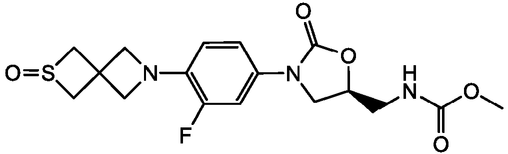

### (S)-Methyl ((3-(3-fluoro-4-(2-oxido-2-thia-6-azaspiro[3.3]heptan-6-yl)phenyl)-2-oxooxazolidin-5-yl) methyl)carbamate

1H-NMR (400 MHz, CDCl3) δ: 7.51 (d, J = 14.4 Hz, 1 H), 7.04 (d, J = 8.4 Hz, 1 H), 6.80 (t, J = 8.8 Hz, 1 H), 5.09 (brs, 1 H), 4.76 (brs, 1 H), 4.16 (d, J = 13.2 Hz, 4 H), 4.01-3.98 (m, 3 H), 3.77-3.75 (m, 1 H), 3.69 (s, 3 H), 3.61-3.55 (m, 2 H), 3.46-3.44 (m, 2 H).

HRMS (ESI): m/z [M + H]+ calcd for C₁₇H₂₁FN₃O₅S: 398.1186; found: 398.1166

### OTB-247

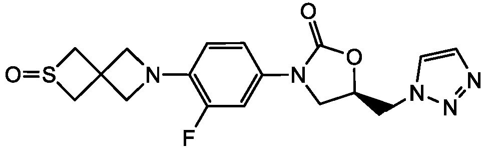

### (R)-5-((1H-1,2,3-Triazol-1-yl)methyl)-3-(3-fluoro-4-(2-oxido-2-thia-6-azaspiro[3.3]heptan-6-yl) phenyl)oxazolidin-2-one

HNMR(400 MHz, CDCl3) δ: 7.78 (d, J = 0.8 Hz, 1H), 7.74 (d, J = 0.8 Hz, 1H), 7.18 (dd, J = 13.6, 2.4 Hz, 1H), 6.87 (dd, J = 8.8, 1.6 Hz, 1H), 6.37 (t, J = 9.2 Hz, 1H), 5.04 - 5.00 (m, 1H), 4.77 (d, J = 3.6 Hz, 2H), 4.08 (t, J = 9.2 Hz, 1H), 3.96 (dd, J = 10.4, 1.6 Hz, 4H), 3.92 - 3.89 (m, 3H), 3.42 - 3.39 (m, 2H).

m/z [M + H]+ calcd for C₁₇H₁₈FN₅O₃S: 391.1114; found: 392.0

### OTB-249

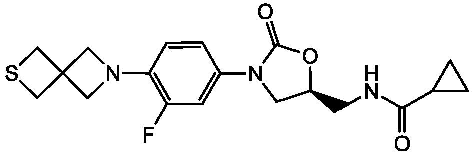

### (S)-N-((3-(3-Fluoro-4-(2-thia-6-azaspiro[3.3]heptan-6-yl)phenyl)-2-oxooxazolidin-5-yl) methyl)cyclopropanecarboxamide

1H-NMR (400 MHz, CDCl3) δ: 7.33 (d, J = 14.0 Hz, 1 H), 6.99 (d, J = 8.8 Hz, 1 H), 6.47 (t, J = 9.2 Hz, 1 H), 6.13 (brs, 1 H), 4.73 (m, 1 H), 3.98-3.95 (m, 5 H), 3.73-3.66 (m, 3 H), 3.42 (s, 4 H), 1.39-1.37 (m, 1 H), 0.97-0.91 (m, 2 H), 0.78-0.76 (m, 2 H).

HRMS (ESI): m/z [M + H]+ calcd for C₁₉H₂₃FN₃O₃S: 392.1444; found: 392.1426

### OTB-255

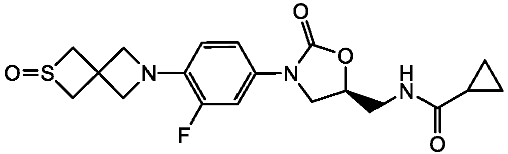

### (S)-N-((3-(3-Fluoro-4-(2-oxido-2-thia-6-azaspiro[3.3]heptan-6-yl)phenyl)-2-oxooxazolidin-5-yl) methyl)cyclopropanecarboxamide

1H-NMR (400 MHz, CDCl3) δ: 7.35 (d, J = 14.0 Hz, 1 H), 7.01 (d, J = 8.4 Hz, 1 H), 6.44 (t, J = 9.2 Hz, 1 H), 6.13 (brs, 1 H), 4.74 (m, 1 H), 4.00-3.91 (m, 7 H), 3.75-3.62 (m, 3 H), 3.47-3.41 (m, 2 H), 1.38-1.37 (m, 1 H), 0.97-0.92 (m, 2 H), 0.78-0.76 (m, 2 H).

HRMS (ESI): m/z [M + H]+ calcd for C19H23FN304S: 408.1393; found: 408.1378

### OTB-250

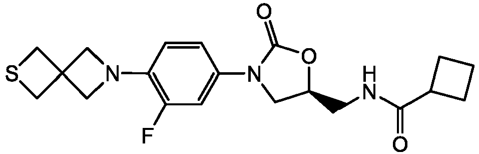

### (S)-N-((3-(3-Fluoro-4-(2-thia-6-azaspiro[3.3]heptan-6-yl)phenyl)-2-oxooxazolidin-5-yl) methyl)cyclobutanecarboxamide

HNMR (400 MHz, CDCl3) δ: 7.76 (d, J = 8.8 Hz, 2 H), 6.92-6.83 (m, 2 H), 5.04 (m, 1 H), 4.78 (q, J = 0.8 Hz, J = 3.2 Hz 2 H), 4.15 (t, J = 2.4 Hz, 4 H), 3.85 (t, J = 6.0 Hz, 1 H), 3.72-3.63 (m, 3 H), 3.40 (s, 4 H), 3.05-2.99 (m, 1 H), 2.24-2.13 (m, 4 H), 1.97-1.60 (m ,2 H).

m/z [M + Na]+ calcd for C₂₀H₂₄FN₃O₃S: 405.1522; found: 428.2

### OTB-254

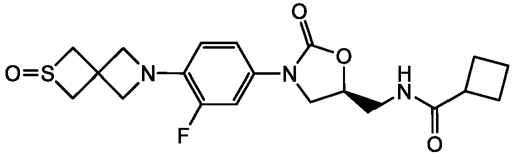

### (S)-N-((3-(3-fluoro-4-(2-oxido-2-thia-6-azaspiro[3.3]heptan-6-yl)phenyl)-2-oxooxazolidin-5-yl) methyl)cyclobutanecarboxamide

1H-NMR (400 MHz, CDCl3) δ: 7.36 (d, J = 14.4 Hz, 1 H), 7.00 (d, J = 8.0 Hz, 1 H), 6.47 (t, J = 9.2 Hz, 1 H), 5.80 (brs, 1 H), 4.73 (brs, 1 H), 4.00 (d, J = 12.0 Hz, 4 H), 3.95-3.92 (m, 3 H), 3.76-3.72 (m, 1 H), 3.65-3.62 (m, 2 H), 3.47-3.41 (m, 2 H), 3.02-2.96 (m, 1 H), 2.26-2.13 (m, 4 H), 1.98-1.84 (m, 2 H).

HRMS (ESI): m/z [M + H]+ calcd for C₂₀H₂₅FN₃O₄S: 422.1549; found: 422.1531

### OTB-260-2A

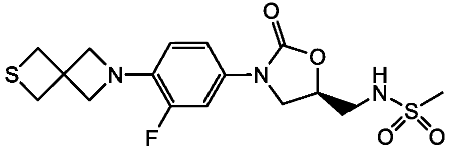

### (R)-N-((3-(3-Fluoro-4-(2-thia-6-azaspiro[3.3]heptan-6-yl)phenyl)-2-oxooxazolidin-5-yl)methyl) methanesulfonamide

HNMR (400 MHz, CDCl3) δ: 7.04 - 6.95 (m, 2H), 5.05 (s, 1H), 4.80 - 4.77 (m, 1H), 4.15 (t, J = 2.4 Hz, 4H), 3.97 (t, J = 8.8 Hz, 1H), 3.86 (dd, J = 6.4, 8.8 Hz, 1H), 3.56 (dd, J = 3.6, 14.4 Hz, 1H), 3.43 - 3.39 (m, 5H), 3.01 (s, 3H).

m/z [M + H]+ calcd for C₁₆H₂₀FN₃O₄S₂: 401.0879; found: 402.1

### OTB-260-2B

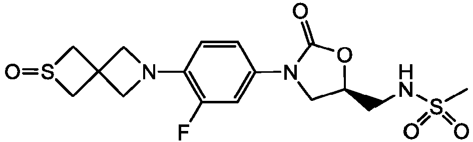

### (R)-N-((3-(3-Fluoro-4-(2-oxido-2-thia-6-azaspiro[3.3]heptan-6-yl)phenyl)-2-oxooxazolidin-5-yl) methyl)methanesulfonamide

HNMR (400 MHz, CDCl₃) δ: 7.32 (dd, J = 14.0, 2.4 Hz, 1H), 7.04 - 7.02 (m, 1H), 6.41 (t, J = 9.6 Hz, 1H), 4.86 - 4.77 (m, 2H), 4.04 - 3.95 (m, 8H), 3.93 (dd, J = 9.6, 3.2 Hz, 1H), 3.43 - 3.40 (m, 3H), 3.02 (s, 3H).

m/z [M + H]+ calcd for C₁₆H₂₀FN₃O₅S₂: 417.0828; found: 418.0

### OTB-260-5A

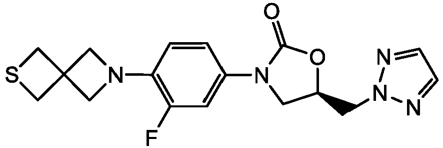

### (R)-5-((2H-1,2,3-Triazol-2-yl)methyl)-3-(3-fluoro-4-(2-thia-6-azaspiro[3.3]heptan-6-yl) phenyl)oxazolidin-2-one

HNMR (400 MHz, CDCl₃) δ: 7.64 (s, 2H), 7.24 - 7.21 (m, 1H), 6.99 (dd, J = 8.8, 1.6 Hz, 1H), 6.41 (t, J = 9.6 Hz, 1H), 5.12 - 5.06 (m, 1H), 4.87 - 4.82 (m, 1H), 4.75 - 4.72 (m, 1H), 4.05 - 4.01 (m, 1H), 3.96 - 3.93 (m, 5H), 3.40 (s, 4H).

m/z [M + H]+ calcd for C₁₇H₁₈FN₅O₂S: 375.1165; found: 376.1

### OTB-260-5B

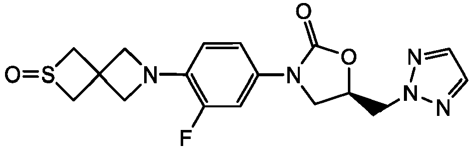

### (R)-5-((2H-1,2,3-Triazol-2-yl)methyl)-3-(3-fluoro-4-(2-oxido-2-thia-6-azaspiro[3.3]heptan-6-yl) phenyl)oxazolidin-2-one

HNMR (400 MHz, CDCl₃) δ: 7.64 (s, 2H), 7.27 - 7.23 (m, 1H), 6.99 (dd, J = 8.8, 2.0 Hz, 1H), 6.39 (t, J = 9.2 Hz, 1H), 5.11 - 5.06 (m, 1H), 4.87 - 4.82 (m, 1H), 4.76 - 4.70 (m, 1H), 4.06 - 4.03 (m, 1H), 3.98 - 3.90 (m, 7H), 3.43 - 3.40 (m, 2H).

m/z [M + H]+ calcd for C₁₇H₁₈FN₅O₃S: 391.1114; found: 392.1

### OTB-260-4A

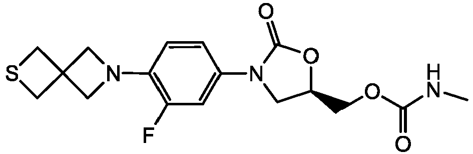

### (R)-(3-(3-Fluoro-4-(2-thia-6-azaspiro[3.3]heptan-6-yl)phenyl)-2-oxooxazolidin-5-yl)methyl methylcarbamate

HNMR (400 MHz, CDCl₃) δ: 7.33 (dd, J = 2.4, 13.6 Hz, 1H), 7.04 (dd, J = 1.6, 8.4 Hz, 1H), 6.44 (t, J = 9.2 Hz, 1H), 4.88 - 4.72 (m, 2H), 4.33 (t, J = 4.0 Hz, 2H), 4.02 (t, J = 9.2 Hz, 1H), 3.97 (d, J = 1.6 Hz, 4H), 3.77 (dd, J = 6.4, 8.8 Hz, 1H), 3.42 (s, 4H), 2.80 (d, J = 4.8 Hz, 3H).

m/z [M + H]+ calcd for C₁₇H₂₀FN₃O₄S: 381.1159; found: 382.0

### OTB-260-4B

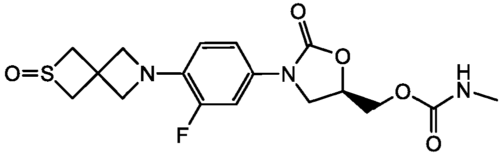

### (R)-(3-(3-Fluoro-4-(2-oxido-2-thia-6-azaspiro[3.3]heptan-6-yl)phenyl)-2-oxooxazolidin-5-yl)methyl methylcarbamate

HNMR (400 MHz, CDCl₃) δ: 7.35 (dd, J = 2.0, 11.6 Hz, 1H), 7.06 (d, J = 8.4 Hz, 1H), 6.42 (t, J = 9.2 Hz, 1H), 4.89 - 4.70 (m, 2H), 4.42 - 4.26 (m, 2H), 4.08 - 3.88 (m, 7H), 3.84 - 3.71 (m, 1H), 3.47 - 3.37 (m, 2H), 2.81 (m, 3H).

m/z [M + H]+ calcd for C₁₇H₂₀FN₃O₅S: 397.1108; found: 398.0

### OTB-520

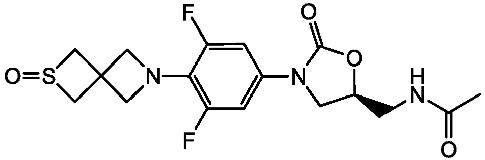

### (S)-N-((3-(3,5-Difluoro-4-(2-oxido-2-thia-6-azaspiro[3.3]heptan-6-yl)phenyl)-2-oxooxazolidin-5-yl)methyl)acetamide

1H-NMR (400 MHz, CDCl3) δ: 7.02 (d, J = 12.0 Hz, 2 H), 5.92 (brs, 1 H), 4.75-4.74 (m, 1 H), 4.16 (d, J = 12.0 Hz, 4 H), 3.97-3.90 (m, 2 H), 3.72-3.65 (m, 4 H), 3.41-3.37 (m, 2 H), 2.02 (s, 3 H).

HRMS (ESI): m/z [M + H]+ calcd for C₁₇H₂₀F₂N₃O₄S: 400.1143; found: 400.1158

### OTB-253

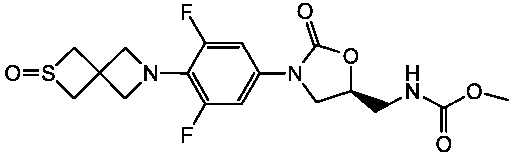

### (S)-Methyl ((3-(3,5-difluoro-4-(2-oxido-2-thia-6-azaspiro[3.3]heptan-6-yl)phenyl)-2-oxooxazolidin-3-yl)methyl)carbamate

1H-NMR (400 MHz, CDCl3) δ: 7.00 (d, J = 10.8 Hz, 2H), 5.36 (m, 1H), 4.72 (m, 1H), 4.14 (d, J = 12.0 Hz, 4H), 3.92 (m, 3H), 3.69 (m, 1H), 3.67 (s, 3H), 3.52 (m, 2H), 3.39 (d, J = 12.4 Hz, 2H).

HRMS (ESI) calcd for C₁₇H₂₀F₂N₃O₅S [M+H]+ 416.1086, found: 416.1073

### OTB-522

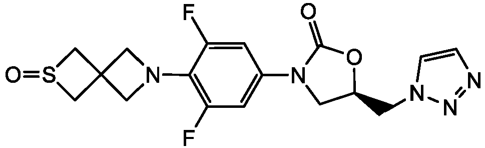

HNMR (400 MHz, CDCl₃) δ: 7.75 (d, J = 8.0 Hz, 2H), 6.92 - 6.83 (m, 2H), 5.05 - 5.01 (m, 1H), 4.77 (d, J = 4.0 Hz, 2H), 4.15 (dt, J = 11.6, 2.4 Hz, 4H), 4.05 (t, J = 9.2 Hz, 1H), 3.92 - 3.89 (m, 3H), 3.40 - 3.37 (m, 2H).

m/z [M + H]+ calcd for C₁₇H₁₇F₂N₅O₃S: 409.1020; found: 410.1

### OTB-252

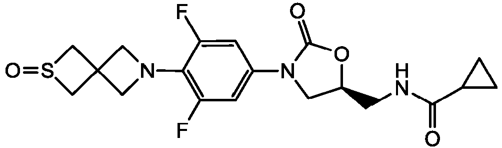

### (S)-N-((3-(3,5-Difluoro-4-(2-oxido-2-thia-6-azaspiro[3.3]heptan-6-yl)phenyl)-2-oxooxazolidin-5-yl)methyl)cyclopropanecarboxamide

1H-NMR (400 MHz, CDCl3) δ: 6.98 (d, J = 11.6 Hz, 2H), 6.60 (m, 1H), 4.73 (m, 1H), 4.13 (d, J = 12.4 Hz, 4H), 3.91 (m, 3H), 3.70 (m, 1H), 3.64 (m, 2H), 3.79 (d, J = 10.4 Hz, 2H),1.41 (m, 1H), 0.94 (m, 1H), 0.87 (m, 1H), 0.74 (m, 2H)
HRMS (ESI) calcd for C₁₉H₂₂F₂N₃O₄S [M+H]+ 426.1294, found: 426.1278

### OTB-251

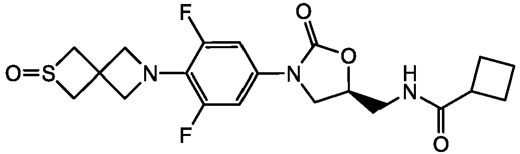

### (S)-N-((3-(3,5-Difluoro-4-(2-oxido-2-thia-6-azaspiro[3.3]heptan-6-yl)phenyl)-2-oxooxazolidin-5-yl)methyl)cyclobutanecarboxamide

1H-NMR (400 MHz, CDCl3) δ: 6.99 (d, J = 10.4 Hz, 2H), 6.06 (m, 1H), 4.74 (m, 1H), 4.14 (d, J = 12.4 Hz, 4H), 3.93 (m, 3H), 3.70 (m, 1H), 3.62 (m, 2H), 3.38 (d, J = 12.4 Hz, 2H), 3.00 (m, 1H), 2.21 (m, 1H), 2.11 (m, 3H), 1.92 (m, 1H), 1.83 (m, 1H)
HRMS (ESI) calcd for C₂₀H₂₄F₂N₃O₄S [M+H]+ 440.1450, found: 440.1441

### OTB-516-2A

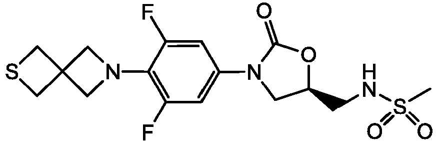

### (R)-N-((3-(3,5-Difluoro-4-(2-thia-6-azaspiro[3.3]heptan-6-yl)phenyl)-2-oxooxazolidin-5-yl)methyl)methanesulfonamide

HNMR (400 MHz, CDCl3) δ: 7.04 - 6.95 (m, 2H), 5.05 (s, 1H), 4.80 - 4.77 (m, 1H), 4.15 (t, J = 2.4 Hz, 4H), 3.97 (t, J = 8.8 Hz, 1H), 3.86 (dd, J = 6.4, 8.8 Hz, 1H), 3.56 (dd, J = 3.6, 14.4 Hz, 1H), 3.43 - 3.39 (m, 5H), 3.01 (s, 3H).

m/z [M + H]+ calcd for C₁₆H₁₉F₂N₃O₄S₂: 419.0785; found: 420.1

### OTB-516-2B

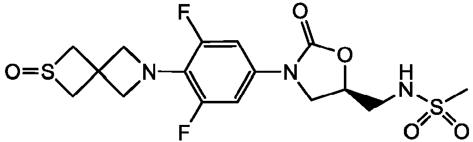

### (R)-N-((3-(3,5-Difluoro-4-(2-oxido-2-thia-6-azaspiro[3.3]heptan-6-yl)phenyl)-2-oxooxazolidin-5-yl)methyl)methanesulfonamide

HNMR (400 MHz, CDCl3) δ: 7.03 - 7.00 (m, 2H), 4.81 - 4.78 (m, 2H), 4.18 - 4.14 (m, 4H), 3.98 - 3.93 (m, 1H), 3.91 - 3.85 (m, 3H), 3.60-3.41 (m, 4H), 3.40 - 3.37 (m, 3H), 3.02 (s,3H).

m/z [M + H]+ calcd for C₁₆H₁₉F₂N₃O₅S₂: 435.0734; found: 436.0

### OTB-516-4A

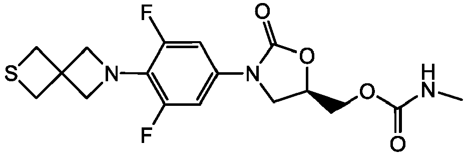

### (R)-(3-(3,5-Difluoro-4-(2-thia-6-azaspiro[3.3]heptan-6-yl)phenyl)-2-oxooxazolidin-5-yl)methyl methylcarbamate

HNMR (400 MHz, CDCl₃) δ: 7.08 - 6.91 (m, 2H), 4.91 - 4.70 (m, 2H), 4.38 - 4.28 (m, 2H), 4.16 (t, J = 2.4 Hz, 4H), 3.98 (t, J = 9.2 Hz, 1H), 3.74 (dd, J = 6.4, 8.8 Hz, 1H), 3.43 - 3.37 (m, 4H), 2.81 (d, J = 4.8 Hz, 3H).

m/z [M + H]+ calcd for C₁₇H₁₉F₂N₃O₄S: 399.1064; found: 400.1

### OTB-516-4B

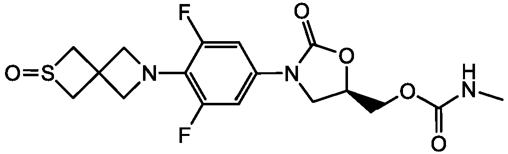

### (R)-(3-(3,5-Difluoro-4-(2-oxido-2-thia-6-azaspiro[3.3]heptan-6-yl)phenyl)-2-oxooxazolidin-5-yl) methyl methylcarbamate

HNMR (400 MHz, CDCl₃) δ: 7.10 - 6.95 (m, 2H), 4.89 - 4.69 (m, 2H), 4.30 - 4.36 (m, 2H), 4.17 (d, J = 11.6 Hz, 4H), 4.02 - 3.89 (m, 3H), 3.74 (dd, J = 6.4, 8.6 Hz, 1H), 3.44 - 3.33 (m, 2H), 2.81 (d, J = 4.8 Hz, 3H).

m/z [M + H]+ calcd for C₁₇H₁₉F₂N₃O₅S: 415.1013; found: 416.0

### OTB-204

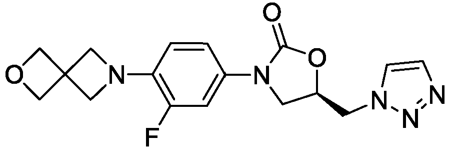

### (R)-5-((1H-1,2,3-tTriazol-1-yl)methyl)-3-(3-fluoro-4-(2-oxa-6-azaspiro[3.3]heptan-6 -yl)phenyl)oxazolidin-2-one

1H-NMR (400 MHz, CDCl3) δ: 7.65 (s, 2 H), 7.31 (d, J = 14.4, 2.4 Hz, 1 H), 6.99 (d, J = 8.4 Hz, 1 H), 6.59 (t, J = 9.2 Hz, 1 H), 5.14-5.07 (m, 1 H), 4.88-4.83 (m, 5H), 4.77-4.71 (m, 1 H), 4.16 (s, 4 H), 4.07-4.02 (m, 1 H), 3.98-3.92 (m, 1 H)
HRMS (ESI): m/z [M + H]+calcd for C₁₇H₁₉FN₅O₃: 360.1472; found: 360.1451

## Claims

1. A compound of Formula I, or a pharmaceutically acceptable salt, hydrate, or solvate of: 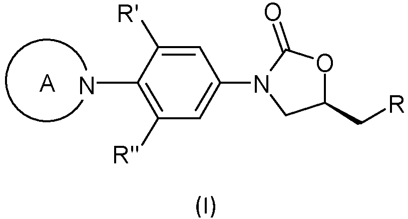 wherein:
R is independently OR₁, OC(O)R₂, OC(O)NHR₂, OS(O₂)R₂, NHS(O)₂R₂, NR₃R₄;
R' and R" are independently H, F, Cl or OMe;
each R₁ is independently H, C₁-C₆ alkyl, C₃-C₈ cycloalkyl, wherein said alkyl, cycloalkyl are optionally substituted with 1 to 4 groups selected from halo, hydroxy, C₁-C₆ alkyl, C₁-C₆ alkyloxy;
each R₂ is independently C₁-C₆ alkyl, C₃-C₈ cycloalkyl, heterocyclyl, heteroaryl or aryl, wherein said alkyl, cycloalkyl, heterocyclyl, heteroaryl, or aryl are optionally substituted with 1 to 4 groups selected from halo, hydroxyl, C₁-C₆ alkyl, C₁-C₆ alkoxy, C₁-C₆ acyloxy, CF₃, NO₂, CN and NH₂;
each R₃ is C₁-C₆ alkyl, C₃-C₈ cycloalkyl, heterocyclyl, heteroaryl, or aryl, and R₄ is H, C₁-C₆ alkyl, C₃-C₈ cycloalkyl, heterocyclyl, heteroaryl, or aryl; wherein said alkyl, cycloalkyl, heterocyclyl, heteroaryl, or aryl are optionally substituted with 1 to 4 groups selected from halo, C₁-C₆ alkyl, C₁-C₆ alkoxy, CF₃, NO₂, CN;
Ring A is 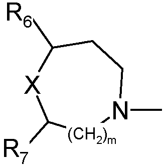
wherein,
each R₆ and R₇ is independently H, F, CH₃, CH₂CH₃, CF₃, phenyl;
X = O, S, SO, SO₂;
m is 1, or 2.

2. The compound of claim 1, wherein the compound is represented by Formula II: 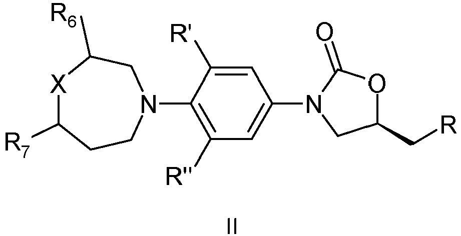 wherein,
R is independently OR₁, OC(O)R₂, NR₃R₄, NHS(O)₂R₂;
R' and R" are independently H, or F;
R₁ is independently H, C₁-C₆ alkyl, C₃-C₆ cycloalkyl;
R₂ is independently C₁-C₆ alkyl, C₃-C₆ cycloalkyl;
R₃ is C₁-C₆ alkyl, C₃-C₆ cycloalkyl, phenyl, and R₄ is H, C₁-C₆ alkyl, C₃-C₆ cycloalkyl, phenyl;
R₆ and R₇ is independently H, F, CH₃, CH₂CH₃, CF₃;
X = O, S, SO, SO₂.

3. The compound of claim 1, wherein the compound is represented by Formula III: 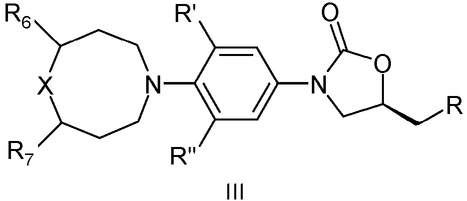 wherein,
R is independently OR₁, OC(O)R₂, NR₃R₄, NHS(O)₂R₂;
R' and R" are independently H, or F;
R₁ is independently H, C₁-C₆ alkyl, C₃-C₆ cycloalkyl;
R₂ is independently C₁-C₆ alkyl, C₃-C₆ cycloalkyl;
R₃ is C₁-C₆ alkyl, C₃-C₆ cycloalkyl, or phenyl, and R₄ is H, C₁-C₆ alkyl, C₃-C₆ cycloalkyl, or phenyl;
R₆ and R₇ is independently H, F, CH₃, CH₂CH₃, CF₃;
X = O, S, SO, SO₂;

4. The compound of claim 1, the compound is represented by Formula Ila, IIb, Illa, Illb, IVa, IVb, Va, Vb, IXa, or IXb: 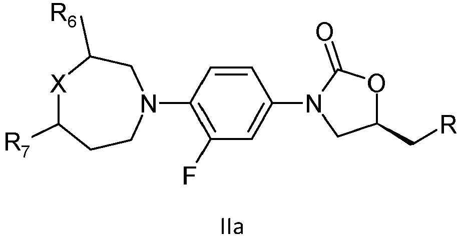 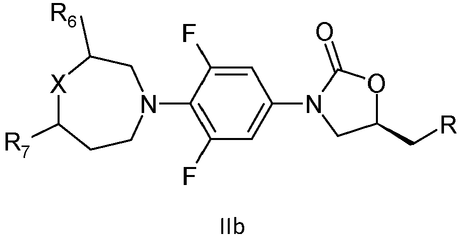 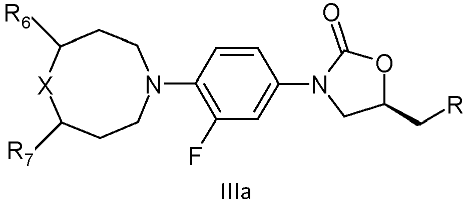 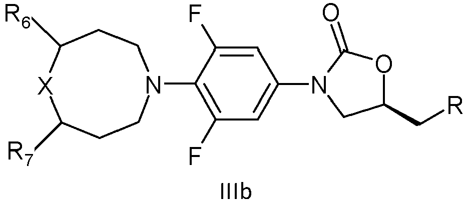 wherein,
R is independently OH, OCH₃, OCH₂CH₃, OC(O)CH₃, NHCH₃, NHC₆H₅, NHS(O)₂R₃;
R₂ is independently C₁-C₆ alkyl; and
X = O, S, SO, SO₂.

5. The compound of claim 1, wherein the compound is:
| | |
|---|---|
| OTB-109 | 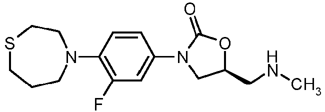 |
| OTB-108 | 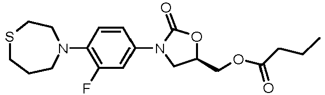 |
| OTB-116 | 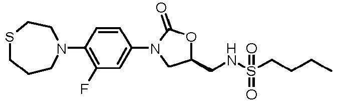 |
| OTB-126 | 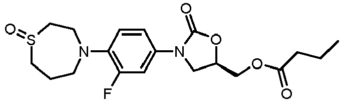 |
| OTB-110 | 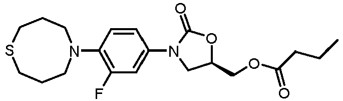 and |
| OTB-120 | 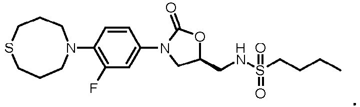 |

6. A pharmaceutical composition comprising at least one compound of Formula I, or a salt, hydrate, or solvate thereof, as defined in any one of claims 1 to 5, and one or more pharmaceutically acceptable carriers and/or additives.

7. The pharmaceutical composition comprising at least one compound of Formula I, or a salt, hydrate, or solvate thereof, as defined in claim 6, further comprising one or more additional anti-infective treatments.

8. A compound of Formula I, or a salt, hydrate, or solvate thereof, as defined in any one of claims 1 to 5, for use in preventing or treating microbial infections in humans.

9. The compound for use of claim 8, wherein the microbial infection is caused by *Mycobacterium tuberculosis.*

## Patentansprüche

1. Eine Verbindung der Formel I oder ein pharmazeutisch verträgliches Salz, Hydrat oder Solvat davon: 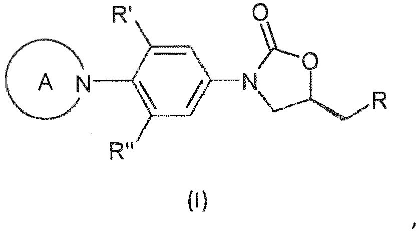 wobei:
R unabhängig OR₁, OC(O)R₂, OC(O)NHR₂, OS(O₂)R₂, NHS(O)₂R₂, NR₃R₄ ist;
R' und R" unabhängig H, F, Cl oder OMe sind;
jedes R₁ unabhängig H, C₁-C₆-Alkyl, C₃-C₈-Cycloalkyl ist, wobei Alkyl, Cycloalkyl gegebenenfalls mit 1 bis 4 Gruppen substituiert sind, die aus Halogen, Hydroxy, C₁-C₆-Alkyl, C₁-C₆-Alkyloxy ausgewählt sind;
jedes R₂ unabhängig C₁-C₆-Alkyl, C₃-C₈-Cycloalkyl, Heterocyclyl, Heteroaryl oder Aryl ist, wobei Alkyl, Cycloalkyl, Heterocyclyl, Heteroaryl oder Aryl gegebenenfalls mit 1 bis 4 Gruppen substituiert sind, die aus Halogen, Hydroxyl, C₁-C₆-Alkyl, C₁-C₆-Alkoxy, C₁-C₆-Acyloxy, CF₃, NO₂, CN und NH₂ ausgewählt sind;
jedes R₃ C₁-C₆-Alkyl, C₃-C₈-Cycloalkyl, Heterocyclyl, Heteroaryl oder Aryl ist, und R₄ H, C₁-C₆-Alkyl, C₃-C₈-Cycloalkyl, Heterocyclyl, Heteroaryl oder Aryl ist; wobei Alkyl, Cycloalkyl, Heterocyclyl, Heteroaryl oder Aryl gegebenenfalls mit 1 bis 4 Gruppen substituiert sind, die aus Halogen, C₁-C₆-Alkyl, C₁-C₆-Alkoxy, CF₃, NO₂, CN ausgewählt sind; 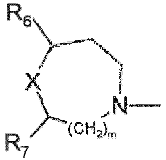 Ring A ist,
wobei
jedes R₆ und R₇ unabhängig H, F, CH₃, CH₂CH₃, CF₃, Phenyl ist;
X = O, S, SO, SO₂;
m 1 oder 2 ist.

2. Die Verbindung nach Anspruch 1, wobei die Verbindung dargestellt ist durch Formel II: 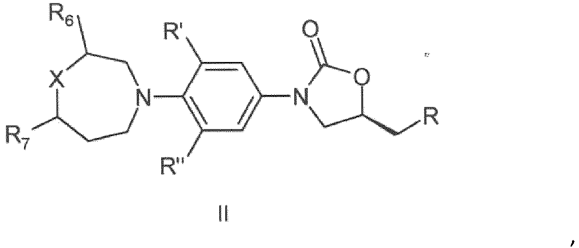 wobei
R unabhängig OR₁, OC(O)R₂, NR₃R₄, NHS(O)₂R₂ ist;
R' und R" unabhängig H oder F sind;
R₁ unabhängig H, C₁-C₆-Alkyl, C₃-C₆-Cycloalkyl ist;
R₂ unabhängig C₁-C₆-Alkyl, C₃-C₆-Cycloalkyl ist;
R₃ C₁-C₆-Alkyl, C₃-C₆-Cycloalkyl, Phenyl ist, und R₄ H, C₁-C₆-Alkyl, C₃-C₆-Cycloalkyl, Phenyl ist;
R₆ und R₇ unabhängig H, F, CH₃, CH₂CH₃, CF₃ ist;
X = O, S, SO, SO₂.

3. Die Verbindung nach Anspruch 1, wobei die Verbindung dargestellt ist durch Formel III: 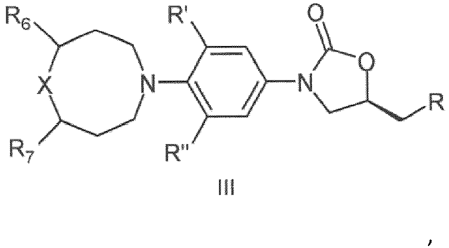 wobei
R unabhängig OR₁, OC(O)R₂, NR₃R₄, NHS(O)₂R₂ ist;
R' und R" unabhängig H oder F sind;
R₁ unabhängig H, C₁-C₆-Alkyl, C₃-C₆-Cycloalkyl ist;
R₂ unabhängig C₁-C₆-Alkyl, C₃-C₆-Cycloalkyl ist;
R₃ C₁-C₆-Alkyl, C₃-C₆-Cycloalkyl oder Phenyl ist, und R₄ H, C₁-C₆-Alkyl, C₃-C₆-Cycloalkyl oder Phenyl ist;
R₆ und R₇ unabhängig H, F, CH₃, CH₂CH₃, CF₃ ist;
X = O, S, SO, SO₂.

4. Die Verbindung nach Anspruch 1, wobei die Verbindung dargestellt ist durch Formel IIa, IIb, IIIa, IIIb, IVa, IVb, Va, Vb, IXa oder IXb: 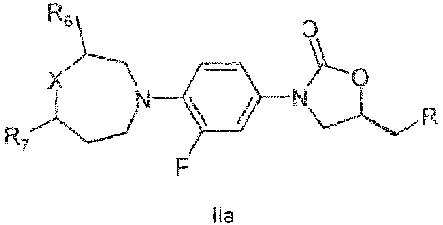 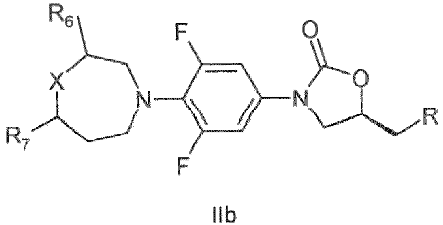 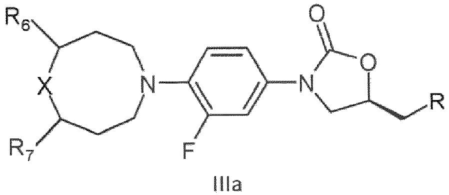 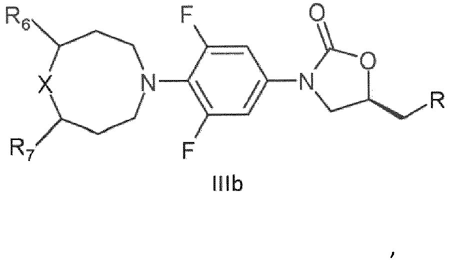 wobei
R unabhängig OH, OCH₃, OCH₂CH₃, OC(O)CH₃, NHCH₃, NHC₆H₅, NHS(O)₂R₂ ist;
R₂ unabhängig C₁-C₆-Alkyl ist; und
X = O, S, SO, SO₂.

5. Die Verbindung nach Anspruch 1, wobei die Verbindung ist:
| | |
|---|---|
| OTB-109 | 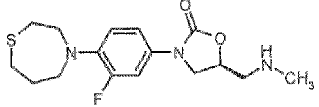 |
| OTB-108 | 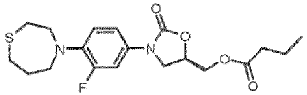 |
| OTB-116 | 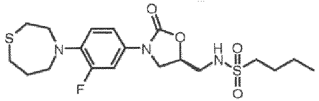 |
| OTB-126 | 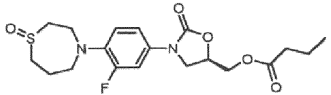 |
| OTB-110 | 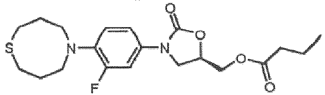 und |
| OTB-120 | 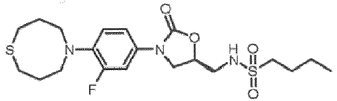 |

6. Ein Arzneimittel, umfassend mindestens eine Verbindung der Formel I oder ein Salz, Hydrat oder Solvat davon, wie in einem der Ansprüche 1 bis 5 definiert, und einen oder mehrere pharmazeutisch verträgliche Träger und/oder Additive.

7. Das Arzneimittel, umfassend mindestens eine Verbindung der Formel I oder ein Salz, Hydrat oder Solvat davon, wie in Anspruch 6 definiert, ferner umfassend eine oder mehrere zusätzliche antiinfektive Therapien.

8. Eine Verbindung der Formel I oder ein Salz, Hydrat oder Solvat davon, wie in einem der Ansprüche 1 bis 5 definiert, zur Verwendung bei der Prävention oder Behandlung von mikrobiellen Infektionen beim Menschen.

9. Die Verbindung zur Verwendung nach Anspruch 8, wobei die mikrobielle Infektion durch *Mycobacterium tuberculosis* hervorgerufen wird.

## Revendications

1. Un composé de formule I, ou un sel, hydrate ou solvate pharmaceutiquement acceptable de : 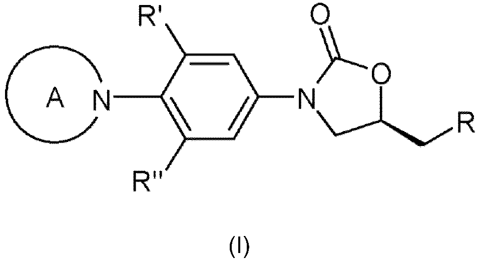 dans lequel :
R est indépendamment OR₁, OC(O)R₂, OC(O)NHR₂ ,OS(O₂)R₂, NHS(O)₂R₂, NR₃R₄;
R' et R" sont indépendamment H, F, CI ou OMe;
chaque R₁ est indépendamment H, alkyle en C₁-C₆, cycloalkyle en C₃-C₈, dans lesquels lesdits alkyle, cycloalkyle sont éventuellement substitués par 1 à 4 groupes choisis parmi halogéno, hydroxy, alkyle en C₁-C₆, acyloxy en C₁-C₆ ;
chaque R₂ est indépendamment alkyle en C₁-C₆, cycloalkyle en C₃-C₈, hétérocyclyle, hétéroaryle ou aryle,
dans lesquels lesdits alkyle, cycloalkyle, hétérocyclyle, hétéroaryle ou aryle sont éventuellement substitués par 1 à 4 groupes choisis parmi halogéno, hydroxyle, alkyle en C₁-C₆, alcoxy en C₁-C₆, acyloxy en C₁-C₆, CF₃, NO₂, CN et NH₂;
chaque R₃ est un alkyle en C₁-C₆, un cycloalkyle en C₃-C₈, un hétérocyclyle, un hétéroaryle ou un aryle, et R₄ est H, un alkyle en C₁-C₆, un cycloalkyle en C₃-C₈, un hétérocyclyle, un hétéroaryle ou un aryle; dans lesquels lesdits alkyle, cycloalkyle, hétérocyclyle, hétéroaryle ou aryle sont éventuellement substitués par 1 à 4 groupes choisis
parmi halogéno, alkyle en C₁-C₆, alcoxy en C₁-C₆, CF₃, NO₂, CN;
Le cycle A est 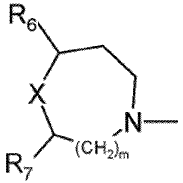
dans lequel,
chacun de R₆ et R₇ est indépendamment H, F, CH₃, CH₂CH₃, CF₃, phényle ;
X = O, S, SO, SO₂;
m est 1, ou 2.

2. Le composé de la revendication 1, dans lequel le composé est représenté par la formule Il : 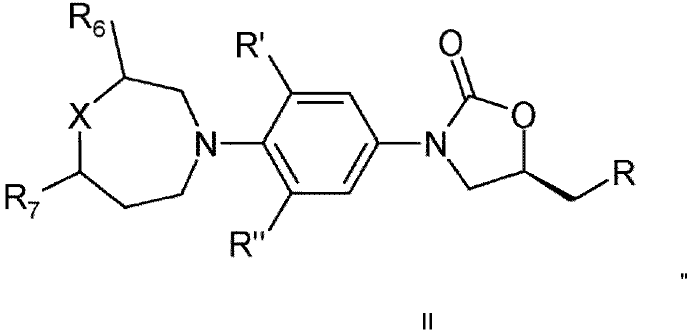 dans lequel,
R est indépendamment OR₁, OC(O)R₂, NR₃R₄, NHS(O)₂R₂;
R' et R" sont indépendamment H, ou F ;
R₁ est indépendamment H, alkyle en C₁-C₆, cycloalkyle en C₃-C₆ ;
R₂ est indépendamment alkyle en C₁-C₆, cycloalkyle en C₃-C₆ ;
R₃ est un alkyle en C₁-C₆, un cycloalkyle en C₃-C₆, un phényle, et R₄ est H, un alkyle en C₁-C₆, un cycloalkyle en C₃-C₆, un phényle ;
R₆ et R₇ est indépendamment H, F, CH₃, CH₂CH₃, CF₃ ;
X =O, S, SO, SO₂.

3. Le composé de la revendication 1, dans lequel le composé est représenté par la formule III : 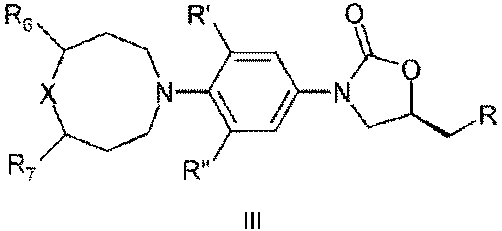 dans lequel,
R est indépendamment OR₁, OC(O)R₂, NR₃R₄, NHS(O)₂R₂;
R' et R" sont indépendamment H, ou F ;
R₁ est indépendamment H, alkyle en C₁-C₆, cycloalkyle en C₃-C₆ ;
R₂ est indépendamment alkyle en C₁-C₆, cycloalkyle en C₃-C₆ ;
R₃ est alkyle en C₁-C₆, cycloalkyle en C₃-C₆, ou phényle, et R₄ est H, alkyle en C₁-C₆, cycloalkyle en C₃-C₆, ou phényl;
R₆ et R₇ sont indépendamment H, F, CH₃, CH₂CH₃, CF₃ ;
X = O, S, SO, SO₂ .

4. Le composé de la revendication 1, le composé est représenté par la Formule Ila, IIb, Illa, IIIb,
IVa, IVb, Va, Vb, IXa, ou IXb: 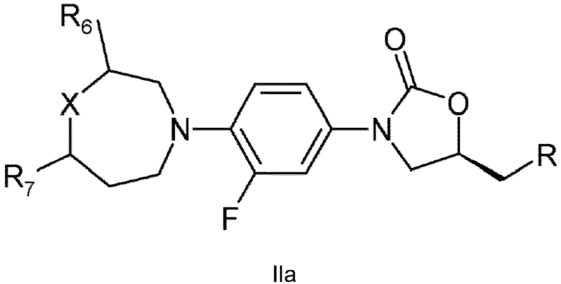 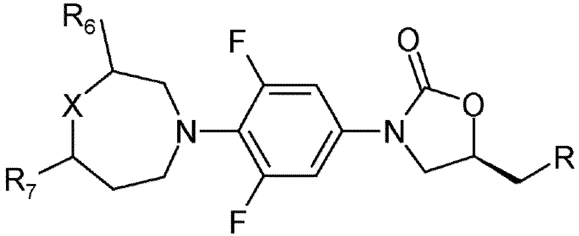 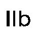 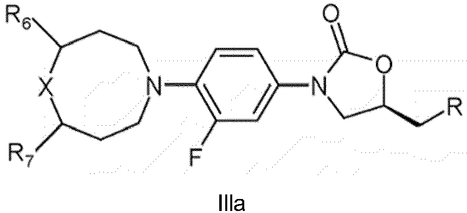 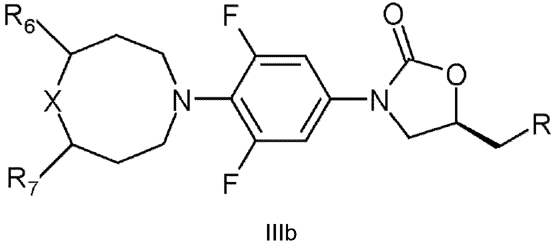
dans lequel,
R est indépendamment OH, OCH₃, OCH₂CH₃, OC(O)CH₃, NHCH₃, NHC₆H₅, NHS(O)₂R₂;
R₂ est indépendamment alkyle en C₁-C₆ ; et
X = O, S, SO, SO₂.

5. Le composé de la revendication 1, dans lequel le composé est :
| | |
|---|---|
| OTB-109 | 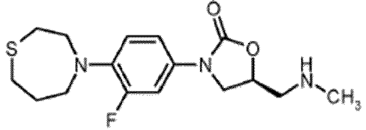 |
| OTB-108 | 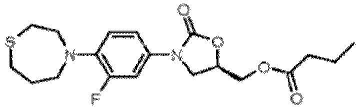 |
| OTB-116 | 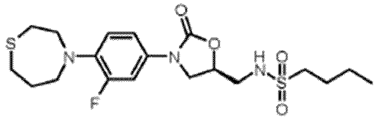 |
| OTB-126 | 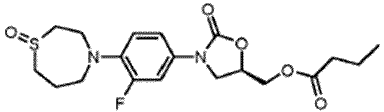 |
| OTB-110 | 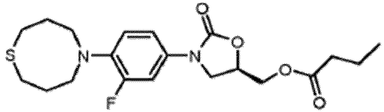 et |
| OTB-120 | 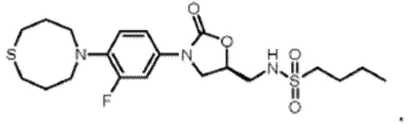 |

6. Une composition pharmaceutique comprenant au moins un composé de Formule I, ou un sel, un hydrate, ou un solvate de celui-ci, tel que défini dans l'une quelconque des revendications 1 à 5, et un ou plusieurs excipients et/ou additifs pharmaceutiquement acceptables.

7. La composition pharmaceutique comprenant au moins un composé de Formule I, ou un sel, un hydrate, ou un solvate de celui-ci, tel que défini dans la revendication 6, comprenant en outre un ou plusieurs traitements anti-infectieux supplémentaires.

8. Un composé de Formule I, ou un sel, un hydrate, ou un solvate de celui-ci, tel que défini dans l'une quelconque des revendications 1 à 5, destiné à être utilisé dans la prévention ou le traitement d'infections microbiennes chez l'être humain.

9. Le composé destiné à être utilisé selon la revendication 8, dans lequel l'infection microbienne est causée par *Mycobacterium tuberculosis*.
